# EUROPEAN PATENT SPECIFICATION

(11) **EP 2 961 404 B1**
(45) Date of publication and mention of the grant of the patent: **02.09.2020**
(21) Application number: 14757145.9
(22) Date of filing: 28.02.2014
(51) Int. Cl.: A61K 31/428, A61P 25/28

(54) **COMPOSITIONS AND METHODS FOR TREATING AMYOTROPHIC LATERAL SCLEROSIS IN RESPONDERS**
ZUSAMMENSETZUNGEN UND VERFAHREN ZUR BEHANDLUNG VON AMYOTROPHER LATERALSKLEROSE BEI REAGIERENDEN PATIENTEN
COMPOSITIONS ET PROCÉDÉS DE TRAITEMENT DE LA SCLÉROSE LATÉRALE AMYOTROPHIQUE CHEZ LES PATIENTS RÉPONDANT AU TRAITEMENT

(30) Priority: 28.02.2013 US 201361771032 P; 04.03.2013 US 201361772328 P; 14.03.2013 US 201361786236 P; 03.10.2013 US 201361886598 P; 04.12.2013 US 201361911984 P
(43) Date of publication of application: 06.01.2016
(73) Proprietor: Knopp Biosciences LLC, Pittsburgh, PA 15203 (US)
(72) Inventor: BOZIK, Michael E., Pittsburgh, PA 15203 (US); PETZINGER JR., Thomas, Pittsburgh, PA 15203 (US); MATHER, James L., Pittsburgh, PA 15203 (US); ARCHIBALD, Donald, Pittsburgh, PA 15203 (US)
(74) Representative: HGF
(86) International application number: PCT/US2014/019668
(87) International publication number: WO 2014/134569

(56) References cited:
- US-A1- 2011 009 460
- US-A1- 2012 258 994
- RUDNICKI STACY A ET AL: "Dexpramipexole effects on functional decline and survival in subjects with amyotrophic lateral sclerosis in a Phase II study: subgroup analysis of demographic and clinical characteristics.", AMYOTROPHIC LATERAL SCLEROSIS & FRONTOTEMPORAL DEGENERATION JAN 2013, vol. 14, no. 1, January 2013 (2013-01), pages 44-51, XP009191645, ISSN: 2167-9223
- BOZIK M ET AL: "Phase 2 study of the safety, tolerability and clinical effects of KNS 760704 in ALS subjects", 20TH INTERNATIONAL SYMPOSIUM ON ALS/MND. BERLIN 8-10 DECEMBER 2009, , 8 December 2009 (2009-12-08), pages 28-29, XP002692344, Retrieved from the Internet: URL:http://www.alsliga.be/uploads/media/AL S_symposium_2009.pdf [retrieved on 2013-01-29]

## Description

### B. Cross Reference:

This application claims the benefit of U.S. Provisional Application No. 61/771,032 entitled "Compositions and methods for treating amyotrophic lateral sclerosis in responders" filed February 28, 2013, U.S. Provisional Application No. 61/772,328 entitled "Compositions and methods for treating amyotrophic lateral sclerosis in responders" filed March 4, 2013, and U.S. Provisional Application No. 61/786,236 entitled "Compositions and methods for treating amyotrophic lateral sclerosis in responders" filed March 14, 2013, and U.S. Provisional Application No. 61/886,598 entitled "Compositions and methods for treating amyotrophic lateral sclerosis in responders" filed October 3, 2013.

### C. Government Interests: Not Applicable.

### D. Parties to a Joint Research Agreement: Not Applicable.

### E. Incorporation by Reference of Material Submitted on a CD: Not Applicable.

### F. Background:

US2011009460, Gribkoff, et al. discloses pharmaceutical compositions of dexpramipexole and methods of using such compositions for the treatment of ALS.

US2012258994, McKinney et al. discloses (+)-1-(3,4-dichlorophenyl)-3-azabicyclo[3.1.0]hexane and pharmaceutically acceptable active salts, polymorphs, glycosylated derivatives, metabolites, solvates, hydrates, and/or prodrugs of (+)-1-(3,4-dichlorophenyl)-3-azabicyclo[3.1.0]hexane and their use alone or in combination with additional psychotherapeutic compositions in the treatment of conditions affected by monoamine neurotransmitters, including treatment of refractory individuals.

### G. Summary of the Invention:

The invention provides a therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject, wherein the subject is a subject with definite ALS, amyotrophic lateral sclerosis symptom onset duration of less than about 18 months, a creatinine value of greater than 72.0 µmol/L, and concomitant riluzole administration; and wherein the amyotrophic lateral sclerosis is treated.

Disclosures herein are directed to a method for treating amyotrophic lateral sclerosis in a subject in need thereof comprising: administering to the subject a therapeutically effective amount of (6R)-2-amino-4,5,6,7-tetrahydro-6-(propylamino)benzothiazole or a pharmaceutically acceptable salt thereof, wherein the subject is a subject with definite ALS, amyotrophic lateral sclerosis symptom onset duration of less than about 18 months, and concomitant riluzole administration and wherein the amyotrophic lateral sclerosis is treated.

In some embodiments, definite ALS is defined by the El Escorial diagnosis criteria. The subject is a subject with a creatine value of greater than 72.0 µmol/L. In some embodiments, said subject with concomitant riluzole administration is a subject who has been receiving riluzole for more than about thirty days. In some embodiments, the subject is a subject with a pulse rate of greater than 81.0 beats per minute, a cholesterol value of less than or equal to 5.3 mmol/L, a creatine phosphokinase value of less than or equal to 184.0 U/L, a bicarbonate value of less than or equal to 21.6 mmol/L, or any combination thereof. In some embodiments, said subject with amyotrophic lateral sclerosis symptom onset duration of less than about 18 months is a subject with symptom onset selected from about 18 months, about 17 months, about 16 months, about 15 months about 14 months, about 13 months, about 12 months, about 11 months, about 10 months, about 9 months, about 8 months, about 7 months, about 6 months, about 5 months, about 4 months, about 3 months, about 2 months, and about 1 month.

In some embodiments, the therapeutically effective amount of (6R)-2-amino-4,5,6,7-tetrahydro-6-(propylamino)benzothiazole or a pharmaceutically acceptable salt thereof is from 150 milligrams to 3,000 milligrams per day. In some embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In some embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In some embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In some embodiments, the therapeutically effective amount of (6R)-2-amino-4,5,6,7-tetrahydro-6-(propylamino)benzothiazole or a pharmaceutically acceptable salt thereof is 150 milligrams twice daily. In some embodiments, the therapeutically effective amount of (6R)-2-amino-4,5,6,7-tetrahydro-6-(propylamino)benzothiazole or a pharmaceutically acceptable salt thereof is from 300 milligrams twice daily.

In some embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. In some embodiments, said pharmaceutical composition is chirally pure for (6R)-2-amino-4,5,6,7-tetrahydro-6-(propylamino)benzothiazole or a pharmaceutically acceptable salt thereof. In some embodiments, the chiral purity for (6R)-2-amino-4,5,6,7-tetrahydro-6-(propylamino)benzothiazole or a pharmaceutically acceptable salt thereof is about 99% or more. In some embodiments, said pharmaceutical composition is selected from a tablet, a capsule and a liquid. In some embodiments, said (6R)-2-amino-4,5,6,7-tetrahydro-6-(propylamino)benzothiazole or a pharmaceutically acceptable salt thereof is administered orally.

In some instances disclosed herein, said subject is diagnosed with one of the following diseases: definite amyotrophic lateral sclerosis, probable amyotrophic lateral sclerosis, possible amyotrophic lateral sclerosis and suspected amyotrophic lateral sclerosis. Treating amyotrophic lateral sclerosis in said subject may be selected from improved ALSFRS-R score, improved CAFS rank, decreased mortality, increased life expectancy, and combinations thereof.

Disclosures herein are directed to a method of treating a patient comprising: identifying said patient as a responder; and administering to said subject a therapeutically effective amount of (6R)-2-amino-4,5,6,7-tetrahydro-6-(propylamino)benzothiazole or a pharmaceutically acceptable salt thereof. The invention provides a therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject identified as a responder; wherein the responder is El Escorial definite ALS and has a serum creatinine level greater than 72.0 µmol/L.

The step of identifying said patient as a responder may comprise diagnosing said patient with El Escorial definite ALS.

The step of identifying the patient with El Escorial definite ALS as a responder may further include measuring the serum creatinine levels of the patient; and identifying the patient as a responder if the serum creatinine levels are greater than about 72 µmol/L.

The step of identifying the said patient as a responder may comprise identifying the presence of the presence of amyotrophic lateral sclerosis symptom onset duration of less than about 18 months, concomitant riluzole administration, a pulse rate of greater than 81.0 beats per minute, a creatinine value of greater than 72.0 µmol/L, a cholesterol value of less than or equal to 5.3 mmol/L, a creatine phosphokinase value of less than or equal to 184.0 U/L, a bicarbonate value of less than or equal to 21.6 mmol/L, or any combination thereof.

The step of identifying the said patient as a responder may comprise diagnosing said patient with EEC definite ALS, identifying the presence of the presence of amyotrophic lateral sclerosis symptom onset duration of less than about 18 months and concomitant riluzole administration.

In some embodiments, the step of identifying the said patient as a responder comprises diagnosing said patient with EEC definite ALS, identifying the presence of amyotrophic lateral sclerosis symptom onset duration of less than about 18 months, concomitant riluzole administration, and a creatinine value of greater than 72.0 µmol/L.

### H. Description of the Drawings:

Figure 1 depicts a flow chart of study subject disposition for a clinical trial.
Figure 2 shows recruitment rate in the clinical trial.

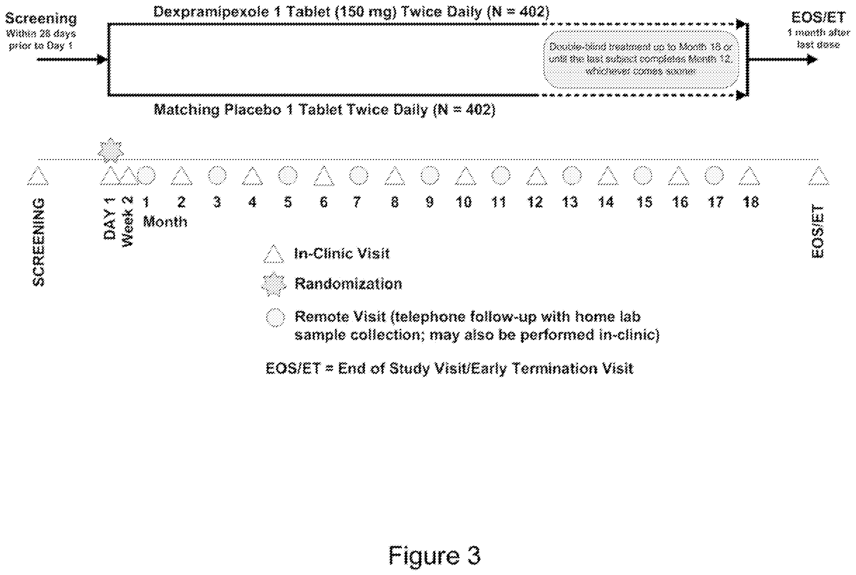

Figure 3 depicts a study schedule of the clinical trial.

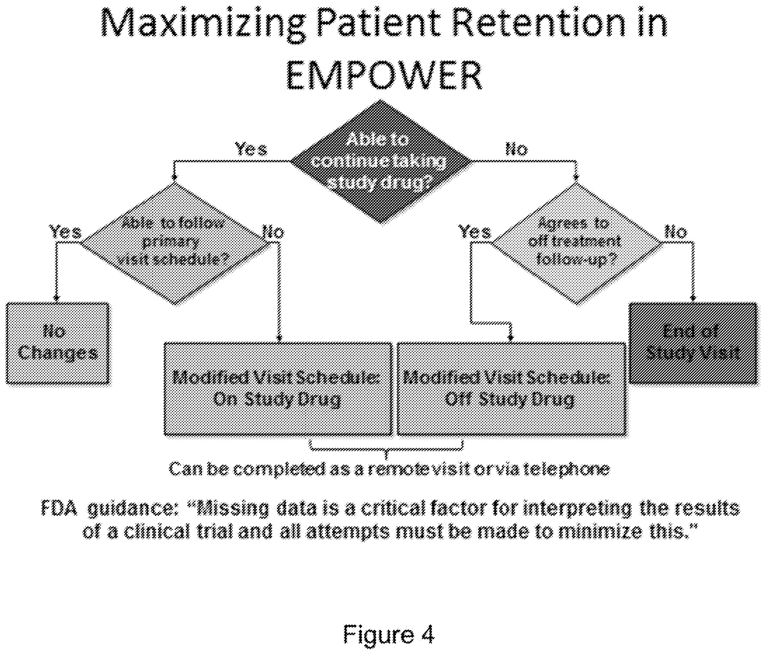

Figure 4 shows a flow chart of maximizing subject retention in the clinical trial.

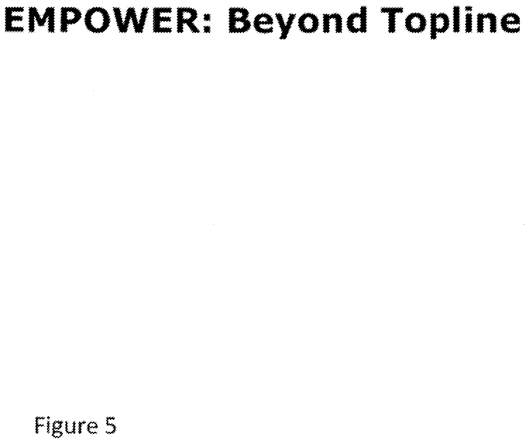

Figures 5-23 show exemplary methods of treating amyotrophic lateral sclerosis.

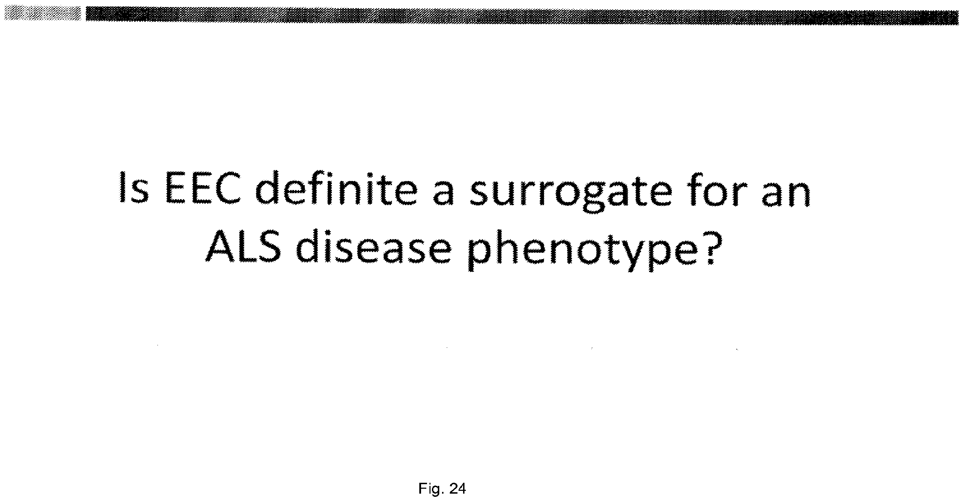

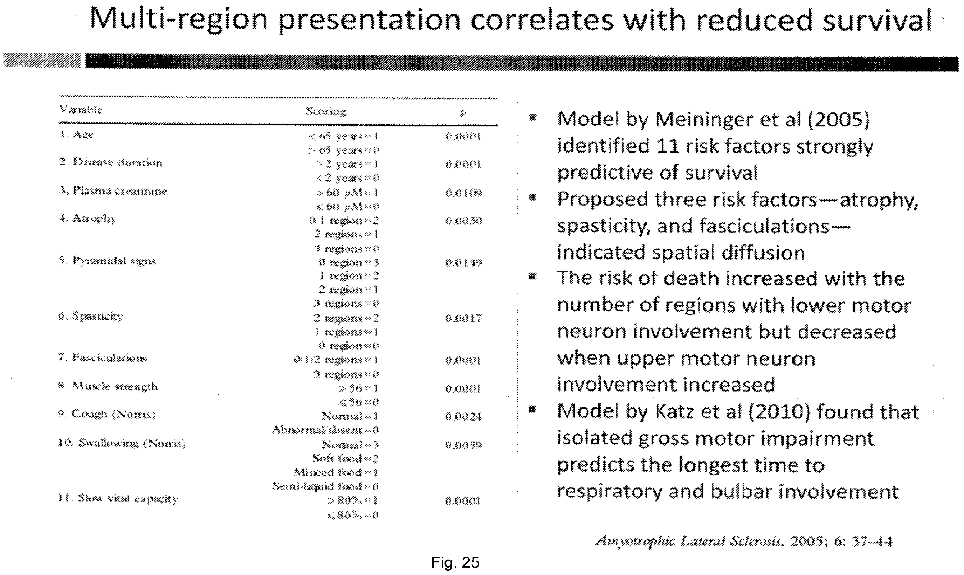

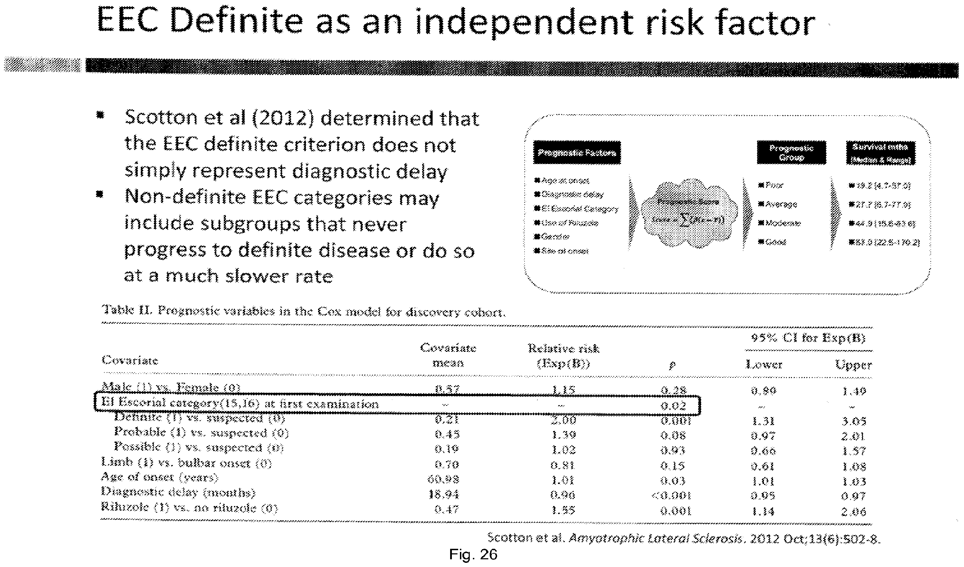

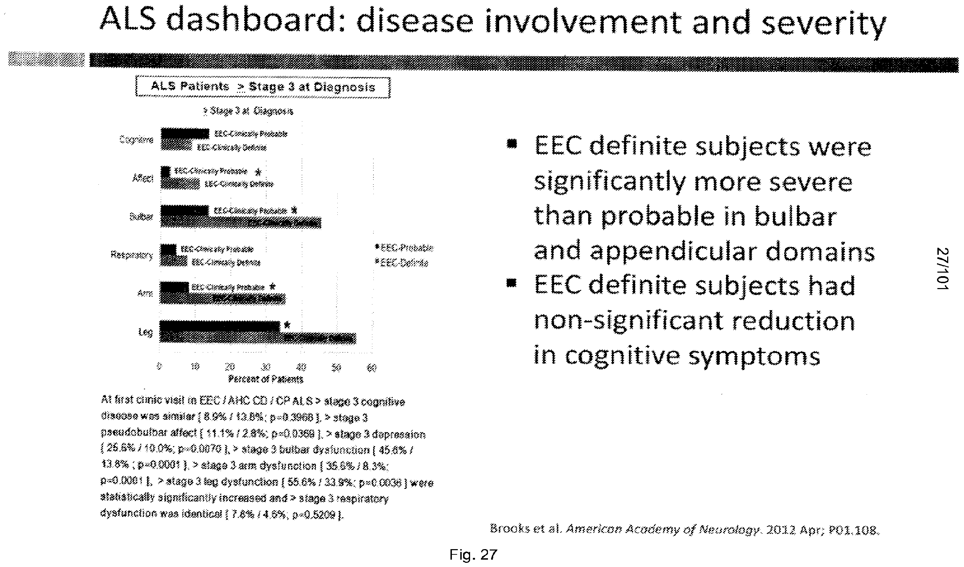

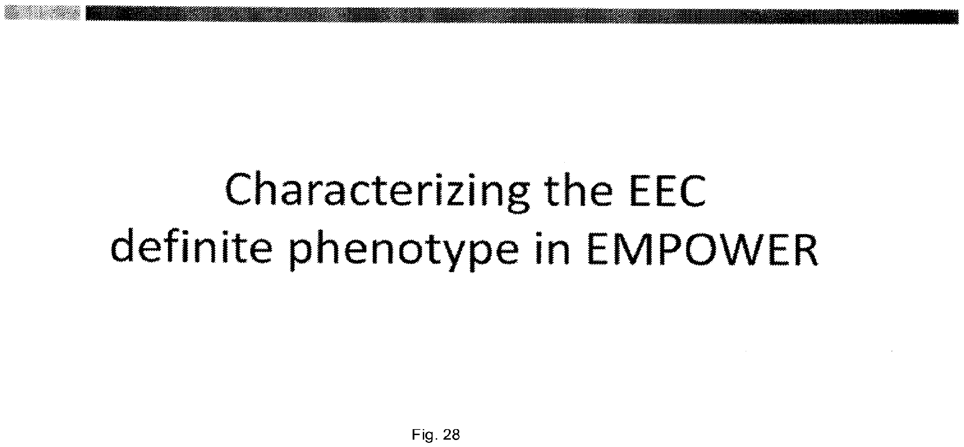

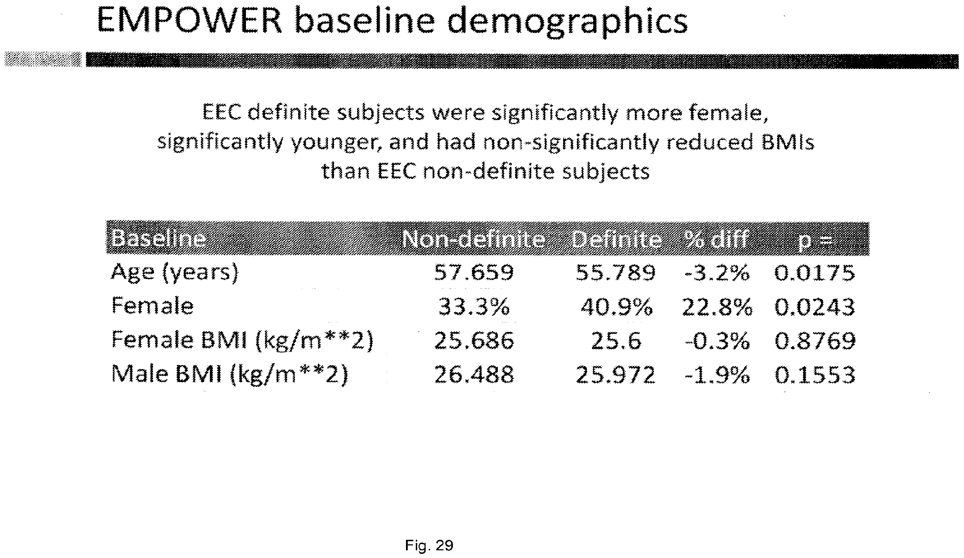

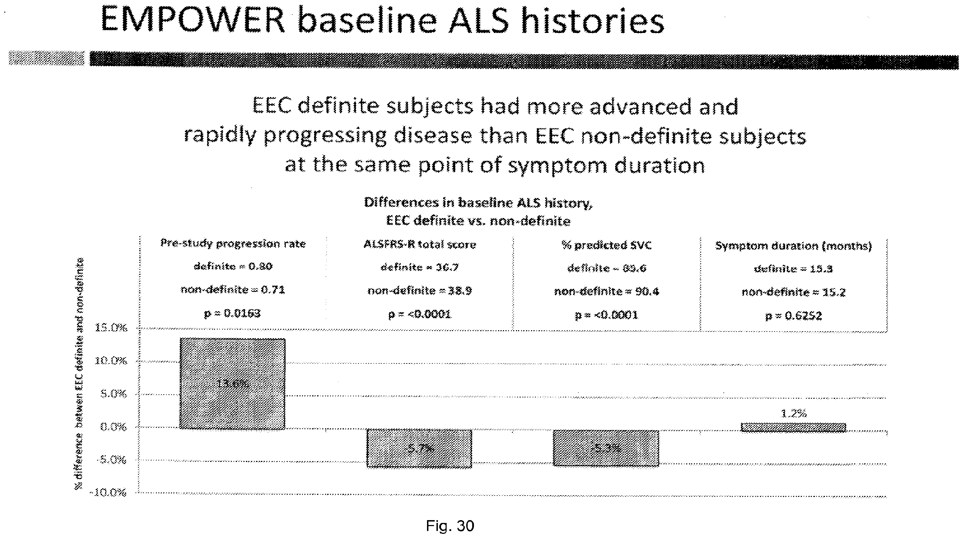

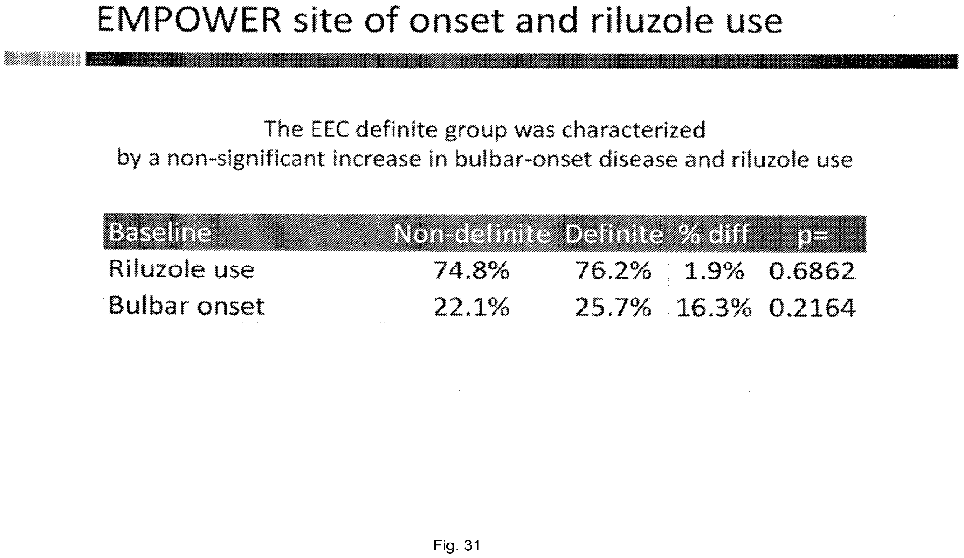

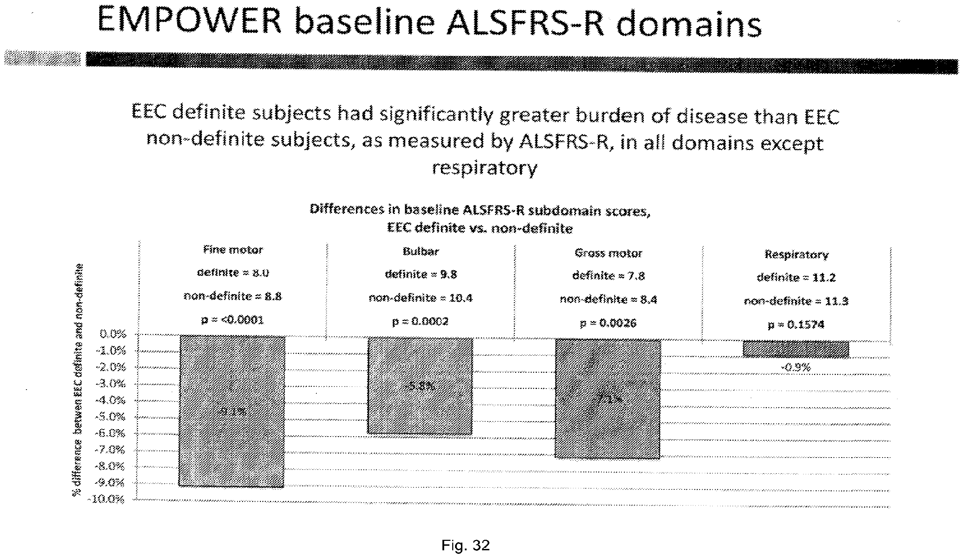

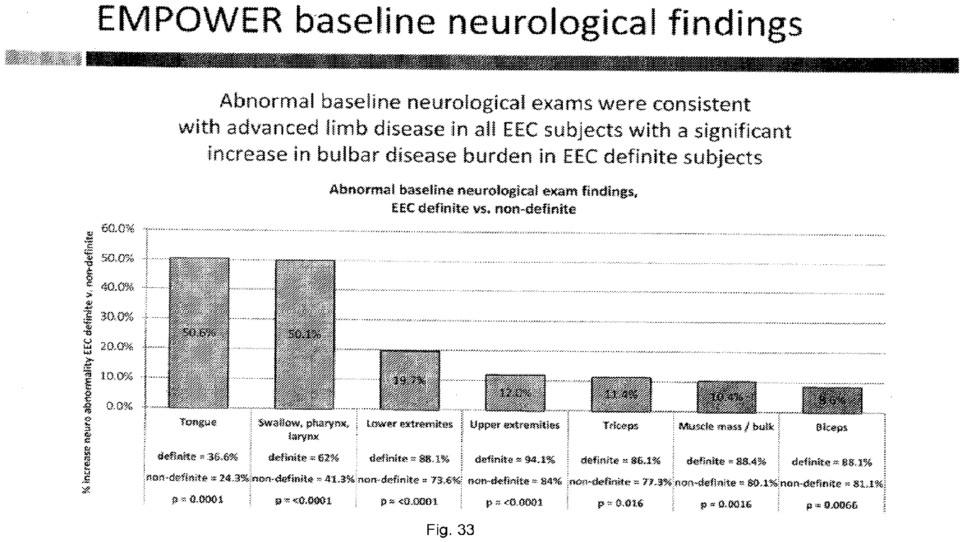

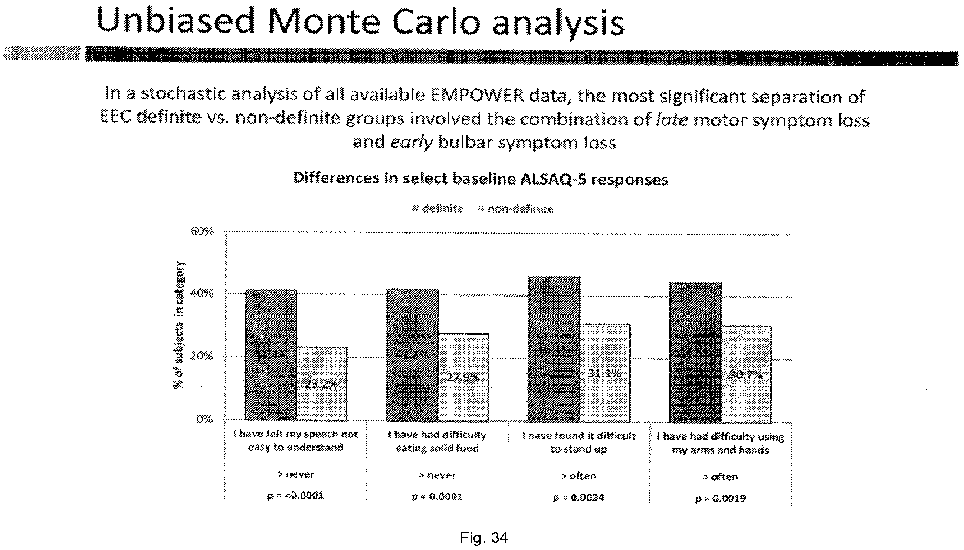

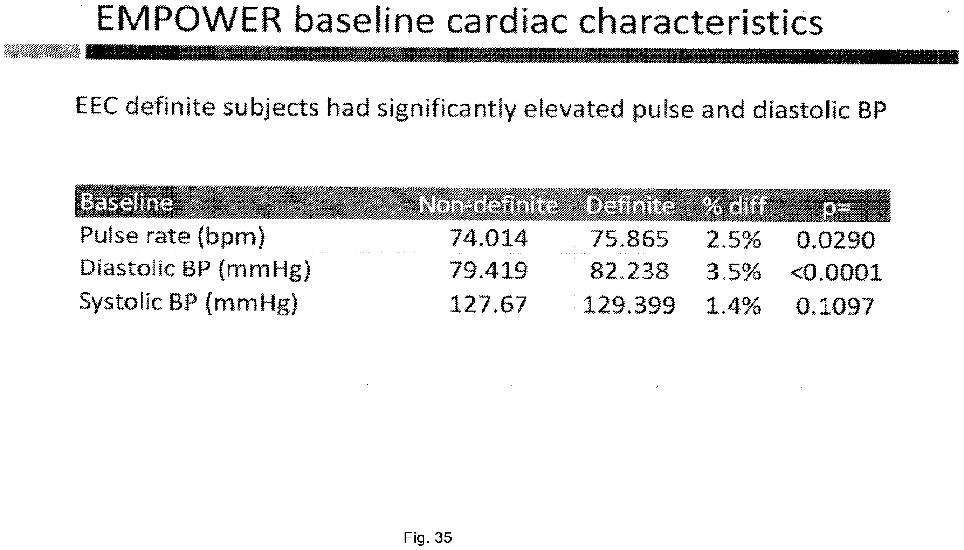

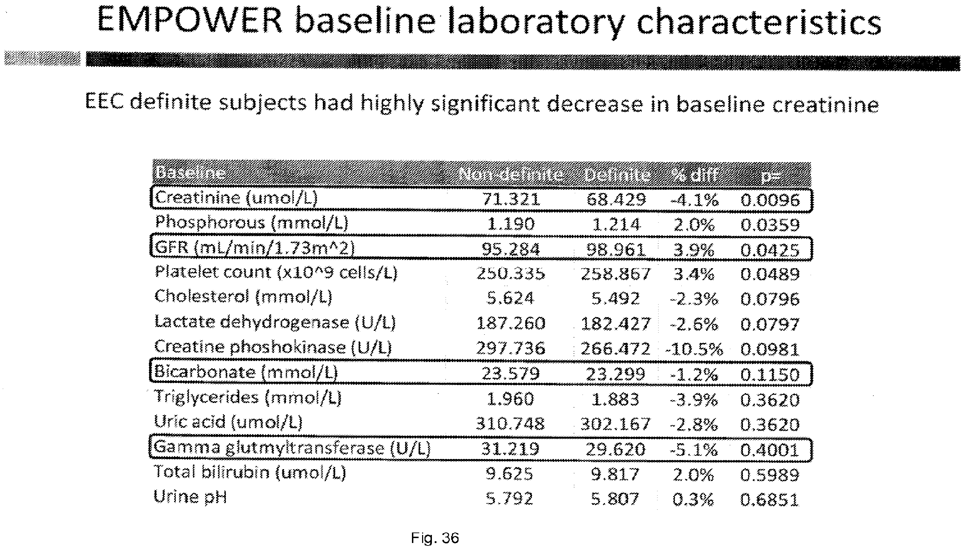

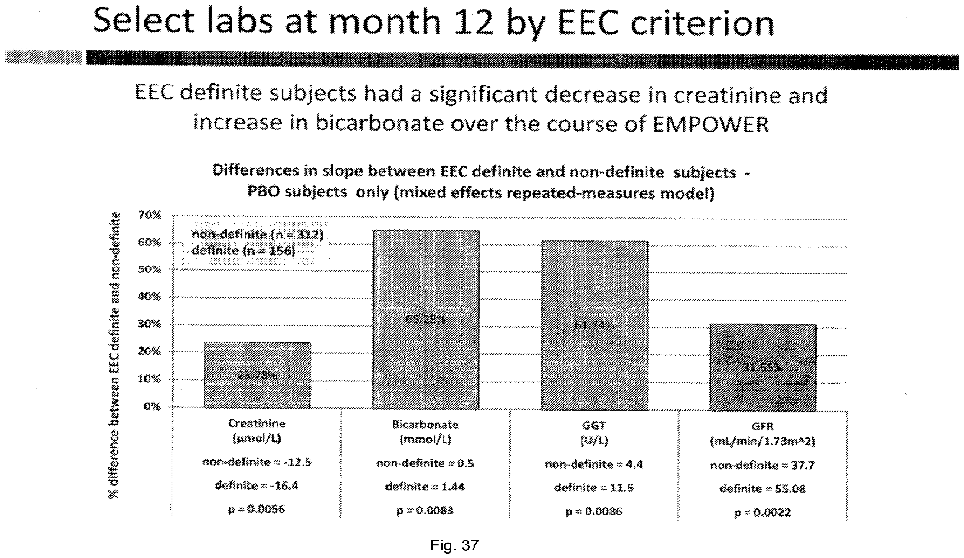

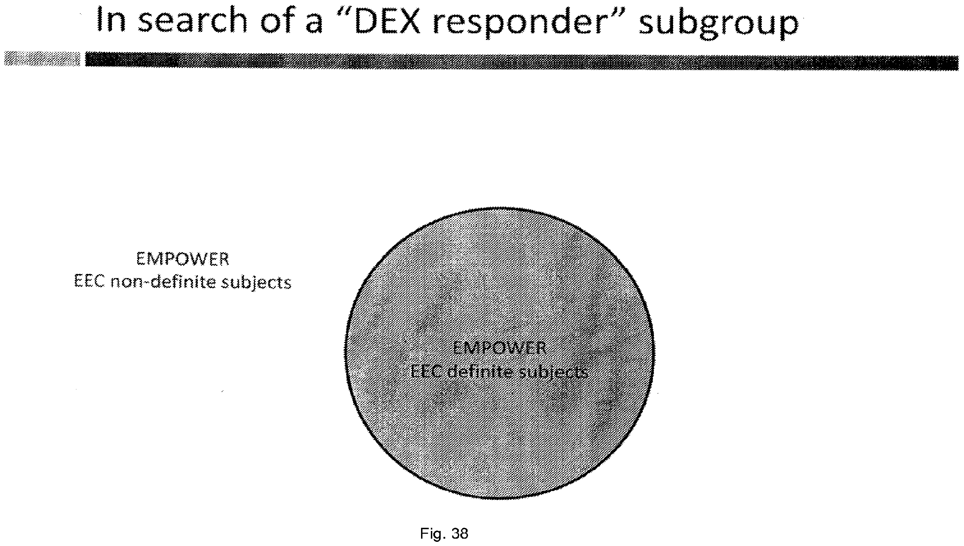

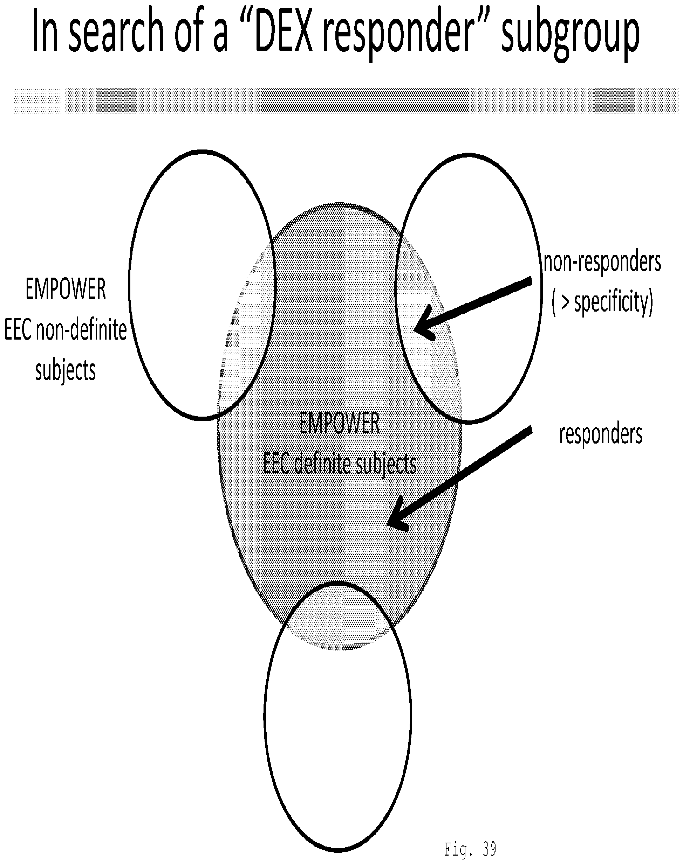

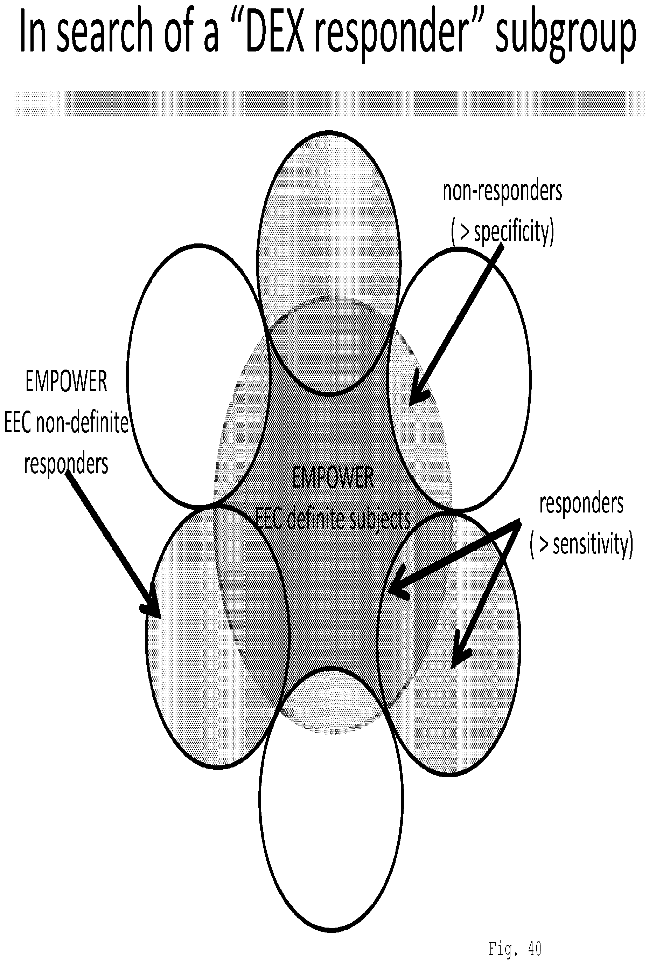

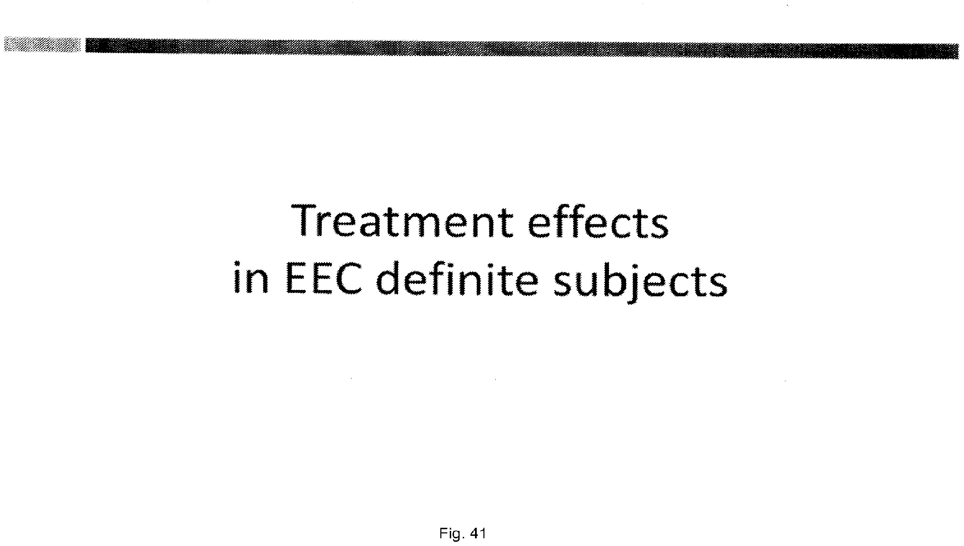

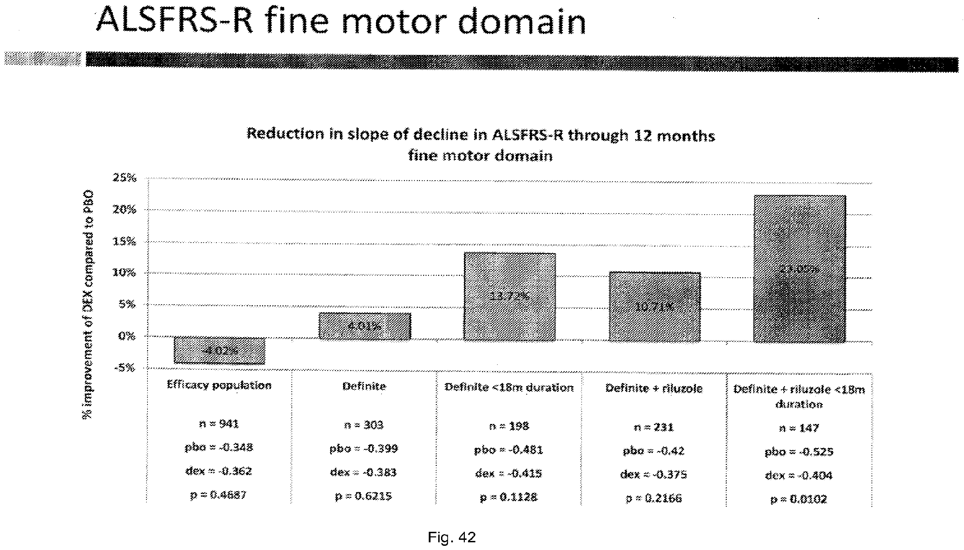

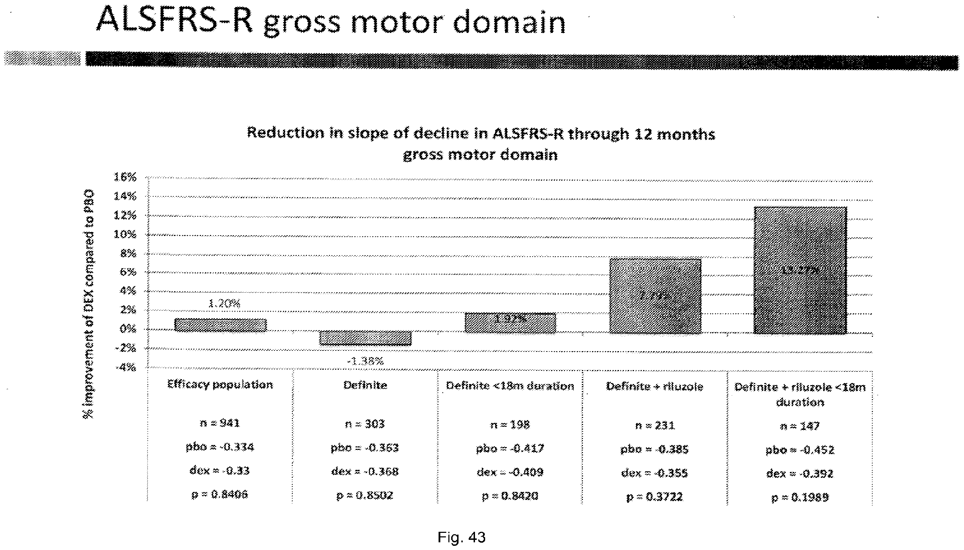

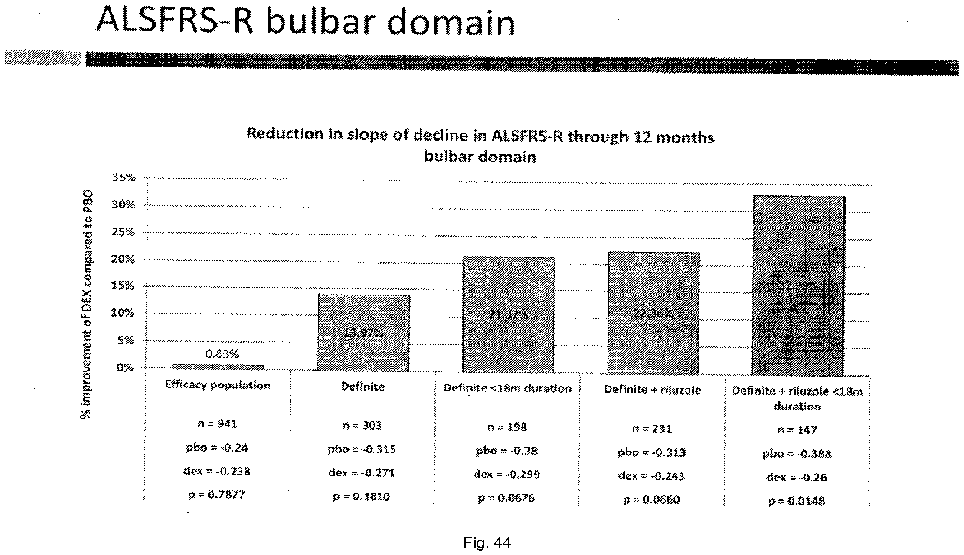

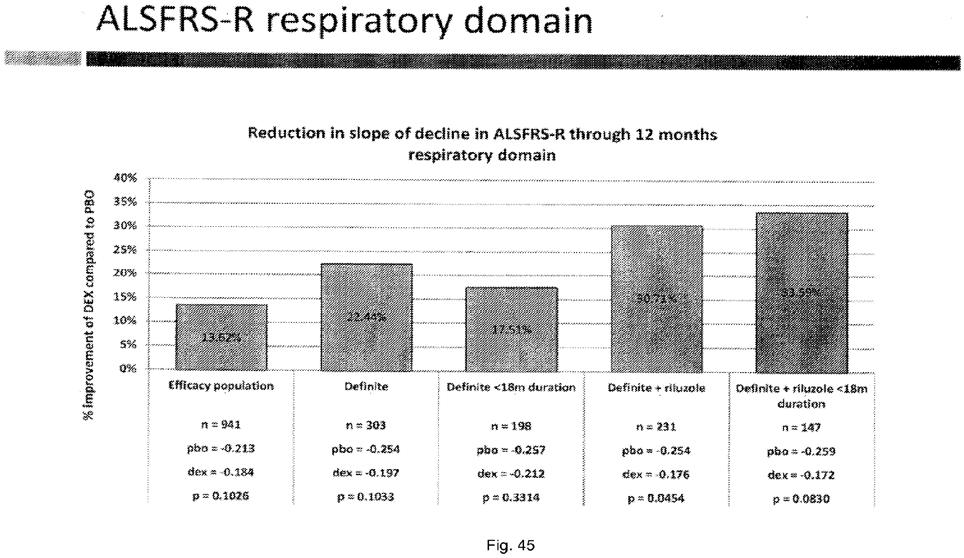

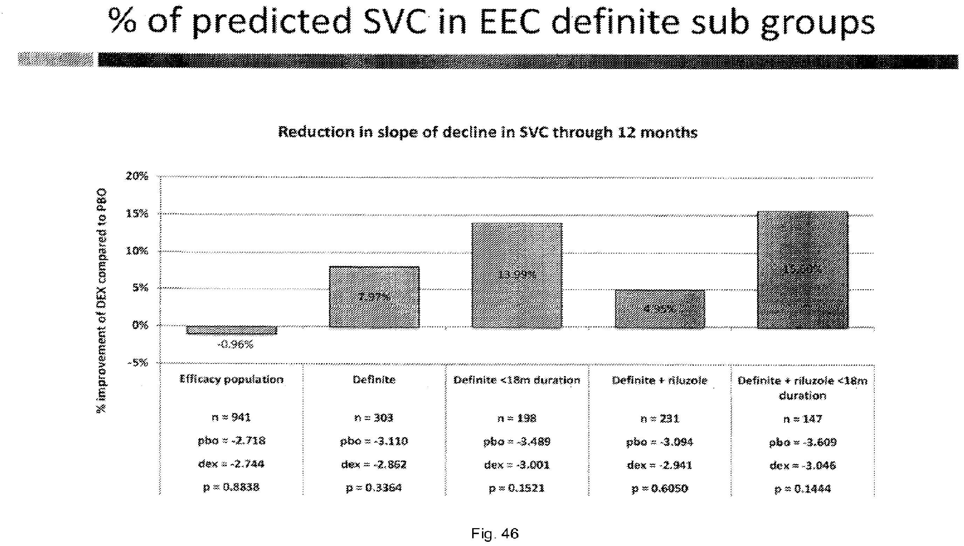

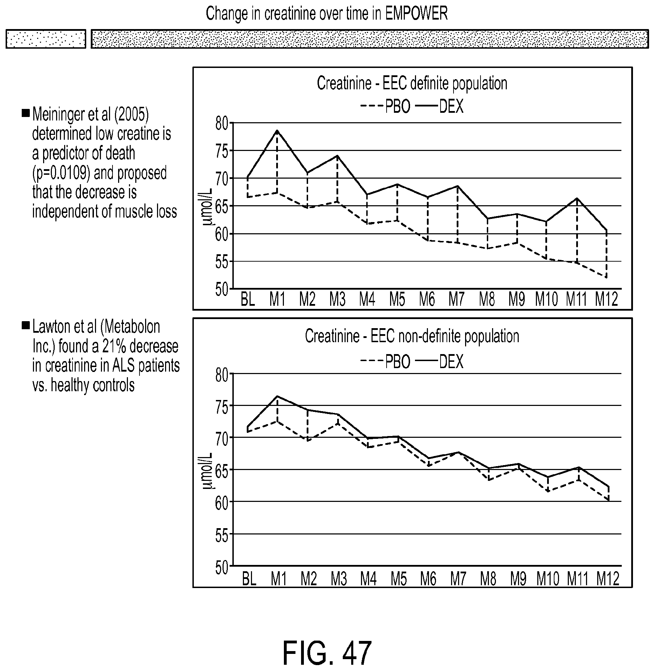

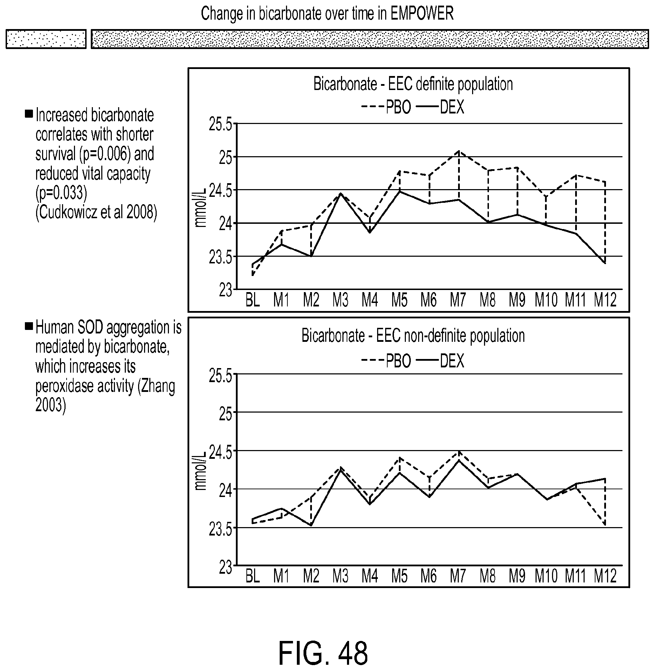

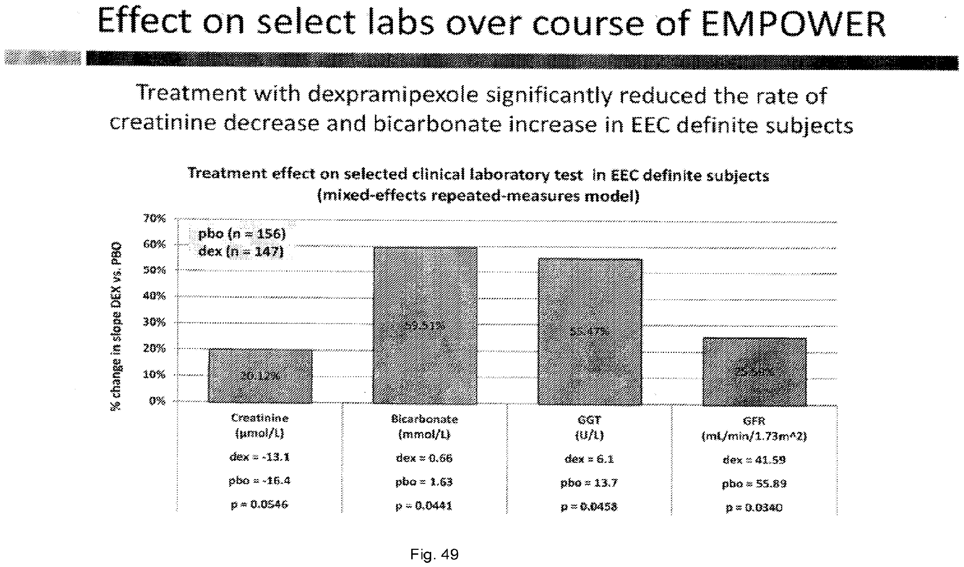

Figures 24-49 show exemplary methods of treating amyotrophic lateral sclerosis.
Figures 50-55 show data tables of the Kaplan-Meier estimates and hazard rations for time to death, slope of ALSFRS-R total score, and summary of joint rank (CAFS) through twelve months for subjects with a baseline creatinine of greater than about 72 µmol/L.
Figures 56-67 show data tables for the change from baseline in serum creatinine results by treatment group.
Figure 68 shows that dexpramipexole at 300 mg/day was well tolerated.
Figure 69 shows that Dexpramipexole 300 mg/day failed to show efficacy on any prespecified endpoint.
Figure 70 shows that dexpramipexole had no significant effect over placebo in the EMPOWER study.
Figure 71 shows A *post hoc* analysis of EMPOWER subgroups and that there are significant inter-study differences despite nearly identical enrollment criteria.
Figure 72 shows enrollment kinetics led to milder EMPOWER disease severity.
Figure 73 shows that El Escorial definite EMPOWER placebo subjects were fast decliners.
Figure 74 shows the apparent riluzole benefit increased in the dexpramipexole population versus placebo population.
Figure 75 shows the dexpramipexole effect on CAFS in EEC definite subjects and that the effect increased on background of riluzole and with shorter symptom duration.
Figure 76 shows the dexpramipexole effect on ALSFRS-R slopes in EEC definite versus efficacy population and the effect increased on background of riluzole and with shorter symptom duration.
Figure 77 shows the dexpramipexole effect on hazard for mortality in EEC definite versus efficacy population and the effect increased on background of riluzole and with shorter symptom duration.
Figure 78 shows a scatter plot of baseline: creatinine vs. ALSFRS-R total score in the safety population and creatinine as a candidate biomarker.
Figure 79 shows A *post hoc* analysis of dexpramipexole effects on time average difference change in creatinine and shows creatinine loss significantly reduced in dexpramipexole-treated EEC definite subgroup.
Figure 80 shows scatter plots of change from baseline to month 6 (creatinine vs. ALSFRS-R total score; safety population) and depicts the dexpramipexole effect on correlation of change in creatinine to change in ALSFRS-R score in placebo subjects and subjects receiving 150 mg BID from the ITT population.
Figure 81 shows the creatinine-sparing effects of dexpramipexole observed in full ITT population and the magnitude of effect smaller than in EEC definite subgroup but still significant.
Figure 82 shows the creatinine-sparing effects of dexpramipexole not a passive marker of weight preservation and the effect adjusted for weight by taking ratio of creatinine to weight per visit.
Figure 83 shows the effects of dexpramipexole and riluzole on change in creatinine and additive effects on creatinine-sparing seen with riluzole and dexpramipexole.
Figure 84 shows the dexpramipexole effects in increasing ATP synthesis confirmed in three independent labs.
Figure 85 shows increased ATP synthesis with a corresponding increase in phosphocreatine could account for creatinine sparing.
Figure 86 shows riluzole and dexpramipexole showed additive creatinine-preserving effects. Shading indicates creatinine preservation greater than for subjects receiving neither riluzole nor dexpramipexole.

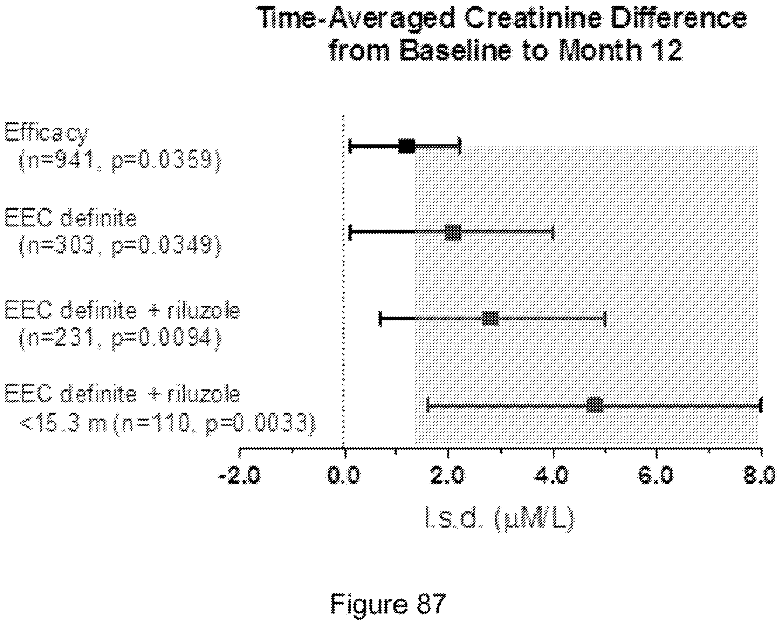

Figure 87 shows dexpramipexole-responder subgroups experienced reduced creatinine loss. Shading indicates improvement in creatinine-sparing effects over efficacy population.
Figure 88 shows a scatter plot of baseline: weight vs. ALSFRS-R total score (Safety Population). Pearson Correlation coefficient = 0.05 (p=0.238).
Figure 89 shows data supporting a correlating between creatinine loss and energy deficiency in ALS.
Figure 90 shows that creatinine loss significantly reduced in dexpramipexole-treated EEC definite subgroups in a *post hoc* analysis of dexpramipexole effects on time average difference change in creatinine.
Figure 91 shows the additive effects on creatinine-sparing seen with riluzole and dexpramipexole.
Figure 92 shows the creatinine-sparing effect of dexpramipexole adjusted for weight by taking ratio of creatinine to weight per visit based on mixed-effects repeated-measures model which includes treatment, visit, and baseline value as fixed effects.
Figure 93 shows a summary of the *post hoc* analysis of EMPOWER creatine supplementation (5g/day).
Figure 94 shows that less weight loss observed in dexpramipexole subjects on creatine supplementation (5g/day).
Figure 95 shows EMPOWER Baseline Covariates in EEC Definite and Not Definite Participants.

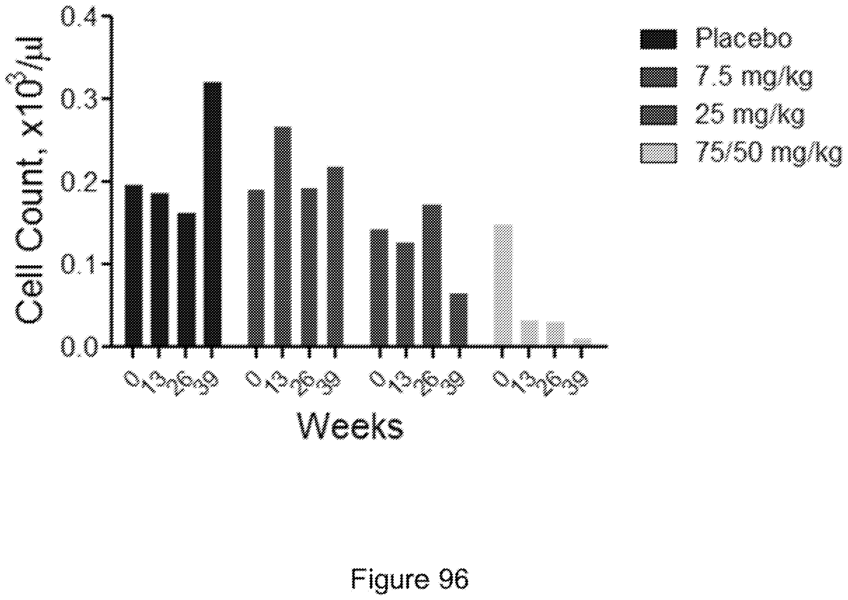

Figure 96 shows dose- and time-dependent effects of dexpramipexole on eosinophil counts in mini-pigs.
Figure 97 shows dose- and time-dependent effects of dexpramipexole on eosinophil counts in phase 2.
Figure 98 shows EMPOWER hematology data: eosinophil count was robustly affected.
Figure 99 shows EMPOWER hematology data: dexpramipexole effect on other white blood cells.
Figure 100 shows time- and dose-dependent eosinophil lowering effects of dexpramipexole in Phase 2 and Phase 3 clinical trials.

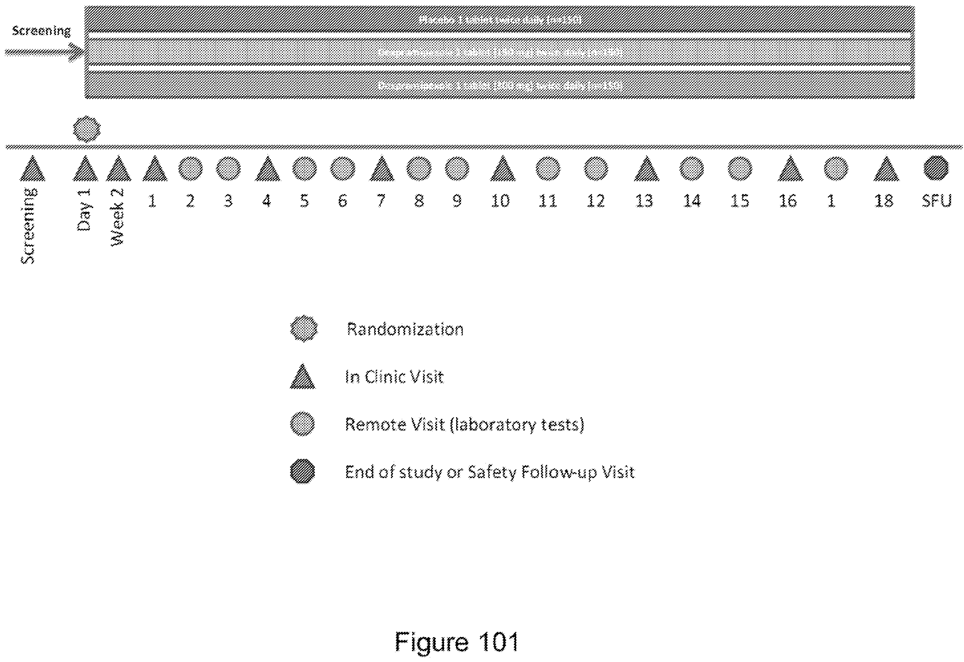

Figure 101 shows a study design schematic for a randomized, double-blind, placebo-controlled, multicenter study of the efficacy and safety of dexpramipexole (kns-760704) in subjects with amyotrophic lateral sclerosis.

### I. Detailed Description

Before the present compositions and methods are described, it is to be understood that any invention is not limited to the particular processes, compositions, or methodologies described, as these may vary. Moreover, the processes, compositions, and methodologies described in particular embodiments are interchangeable. Therefore, for example, a composition, dosage regimen, route of administration, and so on described in a particular embodiment may be used in any of the methods described in other particular embodiments. It is also to be understood that the terminology used in the description is for the purpose of describing the particular versions or embodiments only, and is not intended to limit the scope of the present invention, which will be limited only by the appended claims. Unless clearly defined otherwise, all technical and scientific terms used herein have the same meanings as commonly understood by one of ordinary skill in the art. Although any methods similar or equivalent to those described herein can be used in the practice or testing of embodiments of the present invention, the preferred methods are now described. Nothing herein is to be construed as an admission that the invention is not entitled to antedate such disclosure by virtue of prior invention.

It must be noted that, as used herein, and in the appended claims, the singular forms "a", "an", and "the" include plural reference unless the context clearly dictates otherwise.

Embodiments including the transition phrase "consisting of" or "consisting essentially of" include only the recited components and inactive ingredients. For example, a composition "consisting essentially of" dexpramipexole can include dexpramipexole and inactive excipients, which may or may not be recited, but may not contain any additional active agents or neuroprotectants. A composition "consisting of" dexpramipexole may include only the components specifically recited.

As used herein, the term "about" means plus or minus 10% of the numerical value of the number with which it is being used. Therefore, about 50% means in the range of 45%-55%.

"Optional" or "optionally" may be taken to mean that the subsequently described structure, event or circumstance may or may not occur, and that the description includes both instances where the event occurs and instances where it does not.

"Administering", when used in conjunction with a therapeutic, means to administer a therapeutic directly into or onto a target tissue or to administer a therapeutic to a subject whereby the therapeutic positively impacts the tissue to which it is targeted. "Administering" a composition may be accomplished by oral administration, injection, infusion, absorption or by any method in combination with other known techniques. "Administering" may include the act of self-administration or administration by another person such as a healthcare provider or a device.

The term "improves" is used to convey that the present invention refers to the overall physical state of an individual to whom an active agent has been administered. For example, the overall physical state of an individual may "improve" if one or more symptoms of a neurodegenerative disorder are alleviated by administration of an active agent. "Improves may also refer to changes in the appearance, form, characteristics, and/or physical attributes of tissue, or any combination thereof, to which it is being provided, applied, or administered.

As used herein, the term "therapeutic" means an agent utilized to treat, combat, ameliorate, or prevent, or any combination thereof, an unwanted condition or disease of a subject.

The terms "therapeutically effective amount" or "therapeutic dose" as used herein are interchangeable and may refer to the amount of an active agent or pharmaceutical compound or composition that elicits a biological and/or medicinal response in a tissue, system, animal, individual or human that is being sought by a researcher, veterinarian, medical doctor or other clinician, or any combination thereof. A biological or medicinal response may include, for example, one or more of the following: (1) preventing a disorder, disease, or condition in an individual that may be predisposed to the disorder, disease, or condition but does not yet experience or display pathology or symptoms of the disorder, disease, or condition, (2) inhibiting a disorder, disease, or condition in an individual that is experiencing or displaying the pathology or symptoms of the disorder, disease, or condition or arresting further development of the pathology and/or symptoms of the disorder, disease, or condition, and/or (3) ameliorating a disorder, disease, or condition in an individual that is experiencing or exhibiting the pathology or symptoms of the disorder, disease, or condition or reversing the pathology and/or symptoms disorder, disease, or condition experienced or exhibited by the individual. A biological or medicinal response may include, for example, one or more of the following: (1) inhibiting a disorder, disease, or condition in an individual that is experiencing or displaying the pathology or symptoms of the disorder, disease, or condition or arresting further development of the pathology and/or symptoms of the disorder, disease, or condition, and/or (2) ameliorating a disorder, disease, or condition in an individual that is experiencing or exhibiting the pathology or symptoms of the disorder, disease, or condition or reversing the pathology and/or symptoms disorder, disease, or condition experienced or exhibited by the individual.

As used herein, the term "neuroprotectant" refers to any agent that may prevent, ameliorate or slow the progression of neuronal degeneration and/or neuronal cell death.

The term "treating" may be taken to mean prophylaxis of a specific disorder, disease, or condition, alleviation of the symptoms associated with a specific disorder, disease, or condition and/or prevention of the symptoms associated with a specific disorder, disease or condition. In some embodiments, the term refers to slowing the progression of the disorder, disease, or condition or alleviating the symptoms associated with the specific disorder, disease, or condition. In some embodiments, the term refers to slowing the progression of the disorder, disease, or condition. In some embodiments, the term refers to alleviating the symptoms associated with the specific disorder, disease, or condition. In some embodiments, the term refers to restoring function which was impaired or lost due to a specific disorder, disease, or condition.

The term "subject" or "patient" generally refers to any living organism to which compounds described herein are administered and may include, but is not limited to, any human, primate, or non-human mammal. A "subject" may or may not be exhibiting the signs, symptoms, or pathology of amyotrophic lateral sclerosis (ALS) at any stage of any embodiment.

As used herein, the term "naive subject" refers to a subject that has not previously received pramipexole treatment (either (R)-pramipexole or (S)-pramipexole), particularly, (R)-pramipexole, or who has not received a titration regimen of pramipexole previous to receiving a starting dose of pramipexole.

As used herein, the terms "enantiomers", "stereoisomers", and "optical isomers" may be used interchangeably and refer to molecules which contain an asymmetric or chiral center and are mirror images of one another. Further, the terms "enantiomers", "stereoisomers", or "optical isomers" describe a molecule which, in a given configuration, cannot be superimposed on its mirror image.

As used herein, the terms "chirally pure", "optically pure", or "enantiomerically pure" may be taken to indicate that a composition contains a greatly enhanced percentage of a single optical isomer. In some embodiments, the chirally pure (6S)-dexpramipexole has 99% chiral purity or greater. In some embodiments, the chirally pure (6S)-dexpramipexole has 99.5% chiral purity or greater. In some embodiments, the chirally pure (6S)-dexpramipexole has 99.7% chiral purity or greater. In some embodiments, the chirally pure (6S)-dexpramipexole has 99.9% chiral purity or greater. In some embodiments, the chirally pure (6S)-dexpramipexole has 99.95% chiral purity or greater. In some embodiments, the chirally pure (6S)-dexpramipexole has 99.99% chiral purity or greater. Such chirally pure dexpramipexole allows for therapeutic and pharmaceutical compositions that may have a wide individual and daily dose range. As such, the various methods provide a composition including only dexpramipexole in a pharmaceutically acceptable dosage, and in some embodiments, such pharmaceutical compositions may further include a pharmaceutically acceptable carrier, excipient, and/or diluent.

The term "enantiomerically enriched" may be taken to indicate that at least 51% of a composition is a single optical isomer or enantiomer. The term "enantiomeric enrichment" as used herein refers to an increase in the amount of one enantiomer as compared to the other. A "racemic" mixture is a mixture of about equal amounts of (6R) and (6S) enantiomers of a chiral molecule.

The term "pharmaceutical composition" shall mean a composition including at least one active ingredient, whereby the composition is amenable to investigation for a specified, efficacious outcome in a mammal (for example, without limitation, a human). Those of ordinary skill in the art will understand and appreciate the techniques appropriate for determining whether an active ingredient has an efficacious outcome based upon the needs of the artisan. A pharmaceutical composition may, for example, contain dexpramipexole or a pharmaceutically acceptable salt of dexpramipexole as the active ingredient.

For the purposes of this disclosure, a "salt" is any acid addition salt, preferably a pharmaceutically acceptable acid addition salt, including but not limited to, halogenic acid salts such as hydrobromic, hydrochloric, hydrofluoric, and hydroiodic acid salts; any pharmaceutically acceptable inorganic acid salt such as, for example, nitric, perchloric, sulfuric, and phosphoric acid salts; any pharmaceutically acceptable organic acid salt such as, for example, sulfonic acid salts (methanesulfonic, trifluoromethane sulfonic, ethanesulfonic, benzenesulfonic or p-toluenesulfonic), acetic, malic, fumaric, succinic, citric, benzoic, gluconic, lactic, mandelic, mucic, pamoic, pantothenic, oxalic, and maleic acid salts; and any pharmaceutically acceptable amino acid salt such as aspartic or glutamic acid salts. Further, the acid addition salt may be a mono- or di-acid addition salt, such as a di-hydrohalogenic, di-sulfuric, di-phosphoric or di-organic acid salt. In all cases, the acid addition salt is used as an achiral reagent that is not selected on the basis of any expected or known preference for interaction with or precipitation of a specific optical isomer of the products of this disclosure.

"Pharmaceutically acceptable salt" is meant to indicate those salts which are, within the scope of sound medical judgment, suitable for use in contact with the tissues of a subject without undue toxicity, irritation, allergic response, and the like, and are commensurate with a reasonable benefit/risk ratio. Pharmaceutically acceptable salts are well known in the art. For example, Berge et al. 6, J. Pharm. Sciences, 1-19 (1977), describes pharmaceutically acceptable salts in detail.

As used herein, the term "daily dose amount" refers to the amount of dexpramipexole per day that is administered and/or prescribed to a subject. This amount can be administered in multiple unit doses or in a single unit dose, and at a single time during the day or at multiple times throughout the day.

A "dose amount" or "dose" as used herein, is generally equal to the dosage of the active ingredient which may be administered per day. For example, a dose amount of dexpramipexole may be 50 milligrams, 100 milligrams, 150 milligrams, 300 milligrams, 450 milligrams, 600 milligrams, or an amount between any two of these amounts per day.

The term "unit dose" as used herein may be taken to indicate a discrete amount of the therapeutic composition that contains a predetermined amount of the active compound. The amount of the active compound is generally equal to the dosage of the active ingredient which may be administered at a single time during the day or at multiple times throughout the day. The unit dose may be a fraction of the desired daily dose which may be given in fractional increments, such as, for example, one-half or one-third the desired daily dose. For example, a 150 milligram per day dose amount of dexpramipexole may be administered as two unit doses of 75 milligrams each, three unit doses of 50 milligrams each, or four unit doses of 37.5 milligrams each.

Throughout the application, the term "dopaminergic activity equivalent" (DAE) will be referred to, which means the measure of activity at the dopamine receptors equivalent to the activity of 1 milligram of pramipexole at the dopamine receptors. For example, a dosage of dexpramipexole having a "dopaminergic activity equivalent" (DAE) of 0.01 would have activity at the dopamine receptors which is equivalent to the activity of 0.01 milligrams of pramipexole. The DAE can also be related to a variety of pharmaceutical terms, including maximum tolerated dose (MTD), no observable adverse effect level (NOAEL), and non-effective dose amount for the sake of clarity. For example, the no observable adverse effect level (NOAEL) dose amount for pramipexole is most preferably below 0.05 milligrams. This, in turn, corresponds to a DAE of below 0.05. A dose amount of dexpramipexole having a DAE of 0.01 would, therefore, be below the DAE for the most preferable pramipexole NOAEL dose amount of 0.05 milligrams. In some embodiments, DAE is determined by measuring the binding affinity (IC₅₀) or activity (EC₅₀) at the D₂ and/or D₃ receptors relative to the same parameter for 1 milligrams of pramipexole.

The degree to which dosing of a molecule has demonstrable phenotypic activity resulting from affinity to particular receptors or other pharmaco-effective proteins, even when the activity results from affinities to unknown targets, can be operationally defined in terms of whether this activity contributes in a positive way ("on-target" activity) or a negative way ("off-target" activity) to a specific and desired therapeutic effect. For any given molecule, a number of "off-target" activities can theoretically be identified, but "on-target" activity is restricted to the desired therapeutic effect. To the extent that these activities can be measured and quantified, or comparisons be made with known standards, an index of activity can be generated for each of these categories (the "activity equivalent", or "AE"), and one or more ratios generated to compare "off-target" to "on-target" activities, useful to compare potential risk-benefit ratios between molecules.

Unless specifically indicated otherwise, reference to "EMPOWER", including in the Figures, shall mean Study 223AS302, which is a randomized, placebo-controlled study in which subjects with ALS have been randomized in a 1:1 ratio to placebo or dexpramipexole 150 mg every 12 hours. Subjects with possible, probable, probable laboratory-supported, or definite ALS as defined by the El Escorial criteria who had ALS symptoms for ≤24 months at the time of entry into the study and a slow vital capacity of ≥65% of predicted were eligible to participate in this study. This study is described in more detail in the Examples.

Unless specifically indicated otherwise, reference to "dex", including in the Figures, shall mean dexpramipexole, including (6R)-2-amino-4,5,6,7-tetrahydro-6-(propylamino)-benzothiazole and any pharmaceutically acceptable salt thereof, particularly (6R)-2-amino-4,5,6,7-tetrahydro-6-(propylamino)-benzothiazole dihydrochloride monohydrate.

Unless specifically indicated otherwise, reference to "CL201", including in the Figures, shall mean a 2-part, double-blind, randomized study of the safety, tolerability, and clinical effects of dexpramipexole in subjects with ALS. In Study CL201 Part 1, subjects with ALS received daily doses of either placebo (27 subjects) or dexpramipexole (75 subjects: 23 subjects, 50 mg per day; 26 subjects, 150 mg per day, or 26 subjects, 300 mg per day) for up to 12 weeks. In Part 2, 97 subjects continued on to the 4-week placebo washout phase; 92 subjects were subsequently re-randomized to double-blind active treatment with 50 mg per day or 300 mg per day dexpramipexole for at least 24 weeks. According to the protocol for Part 2, subjects were to receive 2 dosage levels of dexpramipexole for up to 76 weeks (4 weeks of placebo washout followed by up to 72 weeks of active, double-blind treatment). However, Part 1 results were analyzed and, subsequently, an administrative decision was made to close Study CL201 after all active subjects completed their Part 2 Week 28 visit. Therefore, once the last subject had completed at least 24 weeks of double-blind active treatment in Study CL201 Part 2 (Week 28), all active subjects were offered the opportunity to continue receiving open-label treatment with 300 mg per day dexpramipexole in the safety extension study, CL211. The administrative closure of the study created significant artificial attrition at study visits beyond Week 28, with a
corresponding increase in missing safety and efficacy data. Therefore, the Part 2, Week 28 visit was pre-specified as the primary time-point for all Part 2 data analyses.

Unless specifically indicated otherwise, reference to "CAFS", including in the Figures, shall mean Combined Assessment of Function and Survival.

Unless specifically indicated otherwise, reference to "El Escorial" or "EEC", including in the Figures, shall mean the El Escorial World Federation of Neurology Criteria for the Diagnosis of ALS, as further described herein.

Unless specifically indicated otherwise, reference to "El Escorial (definite)", including in the Figures, shall mean a diagnosis of definite ALS in accordance with the El Escorial World Federation of Neurology Criteria for the Diagnosis of ALS, as further described herein.

Unless specifically indicated otherwise, reference to "riluzole (yes)", including in the Figures, shall mean a subject is also taking (i.e., administering) riluzole.

Unless specifically indicated otherwise, reference to "symptom duration", including in the Figures, shall mean the time from the onset of an amyotrophic lateral sclerosis symptom(s) in a subject or observed in a subject.

Unless specifically indicated otherwise, reference to "ALSFRS-R", including in the Figures, shall mean ALS Functional Rating Scale, Revised.

Amyotrophic lateral sclerosis (ALS; Lou Gehrig's disease) is a progressively debilitating motor neuron disease, characterized by degeneration and dysfunction/death of upper and lower motor neurons. ALS is universally fatal but the rate of disease progression may not be linear. Many pathogenic mechanisms of ALS have been implicated and mitochondrial dysfunction likely plays a central role. There is currently no cure for ALS or treatments that attenuate functional decline.

Since 1994, multiple compounds have been evaluated as potential treatments to modify disease progression for ALS in randomized controlled trials with positive Phase III results only demonstrated with riluzole. However, riluzole has modest effects on survival and no demonstrated effect on muscle strength or functional decline.

Dexpramipexole is currently being evaluated for the treatment of ALS. While not wishing to be bound by theory, dexpramipexole is thought to work by increasing the bioenergetic efficiency of dysfunctional mitochondria. Dexpramipexole has shown efficacy in vitro and in vivo neurodegenerative disease models.

Dexpramipexole ((6R)-2-amino-4,5,6,7-tetrahydro-6-(propylamino)-benzothiazole) is a synthetic aminobenzothiazole derivative. The (6S) enantiomer of dexpramipexole, commonly known as "pramipexole" and commercially available under the Mirapex® name (Mirapex® is a registered trademark), is a potent dopamine agonist, which mimics the effects of the neurotransmitter dopamine. Throughout this disclosure, the word "pramipexole" will refer to (6S) enantiomer of 2-amino-4,5,6,7-tetrahydro-6-(propylamino)benzothiazole unless otherwise specified. Pramipexole has also been shown to have both neuroprotective and dopaminergic activities. Therefore, pramipexole may have utility as an inhibitor of the cell death cascades and loss of cell viability observed in neurodegenerative diseases such as Parkinson's disease. Additionally, oxidative stress caused by an increase in oxygen and other free radicals has been associated with the fatal neurodegenerative disorder amyotrophic lateral sclerosis (ALS), a progressive neurodegenerative disorder involving the motor neurons of the cortex, brain stem, and spinal cord.

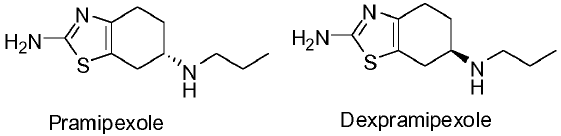

The neuroprotectant activity of both enantiomers may require therapeutic doses in the range of about 10 milligrams per day to about 1,500 milligrams per day. Despite this, pramipexole's dosage is limited to a range between 0.5 and 4.5 milligrams per day. Moreover, even at these low doses, significant adverse side effects have been reported. For example, the Boehringer Ingelheim product insert for Mirapex® sets the maximally tolerated dose for humans at 4.5 milligrams per day. Further, a dose of pramipexole as low as 1.5 milligrams has been shown to cause somnolence in humans. Single dose toxicity of pramipexole after oral administration has been studied in rodents, dogs, monkeys and humans. In rodents, death occurred at doses of 70-105 milligrams per kilogram and above, which is equivalent to a human dose of 7-12 milligrams per kilogram or approximately 500-850 milligrams for a 70 kilogram (approximately 150 pounds) individual. In dogs, vomiting occurred at 0.0007 milligrams per kilogram and above, while monkeys displayed major excitation at 3.5 milligrams per kilogram. In human subjects, an initial single dose of pramipexole of greater than 0.20 milligrams was not tolerated. All species showed signs of toxicity related to exaggerated pharmacodynamic responses to the dopaminergic agonism of pramipexole.

Adverse side-effects associated with low dose pramipexole treatment (less than 5 milligrams per day) include, but are not limited to, dizziness, hallucination, nausea, hypotension, somnolence, constipation, headache, tremor, back pain, postural hypotension, hypertonia, depression, abdominal pain, anxiety, dyspepsia, flatulence, diarrhea, rash, ataxia, dry mouth, extrapyramidal syndrome, leg cramps, twitching, pharyngitis, sinusitis, sweating, rhinitis, urinary tract infection, vasodilatation, flu syndrome, increased saliva, tooth disease, dyspnea, increased cough, gait abnormalities, urinary frequency, vomiting, allergic reaction, hypertension, pruritus, hypokinesia, nervousness, dream abnormalities, chest pain, neck pain, paresthesia, tachycardia, vertigo, voice alteration, conjunctivitis, paralysis, tinnitus, lacrimation, mydriasis and diplopia.

Thus, clinical use of pramipexole as a mitochondria-targeted neuroprotectant is unlikely, as the high dosage needed for the neuroprotective or anti-oxidative/mitochondrial normalization action are not accessible due to high dopamine receptor affinity associated with the (6S) enantiomer. In contrast, (6R)-2-amino-4,5,6,7-tetrahydro-6-(propylamino)benzothiazole ("dexpramipexole") is an effective mitochondria-targeted agent; when administered, dexpramipexole exhibits excellent neuroprotective properties without adverse side effects. Additionally, the functional affinity difference between pramipexole and dexpramipexole (e.g. 10,000-20,000 folds) for dopamine receptors is much greater than previously reported. Thus, higher doses of dexpramipexole can be tolerated by a subject, and will allow for greater brain, spinal cord and mitochondrial concentrations, ultimately increasing the degree to which oxidative stress and/or mitochondrial dysfunction may be reduced. The neuroprotective effect of dexpramipexole may occur by at least one of three mechanisms. First, dexpramipexole may be capable of reducing the formation of reactive oxygen species in cells with impaired mitochondrial energy production. Second, dexpramipexole may partially restore the reduced mitochondrial membrane potential that is correlated with Alzheimer's, Parkinson's, Huntington's, and amyotrophic lateral sclerosis (ALS) diseases. Third, dexpramipexole may block or attenuate the apoptotic cell death pathways that are produced by pharmacological models of Alzheimer's disease, Parkinson's disease, Huntington's disease, amyotrophic lateral sclerosis diseases (ALS), and mitochondrial impairment. The high doses of dexpramipexole required to elicit these neuroprotective effects generally require highly pure preparations of dexpramipexole which take into account the biologically tolerable upper limit of (6S) enantiomer contamination (0.5 milligrams to 4.5 milligrams).

In accordance with embodiments described herein, the El Escorial World Federation of Neurology's Criteria for the Diagnosis of ALS is as follows (See also Brooks, B. R., R. G. Miller, et al. (2000). "El Escorial revisited: revised criteria for the diagnosis of amyotrophic lateral sclerosis." Amyotroph Lateral Scler Other Motor Neuron Disord 1(5): 293-9).

The diagnosis of ALS requires the presence of: 1.) Signs of lower motor neuron (LMN) degeneration by clinical, electrophysiological or neuropathologic examination, 2.) Signs of upper motor neuron (UMN) degeneration by clinical examination, and 3.) Progressive spread of signs within a region or to other regions, together with the absence of: Electrophysiological evidence of other disease processes that might explain the signs of LMN and/or UMN degenerations; and Neuroimaging evidence of other disease processes that might explain the observed clinical and electrophysiological signs.

Furthermore, the El Escorial World Federation of Neurology's Criteria for the Diagnosis of ALS set forth the following steps in the diagnosis of Amyotrophic Lateral Sclerosis. The diagnosis of ALS is made possible by 1.) History, physical and appropriate neurological examinations to ascertain clinical finding which may suggest suspected, possible, probable or definite ALS, 2.) Electrophysiological examinations to ascertain findings which confirm LMN degeneration in clinically involved regions, identify LMN degeneration in clinically uninvolved regions and exclude other disorders, 3.) Neuroimaging examinations to ascertain findings which may exclude other disease processes, 4.) Clinical laboratory examinations, determined by clinical judgment, to ascertain possible ALS-related syndromes, 5.) Neuropathologic examinations, where appropriate, to ascertain findings which may confirm or exclude sporadic ALS, coexistent sporadic ALS, ALS-related syndromes or ALS variants, 6.) Repetition of clinical and electrophysiological examinations at least six months apart to ascertain evidence of progression.

### Clinical features in the diagnosis of ALS

Patients with signs of LMN degeneration (weakness, atrophy and clinical fasciculation's) and UMN degeneration (spasticity, pathologic reflexes, etc.) may be suspected as having ALS. Careful history, physical and neurological examination must search for further clinical evidence of LMN and UMN signs in four regions of the central nervous system.

Clinical features required for the diagnosis of ALS: 1.) Signs of LMN degeneration (weakness, wasting and fasciculation) in one or more of the four regions (bulbar, cervical, thoracic, lumbosacral). LMN findings in a region are without regard to right or left, but are indicative of the level of neuroaxis involved. Therefore, spread of weakness, wasting and fasciculation's to another region is more important than spread from right to left or vice-versa. 2.) Signs of UMN degeneration (increased or donic tendon reflexes, spasticity, pseudo bulbar features, Hoffmann reflex and extensor plantar response) in one or more of the four regions. These UMN signs are clinically appreciated best in the bulbar, cervical and lumbosacral regions. UMN findings in a region are also without regard to right or left. Once the physical and neurological examinations provide information on the presence or absence of LMN and UMN signs in the four regions (bulbar, cervical, thoracic, lumbosacral) they must be ordered topographically in the manner to determine the certainty of the diagnosis of ALS. 3.) The topographical location of certain UMN and LMN signs in four regions of the CNS together with progression of these signs determines the certainty of the diagnoses of ALS. Progression is a cardinal feature of the clinical diagnosis of ALS. Progression of signs within a region and progression of signs to involve other regions are crucial to the diagnosis.

Clinical examinations should be repeated at least every six (6) months to assess progression.

Cases which meet the topographical criteria for probable or definite ALS but which lack progression during the twelve (12) month period diagnosis should be designated as possible ALS.

Definite ALS is defined on clinical grounds alone by the presence of UMN as well as LMN signs in the bulbar region and at least two of the other spinal regions or the presence of UMN and LMN signs in three spinal regions. The important determinants of diagnosis of definite ALS in the absence of electrophysiological, neuroimaging and laboratory examinations are the presence of UMN and LMN signs together in multiple regions.

Probable ALS is defined on clinical grounds alone by UMN and LMN signs in at least two regions. While the regions may be different, some UMN signs must be rostral (above) the LMN signs. Multiple different combinations of UMN and LMN signs may be present in patients with probable ALS.

Possible ALS is defined on clinical grounds alone when the UMN and LMN signs are in only one region or UMN signs alone are present in 2 or more regions or LMN signs are rostral to UMN signs (the latter distribution of signs needs to be differentiated from multiple non-ALS processes). Monomelic ALS, progressive bulbar palsy without spinal UMN and/or LMN signs and progressive primary lateral sclerosis without spinal LMN signs and progressive primary lateral sclerosis without spinal LMN signs constitute special cases which may develop LMN or UMN signs to meet the criteria for probable ALS with time or be subsequently confirmed at autopsy by specific LMN and UMN neuropathologic findings.

Suspected ALS will manifest only LMN signs in 2 or more regions, although UMN pathology might be demonstrated at autopsy. However, only clinical signs are considered pertinent to this classification at the time of diagnostic evaluation.

### Supportive clinical features

Clinical features that support the diagnosis of ALS include one or more of the following: 1.) abnormal pulmonary function test not explained by other causes, 2.) abnormal speech studies not explained by other causes, 3.) abnormal swallowing studies not explained by other causes, 4.) abnormal larynx function studies not explained by other causes, 5.) abnormal isokinetic or isometric strength test in clinically uninvolved muscles, 6.) abnormal muscle biopsy with evidence of denervation.

### Inconsistent clinical features

Clinical findings inconsistent with the diagnoses of ALS include one or more of the following not explained by physiological changes associated with aging or other disease processes: 1.) sensory dysfunction, 2.) sphincter abnormalities, 3.) autonomic nervous system dysfunction, 4.) anterior visual pathway abnormalities, 5.) movement abnormalities associated with probable Parkinson's disease defined by DATATOP criteria, 6.) cognitive abnormalities associated with clinical Alzheimer's disease as defined by NINCDS-ADRDA criteria.

If these clinical findings occur, then close attention should be paid to the possible diagnosis of other disease processes.

Lower motor neuron and upper motor neuron signs may occur together with other clinical signs in disease where the pathologic process is not primary motor neuron degeneration.

### Types of ALS

The clinical signs of progressive LMN and UMN degeneration seen in ALS may a.) occur alone (sporadic ALS), b.) be present incidentally with other pre-existing disease processes that have not developed in parallel with the ALS (coexistent sporadic ALS), c.) Occur in association with laboratory-defined or epidemiologically defined abnormalities that are time-linked to the ALS (ALS-related syndromes), or d.) Occur in association with clinical, genetic or epidemiological features which develop in parallel with the ALS (ALS variants).

The physical and neurological examinations will allow for the clinical diagnosis of ALS to a particular degree of certainty as defined above; however, the history of the disease onset, toxic exposures, past medical history, injuries, family history, geographic location, etc., must be incorporated with the clinical examinations in determining whether the patient may have an ALS related syndrome or an ALS variant.

ALS-related syndromes must meet the clinical, electrophysiological and neuroimaging criteria for possible, probable or definite ALS. ALS-related syndromes have unique laboratory-defined or epidemiologically defined features which are time-linked to the development of the ALS phenotype. If correction of the associated laboratory-defined feature does not result in correction of the ALS phenotype, then the patient with an ALS-related syndrome should be considered in the same way as a patient with sporadic ALS.

ALS-related syndromes include: 1.) Monoclonal gammopathy (monoclonal gammopathy of unknown significance, Waldenstroms's macroglobulinemia, osteosclerotic myeloma, etc.), 2.) Dysimmune motor system degeneration (autoimmune; high-titer GMI ganglioside antibody; etc.), 3.) Nonmalignant endocrine abnormalities (hyperthyroidism, hyperparathyroidism, hypogonadism, etc.), 4.) Lymphoma (Hodgkin's and non-Hodgkin's lymphoma). Cases of sporadic ALS associated with insulinoma, lung, colon or thyroid cancer are thought not to be casually related, 5.) Infection (HIV-1, HTLV-I, encephalitis lethargica, varicella-zoster, brucellosis, cat-scratch disease, Creutzfeldt-Jakob disease, syphilis, delayed post-poliomyelitis, etc.), 6.) Acquired enzyme defects (detoxification enzymes, etc.), 7.) Exogenous toxins (lead, mercury, arsenic, thallium, cadmium, manganese, aluminum, organic pesticides, lupin seeds, etc.), 8.) Physical injury (electric shock, radiation therapy, etc.), 9.) Vascular (vasculitis; ischemic (Dejerine anterior bulbar artery syndrome, etc.), 10.) Spondylotic myelopathy (painless myelopathy with no sensory signs, stabilization or progression post-surgery).

ALS Variants must meet the clinical, electrophysiological and neuroimaging criteria for possible, probable or definite ALS. The predominant presentation is that seen in sporadic ALS, but includes one or more features such as: Familial pattern of inheritance
(multiple phenol-types characterized by age of onset; site of onset; length of survival; and presumed type of inheritance.)

Familial ALS variants in genetic linkage studies should be characterized by an established genetic mode of inheritance over at least two generations and at least one clinically definite or autopsy confirmed case and compelling evidence excluding other possible causes. Affected sub pairs occurring in one generation alone may not result from a single gene effect.

Examples: a.) ALS with defined inheritance and known gene product (hexosaminidase A/B deficiency, superoxide dismutase deficiency); b.) ALS with defined inheritance and chromosome linkage but no gene product (chromosome 21 associated familial ALS or chromosome 2 associated juvenile familial ALS); c.) ALS with defined inheritance and no known linkage or gene product (most cases or familial ALS); d.) Geographic clustering (including disorders seen in the Western Pacific, Guam, Kii Peninsula, North Africa, Madras, etc.); e.) Extrapyramidal signs (bradykinesia; cogwheel rigidity; tremor; clinically significant onset of supranuclear eye signs (pursuit abnormalities); familial or sporadic); f.) Cerebellar degeneration (spinocerebellar abnormalities; familial or sporadic); g.) Dementia (progressive cognitive abnormalities; familial or sporadic); h.)Autonomic nervous system involvement (clinically significant abnormal cardiovascular reflexes; bowel or bladder control problems; familial or sporadic); i.) Objective sensory abnormalities (decreased vibration; sharp-dull discrimination; blunting of cold sensation; familial or sporadic); j.) Electrophysiological features in the diagnoses of ALS

Patients with suspected, possible, probable or definite ALS on clinical grounds should have electrophysiological studies performed to confirm LMN degeneration in clinically affected regions, find electrophysiological evidence of LMN degeneration in clinically uninvolved regions and to exclude other pathophysiological processes.

ALS may be most reliably identified when the clinical and electrophysiological findings are widespread, involving a sufficient number of regions so that other possible cause of similar EMG abnormalities are highly unlikely. The confirmation of the diagnosis of ALS depends on finding electrophysiological evidence of LMN degeneration in at least two muscles of different root or spinal nerves and different cranial or peripheral nerve innervation in two or more of the four (bulbar, cervical, thoracic, lumbosacral) regions. The features of LMN degeneration in a particular muscle are defined by electromyographic needle examination and nerve conduction studies using standard methods for each measure.

Electrophysiological features required to identify definite primary LMN degeneration include all of the following: 1.) Reduced recruitment (reduced interference pattern with firing rates over 10 Hz), 2.) Large motor unit action potentials (large amplitude, long duration), and 3.) Fibrillation potentials.

Electrophysiological features that support the identification of possible primary LMN degeneration include one or more of the following: 1.) either reduced recruitment, large motor unit potentials, fibrillation potentials or unstable motor unit potentials alone, 2.) polyphasic motor unit potentials or increased single fiber density alone, 3.) low amplitude compound muscle actions potentials if the disease duration is over 5 years or if there is associated atrophy, 4.) low amplitude compound muscle action potentials, 5.) compound muscle action potential change between proximal and distal sites of stimulation that is uniform along the length of the nerve, 6.) up to 30% decrement in motor conduction velocity below established normal values if a low amplitude compound muscle action potential greater than 10 percent of normal is present, 7.) up to 50% decrement in motor conduction velocity below established normal values if the compound muscle action potential is below 10% or normal, 8.) up to 20% decrement of the compound muscle action potential on 2 Hz repetitive stimulation, 9.) up to 10% decrement in sensory nerve conduction velocity and action potential amplitude from established normal values, 10.) complex repetitive discharges, and 11.) absence of fasciculations.

Electrophysiological features compatible with UMN degeneration and not excluding ALS include one or more of the following: 1.) up to 30% increment in central motor conduction velocity, 2.) up to 10% decrement in somatosensory, evoked potential amplitude and up to 10% increment in somatosensory evoked potential latency, 3.) mild abnormalities of autonomic function, 4.) mild abnormalities of polysomnography, 5.) mild abnormalities of electronystagmography.

Electrophysiological features that are inconsistent with the diagnoses of ALS or suggest the presence of additional other disease processes include one or more of the following: 1.) focal reduction in compound muscle action potential or more than 10% in a 4-cm segment, 2.) motor conduction velocities, F wave latencies or H wave amplitudes which are more than 30% above established normal values, 3.) more than 20% decrement of repetitive stimulation at 2 Hz, 4.) sensory action potential latencies more than 20% above or sensory action potential amplitudes more than 20% below established normal values, 5.) unstable motor unit potentials with no other electromyographic changes, 6.) more than 30% increment of central motor conduction velocity, 7.) more than 10% increment in sensory evoked potential latency or more than 10% decrement in sensory evoked potential amplitude, 8.) moderate or greater abnormalities in autonomic function or electronystagnography.

Employing electrophysiological evidence of LMN degeneration to confirm the diagnosis of ALS

The certainty of LMN degeneration is determined by the presence of the above finding for each muscle tested in the region.

At least two muscles of different root or spinal nerve and different cranial or peripheral nerve innervation in each region should show electrophysiological evidence of either definite, probable or possible LMN degeneration for that region to be ranked as showing definite, probable or possible LMN degeneration.

Definite LMN degeneration by EMG has the same significance as clinical LMN degeneration and can upgrade the certainty of the clinical diagnoses of ALS in the same fashion as if the clinical signs of LMN degeneration were present in that region.

Probable or possible LMN degeneration by EMG does not carry the same weight as either clinical signs of definite electrophysiological evidence of LMN degeneration in a particular region.

However, the involvement of the regions with probable electrophysiological evidence of LMN degeneration or one region with probable and one region with possible electrophysiological evidence of LMN degeneration carries the same weight as one region with definite evidence of LMN degeneration in upgrading the certainty of diagnosis of ALS.

A single region with electrophysiological evidence of probable LMN degeneration or two regions with electrophysiological evidence of possible LMN degeneration can be used to upgrade the certainty of the diagnosis of ALS from possible ALS to probable ALS but not from probable ALS to definite ALS.

### Neuroimaging features in the diagnosis of ALS

Neuroimaging studies should be selected in order to exclude other conditions which may cause UMN and/or LMN signs that may stimulate sporadic ALS.

### Neuroimaging features required for the diagnosis of ALS:

There are no neuroimaging tests which confirm the diagnosis of ALS.

Neuroimaging features that support the diagnosis of ALS include one or more of the following: 1.) minimal bony abnormalities on plain x-ray of skull or spinal canal, 2.) Minimal abnormalities on head or spinal cord MRI scans without spinal cord and/or root compression, 3.) Minimal abnormalities on spinal cord myelography with post-myelography CT tomography showing no spinal cord and/or root compression.

Neuroimaging features that are inconsistent with the diagnosis of ALS include one or more of the following: 1.) significant bony abnormalities on plain x-ray of skull or spinal canal, 2.) minimal abnormalities on head or spinal cord MRI scans without spinal cord and/or root compression, 3.) minimal abnormalities on spinal cord myelography with post-myelography CT tomography showing no spinal cord and/or room compression.

Neuroimaging features that are inconsistent with the diagnosis of ALS include one or more of the following: 1.) significant bony abnormalities on plain x-rays of skull or spinal canal that might explain clinical findings, 2.) significant abnormalities of head or spinal cord MRI suggesting intraparenchymal processes, arteriovenous malformations or compression of brainstem/spinal cord and/or cranial nerve or spinal nerve roots by bony abnormalities, tumor, etc. MRI of craniocervical function if bulbar onset and/or MRI or pertinent spinal region if spinal onset, 3.) significant abnormalities of spinal cord myelography with/without CT tomography or CT tomography alone suggesting lesions as noted above, 4.) significant abnormalities on spinal cord angiography suggesting arteriovenous malformations.

### Employing neuroimaging evidence to confirm the diagnosis of ALS

The absence of abnormalities in appropriately performed neuroimaging studies will raise patients with clinical and/or electrophysiological evidence of probable ALS to definite ALS.

The absence of neuroimaging abnormalities cannot raise possible ALS to probable ALS.

### Clinical laboratory features in the diagnosis of ALS

The diagnostic process employed to confirm the diagnosis of sporadic ALS includes repeated clinical examinations, repeated electrophysiological examinations, neuroimaging to exclude other disorders and clinical laboratory examinations or exclude other disorders or support the diagnosis of ALS related syndromes.

Clinical laboratory features required for the diagnoses of ALS: There are no clinical laboratory tests which confirm the diagnosis of ALS.

Clinical laboratory features that support the diagnosis of ALS include one or more of the following: normal complete blood count, platelet count, sedimentation rate, prothrombin time, normal electrolyte (Na+, K+, C1, C02+, Mg2+, P04) renal (BUN, creatinine) and liver function (bilirubin, SGOT, SGPT, LDH) test, creatine kinase (CK) elevation not more than 5 times upper limit of normal, normal hexosaminidase A and B activity (if possible of deficiency indicated by suggestive family history or onset under 30
years of age),normal cerebrospinal fluid cell count, protein (not more than 65 mg/dl), absence of intrathecal immunoglobulin synthesis, oligoclonal immunoglobulins and evidence of elevated intrathecal antibodies or infectious agents (syphilis, HIV-1, HTLV-I, etc.), if indicated, normal parathyroid hormone level if calcium is borderline elevated, normal free thyroid hormone concentrations if any thyroid function abnormalities (borderline elevations in T4, T3, TSH); normal glycosylated hemoglobin, if indicated, Normal serum protein electrophoresis and serum immunoeletrophresis with immunofixation; normal urine immunoelectrophoresis with immunofixation, if indicated, Minimal abnormalities in screening test for collagen vascular diseases (anti-nuclear antibody; anti-DNA antibodies; rheumatoid factor, complement, anti-tissue specific antibodies), if indicated, Minimal elevation in screening test for anti-neural antigen (GM1, GM2, GD1b gangliosides, myelin-associated glycoprotein, acetylcholine esterase, etc.) or anti-neuromuscular antigen (acetylcholine receptor, striated muscle, etc.) antibodies, if indicated.

Clinical laboratory features that support the diagnosis of ALS related syndromes

Abnormalities consistent with monoclonal gammopathy with/without significant elevation in monoclonal anti-neural antigen antibody; Significant elevations in polyclonal anti-neural antigen (Gm1, Gm2, GD1b gagnliosides, myelin-associated glycoprotein, acetylcholine esterase, etc.) antibody; Significant elevation in parathyroid hormone, thyroid hormone or other significant endocrine abnormalities; Abnormalities consistent with lymphoma (Hodgkins' or non-Hodgkin's lymphoma); Evidence of infection (HIV-1, HTLV-I, borrelia, syphilis, brucellosis, cat-scratch disease, varicella-zoster, influenza, Creutzfeldt-Jakob disease); Evidence of intoxication (epidemiological evidence or elevated blood, urine, tissue or cerebrospinal fluid level of lead, mercury, arsenic, cadmium, manganese, aluminum, organic pesticides, lupin seeds, etc.); Evidence of physical injury (epidemiological evidence of antecedent electrical or radiation injury or severe trauma); Evidence of vasculitis (elevated erythrocyte sedimentation rate and cerebrospinal fluid abnormalities consistent with spinal cord vasculitis, i.e., markedly elevated cerebrospinal fluid protein) or ischemic injury to spinal cord without sensory signs; Evidence of pre-existing mild or moderate spinal cord spondylotic compression, not amenable to surgical correction or not responding to surgical correction, which progressed with clinical signs consistent with at least probable ALS.

### Clinical laboratory features inconsistent with the diagnosis of ALS:

There is no clinical laboratory finding which, if present with the proper clinical and electrophysiological signs of ALS and appropriate neuroimaging studies, rules out the diagnosis of ALS.

ALS-related syndromes which present with the ALS phenotype as defined have been described with laboratory abnormalities (acquired or genetic), time-linked exposure to chemical, physical or infectious agents and pre-existing structural abnormalities. The correction of laboratory abnormalities, removal of chemical or physical agents, treatment of the associated disease (infection, tumor, structural abnormality) may or may not result in correction or stabilization of the ALS phenotype in ALS-related syndromes.

If correction of the abnormality does not result in improvement, the patient will be considered to have an ALS related syndrome.

If correction of the abnormality does not result in improvement, then the patient will be considered to have an ALS related syndrome.

If correction of the abnormality does not result in improvement then the patient will be considered to have sporadic ALS for the purpose of clinical studies and the therapeutic trials.

### Neuropathological features of the diagnosis of ALS:

The clinical diagnosis may be supported or excluded by biopsy studies in the living patient and the pathological diagnosis may be proven or excluded by autopsy examination.

### Pathological studies in the living patient with sporadic ALS:

### Indications of biopsies:

Biopsies of the skeletal muscle, peripheral nerve and other tissues are not required for the diagnosis of amyotrophic lateral sclerosis, unless the clinical, electrophysiological or laboratory studies have revealed changes that are atypical for ALS. In addition, the muscle biopsy may be used to demonstrate LMN involvement in a body region that had not been shown to be involved by other techniques.

### Muscle biopsy:

### Features required for the diagnosis:

Disseminated single angulated atrophic muscle fibers, or small or large groups of such fibers.

### Features that strongly support the diagnosis:

Angulated atrophic muscle fibers that are strongly positive when stained with oxidative enzyme stains and with non-specific esterase or that show immunoreactive surface staining with anti-NCAM antibodies.

Features that are compatible with, and do not exclude the diagnosis: Scattered hypertrophied muscle fibers; No more than a moderate number of target or targeted fibers; Fiber type grouping of not more than mild-to-moderate extent; The presence of a small number of necrotic muscle fibers.

Features that rule out the diagnoses or suggest the presence of additional disease: 1.) Significant infiltration with lymphocytes and other mononuclear inflammatory cells; 2.) Significant arteritis; 3.) Significant numbers of muscle fibers involved with the following structural changes: necrosis; rimmed vacuoles; nemaline bodies; central cores; accumulation of mitochondria (ragged red fibers); 4.) Large fiber type grouping; 5.) Giant axonal swellings from accumulation of masses of neurofilaments, but not of PAS positive bodies, in intramuscular nerves.

Pathological studies at autopsy, other than in cases surviving for prolonged periods on life support systems

### Gross pathological changes

### Features required for the diagnosis:

There are positive diagnostic features on gross pathological examination.

### Features that support the diagnosis:

There are no positive diagnostic features on gross pathological examination.

Features that support the diagnosis: selective atrophy of the motor cortex, grayness and atrophy of the anterior spinal nerve roots compared with normal, grayness of the lateral columns of the spinal cord, atrophy of skeletal muscles.

Features that rule out the diagnosis of ALS or suggest the presence of additional disease: 1.) Plaques of multiple sclerosis; 2.) A focal cause of myelopathy.

### Light microscopic studies

Features required for the diagnosis: 1.) Some degree of loss of both of the following neuronal systems. Large motor neurons of the anterior horns of the spinal cord and motor nuclei of the brainstem (V motor, VII motor, I and X somatic motor, and XII); and large pyramidal neurons of the motor cortex and/or large myelinated axons of the corticospinal tracts. 2.) The following cellular pathological changes in the involved neuronal regions described above; neuronal atrophy with relative increase in lipofuscin and loss of Nissl substance. There should be evidence of different stages of the process of neuronal degeneration, including the presence of normal-appearing neurons, even in the same region. 3.) Evidence of degeneration of the corticospinal tracts and the same level.

Features that strongly support the diagnosis: 1.) Lack of pathological change in the motor neurons of cranial nerves III, IV and VI, the intermediolateral column of the spinal cord, and Onuf s nucleus. 2.) The occurrence of one or more of the following cellular pathological changes in the involved neuronal systems described above: Axonal spheroids with accumulation of masses of neurofilaments, Bunina bodies; Basophilic cytoplasmic inclusions; Non-basophilic hyaline bodies ("Lewy body-like structures") seen in H&E stained sections; Increased immunocytochemical staining for phosphorylated neurofiliments in perikarya of the motor neurons; Atrophy or loss of the arborizations of the dendrites of the motor neurons of the anterior horns of the spinal cord and brainstem motor nuclei; Wallerian degeneration in the anterior roots.

### Features that are compatible with, and do not exclude, the diagnoses:

Variable involvement of Clark's nucleus and the spinocerebellar tracts; posterior root ganglia, the posterior columns of the spinal cord and peripheral sensory nerves; the brainstem reticular neurons and the anterolateral columns of the spinal cord; the thalamus; subthalamic nucleus; and the subastantia nigra.

Features that rule out the diagnosis or suggest the presence of additional disease:

Major pathological involvement of other parts of the nervous system, including: cerebral cortex other than the motor cortex; basal ganglia; substantia nigra; cerebellum; cranial nerves II and VIII; dorsal root ganglia.

The following cellular pathological changes in the involved neuronal systems described above: Extensive central chromatolysis; Extensive active neuronophagia; Neurofibrillary tangles; The presence of abnormal storage material; The presence of significant spongiform change; The presence of extensive inflammatory cell infiltration.

### Electron-microscopic studies

### Features required for the diagnosis:

### Ultrastructural studies are not required for the diagnosis of ALS

Features that strongly support the diagnosis: Accumulation of interwoven bundles of 10 nm neurofilaments in axonal spheroids or motor neuron perikarya, and thicker linear structures associated with dense granules (Hirano et al. 1984 J. Neuropath. Ex. Neurol. 43:461); Bunina bodies (Hart et al. 1944 Acta Neuropath. 38:225).

### Features that are compatible with, and do not exclude the diagnosis:

The presence of intra-axonal polyglucosan bodies.

Features that rule out the diagnosis or suggest the presence of additional disease; 1.) The presence of significant numbers of definite viral particles. 2.) The presence of significant amounts of abnormal storage materials. 3.) Extensive vacuolation of neuronal perikarya.

### Glossary of Terms:

As used in the El Escorial World Federation of Neurology's Criteria for the Diagnosis of ALS, the term "definite" is intended to mean specific clinical exclusionary criteria met; no other diagnosis possible on basis of clinical distribution or laboratory findings.

As used in the El Escorial World Federation of Neurology's Criteria for the Diagnosis of ALS, the term "dementia" is intended to mean progressive deterioration of specific cognitive functions.

As used in the El Escorial World Federation of Neurology's Criteria for the Diagnosis of ALS, the term "extraphyramidal" is intended to mean clinical features localizable to basal ganglia and/or midline cerebellum.

As used in the El Escorial World Federation of Neurology's Criteria for the Diagnosis of ALS, the term "hyperreflexia" is intended to mean the spread of deep tendon reflex outside stimulated territory.

As used in the El Escorial World Federation of Neurology's Criteria for the Diagnosis of ALS, the term "minor" is intended to mean subjective and objective complaints confirmed by examination (utilization of instrumental sensory testing may increase the detection of sensory abnormalities).

As used in the El Escorial World Federation of Neurology's Criteria for the Diagnosis of ALS, the term "onset" is intended to mean time of first subjective symptom noticed by patient which later is confirmed by examination.

As used in the El Escorial World Federation of Neurology's Criteria for the Diagnosis of ALS, the term "possible" is intended to mean specific clinical and exclusionary criteria met.

As used in the El Escorial World Federation of Neurology's Criteria for the Diagnosis of ALS, the term "probable" is intended to mean specific clinical and exclusionary criteria.

As used in the El Escorial World Federation of Neurology's Criteria for the Diagnosis of ALS, the term "radicular" is intended to mean distribution conforming to particular nerve root.

As used in the El Escorial World Federation of Neurology's Criteria for the Diagnosis of ALS, the term "region" is intended to mean brainstem, cervical, thoracic or lumbosacral spinal cord level (regional involvement is defined by either right or left sided signs).

As used in the El Escorial World Federation of Neurology's Criteria for the Diagnosis of ALS, the term "required" is intended to mean necessary or sufficient.

As used in the El Escorial World Federation of Neurology's Criteria for the Diagnosis of ALS, the term "segment" is intended to mean single brainstem or spinal cord level.

As used in the El Escorial World Federation of Neurology's Criteria for the Diagnosis of ALS, the term "spread" is intended to mean involvement of new anatomic segments or regions in the central nervous system.

As used in the El Escorial World Federation of Neurology's Criteria for the Diagnosis of ALS, the term "support" is intended to mean neither necessary nor sufficient, but may suggest.

As used in the El Escorial World Federation of Neurology's Criteria for the Diagnosis of ALS, the term "systemic" is intended to mean non-central nervous system.

As used in the El Escorial World Federation of Neurology's Criteria for the Diagnosis of ALS, the term "weakness" is intended to mean decreased isometric strength.

As used in the El Escorial World Federation of Neurology's Criteria for the Diagnosis of ALS, the term "worsening" is intended to mean increased weakness of muscles in previously affected segment.

The El Escorial ALS Classifications are as follows:
Definite ALS is defined on clinical grounds alone by the presence of UMN as well as LMN signs in the bulbar region and at least two of the other spinal regions or the presence of UMN and LMN signs in three spinal regions. The important determinants of diagnosis of definite ALS in the absence of electrophysiological, neuroimaging and laboratory examinations are the presence of UMN and LMN signs together in multiple regions.

Probable ALS is defined on clinical grounds alone by UMN and LMN signs in at least two regions. While the regions may be different, some UMN signs must be rostral (above) the LMN signs. Multiple different combinations of UMN and LMN signs may be present in patients with probable ALS.

Possible ALS is defined on clinical grounds alone when the UMN and LMN signs are in only one region or UMN signs alone are present in 2 or more regions or LMN signs are rostral to UMN signs (the latter distribution of signs needs to be differentiated from multiple non-ALS processes). Monomelic ALS, progressive bulbar palsy without spinal UMN and/or LMN signs and progressive primary lateral sclerosis without spinal LMN signs and progressive primary lateral sclerosis without spinal LMN signs constitute special cases which may develop LMN or UMN signs to meet the criteria for probable ALS with time or be subsequently confirmed at autopsy by specific LMN and UMN neuropathologic findings.

Suspected ALS will manifest only LMN signs in 2 or more regions, although UMN pathology might be demonstrated at autopsy. However, only clinical signs are considered pertinent to this classification at the time of diagnostic evaluation.

Disclosures herein are directed to methods for treating amyotrophic lateral sclerosis in a subject in need thereof comprising administering to the subject a therapeutically effective amount of (6R)-2-amino-4,5,6,7-tetrahydro-6-(propylamino)benzothiazole or a pharmaceutically acceptable salt thereof, wherein the subject is selected from a subject with definite amyotrophic lateral sclerosis, a subject with limb-onset amyotrophic lateral sclerosis, a subject with amyotrophic lateral sclerosis symptom onset duration of less than about 18 months, a subject with a high level of serum creatinine, a subject with low bicarbonate levels, a subject with concomitant riluzole administration and combinations thereof, and wherein the amyotrophic lateral sclerosis is treated. The method may further comprises monitoring said subject for any clinical features associated with amyotrophic lateral sclerosis. The method may further comprises initiating therapy with a therapeutically effective amount of (6R)-2-amino-4,5,6,7-tetrahydro-6-(propylamino)benzothiazole or a pharmaceutically acceptable salt thereof upon diagnosis of amyotrophic lateral sclerosis. The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis. Treating amyotrophic lateral sclerosis in a subject in need thereof may further comprise administering a therapeutically effective amount of creatine or a pharmaceutically acceptable salt thereof. Treating amyotrophic lateral sclerosis in a subject in need thereof may further comprise administering a therapeutically effective amount of riluzole or a pharmaceutically acceptable salt thereof.

In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. In some embodiments, the pharmaceutical composition further comprises a therapeutically effective amount of creatine or a pharmaceutically acceptable salt thereof. In some embodiments, the pharmaceutical composition further comprises a therapeutically effective amount of riluzole or a pharmaceutically acceptable salt thereof.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

In certain embodiments, the subject with definite amyotrophic lateral sclerosis is a subject diagnosed with definite ALS as defined by the El Escorial diagnosis criteria. In certain embodiments, the subject with definite amyotrophic lateral sclerosis is a subject with upper motor neuron degeneration and lower motor neuron degeneration in the bulbar region and two other spinal regions. In certain embodiments, the subject with definite amyotrophic lateral sclerosis is a subject with upper motor neuron degeneration and lower motor neuron degeneration in three spinal regions. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. In some embodiments, the pharmaceutical composition further comprises a therapeutically effective amount of creatine or a pharmaceutically acceptable salt thereof. In some embodiments, the pharmaceutical composition further comprises a therapeutically effective amount of riluzole or a pharmaceutically acceptable salt thereof.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

In certain embodiments, the subject with amyotrophic lateral sclerosis symptom onset duration of less than about 18 months is a subject with symptom onset selected from less than 18 months, less than about 17 months, less than about 16 months, less than about 15 months, less than 15.3 months, less than about 14 months, less than about 13 months, less than about 12 months, less than about 11 months, less than about 10 months, less than about 9 months, less than about 8 months, less than about 7 months, less than about 6 months, less than about 5 months, less than about 4 months, less than about 3 months, less than about 2 months, and less than about 1 month. In certain embodiments, the subject with amyotrophic lateral sclerosis symptom onset duration of less than about 18 months is a subject with symptom onset selected from about 18 months, about 17 months, about 16 months, about 15 months about 14 months, about 13 months, about 12 months, about 11 months, about 10 months, about 9 months, about 8 months, about 7 months, about 6 months, about 5 months, about 4 months, about 3 months, about 2 months, and about 1 month. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily.

In certain instances disclosed herein, a subject with a high level of serum creatinine is a subject with serum creatinine level selected from greater than about 40 µmol/L, greater than about 45 µmol/L, greater than about 50 µmol/L, greater than about 55 µmol/L, greater than about 60 µmol/L, greater than about 65 µmol/L, greater than about 70 µmol/L, greater than about 72 µmol/L of serum creatinine. In certain instances disclosed herein, a subject with a high level of serum creatinine is a subject with serum creatinine level selected from about 40 µmol/L, about 45 µmol/L, about 50 µmol/L, about 55 µmol/L, about 60 µmol/L, about 65 µmol/L, about 70 µmol/L, about 72 µmol/L of serum creatinine. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

In certain embodiments, the subject with concomitant riluzole administration is a subject on a stable dosing regimen of riluzole. In certain embodiments, the subject with concomitant riluzole administration is a subject receiving about 50 milligrams of riluzole twice daily. In certain embodiments, the subject with concomitant riluzole administration is a subject who has been receiving riluzole for more than about thirty days. In certain embodiments, the subject with concomitant riluzole administration is a subject who has been receiving riluzole for about sixty days or more. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. In some embodiments, the pharmaceutical composition further comprises a therapeutically effective amount of creatine or a pharmaceutically acceptable salt thereof. In some embodiments, the pharmaceutical composition further comprises a therapeutically effective amount of riluzole or a pharmaceutically acceptable salt thereof.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

In certain embodiments, treating amyotrophic lateral sclerosis in said subject is selected from improved ALSFRS-R score, improved CAFS rank, decreased mortality, increased life expectancy, and combinations thereof.

Disclosures herein are directed to methods for treating amyotrophic lateral sclerosis in a subject diagnosed with definite amyotrophic lateral sclerosis comprising administering to the subject a therapeutically effective amount of (6R)-2-amino-4,5,6,7-tetrahydro-6-(propylamino)benzothiazole or a pharmaceutically acceptable salt thereof, wherein the amyotrophic lateral sclerosis is treated. In certain embodiments, the definite amyotrophic lateral sclerosis is definite ALS as defined by the El Escorial diagnosis criteria. In certain embodiments, the subject has upper motor neuron degeneration and lower motor neuron degeneration in the bulbar region and two other spinal regions. In certain embodiments, the subject has upper motor neuron degeneration and lower motor neuron degeneration in three spinal regions. The subject may be selected from a subject with symptom onset duration of less than about 18 months, a subject with a high level of serum creatinine, a subject with low levels of serum sodium bicarbonate, a subject with concomitant riluzole administration and combinations thereof. A subject with a high level of serum creatinine may be a subject with greater than about 72 µmol/L serum creatinine. In certain embodiments, the therapeutically effective amount of (6R)-2-amino-4,5,6,7-tetrahydro-6-(propylamino)benzothiazole or a pharmaceutically acceptable salt
thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily. In some embodiments, treating amyotrophic lateral sclerosis in a subject in need thereof further comprises administering a therapeutically effective amount of creatine or a pharmaceutically acceptable salt thereof. In some embodiments, treating amyotrophic lateral sclerosis in a subject in need thereof further comprises administering a therapeutically effective amount of riluzole or a pharmaceutically acceptable salt thereof.

Disclosures herein are directed to methods for treating amyotrophic lateral sclerosis in a subject diagnosed with definite amyotrophic lateral sclerosis comprising administering to the subject a therapeutically effective amount of (6R)-2-amino-4,5,6,7-tetrahydro-6-(propylamino)benzothiazole or a pharmaceutically acceptable salt thereof, wherein the amyotrophic lateral sclerosis is treated. Definite amyotrophic lateral sclerosis may be the presence of by the El Escorial diagnosis criteria, amyotrophic lateral sclerosis symptom onset duration of less than about 18 months, limb-onset amyotrophic lateral sclerosis, concomitant riluzole administration, an ALSFRS-R score of greater than 36.0, a re-study progression rate greater than or equal to 0.8 points per month, a percentage predicted relaxed (slow) vital capacity (SVC) of less than or equal to 102.0, an ALSFRS-R fine motor domain score of greater than 10.0 points, ALSFRS-R bulbar domain score or greater than 9.0 points, an ALSFRS-R gross motor domain score of greater than 8.0 points, an abnormal neurological exam of the tongue, an abnormal neurological exam of the
pharynx, larynx and swallowing, an abnormal neurological exam of the lower extremities, an abnormal neurological exam of the upper extremities, an abnormal neurological exam of the triceps, an abnormal neurological exam of the muscle mass and bulk, an abnormal neurological exam of the bicep, an abnormal neurological exam, a pulse rate of greater than 81.0 beats per minute, a diastolic blood pressure of greater than 82.0 mmHg, a systolic blood pressure of less than or equal to 117.0 mmHg, a creatinine value of greater than 72.0 µmol/L, a phosphorous value of less than or equal to 1.090 µmol/L, a platelet count of less than or equal to 248.0 x10⁹ cells/L, a cholesterol value of less than or equal to 5.3 mmol/L, a lactate dehydrogenase value of less than or equal to 161.0 U/L, a creatine phosphokinase value of less than or equal to 184.0 U/L, a bicarbonate value of less than or equal to 21.6 mmol/L, a triglyceride level of less than or equal to 1.4mmol/L, a uric acid level of greater than 320.0 µmol/L, a gamma-glutamyltransferase (GGT) level of greater than 37.0 U/L, a total bilirubin level of less than or equal to 6.0 µmol/L, a urine pH of less than or equal to 5.5, or any combination thereof. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily. The method may further comprise monitoring said subject for any clinical features associated with amyotrophic lateral sclerosis. Therapy with a
therapeutically effective amount of (6R)-2-amino-4,5,6,7-tetrahydro-6-(propylamino)benzothiazole or a pharmaceutically acceptable salt thereof may be initiated upon diagnosis of amyotrophic lateral sclerosis. The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis. In some embodiments, treating amyotrophic lateral sclerosis in a subject in need thereof further comprises administering a therapeutically effective amount of creatine or a pharmaceutically acceptable salt thereof. In some embodiments, treating amyotrophic lateral sclerosis in a subject in need thereof further comprises administering a therapeutically effective amount of riluzole or a pharmaceutically acceptable salt thereof.

Disclosures herein are directed to methods for treating amyotrophic lateral sclerosis in a subject exhibiting symptoms of amyotrophic lateral sclerosis comprising administering to the subject a therapeutically effective amount of (6R)-2-amino-4,5,6,7-tetrahydro-6-(propylamino)benzothiazole or a pharmaceutically acceptable salt thereof, wherein the symptoms of amyotrophic lateral sclerosis are treated. The subject may exhibit clinical characteristics selected from definite amyotrophic lateral sclerosis, amyotrophic lateral sclerosis symptom onset duration of less than about 18 months, limb-onset amyotrophic lateral sclerosis, concomitant riluzole administration, an ALSFRS-R score of greater than 36.0, a re-study progression rate greater than or equal to 0.8 points per month, a percentage predicted relaxed (slow) vital capacity (SVC) of less than or equal to 102.0, an ALSFRS-R fine motor domain score of greater than 10.0 points, ALSFRS-R bulbar domain score or greater than 9.0 points, an ALSFRS-R gross motor domain score of greater than 8.0 points, an abnormal neurological exam of the tongue, an abnormal neurological exam of the pharynx, larynx and swallowing, an abnormal neurological exam of the lower extremities, an abnormal neurological exam of the upper extremities, an abnormal neurological exam of the triceps, an abnormal neurological exam of the muscle mass and bulk, an abnormal neurological exam of the bicep, an abnormal neurological exam, a pulse rate of greater than 81.0 beats per minute, a diastolic blood pressure of greater than 82.0 mmHg, a systolic blood pressure of less than or equal to 117.0 mmHg, a creatinine value of greater than 72.0 µmol/L, a phosphorous value of less than or equal to 1.090 µmol/L, a platelet count of less than or equal to 248.0 x10⁹ cells/L, a cholesterol value of less than or equal to 5.3 mmol/L, a lactate dehydrogenase value of less than or equal to 161.0 U/L, a creatine phosphokinase value of less than or equal to 184.0 U/L, a bicarbonate value of less than or equal to 21.6 mmol/L, a triglyceride level of less than or equal to 1.4mmol/L, a uric
acid level of greater than 320.0 µmol/L, a gamma-glutamyltransferase (GGT) level of greater than 37.0 U/L, a total bilirubin level of less than or equal to 6.0 µmol/L, a urine pH of less than or equal to 5.5, or any combination thereof. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily. In some embodiments, treating amyotrophic lateral sclerosis in a subject in need thereof further comprises administering a therapeutically effective amount of creatine or a pharmaceutically acceptable salt thereof. In some embodiments, treating amyotrophic lateral sclerosis in a subject in need thereof further comprises administering a therapeutically effective amount of riluzole or a pharmaceutically acceptable salt thereof.

The methods may further comprise monitoring said subject for any clinical features associated with amyotrophic lateral sclerosis. The methods may further comprise initiating therapy with a therapeutically effective amount of (6R)-2-amino-4,5,6,7-tetrahydro-6-(propylamino)benzothiazole or a pharmaceutically acceptable salt thereof upon diagnosis of amyotrophic lateral sclerosis. The subject may exhibit symptoms of amyotrophic lateral sclerosis.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. In some embodiments, the pharmaceutical composition further comprises a therapeutically effective amount of creatine or a pharmaceutically acceptable salt thereof. In some embodiments, the pharmaceutical composition further comprises a therapeutically effective amount of riluzole or a pharmaceutically acceptable salt thereof.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

Disclosures herein are directed to methods for treating amyotrophic lateral sclerosis in a subject in need thereof comprising administering to the subject a therapeutically effective amount of (6R)-2-amino-4,5,6,7-tetrahydro-6-(propylamino)benzothiazole or a pharmaceutically acceptable salt thereof and administering to the subject a therapeutically effective amount of riluzole, wherein the amyotrophic lateral sclerosis is treated. The therapeutically effective amount of riluzole may be about 50 milligrams twice daily. The (6R)-2-amino-4,5,6,7-tetrahydro-6-(propylamino)benzothiazole or a pharmaceutically acceptable salt thereof and said riluzole may be administered sequentially or simultaneously. The subject may be selected from a subject with definite amyotrophic lateral sclerosis, subject with limb-onset amyotrophic lateral sclerosis, a subject with amyotrophic lateral sclerosis symptom onset duration of less than about 18 months, a subject with low bicarbonate levels, a subject with a high level of serum creatinine, and a combination thereof. A subject with a high level of serum creatinine may be a subject with greater than about 72 µmol/L serum
creatinine. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily. In certain embodiments, the disclosure further comprises monitoring said subject for any clinical features associated with amyotrophic lateral sclerosis. The method may further comprise initiating therapy with a therapeutically effective amount of (6R)-2-amino-4,5,6,7-tetrahydro-6-(propylamino)benzothiazole or a pharmaceutically acceptable salt thereof upon diagnosis of amyotrophic lateral sclerosis. The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis. Treating amyotrophic lateral sclerosis in a subject in need thereof may further comprise administering a therapeutically effective amount of creatine or a pharmaceutically acceptable salt thereof. Treating amyotrophic lateral sclerosis in a subject in need thereof may further comprise administering a therapeutically effective amount of riluzole or a pharmaceutically acceptable salt thereof.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical
composition further comprises a pharmaceutically acceptable excipient. In some embodiments, the pharmaceutical composition further comprises a therapeutically effective amount of creatine or a pharmaceutically acceptable salt thereof. In some embodiments, the pharmaceutical composition further comprises a therapeutically effective amount of riluzole or a pharmaceutically acceptable salt thereof.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

Further disclosures are directed to methods for treating amyotrophic lateral sclerosis in a subject in need thereof comprising administering to the subject with amyotrophic lateral sclerosis symptom duration of less than about 18 months a therapeutically effective amount of (6R)-2-amino-4,5,6,7-tetrahydro-6-(propylamino)benzothiazole or a pharmaceutically acceptable salt thereof, wherein the amyotrophic lateral sclerosis is treated. The subject may be selected from a subject with definite amyotrophic lateral sclerosis, subject with limb-onset amyotrophic lateral sclerosis, a subject with a high level of serum creatinine, a subject with low bicarbonate levels, a subject with concomitant riluzole administration and a combination thereof. A subject with a high level of serum creatinine may be a subject with greater than about 72 µmol/L serum creatinine. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective
amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily. The method may further comprise monitoring said subject for any clinical features associated with amyotrophic lateral sclerosis. Therapy with a therapeutically effective amount of (6R)-2-amino-4,5,6,7-tetrahydro-6-(propylamino)benzothiazole or a pharmaceutically acceptable salt thereof may be initiated upon diagnosis of amyotrophic lateral sclerosis. The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis. Treating amyotrophic lateral sclerosis in a subject in need thereof may further comprise administering a therapeutically effective amount of creatine or a pharmaceutically acceptable salt thereof. Treating amyotrophic lateral sclerosis in a subject in need thereof may further comprise administering a therapeutically effective amount of riluzole or a pharmaceutically acceptable salt thereof.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. In some embodiments, the pharmaceutical composition further comprises a therapeutically effective amount of creatine or a pharmaceutically acceptable salt thereof. In some embodiments, the
pharmaceutical composition further comprises a therapeutically effective amount of riluzole or a pharmaceutically acceptable salt thereof.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

Further disclosures herein are directed to methods for treating amyotrophic lateral sclerosis in a subject in need thereof comprising administering to the subject with a high level of serum creatinine a therapeutically effective amount of (6R)-2-amino-4,5,6,7-tetrahydro-6-(propylamino)benzothiazole or a pharmaceutically acceptable salt thereof, wherein the amyotrophic lateral sclerosis is treated. A subject with a high level of serum creatinine may be a male subject with serum creatinine level selected from greater than about 40 µmol/L, greater than about 45 µmol/L, greater than about 50 µmol/L, greater than about 55 µmol/L, greater than about 60 µmol/L, greater than about 65 µmol/L, greater than about 70 µmol/L, greater than about 72 µmol/L of serum creatinine. The subject may be a male with a high level of serum reatinine is a subject with serum creatinine level selected from about 40 µmol/L, about 45 µmol/L, about 50 µmol/L, about 55 µmol/L, about 60 µmol/L, about 65 µmol/L, about 70 µmol/L, about 72 µmol/L of serum creatinine. A subject with a high level of serum creatinine may be a female subject with serum creatinine level selected from greater than about 40 µmol/L, greater than about 45 µmol/L, greater than about 50 µmol/L, greater than about 55 µmol/L, greater than about 60 µmol/L, greater than about 65 µmol/L, greater than about 70 µmol/L, greater than about 72 µmol/L of serum creatinine. The subject with a high level of serum
creatinine is a female subject with serum creatinine level selected from about 40 µmol/L, about 45 µmol/L, about 50 µmol/L, about 55 µmol/L, about 60 µmol/L, about 65 µmol/L, about 70 µmol/L, about 72 µmol/L of serum creatinine. The subject may be selected from a subject with definite amyotrophic lateral sclerosis, a subject with amyotrophic lateral sclerosis symptom duration of less than about 18 months, a subject with concomitant riluzole administration and a combination thereof.

In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily. Treating amyotrophic lateral sclerosis in a subject in need thereof may further comprise administering a therapeutically effective amount of creatine or a pharmaceutically acceptable salt thereof. Treating amyotrophic lateral sclerosis in a subject in need thereof may further comprise administering a therapeutically effective amount of riluzole or a pharmaceutically acceptable salt thereof.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. In some embodiments, the pharmaceutical composition further comprises a therapeutically effective amount of creatine or a pharmaceutically acceptable salt thereof. In some embodiments, the
pharmaceutical composition further comprises a therapeutically effective amount of riluzole or a pharmaceutically acceptable salt thereof.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

Disclosures herein are directed to methods of treating amyotrophic lateral sclerosis in a subject in need thereof comprising monitoring said subject for any clinical features associated with amyotrophic lateral sclerosis; and initiating therapy with (6R)-2-amino-4,5,6,7-tetrahydro-6-(propylamino)benzothiazole or a pharmaceutically acceptable salt thereof upon diagnosis of definite amyotrophic lateral sclerosis in accordance with the El Escorial diagnostic criteria for amyotrophic lateral sclerosis. The therapy may be administering an effective amount of (6R)-2-amino-4,5,6,7-tetrahydro-6-(propylamino)benzothiazole or a pharmaceutically acceptable salt thereof daily to said subject. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily.

Disclosures herein are directed to methods of treating amyotrophic lateral sclerosis in a subject in need thereof comprising monitoring said subject for any clinical features associated with amyotrophic lateral sclerosis; and initiating therapy with (6R)-2-amino-4,5,6,7-tetrahydro-6-(propylamino)benzothiazole or a pharmaceutically acceptable salt thereof upon diagnosis of definite amyotrophic lateral sclerosis. A diagnosis of definite amyotrophic may be characterized by definite ALS as defined by the El Escorial diagnosis criteria, amyotrophic lateral sclerosis symptom onset duration of less than about 18 months, limb-onset amyotrophic lateral sclerosis, concomitant riluzole administration, an ALSFRS-R score of greater than 36.0, a re-study progression rate greater than or equal to 0.8 points per month, a percentage predicted relaxed (slow) vital capacity (SVC) of less than or equal to 102.0, an ALSFRS-R fine motor domain score of greater than 10.0 points, ALSFRS-R bulbar domain score or greater than 9.0 points, an ALSFRS-R gross motor domain score of greater than 8.0 points, an abnormal neurological exam of the tongue, an abnormal neurological exam of the prharynx, larynx and swallowing, an abnormal neurological exam of the lower extremities, an abnormal neurological exam of the upper extremities, an abnormal neurological exam of the triceps, an abnormal neurological exam of the muscle mass and bulk, an abnormal neurological exam of the bicep, an abnormal neurological exam, a pulse rate of greater than 81.0 beats per minute, a diastolic blood pressure of greater than 82.0 mmHg, a systolic blood pressure of less than or equal to 117.0 mmHg, a creatinine value of greater than 72.0 µmol/L, a phosphorous value of less than or equal to 1.090 µmol/L, a platelet count of less than or equal to 248.0 x10⁹ cells/L, a cholesterol value of less than or equal to 5.3 mmol/L, a lactate dehydrogenase value of less than or equal to 161.0 U/L, a creatine phosphokinase value of less than or equal to 184.0 U/L, a bicarbonate value of less than or equal to 21.6 mmol/L, a triglyceride level of less than or
equal to 1.4mmol/L, a uric acid level of greater than 320.0 µmol/L, a gamma-glutamyltransferase (GGT)level of greater than 37.0 U/L, a total bilirubin level of less than or equal to 6.0 µmol/L, a urine pH of less than or equal to 5.5, or any combination thereof. The therapy may be administering an effective amount of (6R)-2-amino-4,5,6,7-tetrahydro-6-(propylamino)benzothiazole or a pharmaceutically acceptable salt thereof daily to said subject. In certain embodiments, the therapeutically effective amount of (6R)-2-amino-4,5,6,7-tetrahydro-6-(propylamino)benzothiazole or a pharmaceutically acceptable salt thereof is from 50 milligrams to 3,000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 3000 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 900 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 600 milligrams per day. In certain embodiments, the therapeutically effective amount of (6R)-2-amino-4,5,6,7-tetrahydro-6-(propylamino)benzothiazole or a pharmaceutically acceptable salt thereof is from 150 milligrams to 300 milligrams per day. In certain embodiments, the therapeutically effective amount of (6R)-2-amino-4,5,6,7-tetrahydro-6-(propylamino)benzothiazole or a pharmaceutically acceptable salt thereof is 150 milligrams twice daily. In certain embodiments, the therapeutically effective amount of (6R)-2-amino-4,5,6,7-tetrahydro-6-(propylamino)benzothiazole or a pharmaceutically acceptable salt thereof is from 300 milligrams twice daily. In some embodiments, treating amyotrophic lateral sclerosis in a subject in need thereof further comprises administering a therapeutically effective amount of creatine or a pharmaceutically acceptable salt thereof. In some embodiments, treating amyotrophic lateral sclerosis in a subject in need thereof further comprises administering a therapeutically effective amount of riluzole or a pharmaceutically acceptable salt thereof.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. In some embodiments, the pharmaceutical composition further comprises a therapeutically effective amount of creatine or a pharmaceutically acceptable salt thereof. In some embodiments, the
pharmaceutical composition further comprises a therapeutically effective amount of riluzole or a pharmaceutically acceptable salt thereof.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

Disclosures herein are directed to methods of reducing the decline of serum creatinine in a subject having definite amyotrophic lateral sclerosis comprising administering to the subject a therapeutically effective amount of (6R)-2-amino-4,5,6,7-tetrahydro-6-(propylamino)benzothiazole or a pharmaceutically acceptable salt thereof. The subject further may have at least one of the following characteristics: limb-onset amyotrophic lateral sclerosis, low bicarbonate levels, symptom onset duration of less than about 18 months, greater than about 72 µmol/L serum creatinine, and concomitant riluzole administration. The disclosure may further comprise monitoring said subject for any clinical features associated with amyotrophic lateral sclerosis. Therapy with a therapeutically effective amount of (6R)-2-amino-4,5,6,7-tetrahydro-6-(propylamino)benzothiazole or a pharmaceutically acceptable salt thereof may be initiated upon diagnosis of amyotrophic lateral sclerosis. The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis.

There is also disclosed a method of reducing the decline of serum creatinine in a subject in need thereof, comprising administering to the subject a therapeutically effective amount of (6R)-2-amino-4,5,6,7-tetrahydro-6-(propylamino)benzothiazole or a pharmaceutically acceptable salt thereof.

Disclosures herein are directed to methods of reducing the decline of serum creatinine in a subject in need thereof, comprising administering to the subject a therapeutically effective amount of (6R)-2-amino-4,5,6,7-tetrahydro-6-(propylamino)benzothiazole or a pharmaceutically acceptable salt thereof. It is also disclosed that the method further comprises initiating therapy with a therapeutically effective amount of (6R)-2-amino-4,5,6,7-tetrahydro-6-(propylamino)benzothiazole or a pharmaceutically acceptable salt thereof upon diagnosis of a decline of serum creatinine. The subject may exhibit symptoms of a decline of serum creatinine. The subject may have a definite decline of serum creatinine, a probable decline of serum creatinine, a possible a decline of serum creatinine or a suspected a decline of serum creatinine.

In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

In certain embodiments, the pharmaceutical composition is selected from a tablet, a capsule and a liquid.

In certain embodiments, the (6R)-2-amino-4,5,6,7-tetrahydro-6-(propylamino)benzothiazole or a pharmaceutically acceptable salt thereof is administered orally.

Other disclosures herein are directed to methods of reducing the decline of muscle loss in a subject having definite amyotrophic lateral sclerosis comprising administering to the subject a therapeutically effective amount of (6R)-2-amino-4,5,6,7-tetrahydro-6-(propylamino)benzothiazole or a pharmaceutically acceptable salt thereof. The subject may further have at least one of the following characteristics: limb-onset amyotrophic lateral sclerosis, low bicarbonate levels, symptom onset duration of less than about 18 months, greater than about 72 µmol/L serum creatinine, and concomitant riluzole administration. The method may further comprise monitoring said subject for any clinical features associated with amyotrophic lateral sclerosis. Therapy with a therapeutically effective amount of (6R)-2-amino-4,5,6,7-tetrahydro-6-(propylamino)benzothiazole or a pharmaceutically acceptable salt thereof may be initiated upon diagnosis of amyotrophic lateral sclerosis.

The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis.

Other disclosures herein are directed to methods of reducing the decline of muscle loss in a subject in need thereof, comprising administering to the subject a therapeutically effective amount of (6R)-2-amino-4,5,6,7-tetrahydro-6-(propylamino)benzothiazole or a pharmaceutically acceptable salt thereof. The method may further comprise initiating therapy with a therapeutically effective amount of (6R)-2-amino-4,5,6,7-tetrahydro-6-(propylamino)benzothiazole or a pharmaceutically acceptable salt thereof upon diagnosis of muscle loss. The subject may exhibit symptoms of muscle loss. The subject may have definite muscle loss, probable muscle loss, possible muscle loss or suspected muscle loss.

The therapeutically effective amount of (6R)-2-amino-4,5,6,7-tetrahydro-6-(propylamino)benzothiazole or a pharmaceutically acceptable salt thereof may be administered in a pharmaceutical composition, wherein said pharmaceutical composition further comprises a pharmaceutically acceptable excipient.

The pharmaceutical composition may be chirally pure for (6R)-2-amino-4,5,6,7-tetrahydro-6-(propylamino)benzothiazole or a pharmaceutically acceptable salt thereof. The chiral purity for (6R)-2-amino-4,5,6,7-tetrahydro-6-(propylamino)benzothiazole or a pharmaceutically acceptable salt thereof may be about 99% or more, including about 99.95% or more. The chemical purity for (6R)-2-amino-4,5,6,7-tetrahydro-6-(propylamino)benzothiazole or a pharmaceutically acceptable salt thereof may be about 99% or more, including about 99.97% or more.

The pharmaceutical composition may be selected from a tablet, a capsule and a liquid.

The (6R)-2-amino-4,5,6,7-tetrahydro-6-(propylamino)benzothiazole or a pharmaceutically acceptable salt thereof may be administered orally.

Disclosures herein are also directed to methods of treating amyotrophic lateral sclerosis in a subject in need thereof comprising orally administering to said subject 150 milligrams of (6R)-2-amino-4,5,6,7-tetrahydro-6-(propylamino)benzothiazole dihydrochloride monohydrate in a tablet pharmaceutical composition twice daily to said subject, wherein the subject is selected from a subject with definite amyotrophic lateral sclerosis, limb-onset amyotrophic lateral sclerosis a subject with amyotrophic lateral sclerosis symptom onset duration of less than about 18 months, a subject with a high level of serum creatinine, a subject with low bicarbonate levels, a subject with concomitant riluzole administration and combinations thereof, and wherein the amyotrophic lateral sclerosis is treated. The therapeutically effective amount of (6R)-2-amino-4,5,6,7-tetrahydro-6-(propylamino)benzothiazole or a pharmaceutically acceptable salt thereof may be administered in a pharmaceutical composition, wherein said pharmaceutical composition further comprises a pharmaceutically acceptable excipient. A subject with a high level of serum creatinine may be a subject with greater than about 72 µmol/L serum creatinine. The method may further comprise monitoring said subject for any clinical features associated with amyotrophic lateral sclerosis. The method may further comprise initiating therapy with a therapeutically effective amount of (6R)-2-amino-4,5,6,7-tetrahydro-6-(propylamino)benzothiazole or a pharmaceutically acceptable salt thereof upon diagnosis of amyotrophic lateral sclerosis. The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis.

The pharmaceutical composition may be chirally pure for (6R)-2-amino-4,5,6,7-tetrahydro-6-(propylamino)benzothiazole or a pharmaceutically acceptable salt thereof. The chiral purity for (6R)-2-amino-4,5,6,7-tetrahydro-6-(propylamino)benzothiazole or a pharmaceutically acceptable salt thereof may be about 99% or more, including about 99.95% or more. The chemical purity for (6R)-2-amino-4,5,6,7-tetrahydro-6-(propylamino)benzothiazole or a pharmaceutically acceptable salt thereof may be about 99% or more, including about 99.97% or more.

Disclosures herein are also directed to methods of treating amyotrophic lateral sclerosis in a subject in need thereof comprising orally administering to said subject 150 milligrams of (6R)-2-amino-4,5,6,7-tetrahydro-6-(propylamino)benzothiazole dihydrochloride monohydrate in a tablet pharmaceutical composition twice daily to said subject, wherein the subject suffers from definite amyotrophic lateral sclerosis, with a symptom onset duration of less than about 18 months, a subject with a high level of serum creatinine, limb-onset amyotrophic lateral sclerosis, low bicarbonate levels, and concomitant riluzole administration, and wherein the amyotrophic lateral sclerosis is treated. In certain embodiments, the therapeutically effective amount of (6R)-2-amino-4,5,6,7-tetrahydro-6-(propylamino)benzothiazole or a pharmaceutically acceptable salt thereof is administered in a pharmaceutical composition, wherein said pharmaceutical composition further comprises a pharmaceutically acceptable excipient. A subject with a high level of serum creatinine may be a subject with greater than about 72 µmol/L serum creatinine. The method may further comprise monitoring said subject for any clinical features associated with amyotrophic lateral sclerosis. Therapy with a therapeutically effective amount of (6R)-2-amino-4,5,6,7-tetrahydro-6-(propylamino)benzothiazole or a pharmaceutically acceptable salt thereof may be initiated upon diagnosis of amyotrophic lateral sclerosis. The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis.

In certain embodiments, the pharmaceutical composition is chirally pure for (6R)-2-amino-4,5,6,7-tetrahydro-6-(propylamino)benzothiazole or a pharmaceutically acceptable salt thereof. In certain embodiments, the chiral purity for (6R)-2-amino-4,5,6,7-tetrahydro-6-(propylamino)benzothiazole or a pharmaceutically acceptable salt thereof is
about 99% or more, including about 99.95% or more. In certain embodiments, the chemical purity for (6R)-2-amino-4,5,6,7-tetrahydro-6-(propylamino)benzothiazole or a pharmaceutically acceptable salt thereof is about 99% or more, including about 99.97% or more.

Another method for treating amyotrophic lateral sclerosis (ALS) in a subject comprises determining a baseline ALS Functional Rating Scale, Revised (ALSFRS-R) scores for the subject; administering to the subject an effective amount of about chirally pure (6R)-2-amino-4,5,6,7-tetrahydro-6-(propylamino)benzothiazole or pharmaceutically acceptable salt thereof; collecting outcome data from the subject; analyzing the outcome data as a primary endpoint in a joint-rank statistical test adjusted for mortality and adjusted for a change from baseline using ALS Functional Rating Scale, Revised (ALSFRS-R) scores from a plurality of persons for indications of efficacy or non-efficacy; and continuing to administer an effective amount to the subject if the joint-rank statistical test indicates the effective amount is efficacious, but discontinuing to administer an effective amount to the subject if the joint-rank statistical test indicates the effective amount is non-efficacious.

The ALSFRS-R measures 4 domains: pulmonary function, bulbar function, and gross and fine motor skills. There are a total of 12 questions, each scored from 0 to 4 for a total possible score of 48. The twelve questions and rating scale are provided in Cederbaum, et al., 169 J. NEUROL. SCI., 13-21 (1999).

The analysis may utilize a greater negative adjustment for a death of one of the plurality of persons than for a survival with functional decline, if a higher rank is a better global clinical outcome. The analysis may provide a greater negative adjustment for an earlier death of one of the plurality of persons than for a later death of a person, if a higher rank is a better global clinical outcome. The outcome data may be collected by phone interview, home visit, or combinations thereof. The outcome data may be collected in part by phone interview or home visit or combinations thereof. The outcome data may be selected from laboratory tests, ALSFRS-R score, adverse event reporting, or a combination thereof. The method may have the change from baseline determined through twelve months follow-up. The method may further comprise determining a secondary endpoint data of evaluation of time to death or respiratory insufficiency, respiratory decline, muscle strength, quality of life, population PK, and safety. The quality of life may be assessed using an amyotrophic lateral sclerosis (ALS) assessment questionnaire and a change-in-health survey.

In some embodiments, treating ALS can include slowing progression of ALS, reducing intensity of symptoms associated with ALS, reducing onset of symptoms associated with ALS, reducing weight loss associated with ALS, reversing weight loss associated with ALS, delaying mortality, and combinations thereof. In particular embodiments, the symptoms associated with ALS may be, for example, decreases in fine motor function, decreases in gross motor function, decreases in bulbar function, decreases in respiratory function, and combinations thereof. Further, in other embodiments, the symptoms associated with ALS can include difficulty with daily activities, such as, for example, difficulty with walking, speech, eating, swallowing, writing, climbing stairs, cutting food, turning in bed, dressing, maintaining hygiene, and combinations thereof, and may experience other symptoms, such as, for example, difficulty breathing, dyspnea, orthopnea, respiratory insufficiency, increased salivation and combinations thereof.

In some embodiments, the effective amount may be from about 50 milligrams to about 300 milligrams per day, and in other embodiments, the effective amount may be from about 150 milligrams to about 300 milligrams per day. In still other embodiments, the effective amount may be about 300 milligrams or more per day. In other embodiments, the stable daily dose may be one to five unit doses per day, and in particular embodiments, each unit dose may be a solid unit dose. In some embodiments, administering may include administering one unit dose two times per day wherein each unit dose is equal to about one-half of the stable daily dose, and in other embodiments, administering may include administering one unit dose once every twelve hours wherein each unit dose is equal to about one-half of the stable daily dose. In still other embodiments, administering may include administering one unit dose four times per day wherein each unit dose is equal to about one-quarter of the stable daily dose. In yet other embodiments, administering can include administering two unit doses wherein each unit dose is about 150 milligrams two times per day, and in further embodiments, administering may include administering four unit doses wherein each unit dose is about 75 milligrams four times per day.

The method may be carried out for a time period selected from at least about twelve months, at least about eighteen months, at least about two years, at least about four years, at least about six years, at least about eight years, at least about ten
years, at least about twenty years, and until the subject dies. The method may be carried out at least daily for an indefinite amount of time.

The method may further comprise administering one or more other amyotrophic lateral sclerosis (ALS) treatments simultaneously or concurrently with administering about chirally pure (6R)-2-amino-4,5,6,7-tetrahydro-6-(propylamino)benzothiazole or a pharmaceutically acceptable salt thereof. The one or more other amyotrophic lateral sclerosis (ALS) treatments may include riluzole.

The subject may have begun exhibiting symptoms of amyotrophic lateral sclerosis (ALS) less than about two years before beginning administering of (6R)-2-amino-4,5,6,7-tetrahydro-6-(propylamino)benzothiazole or pharmaceutically acceptable salt thereof. The subject may have begun exhibiting symptoms of amyotrophic lateral sclerosis (ALS) at least greater than about two years before beginning administering of (6R)-2-amino-4,5,6,7-tetrahydro-6-(propylamino)benzothiazole or pharmaceutically acceptable salt thereof. In some embodiments, the subject exhibits a greater than 20 % improvement in ALS Functional Rating Scale, Revised (ALSFRS-R) score when compared to baseline. In particular embodiments, the subject exhibits a greater than 30 % improvement in ALS Functional Rating Scale, Revised (ALSFRS-R) score when compared to baseline. In still other embodiments, the improvement is apparent in a time period selected from the group consisting of less than thirty-six months, less than eighteen months, less than twelve months, less than about nine months, less than about six months, less than about three months, and less than about one month. Further, in some embodiments, administering about chirally pure (6R)-2-amino-4,5,6,7-tetrahydro-6-(propylamino)benzothiazole or pharmaceutically acceptable salt thereof results in slowing of a rate of fine motor function loss in the subject.

The method may further comprise administering a daily dose of greater than an effective amount for a period of time before administering an effective amount of about chirally pure (6R)-2-amino-4,5,6,7-tetrahydro-6-(propylamino)benzothiazole or pharmaceutically acceptable salt thereof. In particular embodiments, the greater than an effective amount is greater than 150 milligrams. In still other embodiments, the greater than an effective amount is greater than 300 milligrams. The period of time before administering an effective amount may be from about one week to about eighteen months. The period of time before administering an effective amount may be from about two weeks to about twelve months.
Administering an effective amount of about chirally pure (6R)-2-amino-4,5,6,7-tetrahydro-6-(propylamino)benzothiazole or pharmaceutically acceptable salt thereof may be carried out indefinitely.

In some embodiments, an effective amount of about chirally pure (6R)-2-amino-4,5,6,7-tetrahydro-6-(propylamino)benzothiazole or pharmaceutically acceptable salt thereof is administered in an initial dose and every administration thereafter. In other embodiments, dosing achieves a dose dependent, steady state AUC₀₋₁₂ (hours x ng/mL) selected from the group consisting of 836 ±234 for an effective amount of 50 milligrams, 2803 ±1635 for an effective amount of 150 milligrams, and 6004 ± 2700 for an effective amount of 300 milligrams.

In some embodiments, the effective amount comprises a stable daily dose. In particular embodiments, the stable daily dose comprises from about 50 mg to about 300 mg of about chirally pure (6R)-2-amino-4,5,6,7-tetrahydro-6-(propylamino)benzothiazole or pharmaceutically acceptable salt thereof. In other embodiments, the stable daily dose comprises one to five unit doses per day. In still other embodiments, each unit dose is a solid unit dose. In some embodiments, administering comprises administering one unit dose two times per day wherein each unit dose is equal to about half of the stable daily dose. In other embodiments, administering comprises administering one unit dose once every twelve hours wherein each unit dose is equal to about half of the stable daily dose. In still other embodiments, administering comprises administering one unit dose four times per day wherein each unit dose is equal to about one quarter of the stable daily dose. In yet other embodiments, administering comprises administering two unit doses wherein each unit dose is about 150 milligrams two times per day. Further, some embodiments, administering comprises administering four unit doses wherein each unit dose is about 75 milligrams four times per day. In some embodiments, administering a stable daily dose of about chirally pure (6R)-2-amino-4,5,6,7-tetrahydro-6-(propylamino)benzothiazole or pharmaceutically acceptable salt thereof is carried out for at least about eighteen months. Further, in some embodiments, administering a stable daily dose of about chirally pure (6R)-2-amino-4,5,6,7-tetrahydro-6-(propylamino)benzothiazole or pharmaceutically acceptable salt thereof is carried out for an indefinite amount of time. In some embodiments, the stable daily dose is consistent throughout a treatment regimen. In particular embodiments, an initial daily dose is equal to each daily dose thereafter. In other embodiments, there is not titration before administering the stable daily dose. In yet other embodiments, administering achieves a dose
dependent, steady state AUC₀₋₁₂ (h x ng/mL) selected from 836 ±234 for stable daily dose of 50 milligrams, 2803 ±1635 for stable daily dose of 150 milligrams, or 6004 ± 2700 for stable daily dose of 300 milligrams.

The method may further comprise monitoring the subject. The method may further comprise monitoring the subject for neutropenia. The method may further comprise monitoring the ALS Functional Rating Scale, Revised (ALSFRS-R) score for the subject. The method may further comprise monitoring the subject's fine motor function, gross motor function, bulbar function, respiratory function, and combinations thereof. The method may further comprise monitoring behaviors selected from the group consisting of swallowing, handwriting, speech, ability to walk, ability to climb stairs, ability to dress, ability to maintain hygiene, and combinations thereof. The method may further comprise scheduling a doctor visit every six months for at least twelve months. The subject may be predisposed to amyotrophic lateral sclerosis (ALS) and not exhibiting symptoms of amyotrophic lateral sclerosis (ALS). The method may further comprise administering about chirally pure (6R)-2-amino-4,5,6,7-tetrahydro-6-(propylamino)benzothiazole or pharmaceutically acceptable salt thereof to family members of the subject. Treating may comprise administering the about chirally pure (6R)-2-amino-4,5,6,7-tetrahydro-6-(propylamino)benzothiazole or pharmaceutically acceptable salt thereof to a subject not exhibiting symptoms of amyotrophic lateral sclerosis (ALS). Further, in some instances disclosed herein, treating comprises administering the about chirally pure (6R)-2-amino-4,5,6,7-tetrahydro-6-(propylamino)benzothiazole or pharmaceutically acceptable salt thereof to a subject that is predisposed to amyotrophic lateral sclerosis (ALS).

A method of the instant disclosure is for conducting a clinical trial for treating amyotrophic lateral sclerosis (ALS). The method comprises a clinical trial to evaluate the efficacy of a pharmaceutical agent. The method comprises steps to evaluate the efficacy of dexpramipexole for treating ALS. The method may provide for administering to the plurality of subjects an effective amount of about chirally pure dexpramipexole or pharmaceutically acceptable salt thereof. The clinical trial may be carried out versus placebo in subjects with ALS.

Disclosures herein are directed to methods for conducting a clinical trial for treating ALS in a plurality of subjects. In particular, the ALS Functional Rating Scale, Revised (ALSFRS-R) scores may be determined for the subjects to determine a plurality of baseline numbers. The method may further comprise the collecting of outcome data from the subjects. The method comprises analyzing the outcome data as a primary endpoint in a joint-rank statistical test adjusted for mortality and adjusted for a change from baseline in ALS Functional Rating Scale, Revised (ALSFRS-R) score. The analysis may provide a greater negative adjustment for a death of a subject than for a survival with functional decline, if a higher rank is a better global clinical outcome. The analysis may provide a greater negative adjustment for an earlier death of a subject than for a later death of a subject, if a higher rank is a better global clinical outcome. Outcome data may be collected by phone interview, home visit, or combinations thereof. Outcome data may be collected in part by phone interview or home visit or combinations thereof. The outcome data may be selected from laboratory tests, ALSFRS-R score, adverse event reporting, or a combination thereof. The method may have the change from baseline determined through twelve months follow-up. The method may further comprise determining a secondary endpoint data of evaluation of time to death or respiratory insufficiency, respiratory decline, muscle strength, quality of life, population PK, and safety. The quality of life may be assessed using an amyotrophic lateral sclerosis (ALS) assessment questionnaire and a change-in-health survey.

In various embodiments, dexpramipexole administered or incorporated into the pharmaceutical compositions may be chirally pure or enantiomerically enriched to such an extent that the effects of any dopaminergic agonist activity associated with residual pramipexole is either absent or sufficiently small to allow for high dosage administration of dexpramipexole relative to enantiomerically pure or enantiomerically enriched pramipexole.

Accordingly, the methods for a clinical trial for treating ALS may include administering dexpramipexole for an extended, prolonged, or indefinite period of time. The extended period of time may be about twelve weeks or longer, about six months or longer, about one year or longer, and in other instances disclosed herein, a method of treating ALS comprises administering dexpramipexole on a maintenance dosing regimen. Thus, various disclosures herein are directed to maintenance therapy in which a dosing schedule for dexpramipexole is maintained for an extended period of time without titration or otherwise changing the dosing schedule. In such disclosures, the extended period of time may be about 12 weeks or longer, about 6 months or longer, about one year or longer, two, three, four, five, or ten years or longer, and in certain embodiments, an indefinite period of time.

Dexpramipexole may be administered to a plurality of individuals exhibiting the symptoms of a neurodegenerative disease or to a plurality of individuals predisposed to a neurodegenerative disease. Non-limiting examples of neurodegenerative diseases that may be treated using dexpramipexole include Huntington's Chorea, metabolically induced neurological damage, Alzheimer's disease, senile dementia, age associated cognitive dysfunction, vascular dementia, multi-infarct dementia, Lewy body dementia, neurodegenerative dementia, neurodegenerative movement disorder, ataxia, Friedreich's ataxia, multiple sclerosis, spinal muscular atrophy, primary lateral sclerosis, seizure disorders, motor neuron disorder or disease, inflammatory demyelinating disorder, Parkinson's disease, ALS, hepatic encephalopathy, and chronic encephalitis, or any combination thereof. Thus, the compositions and methods may be used to treat nearly individuals exhibiting symptoms of a neurological disease or susceptible to such diseases.

In further instances, the clinical trial may be carried out with various subjects in combination with other forms of treatment. Such combination therapy may produce synergistic effects, such that the effect of dexpramipexole is augmented wherein one or more symptoms show a dramatic improvement over pre-treatment levels. For example, dexpramipexole treatment may be carried out in combination with (simultaneously or concurrently) with riluzole without adverse effects or reduced symptom relief.

The method will use the efficacy population for analysis of the primary outcome consisting of all subjects who receive at least one dose of drug and have one evaluation. The intent-to-treat (ITT) population, consisting entirely of randomized subjects, will be used for the secondary and tertiary endpoints. Key outcomes data, such as survival status, will continue to be collected for those subjects who discontinue the study up to what would have been their Month 18 visit, or the study completion date, whichever comes first. The impact of any missing data will be assessed in the sensitivity analysis.

The inclusion in the method of outcome measures, such as, for example, respiratory status, quality of life, functional independence, and caregiver burden, yields data to support a thorough evaluation of the efficacy of dexpramipexole in ALS. In addition, ongoing safety data collection in the larger and more geographically diverse subject population augments the safety profile for dexpramipexole based on earlier-phase clinical studies.

The method may include subjects with "possible ALS". Expanding the subject inclusion criteria effects enrollment. Including subjects earlier in the disease process may enhance the potential to observe a response to treatments that are putatively neuroprotective.

The designs of the methods include several features to promote subject retention and decrease the subject and caregiver burden. Retention of study participants is important because early discontinuation creates a missing data dilemma, which may weaken positive results in clinical trials. In ALS, progression of the underlying disease may contribute to dropout rates as subjects are physically challenged to meet requirements for study participation. The method offers accommodation/planning for subjects who are housebound or under hospice care. Once approved by the investigator, continued participation requires only clinical laboratory tests, adverse event (AE) reporting, and ALSFRS-R assessments. All required information can be collected by phone interview or home visit. The discontinuation rate in the method is much lower than that observed in most prior clinical trials in ALS.

The method is one of the larger late-stage interventional clinical trials in ALS to date and is utilizing a novel endpoint (Combined Assessment of Function and Survival) that accounts for treatment effects on both survival and function. The method is designed to provide evidence regarding the efficacy of dexpramipexole in subjects with ALS.

For the first time in ALS clinical studies, function and survival was analyzed in a combined assessment as the primary endpoint. The Combined Assessment of Function and Survival (CAFS) analysis accounts for death by appropriately defining death as a worse outcome than survival with any degree of functional decline and subjects who die early in the study being defined as a worse outcome than those who die later in the study. This innovative method was used to address the challenge, noted in trials of other potential ALS treatments, of how to take mortality into account when looking at a functional outcome measure.

Another method for treating ALS in a subject comprises administering to the subject an effective amount of about chirally pure (6R)-2-amino-4,5,6,7-tetrahydro-6-(propylamino)benzothiazole or pharmaceutically acceptable salt thereof, and continuing to administer an effective amount of about chirally pure (6R)-2-amino-4,5,6,7-tetrahydro-6-(propylamino)benzothiazole or pharmaceutically acceptable salt thereof to the subject if there
is an improvement in the subject's ALSFRS-R score from a previous ALSFRS-R score of the subject. The method may further comprise determining a baseline ALS Functional Rating Scale, Revised (ALSFRS-R) score for the subject. The method may further comprise collecting outcome data from the subject. Further, the method may further comprise analyzing the outcome data as a primary endpoint in a joint-rank statistical test adjusted for mortality and adjusted for a change from baseline using ALS Functional Rating Scale, Revised (ALSFRS-R) scores from a plurality of persons for indications of efficacy or non-efficacy. The improvement in the subject's ALSFRS-R score may be statistically significant. The improvement in the subject's ALSFRS-R score may be statistically significant as defined in the EMPOWER phase III clinical trial. And the improvement in the subject's ALSFRS-R score may be statistically significant compared to the change in baseline ALSFRS-R scores using follow-up data through 12 months in a population of ALS subjects receiving an effective amount of about chirally pure (6R)-2-amino-4,5,6,7-tetrahydro-6-(propylamino)benzothiazole or pharmaceutically acceptable salt thereof.

Another method for treating amyotrophic lateral sclerosis (ALS) in a subject comprises administering to the subject an effective amount of about chirally pure (6R)-2-amino-4,5,6,7-tetrahydro-6-(propylamino)benzothiazole or pharmaceutically acceptable salt thereof; and continuing to administer an effective amount of about chirally pure (6R)-2-amino-4,5,6,7-tetrahydro-6-(propylamino)benzothiazole or pharmaceutically acceptable salt thereof to the subject if there is an improvement in the subject's CAFS ranking from a previous CAFS ranking of the subject.

Yet another method for treating amyotrophic lateral sclerosis (ALS) in a subject with possible ALS comprises administering to the subject with possible ALS an effective amount of about chirally pure (6R)-2-amino-4,5,6,7-tetrahydro-6-(propylamino)benzothiazole or pharmaceutically acceptable salt thereof, and continuing to administer an effective amount of about chirally pure (6R)-2-amino-4,5,6,7-tetrahydro-6-(propylamino)benzothiazole or pharmaceutically acceptable salt thereof to the subject if there is an improvement in the subject's ALS symptoms. The method may further comprise determining a baseline ALS Functional Rating Scale, Revised (ALSFRS-R) score for the subject. Other methods may further comprise collecting outcome data from the subject. The method may further comprise analyzing the outcome data as a primary endpoint in a joint-rank statistical test adjusted for mortality and adjusted for a change from baseline using
ALS Functional Rating Scale, Revised (ALSFRS-R) scores from a plurality of persons for indications of efficacy or non-efficacy. In some other methods, the improvement in the subject's ALSFRS-R score may be statistically significant. In still other methods, the improvement in the subject's ALSFRS-R score may be statistically significant as defined in the EMPOWER phase III clinical trial. In yet other methods, the improvement in the subject's ALSFRS-R score may be statistically significant compared to the change in baseline ALSFRS-R scores using follow-up data through 12 months in a population of ALS subjects receiving an effective amount of about chirally pure (6R)-2-amino-4,5,6,7-tetrahydro-6-(propylamino)benzothiazole or pharmaceutically acceptable salt thereof

The present application discloses methods for treating amyotrophic lateral sclerosis (ALS) in a subject, the method comprising determining a baseline ALS Functional Rating Scale Revised (ALSFRS-S) score for the subject, administering to the subject, one or more single unit doses, each unit dose comprising from about 50 mg to about 150 mg of 99% or greater chirally enriched (6R)-2-amino-4,5,6,7-tetrahydro-6-(propylamino)benzothiazole or a pharmaceutically acceptable salt thereof, providing to the subject, a daily dose of about 50 mg to about 300 mg of the (6R)-2-amino-4,5,6,7-tetrahydro-6-(propylamino)benzothiazole or a pharmaceutically acceptable salt thereof during a treatment time period, collecting outcome data from the subject during the treatment time period, determining an outcome ALSFRS-R score for the subject at the end of the treatment time period, and increasing the amount of (6R)-2-amino-4,5,6,7-tetrahydro-6-(propylamino)benzothiazole or a pharmaceutically acceptable salt thereof in each unit dose if the subject's outcome ALSFRS-R score is lower than the baseline ALSFRS-R score, or maintaining the amount of (6R)-2-amino-4,5,6,7-tetrahydro-6-(propylamino)benzothiazole or a pharmaceutically acceptable salt thereof in each unit dose if the subject's outcome ALSFRS-R score is an improvement over the subject's baseline ALSFRS-R score, wherein the subject is diagnosed with amyotrophic lateral sclerosis (ALS) or is predisposed to amyotrophic lateral sclerosis (ALS) and is not exhibiting any symptoms.

The present application discloses a method of identifying a patient who will respond to treatment with (6R)-2-amino-4,5,6,7-tetrahydro-6-(propylamino)benzothiazole or a pharmaceutically acceptable salt thereof comprising: diagnosing a patient with EEC definite ALS; measuring the serum creatinine levels of the patient; and identifying the patient as a responder if the serum creatinine levels are greater than about 72 µmol/L.

Some instances of each of the aforementioned methods are generally directed to pharmaceutical compositions including an effective amount of dexpramipexole and methods for using such pharmaceutical compositions for the treatment of neurological diseases such as, for example, ALS. In particular, instances of the various methods are directed to methods for treating neurological diseases including the step of administering at least about 150 milligrams of dexpramipexole per day to a subject in need of treatment, and in other embodiments, at least about 300 milligrams of dexpramipexole may be administered to a subject in need of treatment per day. Such administration may be carried out as a single dose once per day, or in certain embodiments, two or more doses of dexpramipexole may be administered two or more times per day. Therefore, instances of the various methods are also directed to pharmaceutical compositions at least including 50 milligrams of dexpramipexole and a pharmaceutically acceptable excipient, and in some embodiments, such pharmaceutical compositions may include at least 75 milligrams, 100 milligrams, 125 milligrams, 150 milligrams, 300 milligrams, 400 milligrams, 500 milligrams, or 600 milligrams of dexpramipexole and one or more pharmaceutically acceptable excipients, which may be administered as described above. Amyotrophic lateral sclerosis (ALS) may be limb-onset ALS or bulbar-onset ALS.

Some instances of the above methods are directed to methods for treating ALS in a plurality of subjects. In particular, the ALS Functional Rating Scale, Revised (ALSFRS-R) scores may be determined for the subjects to determine a plurality of baseline numbers. The method may provide for administering to the plurality of subjects an effective amount of about chirally pure dexpramipexole or pharmaceutically acceptable salt thereof. The method may further comprise the collecting of outcome data from the subjects. The method comprises analyzing the outcome data as a primary endpoint in a joint-rank statistical test adjusted for mortality and adjusted for a change from baseline in ALSFRS-R score. The analysis may provide a greater negative adjustment for a death of a subject than for a survival with functional decline, if a higher rank is a better global clinical outcome. The analysis may provide a greater negative adjustment for an earlier death of a subject than for a later death of a subject, if a higher rank is a better global clinical outcome. Outcome data may be collected by phone interview, home visit, or combinations thereof. Outcome data may be collected in part by phone interview or home visit or combinations thereof. The outcome data may be selected from laboratory tests, ALSFRS-R score, adverse event reporting, or
a combination thereof. The method may have the change from baseline determined through twelve months follow-up. The method may further comprise determining a secondary endpoint data of evaluation of time to death or respiratory insufficiency, respiratory decline, muscle strength, quality of life, population PK, and safety. The quality of life may be assessed using an amyotrophic lateral sclerosis (ALS) assessment questionnaire and a change-in-health survey.

In various instances of the methods above, dexpramipexole administered or incorporated into the pharmaceutical compositions may be enantiomerically pure or enantiomerically enriched to such an extent that the effects of any dopaminergic agonist activity associated with residual (6S)-2-amino-4,5,6,7-tetrahydro-6-(propylamino)benzothiazole (pramipexole) is either absent or sufficiently small to allow for high dosage administration of dexpramipexole relative to enantiomerically pure or enantiomerically enriched pramipexole. A description of methods for producing high purity dexpramipexole can be found in U.S. Application No. 12/049,235,. Treatment with dexpramipexole may include administering daily doses of about 100 milligrams or more, about 125 milligrams or more, about 150 milligrams or more, 300 milligrams or more, 400 milligrams or more, 500 milligrams or more, or 600 milligrams or more without the adverse side effects associated with dopaminergic agonism. For example, daily doses of dexpramipexole of about 150 milligrams or more or about 300 milligrams or more may be administered without an apparent impact on heart rate, blood pressure, or other cardiac activity that can be measured using, for instance, ECG or blood pressure cuffs that would otherwise be indicative of treatment with a dopamine agonist. Administrations of about 100 milligrams or more, about 125 milligrams or more, about 150 milligrams or more, 300 milligrams or more, 400 milligrams or more, 500 milligrams or more, or 600 milligrams or more per day of dexpramipexole have not been shown to cause any of these side-effects.

Moreover, because dexpramipexole is well tolerated, treatment including administration of daily doses of about 100 milligrams or more, about 125 milligrams or more, about 150 milligrams or more, 300 milligrams or more, 400 milligrams or more, 500 milligrams or more, or 550 milligrams or more of dexpramipexole may be carried out for prolonged periods of time such as, for example, twelve weeks or more, six months or more, one year or more and, in certain embodiments, for two, three, five or ten years or more, and in other embodiments, for an
indefinite period of time. Accordingly, methods of treating ALS may include administering dexpramipexole for an extended, prolonged, or indefinite period of time. The extended period of time may be about twelve weeks or longer, about six months or longer, about one year or longer, and in other instances, a method of treating ALS comprises administering dexpramipexole on a maintenance dosing regimen. In such instances, the maintenance dosing regimen may include administering about 100 milligrams or more, about 125 milligrams or more, about 150 milligrams or more, 300 milligrams or more, 500 milligrams or more, or 550 milligrams or more of dexpramipexole per day without any titration (or an initial dosing regimen of less than the maintenance dose). Thus, various instances are directed to maintenance therapy in which a dosing schedule for dexpramipexole is maintained for an extended period of time without titration or otherwise changing the dosing schedule. In such instances, the extended period of time may be about 12 weeks or longer, about 6 months or longer, about one year or longer, two, three, four, five, or ten years or longer, and in certain instances, an indefinite period of time. In other instances, the maintenance dosing may include administering less than the initial daily dose, such as, less than about 150 milligrams or less than about 300 milligrams of dexpramipexole per day. Additionally, without wishing to be bound by theory, the adverse effects associated with dopamine agonist treatment such as those described above may not develop after treatment with dexpramipexole has been carried out for a period of time of at least twelve weeks or more, and in some embodiments at least six months or one, two, three, five or ten years or more.

An initial dosing regimen may be provided. In certain embodiments, the initial dosing regimen may include administering a higher dose of dexpramipexole than the maintenance dosing regimen as either a single administration or by administering an increased dosage for a limited period of time prior to beginning a maintenance dosing regimen. For example, in certain embodiments, the initial dosing regimen may be about 300 milligrams to about 500 milligrams or more of dexpramipexole per day. This initial dosing regimen may continue for one, two, three, four, five, six, or seven days, up to four weeks, up to eight weeks, or up to twelve weeks. Following the initial dosing regimen, the subject may be administered a maintenance dosing regimen of, for example, about 100 milligrams or more, about 125 milligrams or more, about 150 milligrams or more, 300 milligrams or more, 400 milligrams or more, 500 milligrams or more, or 550 milligrams or more of dexpramipexole for an indefinite period of time such as, for example, at least twelve weeks or more, or at least six months, or one, two, three, five or ten years or more. In some embodiments, subjects undergoing maintenance may be administered one or more higher dosage treatments at one or more times during the maintenance dosage regimen.

Dexpramipexole may be administered to any individual exhibiting the symptoms of a neurodegenerative disease or to individuals predisposed to a neurodegenerative disease. Non-limiting examples of neurodegenerative diseases that may be treated using dexpramipexole include Huntington's chorea, metabolically induced neurological damage, Alzheimer's disease, senile dementia, age associated cognitive dysfunction, vascular dementia, multi-infarct dementia, Lewy body dementia, neurodegenerative dementia, neurodegenerative movement disorder, ataxia, Friedreich's ataxia, multiple sclerosis, spinal muscular atrophy, primary lateral sclerosis, seizure disorders, motor neuron disorder or disease, inflammatory demyelinating disorder, Parkinson's disease, ALS, hepatic encephalopathy, and chronic encephalitis, or any combination thereof. Thus, the compositions and methods of the various methods may be used to treat nearly any individual exhibiting symptoms of a neurological disease or susceptible to such diseases.

Dexpramipexole may be used to treat ALS. For example, individuals diagnosed with ALS within two years or less may be treated with dexpramipexole to reduce, eliminate, or slow advancement of ALS or symptoms associated with ALS such as, for example, fine motor function loss, gross motor function loss, loss of bulbar function, and loss of respiratory function. Dexpramipexole may be administered to reduce or slow the advancement of symptoms including, but not limited to, trembling, loss of muscle control, loss of ability to write, loss of ability to move or roll over, loss of speech, inability to swallow, difficulty breathing, and so on. Individuals with advanced symptoms or individuals who were diagnosed with ALS more than two years before beginning treatment may be treated with dexpramipexole, and such individuals may respond to treatment by exhibiting a reduction or elimination of one or more symptoms related to ALS, or in certain instances disclosed herein, the rate of symptom onset or advancement may be reduced, for example; the rate of motor function loss, the rate of loss of speech, and/or difficulty swallowing may be slowed and/or reduced.

In further embodiments, a dose-dependent response may be associated with treatment with dexpramipexole, and in certain embodiments, a dose-dependent response may be enhanced when treatment is carried out for longer periods of time. For example, a naïve subject who is administered a daily dose of, for example, about 300 milligrams of dexpramipexole or more, about 500 milligrams or more, or about 600 milligrams or more, may exhibit greater improvement in one or more symptoms of a neurological disease than a similarly situated naive subject who is administered a daily dose of dexpramipexole less than 300 milligrams, less than 500 milligrams, or less than 600 milligrams. In such embodiments, improvement resulting from higher dosage administration may be apparent after a single treatment. Moreover, in some embodiments, enhanced improvement in one or more symptoms as a result of administration of higher daily doses of dexpramipexole may be observed up to six months or more after beginning such treatment. Thus, in particular embodiments, treatment with higher doses of dexpramipexole may be carried out for prolonged periods of time, and the improvement associated with such dexpramipexole treatment may be realized after treatment has been carried out for a time period of, for example, one, two, three, four, five, six, or seven days, up to one, two, four, six, eight, twelve, twenty-four, or forty-eight weeks, up to five, ten, fifteen, or twenty years, or any length of time between the recited values. In further embodiments, treatment with higher doses of dexpramipexole may be carried out as maintenance therapy, wherein the subject is administered such doses of dexpramipexole at the initiation of treatment and, thereinafter continue such doses of dexpramipexole over time. In each of the methods described herein, any of the doses of dexpramipexole and/or any of the dosing regimens of dexpramipexole described herein may be used in such methods and continued administration of the doses may be continued for any of the described periods of time.

The observed improvement in one or more symptoms may become enhanced as treatment progresses such that after an improvement in one or more symptoms is observed further improvements in one or more symptoms may become evident with continued treatment. Without wishing to be bound by theory, a lag between beginning treatment and the first observation of improvement may be due to a period in which the dexpramipexole concentration in one or more of the subject's tissues increases to a threshold level where symptom improvement is observed. Any lag before observation of improvement may vary between subjects and may vary depending on,
for instance, the subject's demographics or characteristics such as, for example, age, progression of the disease, and/or the time between the onset of symptoms of the disease and beginning treatment, or any combination of the demographics or characteristics thereof.

In additional instances of the various methods, dexpramipexole may be administered to subjects in need of treatment for excessive weight loss associated with ALS. Without wishing to be bound by theory, the precipitous weight loss that is a cardinal symptom of ALS may be associated with increased energy expenditure, skeletal muscle hypermetabolism, and the systematic wasting of muscle tissue known as cachexia. In various embodiments, the total daily dose of dexpramipexole administered may be for example, less than 150 milligrams to 300 milligrams or greater, 400 milligrams or greater, 500 milligrams or greater, or 600 milligrams or greater. Any of the doses of dexpramipexole and/or any of the dosing regimens of dexpramipexole described herein may be used in the methods and continued administration of the doses may be continued for any of the described periods of time.

Dexpramipexole may be administered by titration where one or more initial doses are less than 150 milligrams, less than 300 milligrams, less than 400 milligrams, less than 500 milligrams, less than 600 milligrams, and so on when administered to naive subjects. Dexpramipexole treatment may further comprise titration because pramipexole has a significant adverse impact on naive subjects, and titration over the course of weeks in which the dosage regimen is periodically increased to reach higher dosages purportedly limits these adverse effects. In various instances, of the various methods, no titration of dexpramipexole is required. Thus, if an effective daily dose of dexpramipexole is, for example, 150 milligrams or 300 milligrams, the initial dose of dexpramipexole may be 150 milligrams or 300 milligrams of dexpramipexole, and each daily dose thereafter may be 150 milligrams or 300 milligrams. Accordingly, the daily dose may be considered a "stable daily dose." For example, dexpramipexole treatment can be initiated at high levels without the need for titration. Therefore, a naive subject who requires a dosage greater than about 150 milligrams or more, or about 300 milligrams or more, 400 milligrams or more, or about 500 milligrams or more, or about 600 milligrams or more dexpramipexole for treatment may be administered about 100 milligrams or more, about 125 milligrams or more, about 150 milligrams or more, 300 milligrams or more, 400 milligrams or more, 500 milligrams or more, or 600 milligrams or more of dexpramipexole during the first treatment without triggering the onset of adverse effects, as would be
expected if pramipexole was administered at its terminal level during an initial treatment. Accordingly, disclosures herein are directed to a method of treating a subject with ALS including administering an effective amount of dexpramipexole without titration. The effective amount may be about 100 milligrams or more, about 125 milligrams or more, about 150 milligrams or more, 300 milligrams or more, 400 milligrams or more, 500 milligrams or more, or 600 milligrams or more daily, and in some embodiments, the effective amount may be about 300 milligrams or more daily. In particular instances, the effective amount may be administered in separate equal doses twice daily. In each of the methods described herein, any of the doses of dexpramipexole and/or any of the dosing regimens of dexpramipexole described herein may be used in such methods and continued administration of such doses may be continued for any of the described periods of time.

Instances of the various methods are also directed to a dosage regimen for administering dexpramipexole. For example, the dosage regimen may include an initial dose dexpramipexole in one or more unit doses, then a plurality of daily doses having an equal amount of dexpramipexole as the initial dose in one or more unit doses. Such embodiments are not limited by the amount of the initial dose and daily doses. For example, in particular embodiments, the initial dose and each of the plurality of daily doses may be from about 50 milligrams to about 300 milligrams, or about 400 milligrams, or about 500 milligrams, or about 600 milligrams of dexpramipexole. In other embodiments, the initial dose and each of the plurality of daily doses may be from about 100 milligrams or more to about 300 milligrams, or about 400 milligrams, or about 500 milligrams, or about 600 milligrams of dexpramipexole, and in still other embodiments, the initial dose and each of the plurality of daily doses may be about 300 milligrams or more, about 400 milligrams or more, about 500 milligrams or more, or about 600 milligrams or more of dexpramipexole. In some embodiments, the one or more unit doses of the dosage regimen may be one to five unit doses, and in such embodiments, each of the one or more unit doses may be substantially equal. In other embodiments, each unit dose of the dosage regimen may be a solid unit dose. In each of the methods described herein, any of the dosage regimens for dexpramipexole described herein may be used in any of the methods, and the dosing regimens may be carried out using any of the compositions or any combination thereof described herein.

In particular instancesof the various methods, dexpramipexole may be administered to ALS subjects, and in such embodiments, the improvements observed in ALS subjects treated with dexpramipexole may be significantly better than conventional treatments such as, for example, treatments with riluzole. The improvement may be signified by greater than 20% increase in ALSFRS-R score, when compared to baseline scores taken before treatment, and in other instances, this improvement may be manifested in a greater than 30 % increase in ALSFRS-R score. The improvement in ALSFRS-R score may become apparent in less than 9 months, and in some embodiments, less than six, three, or one months. Riluzole, the only approved treatment for ALS, has not demonstrated any effect on ALSFRS-R score even after prolonged treatment. The majority of clinicians and clinical researchers believe that a therapy that results in a change of 20 % or greater in slope of ALSFRS-R score is clinically meaningful. Therefore, the rate of improvement observed during dexpramipexole treatment is considerably and surprisingly better than that of other ALS treatments or no treatment based on ALSFRS-R score.

Dexpramipexole may be administered for the treatment of ALS without incurring adverse events associated with, for example, riluzole, and the current standard of pharmacological intervention for ALS. For example, the overall rates of adverse events may be higher among subjects receiving riluzole concomitant with dexpramipexole or in conjunction with placebo. Headaches, for example, were reported by four times as many subjects receiving riluzole as those not receiving riluzole.

Dexpramipexole may be administered to improve the general health of individuals having a neurological disease, and in other embodiments, dexpramipexole may be administered to alleviate one or more specific symptoms. For example, dexpramipexole may be administered to ALS subjects to improve symptoms associated with for example, loss of fine motor, loss of speech, and difficulty swallowing or a combination thereof. Without wishing to be bound by theory, in such cases, improvements in fine motor and speech and swallowing related symptoms may become apparent in a shorter period of time following the initiation of dexpramipexole treatment than, for instance, improvements in large motor function and pulmonary related symptoms. Thus, while improvements in large motor function and pulmonary related symptoms may be observed after treatment with dexpramipexole,
dexpramipexole may be administered to alleviate fine motor and speech and swallowing related symptoms more immediately than other ALS symptoms. ALS subjects treated with dexpramipexole may have an increased time before a feeding tube must be employed because such subjects may retain the ability to masticate and swallow food stuffs under their own power.

Dexpramipexole may be administered to slow the rate of decline of a subject exhibiting symptoms of a neurological disease and/or to reduce mortality in such subjects. Populations of subjects diagnosed with a neurological disease such as, for example, ALS, may exhibit an increased time to death, an increased survival rate, and/or a decreased frequency of death as a result of treatment with dexpramipexole. Moreover, even in subjects who succumb to ALS or another neurological disease treated with dexpramipexole, dexpramipexole treatment may improve the quality of life for such subjects up to death.

The foregoing methods may comprise administering dexpramipexole on a dosing regimen to achieve a dose dependent , steady state AUC₀₋₁₂ (h x ng/mL) ranging from 836 ±234 to 2803 ±1635 to 6004 ± 2700 at daily doses of 50 milligrams, 150 milligrams, and 300 milligrams, respectively, when administered in two equal doses twice daily.

In further instances of the various methods, dexpramipexole treatment may be carried out in combination with other forms of treatment. In some embodiments, such combination therapy may produce synergistic effects, such that the effect of dexpramipexole is augmented wherein one or more symptoms show a dramatic improvement over pretreatment levels. For example, in certain embodiments, dexpramipexole treatment may be carried out in combination with (simultaneously or concurrently) with riluzole without adverse effects or reduced symptom relief. In other embodiments, dexpramipexole may be administrated in combination with (simultaneously or concurrently) an additional form of treatment including, but not limited, those set forth in U.S. Patent Application No. 13/059,713 filed April 19, 2011.

The pharmaceutical composition of dexpramipexole may achieve the effects described above by eliciting a neuroprotective, anti-oxidative, anti-apoptotic, or other beneficial cellular effects or combination of effects without the side-effects associated with dopaminergic agonists commonly used to treat neurodegenerative diseases. Without wishing to be bound by theory, the ability to deliver
clinically effective doses of dexpramipexole without dose limiting side effects may be made possible by: (i) the synthesis of dexpramipexole that is pure within limits of the detection; and (ii) that dexpramipexole possesses a substantially lower affinity for dopamine receptors than its enantiomer, pramipexole. Further details regarding the molecular basis for dexpramipexole neuroprotective, anti-oxidative, anti-apoptotic, or other beneficial cellular effects or combination of effects, including a comparison of the activity of dexpramipexole versus pramipexole can be found in U.S. Application No. 11/957,157.

Various instances disclosed herein include methods for treating a neurodegenerative disease by administering a therapeutically effective amount of dexpramipexole such as, for example, about 100 milligrams or more, about 125 milligrams or more, about 150 milligrams, or more or about 300 milligrams or more. In accordance with such instances, dexpramipexole may be formulated as a pharmaceutical or therapeutic composition by combining with one or more pharmaceutically acceptable carriers. Such pharmaceutical or therapeutic compositions may be formulated in tablet or capsule form for use in oral administration routes. The compositions and amounts of non-active ingredients in such a formulation may depend on the amount of the active ingredient, and on the size and shape of the tablet or capsule. Such parameters may be readily appreciated and understood by one of skill in the art.

The amount of pramipexole, (6S)-2-amino-4,5,6,7-tetrahydro-6-(propylamino)benzothiazole, remaining in the chirally pure dexpramipexole may be an amount not exceeding about 1.0 milligram, and in some embodiments, the amount of pramipexole may be an amount not exceeding about 0.75 milligrams, about 0.5 milligrams, about 0.25 milligrams, or about 0.125 milligrams. In particular embodiments, the amount of pramipexole in chirally pure dexpramipexole may be less than about 0.125 milligrams. Therefore, the amount of pramipexole that may be administered in pharmaceutical compositions containing the chirally pure dexpramipexole of various embodiments may be less than 1.0 milligrams per day, less than 0.5 milligrams per day, and in certain embodiments, less than 0.125 milligrams per day. Without wishing to be bound by theory, the amount of pramipexole in chirally pure dexpramipexole may be a non-effective dose such that any pramipexole in such compositions does not elicit a noticeable effect on subjects who are administered the pharmaceutical compositions of the various methods. For example, a 300 milligrams per day dose of dexpramipexole administered to a subject as a single unit dose of about 99.8% chirally pure dexpramipexole may contain a non-effective dose amount of pramipexole less than 1.0 milligrams per day, a 300 milligrams per day dose of about 99.9 % chirally pure dexpramipexole may contain a non-effective dose amount of pramipexole less than 0.5 milligrams per day, and a 300 milligrams per day dose of about 99.98 % dexpramipexole may contain a non-effective dose pramipexole of less than 0.125 mg/day.

Chirally pure dexpramipexole may be prepared or converted to a pharmaceutically acceptable salt of dexpramipexole. For example, in some embodiments, dexpramipexole may be formulated as (6R)-2-amino-4,5,6,7-tetrahydro-6-(propylamino)benzothiazole dihydrochloride, which is a pharmaceutically acceptable salt and may improve solubility of dexpramipexole in water. The conversion of (6R)-2-amino-4,5,6,7-tetrahydro-6-(propylamino)benzothiazole to a pharmaceutically acceptable salt may be accomplished by any method readily appreciated and understood by one of skill in the art. For example, (6R)-2-amino-4,5,6,7-tetrahydro-6-(propylamino)benzothiazole dihydrochloride may be prepared by a one-step method in which (6R)-2-amino-4,5,6,7-tetrahydro-6-(propylamino)benzothiazole or (6R)-2-amino-4,5,6,7-tetrahydro-6-(propylamino)benzothiazole salt is reacted with concentrated HCI in an organic solvent such as, for example, an alcohol, at a reduced temperature range of, for example, from about 0 °C to about 5 °C. An organic solvent, such as methyl tert-butyl ether, may then be added, and the reaction may be stirred for about one hour. The (6R)-2-amino-4,5,6,7-tetrahydro-6-(propylamino)benzothiazole dihydrochloride produced may be recovered from the reaction mixture by filtering, washing with an alcohol, and vacuum drying.

The amount of dexpramipexole in such pharmaceutically acceptable compositions suitable for oral administration may vary. For example, in some embodiments, the amount of dexpramipexole in such compositions may range from about 25 milligrams to about 1000 milligrams, about 50 milligrams to about 1000 milligrams, from about 100 milligrams to about 1000 milligrams, from about 125 milligrams to about 1000 milligrams, from about 150 milligrams to about 1000 milligrams, from about 300 milligrams to about 1000 milligrams, from about 500 milligrams to about 1000 milligrams, from about 600 milligrams to about 1000 milligrams, and in certain embodiments, the amount of dexpramipexole may be from about 60 milligrams to about 300 milligrams. Each of the compositions embodied herein may be used in any of the methods or dosage regimen described herein.

The daily dosage of dexpramipexole may be administered as a single daily dose, or may be divided into two or more doses of equal or unequal amount administered throughout the day. For example, in some embodiments, dexpramipexole may be administered in doses of about 100 milligrams or more, about 125 milligrams or more, about 150 milligrams or more, 300 milligrams or more, 500 milligrams or more, or 600 milligrams or more. Dexpramipexole may be administered in one to five doses each containing an equal amount of dexpramipexole, and in other embodiments, dexpramipexole may be administered in two or three doses throughout the day in doses of about 100 milligrams or more, about 125 milligrams or more, about 150 milligrams or more, 300 milligrams or more, 500 milligrams or more, or 600 milligrams or more. In still other embodiments, dexpramipexole may be administered in two or three doses throughout the day in doses about 100 milligrams or more, about 125 milligrams or more, about 150 milligrams or more, 300 milligrams or more, 500 milligrams or more, or 600 milligrams or more, wherein one dose contains a higher concentration of dexpramipexole. For example, one dose of a 300 mg dexpramipexole regimen may contain 100 milligrams of dexpramipexole and a second dose administered at a different time during the day may contain 200 milligrams of dexpramipexole. The daily doses may be used in any of the methods or dosage regimen described herein.

The pharmaceutical or therapeutic compositions of the various methods may be prepared, packaged, sold in bulk, as a single unit dose, or as multiple unit doses, and can be administered in the conventional manner by any route where they are active. For example, the compositions may be administered orally, opthalmically, intravenously, intramuscularly, intra-arterially, intramedullary, intrathecally, intraventricularly, transdermally, subcutaneously, intraperitoneally, intravesicularly, intranasally, enterally, topically, sublingually, rectally by inhalation, by depot injections, or by implants or by use of vaginal creams, suppositories, pessaries, vaginal rings, rectal suppositories, intrauterine devices, and transdermal forms such as patches and creams. Specific modes of administration will depend on the indication. The selection of the specific route of administration and the dose regimen may be adjusted or titrated by the clinician according to known methods in order to obtain the optimal clinical response. All of the methods described herein may be carried out by administering dexpramipexole by any such route for administration described herein. Additionally, dexpramipexole may be delivered by using any such route of administration for the entire dosage regimen described herein.

Pharmaceutical formulations containing dexpramipexole in a solid dosage may include, but are not limited to, tablets, capsules, cachets, pellets, pills, powders, and granules; topical dosage forms which include, but are not limited to, solutions, powders, fluid emulsions, fluid suspensions, semi-solids, ointments, pastes, creams, gels and jellies, and foams; and parenteral dosage forms which include, but are not limited to, solutions, suspensions, emulsions, and dry powder; comprising an effective amount of a polymer or copolymer. It is also known in the art that the active ingredients can be contained in such formulations with pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. The means and methods for administration are known in the art and an artisan can refer to various pharmacologic references for guidance. For example, Modern Pharmaceutics, Banker & Rhodes, Marcel Dekker, Inc. (1979); and Goodman & Gilman's The Pharmaceutical Basis of Therapeutics, 6th Edition, MacMillan Publishing Co., New York (1980) can be consulted.

For oral administration, the compounds can be formulated readily by combining these compounds with pharmaceutically acceptable carriers well known in the art. Such carriers enable the compounds of the various methods to be formulated as tablets, pills, dragees, capsules, liquids, gels, syrups, slurries, suspensions and the like, for oral ingestion by a subject to be treated. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations including, but not limited to, maize starch, wheat starch, rice starch, potato starch, gelatin, gum tragacanth, methyl cellulose, hydroxypropylmethyl-cellulose, sodium carboxymethylcellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate.

Pharmaceutical compositions may be suitable for oral administration such as, for example, a solid oral dosage form or a capsule, and in certain embodiments, the composition may be a tablet. Such tablets may include any number of additional agents such as, for example, one or more binder, one or more lubricant, one or more diluent, one or more lubricant, one or more surface active agent,
one or more dispersing agent, one or more colorant, and the like. Such tablets may be prepared by any method known in the art, for example, by compression or molding. Compressed tablets may be prepared by compressing in a suitable machine the ingredients of the composition in a free-flowing form such as a powder or granules, and molded tablets may be made by molding in a suitable machine a mixture of the powdered compound moistened with an inert liquid diluent. The tablets, of some embodiments, may be uncoated and, in other embodiments, they may be coated by known techniques.

In other embodiments prepared for oral administration, the pharmaceutical compositions may be provided in a dragee cores with suitable coatings. In such embodiments, dragee cores may be prepared suing concentrated sugar solutions, which may optionally contain gum arabic, talc, polyvinyl pyrrolidone, carbopol gel, polyethylene glycol, and/or titanium dioxide, lacquer solutions, and suitable organic solvents or solvent mixtures. In some embodiments, dyestuffs or pigments may be added to the tablets or dragee coatings for identification or to characterize different combinations of active compound doses. In yet other embodiments, pharmaceutical compositions including an effective amount of dexpramipexole prepared for oral administration may include, but are not limited to, push-fit capsules made of gelatin, as well as soft, sealed capsules made of gelatin and a plasticizer, such as glycerol or sorbitol. The push-fit capsules can contain the active ingredients in admixture with filler such as, for example, lactose, binders such as, for example, starches, and/or lubricants such as, for example, talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

In embodiments in which the tablets and dragee cores are coated, the coatings may delay disintegration and absorption in the gastrointestinal tract and thereby provide a sustained action over a longer period. Additionally, such coatings may be adapted for releasing dexpramipexole in a predetermined pattern, for example, in order to achieve a controlled release formulation, or it may be adapted to not release the active compound until after passage of the stomach by including, for example, an enteric coating. Suitable coatings encompassed by such embodiments may include, but are not limited to, sugar coating, film coating, such as, for example, hydroxypropyl methylcellulose, methyl-cellulose, methyl hydroxyethylcellulose, hydroxypropylcellulose, carboxymethylcellulose,
acrylate copolymers, polyethylene glycols, and/or polyvinylpyrrolidone, or an enteric coating, such as, for example, methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose. Furthermore, a time delay material such as, for example, glyceryl monostearate or glyceryl distearate may be incorporated into the coatings of some embodiments. In still other embodiments, solid tablet compositions may include a coating adapted to protect the composition from unwanted chemical changes, for example, to reduce chemical degradation prior to the release of the active drug substance.

Pharmaceutical compositions suitable for oral administration may include a therapeutically effective amount of dexpramipexole and a non-effective dose amount of pramipexole and may further include one or more diluents, one or more disintegrants, one or more lubricants, one or more pigments or colorants, one or more gelatins, one or more plasticizers, and the like. For example, in some embodiments, a tablet may include an effective amount of dexpramipexole, from about 20% to about 50% by weight of diluent in an amount, from about 10% to about 30% by weight of a second diluent, from about 2% to about 6% by weight of a disintegrant, and from about 0.01% to about 2% by weight of a lubricant, and in particular embodiments, such tablets may include an effective amount of dexpramipexole, from about 20% to about 50% by weight microcrystalline cellulose, about 10% to about 30% by weight mannitol, from about 2% to about 6% crospovidone or croscarmellose, and from about 0.01% to about 2% by weight magnesium stearate. In further embodiments, the pharmaceutical composition may include any amount or combination of microcrystalline cellulose, mannitol, sodium, crospovidone, croscarmellose magnesium stearate, or any combination thereof.

In such embodiments, the pharmaceutical composition suitable for oral administration may include at least about 50 milligrams of dexpramipexole, and in some embodiments, such pharmaceutical compositions may include at least about 75 milligrams of dexpramipexole, at least about 100 milligrams of dexpramipexole, at least about 150 milligrams of dexpramipexole, at least about 200 milligrams of dexpramipexole, at least about 250 milligrams of dexpramipexole, at least about 300 milligrams of dexpramipexole, at least about 500 milligrams of dexpramipexole, at least about 600 milligrams of dexpramipexole, at least about 750 milligrams of dexpramipexole, or at least about 1000 milligrams of dexpramipexole. In certain embodiments, such pharmaceutical
compositions suitable for oral administration prepared at any dosage described above may include a non-effective dose amount of pramipexole of less than about 0.125 milligrams.

The pharmaceutical compositions including dexpramipexole may be prepared as suspensions, solutions, or emulsions in oily or aqueous vehicles suitable for injection. In such embodiments, such liquid formulations may further include formulatory agents such as suspending, stabilizing, and/or dispersing agents formulated for parenteral administration. Such injectable formulations may be administered by any route, including but not limited to, for example, subcutaneous, intravenous, intramuscular, intra-arterial, bolus injection, or continuous infusion, and in embodiments in which injectable formulations are administered by continuous infusion, such infusion may be carried out for a period of about fifteen minutes to about twenty-four hours. In certain embodiments, formulations for injection can be presented in unit dosage form, such as, for example, in ampoules or in multi-dose containers, with an added preservative.

In other embodiments, dexpramipexole may be formulated as a depot preparation, and such long acting formulations can be administered by implantation, such as, for example, subcutaneously or intramuscularly, or by intramuscular injection. Depot injections can be administered at about one to about six months or longer intervals. Thus, for example, the compounds can be formulated with suitable polymeric or hydrophobic materials, for example, as an emulsion in an acceptable oil, or with ion exchange resins, or as sparingly soluble derivatives, for example, as a sparingly soluble salt.

In still other embodiments, pharmaceutical compositions including dexpramipexole may be formulated for buccal or sublingual administration. In such embodiments, the pharmaceutical compositions may be prepared as chewable tablets, flash melts, or lozenges formulated in any conventional manner known in the art.

In yet other embodiments, pharmaceutical compositions including dexpramipexole may be formulated for administration by inhalation. In such embodiments, pharmaceutical compositions may be delivered in the form of an aerosol spray presentation from pressurized packs or a nebulizer with the use of a suitable propellant, such as, for example, dichlorodifluoromethane, trichlorofluoromethane, dichlorotetrafluoroethane, carbon dioxide, or other suitable gas. In the case of a pressurized aerosol pack, the dosage unit can be determined by providing a valve to deliver a metered amount. Capsules and cartridges of, for example, gelatin for use in an inhaler or insufflator can be formulated containing a powder mix of the compound and a suitable powder base such as lactose or starch.

In further embodiments, pharmaceutical compositions including dexpramipexole can be formulated in rectal compositions such as suppositories or retention enemas, for example, containing conventional suppository bases such as cocoa butter or other glycerides.

In some embodiments, pharmaceutical compositions including dexpramipexole may be formulated for transdermal administration. Such pharmaceutical compositions may be prepared, for example, to be applied to a plaster or applied by transdermal, therapeutic systems that are supplied to the subject. In other embodiments, pharmaceutical and therapeutic compositions including dexpramipexole for transdermal administration may include suitable solid or gel phase carriers or excipients such as, but not limited to, calcium carbonate, calcium phosphate, various sugars, starches, cellulose derivatives, gelatin, and polymers such as, for example, polyethylene glycols.

In some embodiments, pharmaceutical compositions including dexpramipexole may be administered alone as a single therapeutic agent. In other embodiments, pharmaceutical compositions including dexpramipexole may be administered in combination with one or more other active ingredients, such as, for example, adjuvants, protease inhibitors, or other compatible drugs or compounds where such combination is seen to be desirable or advantageous in achieving the desired effects of the methods described herein.

In contrast to many other studies, in certain instances disclosed herein the various methods include subjects with "possible ALS". Historically, the inclusion of subjects in clinical trials in ALS has been limited to those who meet laboratory-supported probable, probable, or definite ALS, either familial or sporadic, in accordance with the revised El Escorial criteria. When these criteria were applied to data obtained from the Irish ALS register, 43% of subjects with ALS would not have been eligible for clinical studies at the time of diagnosis and 10% of subjects remained ineligible at death. In these subjects, the median time from onset of symptoms to eligibility for trial participation was thirteen months. Expanding the subject inclusion criteria impacts enrollment. In addition, including subjects earlier in the disease process may enhance the potential to observe a response to treatments that are
putatively neuroprotective. This is a valid way to capture subjects earlier in their disease progression.

Clinical studies are critical for establishing the safety and efficacy of potential treatments, as Phase II studies are generally smaller and designed to provide data supporting the safety and tolerability of a compound. An uncontrolled study of a compound without Phase III study results, particularly in a serious illness such as ALS, has the potential to cause untoward effects on subjects. Moreover, the interpretation of smaller studies, such as Phase II studies, and their use to determine whether or not to conduct larger studies must be carefully considered in any disease area. Studies are therefore critical to confirm any potential efficacy observed in smaller studies and for assessment of long-term safety.

Several compounds initially thought to be effective in ALS were used by individuals with ALS outside of studies because of the dearth of effective therapies. Early reports of a beneficial effect of lithium from a small exploratory study were not confirmed. In fact in one study more subjects who received lithium in combination with riluzole experienced an operationally defined "treatment failure" event (either a decrease of ≥ 6 points on the ALSFRS-R rating scale or death) than subjects who received placebo/riluzole. A small phase II study of talampanel also did not indicate statistically significant differences from placebo although trends in slowing ALSFRS-R decline and muscle strength were observed (Pascuzzi RM et al, 2010); talampanel subsequently failed to show any treatment effects in a larger Phase III study in ALS. In a phase III clinical study of minocycline for ALS, the rate of decline in the ALSFRS-R score was faster for subjects treated with minocycline than for subjects treated with placebo (-1.3 versus -1.04 units/month, [95%CI -0.44, -0.81], p=0.11) despite positive studies demonstrating the beneficial effects of minocycline in laboratory animals. Off-label use of the available compounds may have exposed subjects with ALS to possible detrimental effects. Off-label use also has the potential to impact clinical development and clinical trial conduct.

The instances for disease states, subject type, naive vs. not naïve, daily dose amounts, no observable adverse effect level dose amounts, non-effective dose amounts, and chiral purities for the methods of the various methods, which are described herein separately for the sake of brevity, can be joined in any suitable combination or combinations. Some disclosures herein include methods of identifying a subject that will likely respond to treatments described herein. The subject may exhibit one or more of the
following characteristics: definite ALS as defined by the El Escorial diagnosis criteria, amyotrophic lateral sclerosis symptom onset duration of less than about 18 months, limb-onset amyotrophic lateral sclerosis, concomitant riluzole administration, an ALSFRS-R score of greater than 36.0, a re-study progression rate greater than or equal to 0.8 points per month, a percentage predicted relaxed (slow) vital capacity (SVC) of less than or equal to 102.0, an ALSFRS-R fine motor domain score of greater than 10.0 points, ALSFRS-R bulbar domain score or greater than 9.0 points, an ALSFRS-R gross motor domain score of greater than 8.0 points, an abnormal neurological exam of the tongue, an abnormal neurological exam of the pharynx, larynx and swallowing, an abnormal neurological exam of the lower extremities, an abnormal neurological exam of the upper extremities, an abnormal neurological exam of the triceps, an abnormal neurological exam of the muscle mass and bulk, an abnormal neurological exam of the bicep, an abnormal neurological exam, a pulse rate of greater than 81.0 beats per minute, a diastolic blood pressure of greater than 82.0 mmHg, a systolic blood pressure of less than or equal to 117.0 mmHg, a creatinine value of greater than 72.0 µmol/L, a phosphorous value of less than or equal to 1.090 µmol/L, a platelet count of less than or equal to 248.0 x10⁹ cells/L, a cholesterol value of less than or equal to 5.3 mmol/L, a lactate dehydrogenase value of less than or equal to 161.0 U/L, a creatine phosphokinase value of less than or equal to 184.0 U/L, a bicarbonate value of less than or equal to 21.6 mmol/L, a triglyceride level of less than or equal to 1.4mmol/L, a uric acid level of greater than 320.0 µmol/L, a gamma-glutamyltransferase (GGT)level of greater than 37.0 U/L, a total bilirubin level of less than or equal to 6.0 µmol/L, a urine pH of less than or equal to 5.5, or any combination thereof. The subject may have at least one of the above characteristics. The subject may have more than one of the above characteristics. The method may further comprises monitoring said subject for any clinical features associated with amyotrophic lateral sclerosis. Therapy with a therapeutically effective amount of (6R)-2-amino-4,5,6,7-tetrahydro-6-(propylamino)benzothiazole or a pharmaceutically acceptable salt thereof may be initiated upon diagnosis of amyotrophic lateral sclerosis. The subject may exhibit symptoms of amyotrophic lateral sclerosis. The subject may have definite amyotrophic lateral sclerosis, probable amyotrophic lateral sclerosis, possible amyotrophic lateral sclerosis or suspected amyotrophic lateral sclerosis.

Disclosures herein are directed to creatinine as a biomarker, indicative of effective treatment. Disclosures herein are directed to a method of determining the efficacy of an amyotrophic lateral sclerosis treatment in a subject, comprising measuring serum creatinine levels in said subject, wherein a reduction in the decline of the serum creatinine in said subject is indicative of effective treatment of amyotrophic lateral sclerosis. The treatment may comprise administering to the subject a therapeutically effective amount of (6R)-2-amino-4,5,6,7-tetrahydro-6-(propylamino)benzothiazole or a pharmaceutically acceptable salt thereof. to the subject a therapeutically effective amount of riluzole or a pharmaceutically acceptable salt thereof. The treatment may comprise to the subject a therapeutically effective amount of (6R)-2-amino-4,5,6,7-tetrahydro-6-(propylamino)benzothiazole or a pharmaceutically acceptable salt thereof and a to the subject a therapeutically effective amount of riluzole or a pharmaceutically acceptable salt thereof. The treatment may comprise administering a pharmaceutical composition as described herein

Disclosures herein are directed to a method of monitoring disease progression in a subject comprising measuring a baseline serum creatinine level in the subject, measuring a serum creatinine level after a pre-determined time period and comparing the serum creatinine level after a pre-determined time period with the baseline serum creatinine level in the subject. A decrease in serum creatinine levels after a pre-determined period of time may be compared with the baseline serum creatinine level is indicative of disease progression. An increase in serum creatinine levels after a pre-determined period of time may be compared with the baseline serum creatinine level is indicative of the absence of disease progression. The pre-determined period of time may be about 2 weeks, about 1 month, or about 1 year. A serum creatinine level may be measured after a pre-determined period of time after the measuring of a baseline serum creatinine level can be used as an intermediate baseline for another serum creatinine level measurement after another pre-determined period of time. The subject may be a subject with definite ALS . The subject may be a subject with definite ALS, amyotrophic lateral sclerosis symptom onset duration of less than about 18 months, concomitant riluzole administration, a creatinine value of greater than 72.0 µmol/L or any combination thereof. Definite ALS may be defined by the El Escorial diagnosis criteria. The subject may be a subject being administered (6R)-2-amino-4,5,6,7-tetrahydro-6-(propylamino)benzothiazole or a pharmaceutically acceptable salt thereof.

Disclosures herein are directed to a method of monitoring treatment response in a subject comprising measuring a baseline serum creatinine level in the subject, measuring a serum creatinine level after a pre-determined time period and comparing the serum creatinine level after a pre-determined time period with the baseline serum creatinine level in the subject. A decrease in serum creatinine levels after a pre-determined period of time compared with the baseline serum creatinine level may be indicative of treatment response. An increase in serum creatinine levels after a pre-determined period of time compared with the baseline serum creatinine level may be indicative of the absence of a treatment response. The pre-determined period of time may be about 2 weeks, about 1 month, or about 1 year. A serum creatinine level measured after a pre-determined period of time after the measuring of a baseline serum creatinine level may be be used as an intermediate baseline for another serum creatinine level measurement after another pre-determined period of time. The subject may be a subject with definite ALS .The subject may be a subject with definite ALS, amyotrophic lateral sclerosis symptom onset duration of less than about 18 months, concomitant riluzole administration, a creatinine value of greater than 72.0 µmol/L or any combination thereof. Definite ALS may be defined by the El Escorial diagnosis criteria. The subject may be a subject being administered (6R)-2-amino-4,5,6,7-tetrahydro-6-(propylamino)benzothiazole or a pharmaceutically acceptable salt thereof.

### EXAMPLE 1 - POST HOC EMPOWER ANALYSES

A study design addressed the assessment of both function and survival as a primary outcome.

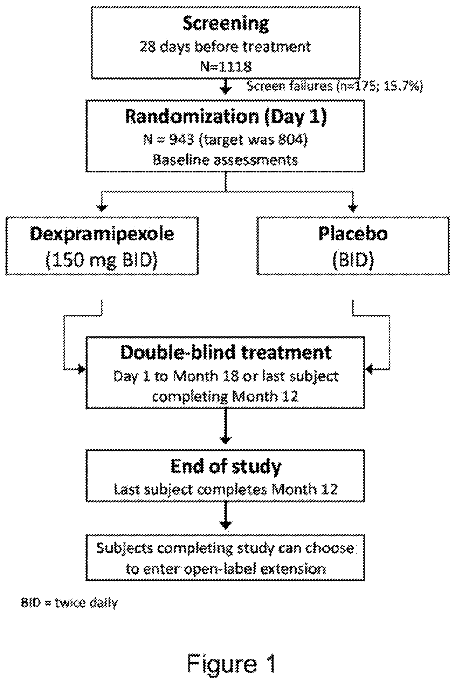

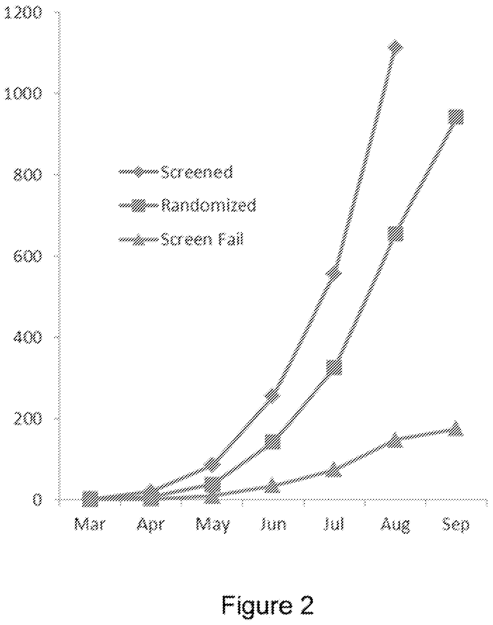

There is provided a multicenter, prospective, randomized, double-blind, placebo-controlled trial to determine the efficacy and safety of dexpramipexole. Eligible subjects were between 18-80 years of age with a clinical diagnosis of possible, laboratory-supported probable, probable, or definite ALS, either familial or sporadic, in accordance with the revised El Escorial criteria. Subjects were required to have had onset of first ALS symptoms ≤24 months before Day 1 and an upright vital capacity of ≥65% of predicted value for age, height, and gender at screening. Subjects not using riluzole for ≥30 days or those taking a stable dose for ≥60 days before Day 1 were allowed in the study. Riluzole-naïve subjects were also eligible, which allowed exploratory comparisons of the efficacy of
dexpramipexole alone vs. dexpramipexole with concomitant riluzole treatment. Subjects had to be able to swallow oral medications on Day 1 (Figure 1). Screening results in 175 failures reduced the number before the screening of 1118 subjects to 943 subjects (Figures 1 and 2).

Subjects not receiving riluzole or those taking riluzole at a stable dose for ≥60 days before study start were eligible. Subjects in a randomized 1:1 ratio received oral 150 mg dexpramipexole or placebo twice daily for 12 to 18 months. Use of placebo was ethical because the safety and efficacy of dexpramipexole in ALS have not yet been conclusively demonstrated and concomitant treatment with riluzole is permitted if desired by the subject and if initiated prior to enrollment in the study.

The key exclusion criteria in the embodiment were: 1) presence of significant cognitive impairment, clinical dementia, or psychiatric illness; other neurodegenerative disease, such as, for example, Parkinson's disease or Alzheimer disease; 2) a clinically significant history of unstable/severe cardiac, oncologic, hepatic, or renal disease or other medically significant illness; 3) pre-existing pulmonary disorder not attributed to ALS; 4) a neutrophil count <1.96 x 103/µL at screening or a documented history of neutropenia; 5) aspartate aminotransferase (AST) or alanine aminotransferase (ALT) levels >3.0 x upper limit of normal; 6) creatinine clearance ≤50 mL/min; 7) previous exposure to dexpramipexole or other experimental agent (off-label use or investigational) ≤30 days prior to Day 1; 8) current use of pramipexole or other dopamine agonists.

The primary efficacy analysis was a Combined Assessment of Function and Survival (CAFS), based on changes from baseline in the ALS Functional Rating Score-Revised (ALSFRS-R) scores or time to death using follow-up data through 12 months. Key secondary endpoints included time to death or respiratory insufficiency, such as combined endpoint: time to tracheostomy, or the use of non-invasive ventilation for greater than or equal to 22 hours per day for greater than or equal to ten consecutive day, or death), time to death, respiratory decline, (time to reach less than or equal to 50% of predicted upright slow vital capacity or death), changes in muscle strength quantified by hand-held dynamometry, and changes in ALS-related quality of life measured by the ALS Assessment Questionnaire (ALSAQ-5). Efficacy data was evaluated using 2-sided tests with α = 0.05 for the primary and secondary endpoints. Adverse events and other safety indices were monitored throughout the study.

Double-blind treatment continued up to 18 months or until the last subject completed 12 months, whichever came first. This allowed the collection of maximum information on the effect of dexpramipexole on survival and safety. The operational conduct of the study included a managed recruitment process, a robust communication plan with study sites, and an actively involved study team that closely monitored study sites.

Nine hundred forty three (943) subjects were enrolled. Mean baseline ALSFRS-R score was 38.2 ± 5.5, and mean symptom duration was 15.3 ± 5.4 months; most subjects, about 75%, entered the study receiving concomitant riluzole.

### Efficacy Assessments

The primary endpoint of the method of Example 1 was the Combined Assessment of Function and Survival (CAFS), a joint-rank test that is a valid statistical approach for analyzing functional outcomes adjusted for mortality. The CAFS ranks subjects' outcomes based on both time to death and change from baseline in ALSFRS-R score using follow-up data through 12 months. CAFS is predicated on the comparison of outcomes of each subject to all other subjects in the study. A higher rank is assigned to subjects who survive versus those who die, and the change from baseline in the ALSFRS-R is used to rank each survivor. A higher CAFS ranking indicates a better global clinical outcome. At the end of the trial, each subject's CAFS score was used to calculate average scores for each treatment group in order to evaluate the effect of dexpramipexole versus placebo on both function and survival.

Secondary and tertiary endpoints included evaluation of time to death or respiratory insufficiency, (combined endpoint: time to tracheostomy, or the use of non-invasive ventilation for ≥22 hours per day for ≥10 consecutive days, or death), time to death, respiratory decline (time to reach ≤50% of predicted upright slow vital capacity or death), muscle strength, quality of life, population pharmacokinetics, and safety (Table 2). Quality of life was assessed using the Amyotrophic Lateral Sclerosis Assessment Questionnaire (5-item Form) (ALSAQ-5) and the change in the Short Form 36 (SF-36) total score. The change in the Caregiver Burden Inventory (CBI) total score is used to evaluate potential impact on caregiver burden. Safety assessments included physical examinations, clinical laboratory evaluations, vital signs, and adverse/serious adverse event monitoring. Because a small number of cases of reversible neutropenia were observed in a previous study, monthly blood draws (in clinic alternating with home visits) including absolute neutrophil counts was obtained for all subjects.

**Table 2: EMPOWER Study Endpoints**

| |
|---|
| Primary Efficacy Endpoint (at 12 months) |
| • CAFS is a joint rank endpoint: based on change from baseline in ALSFRS-R score and time to death using follow-up data through 12 months |
| Secondary and Tertiary Endpoints (at 18 months) |
| Efficacy |
| • Time to death or respiratory insufficiency |
| • Time to death |
| • Respiratory decline: time to 50% of predicted upright VC or death; decline in predicted SVC and SNIP |
| • Change in MSM, determined by overall mega score for HHD |
| • Ambulatory decline: time to recommended wheelchair use for out-of-home ambulation or death |
| • Time to death or death equivalence (tracheostomy or permanent assisted ventilation, defined as use of NIV for ≥22 hours per day for ≥10 days), using all available follow-up data |
| • Time to recommended gastrostomy tube placement or death |
| • Time to treatment failure, calculated from randomization to date of death or decline in ALSFRS-R |
| Population Pharmacokinetics |
| • Final parameters dependent on the population PK model |
| ∘ Relationship between population PK parameters and potential determinant covariates (e.g., age, gender, race, co-medication, renal function) |
| ∘ Changes from baseline in efficacy endpoints (e.g., ALSFRS-R) |
| Safety (assessment visits) |
| • Monitor AEs and SAEs (throughout study duration) |
| • Vital signs: SBP/DBP, RR, HR, temperature (screening, BL, Week 2, Months 2, 4, 6, 8, 10, 12, 14, 16, 18 and EOS/ET) |
| • Clinical laboratory assessments: hematology, blood chemistry, urinalysis (every clinic visit) |
| • Physical examination (screening, BL, Months 2, 6, 12, 18 and EOS/ET) |
| • 12-lead ECGs (screening, BL, Week 2, Months 6, 12, 18 and EOS/ET) |
| • Body weight (screening, BL, Week 2, Months 2, 4, 6, 8, 10, 12, 14, 16, 18 and EOS/ET) |
| Quality of life (assessment visits) |
| • ALS Assessment Questionnaire, 5-Item Form (ALSAQ-5) (BL, Months 6, 12, 18 and EOS/ET) |
| • European Quality of Life - 5 Dimensions (EQ-5D) (BL, Months 6, 12, 18 and EOS/ET) |
| • Caregiver Burden Inventory (CBI) (BL, Months 6, 12, 18 and EOS/ET) |
| • Health Resource Use Questionnaire (BL, Months 6, 12, 18 and EOS/ET) |

| |
|---|
| CAFS = combined assessment of function and survival; AE = adverse event; BL = baseline; DBP = diastolic blood pressure; ECG = electrocardiogram; EOS = end of study; ET = early termination; HHD = hand-held dynamometry; HR = heart rate; MSM = muscle strength measurement; NIV = non-invasive ventilation; RR = respiratory rate; SAE = serious adverse event; SNIP = sniff nasal inspiratory pressure; SVC = slow vital capacity; SBP = systolic blood pressure; VC= vital capacity |

### Statistical Analysis

The study is able to independently evaluate a potential benefit of dexpramipexole versus placebo on ALSFRS-R scores, survival, and CAFS ranking. Analysis of the primary endpoint was based on the efficacy population, defined as all randomized subjects who received ≥1 dose of study drug and with ≥1 post-dosing efficacy evaluation or who died during the study. Analysis of secondary and tertiary endpoints was done using the
intent-to-treat population (ITT), defined as all randomized subjects who received ≥1 dose of study drug. All efficacy comparisons will be 2-sided statistical tests with α = 0.05 for the primary endpoint, CAFS ranking, and secondary endpoints.

For the survival analysis, the study was powered such that it would have an 80% probability of detecting a 37% difference between dexpramipexole and placebo, based on a sample size of 402 subjects per treatment group. A hazard ratio reduction of about 37% represents a clinically meaningful survival benefit. For about 90% power to detect a mean difference between groups of about 2.13 on the ALSFRS-R score at 12 months, it was estimated that a sample size of about 402 subjects per treatment group is required, with about a 20% drop-out rate. This dropout rate was derived by an assessment of the dropout rates of several large ALS trials conducted since 1996 which indicated that the placebo drop-out rate was below 20% annually. Discontinuation rates in pivotal trials have historically been high for ALS treatment. See for example Figure 1 of Aggarwal et al., "ALS Drug Development: Reflections from the Past and a Way Forward", 5 NEUROTHERAPEUTICS, 516-527 (2008).

The study power calculation used a 2-sided Wilcoxon test with α = 0.05 and standard deviation (σ) of about 8.1. The standard deviation (σ) was based on results of a study from U.S. patent application no. 12/819,990 and published studies of minocycline and glatiramer acetate, chosen as being more reflective of the current care of ALS subjects and their associated disease progression rates than older studies. As the number of subjects (943) enrolled exceeds the planned sample size (804) for 90% power, the study adequately will assess the *a priori* endpoints.

To understand drivers for the effect on CAFS rankings, evaluations of ALSFRS-R scores and time to death will be conducted as component analyses for CAFS rankings. The mixed effects repeated measures model was performed on the change from baseline in ALSFRS-R functional scores using data through 12 months. The mixed-effects slope model is not the primary analysis method, as the mixed-effects slope model assumes linearity in the decline of function over time, which may or may be observed in a 12-18month study, and moreover, assumes that all discontinuations are random and non-informative, which is not the case for deaths. However, the mixed-effects slope model will be used to assess the slope of decline in determining ALSFRS-R scores as a sensitivity analysis.

Clinical status was assessed by administration of (1) the ALSFRS-R to assess functional status; (2) vital capacity (VC) to assess pulmonary function; and (3) the McGill single-item scale (SIS) to assess general quality-of-life. Plasma and CSF samples were collected to assess potential drug-related changes in potential surrogate markers of motor neuron stress and damage, such as levels of cystatin C.

### Results

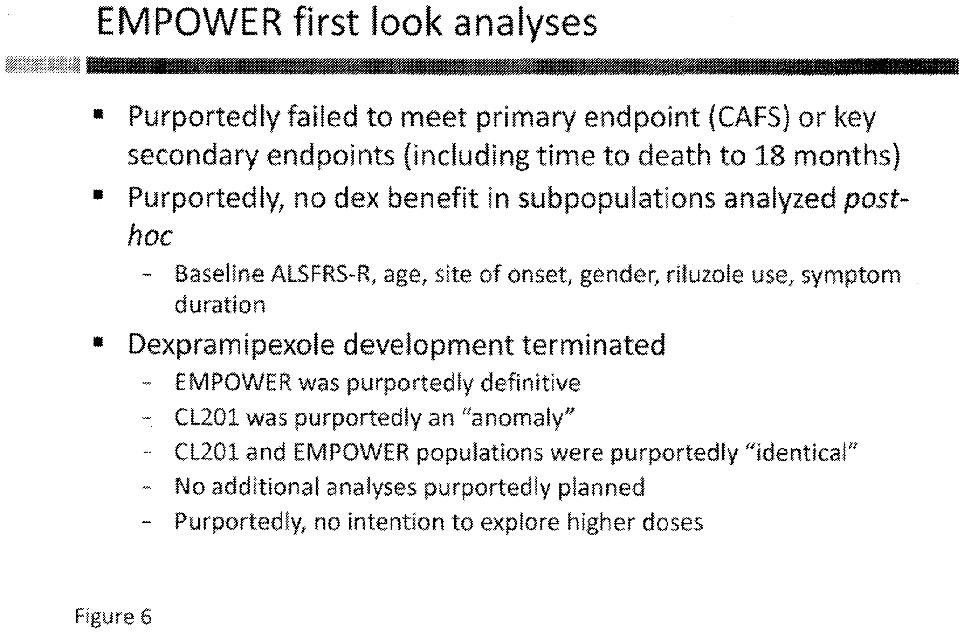

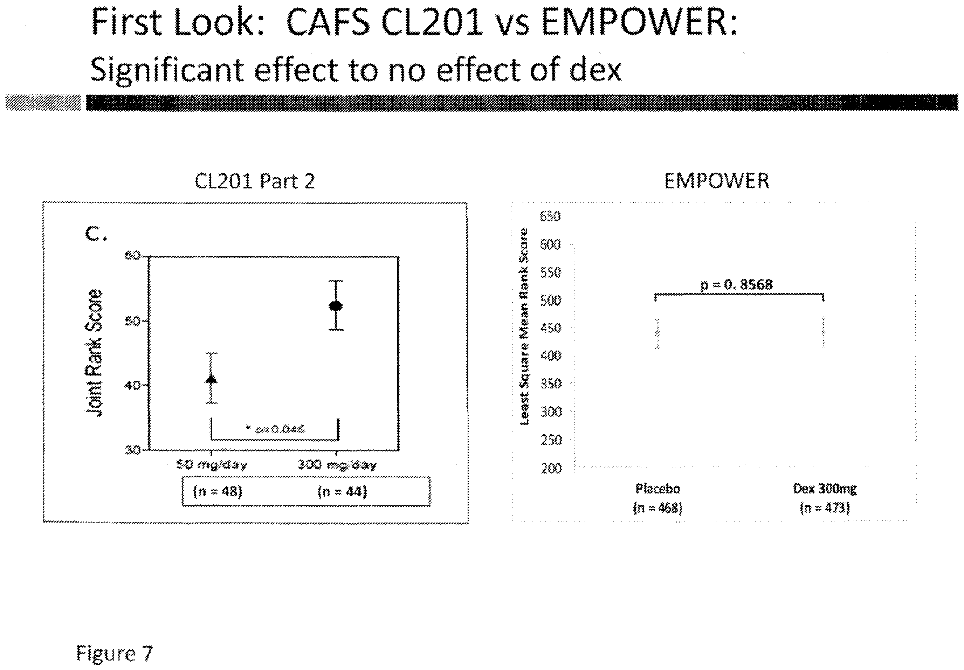

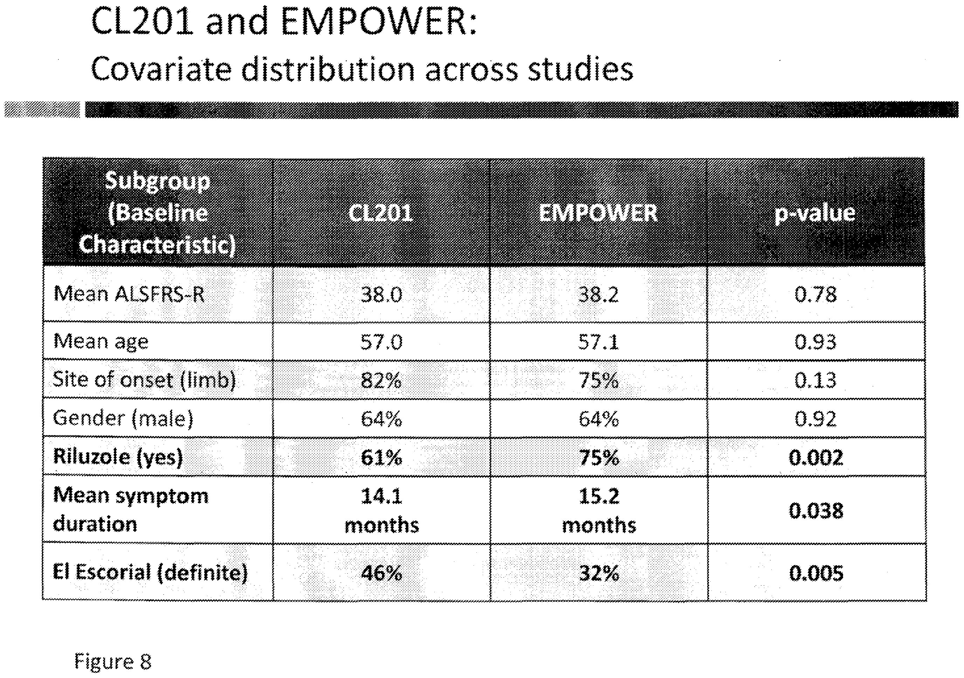

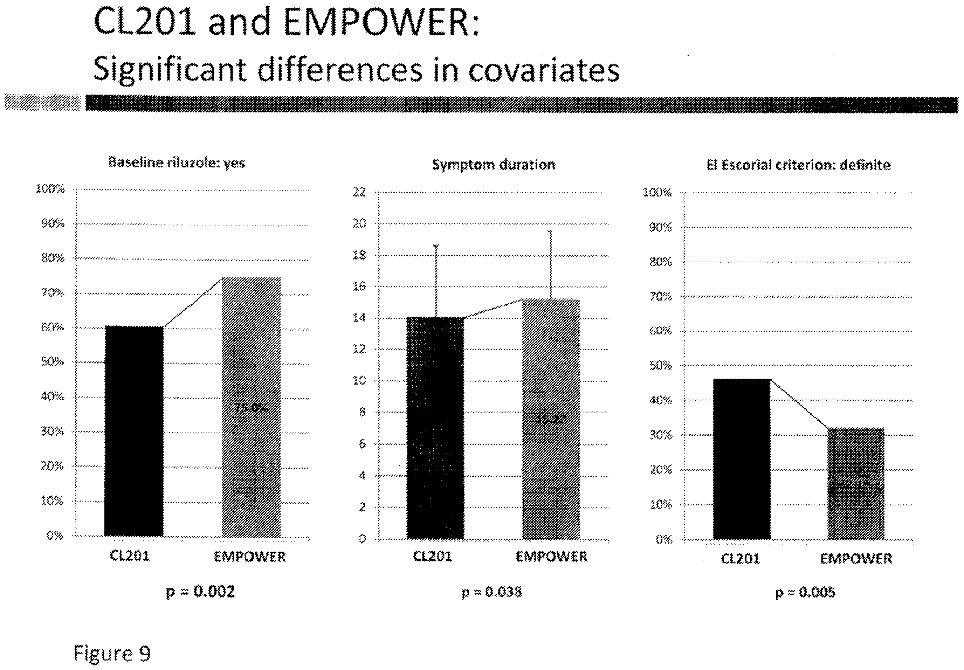

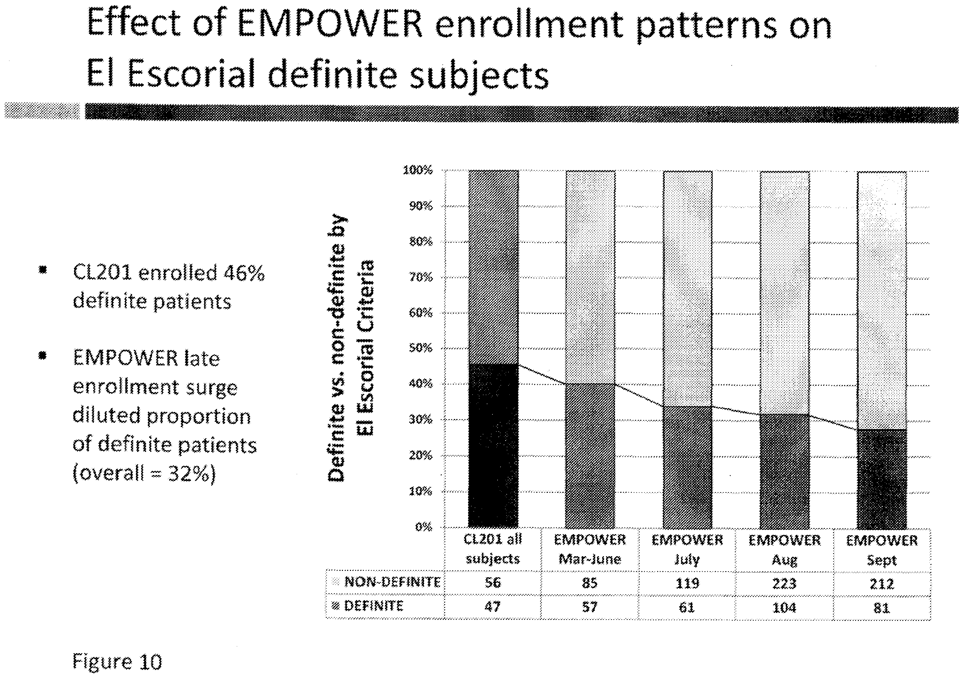

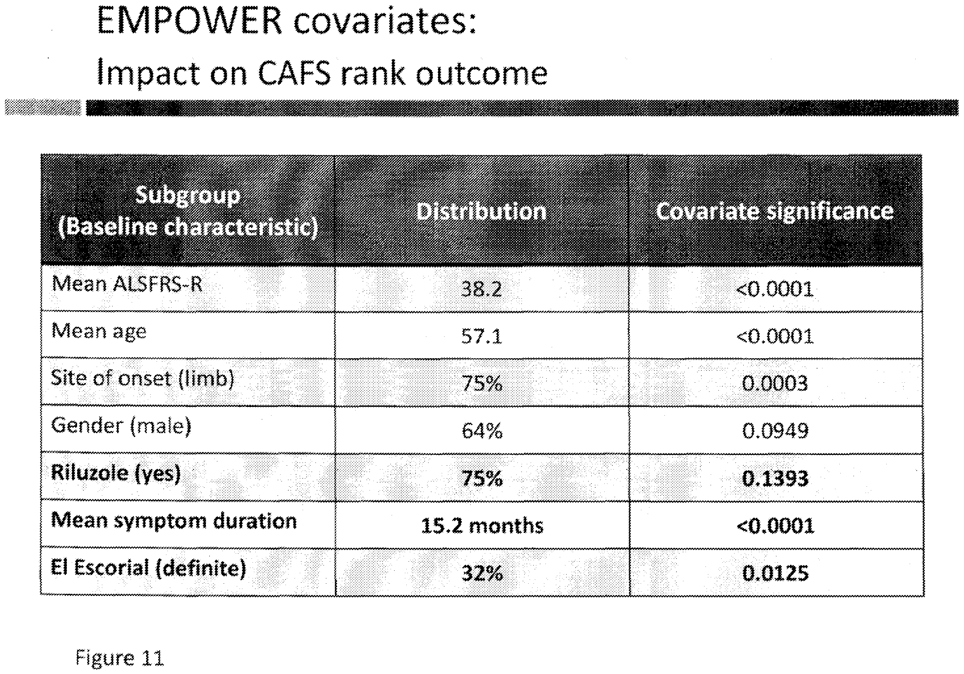

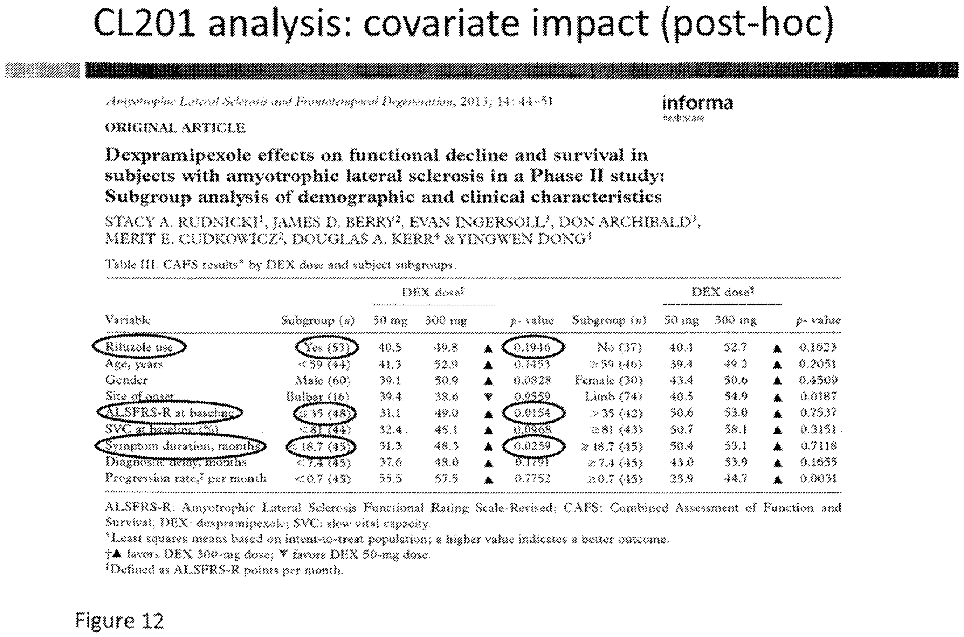

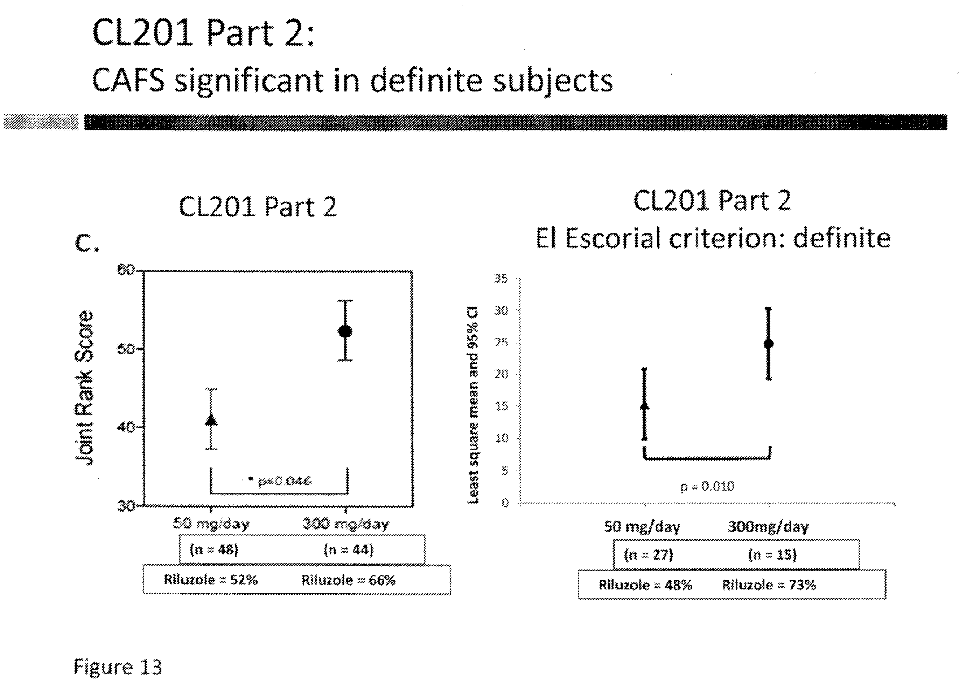

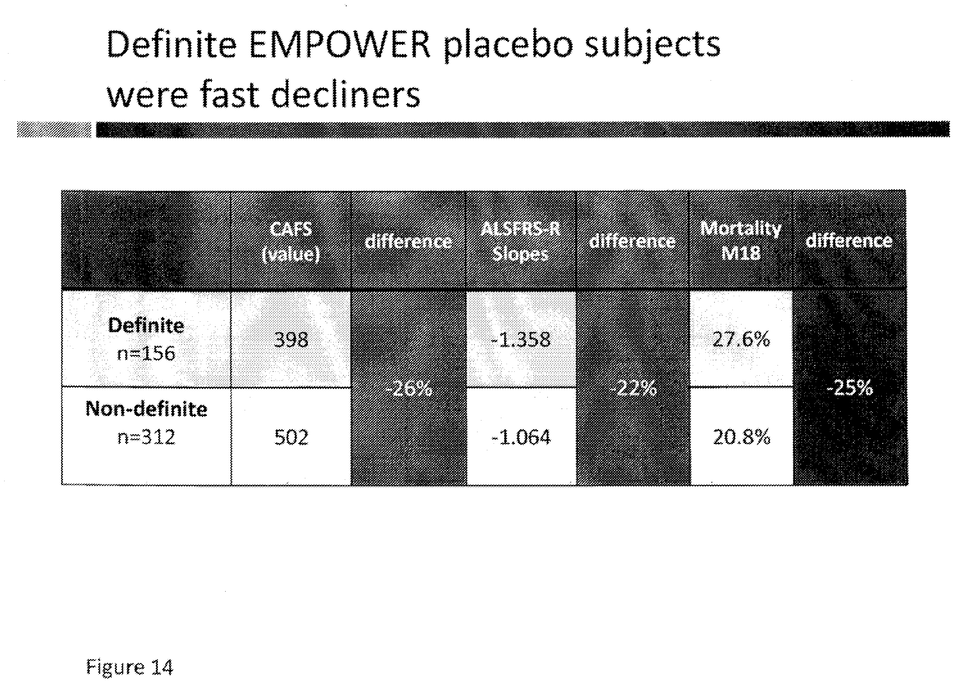

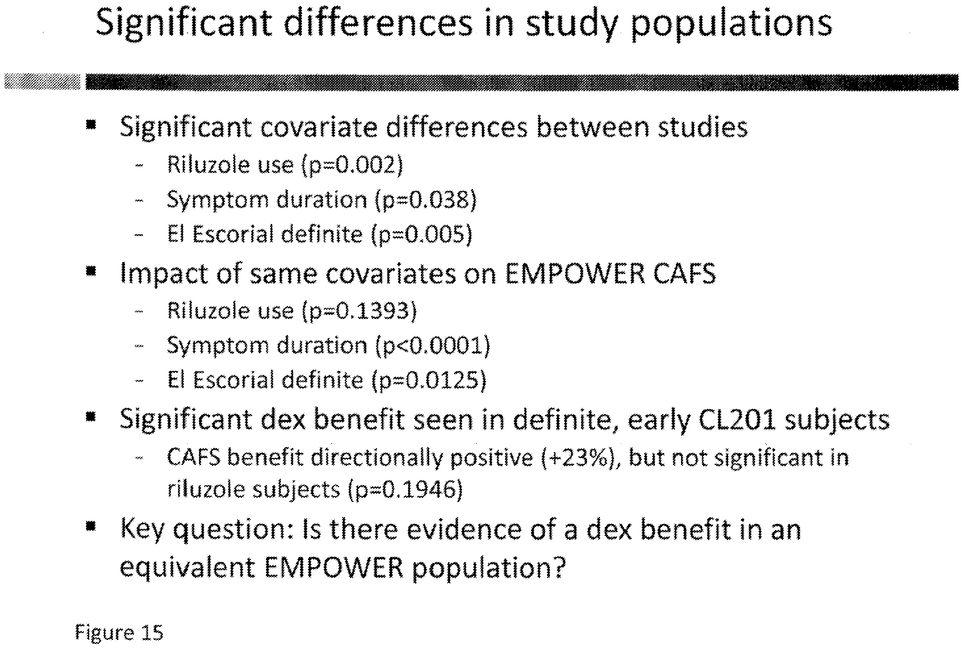

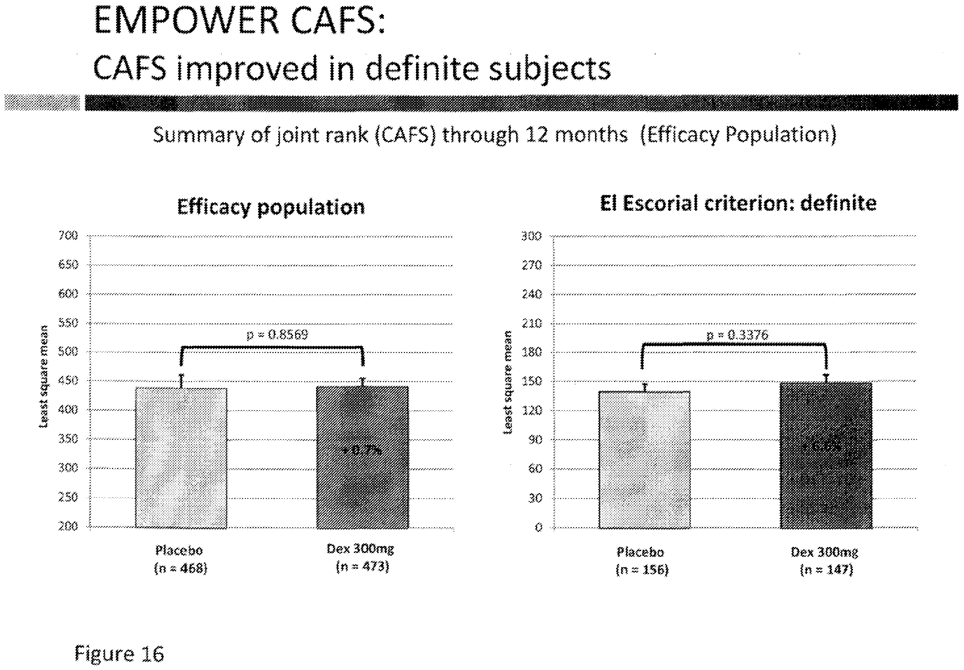

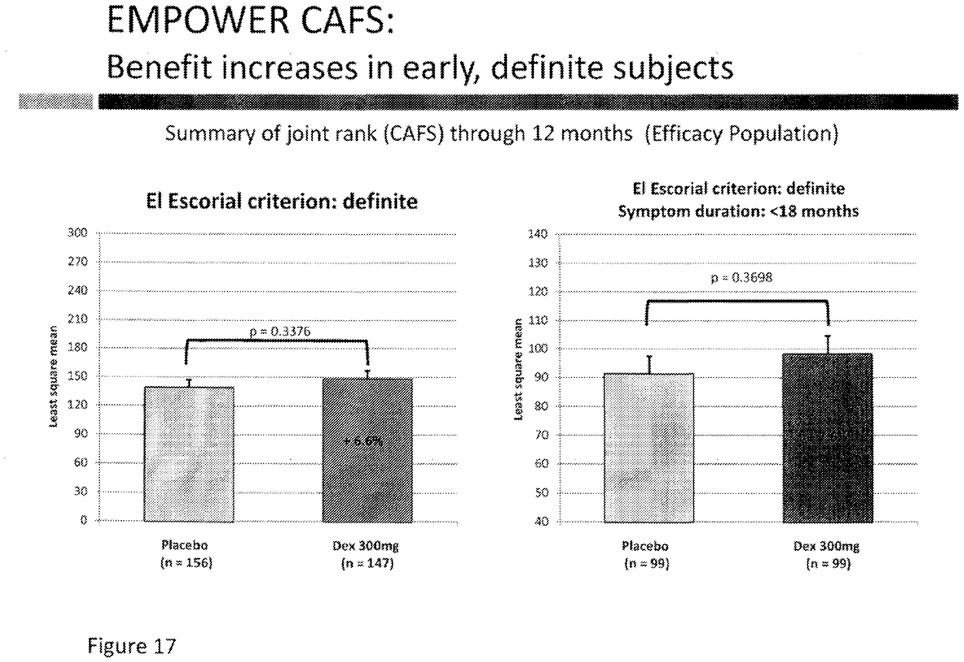

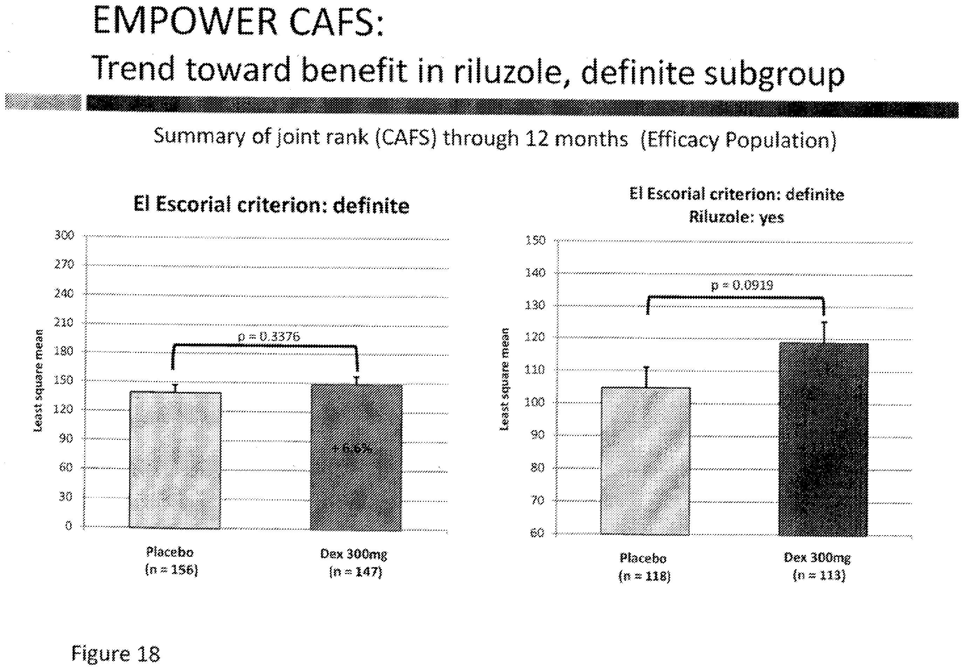

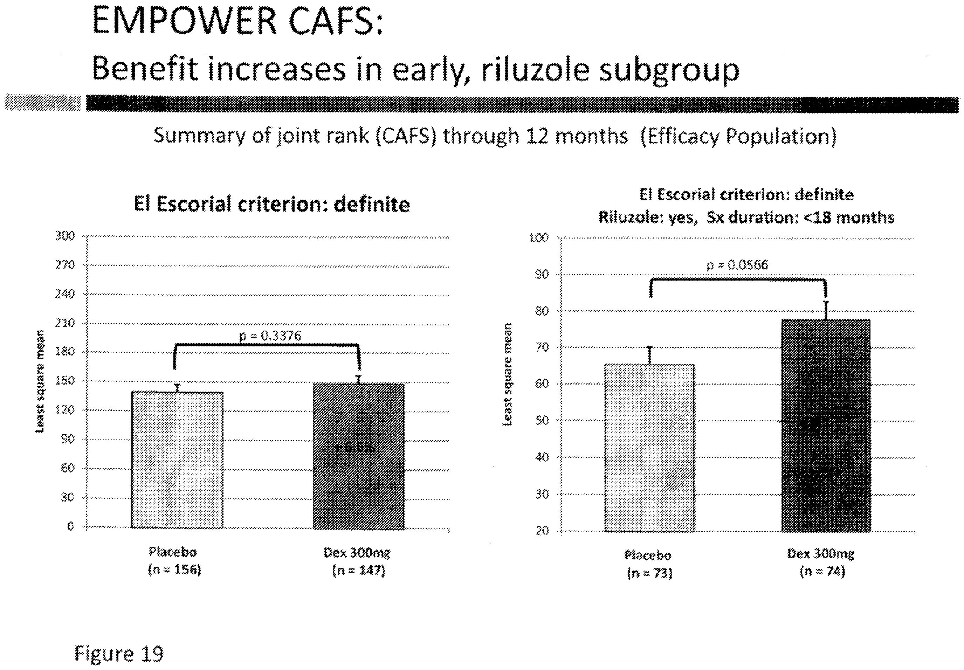

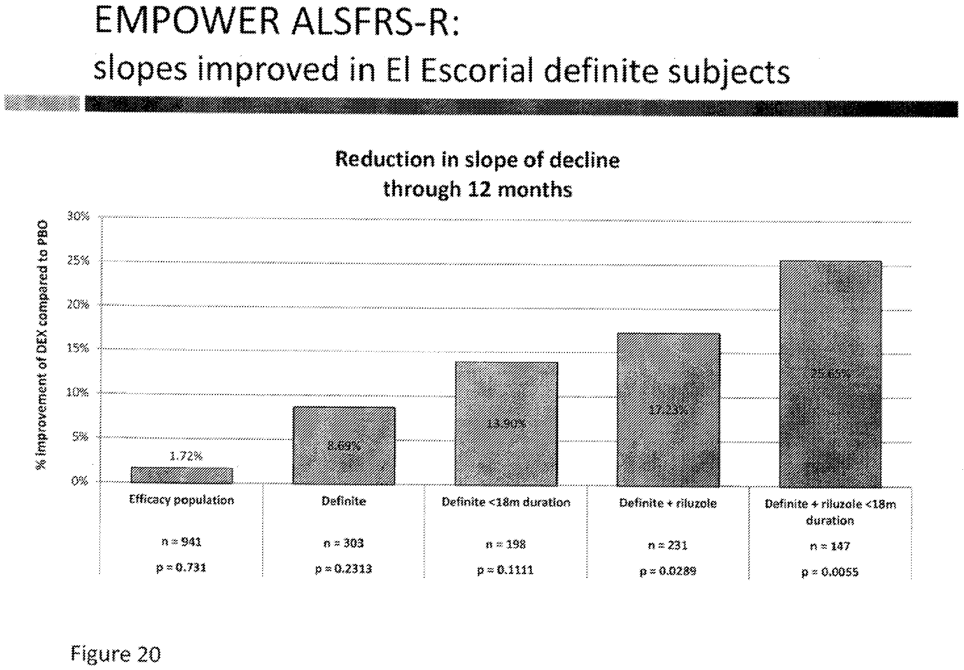

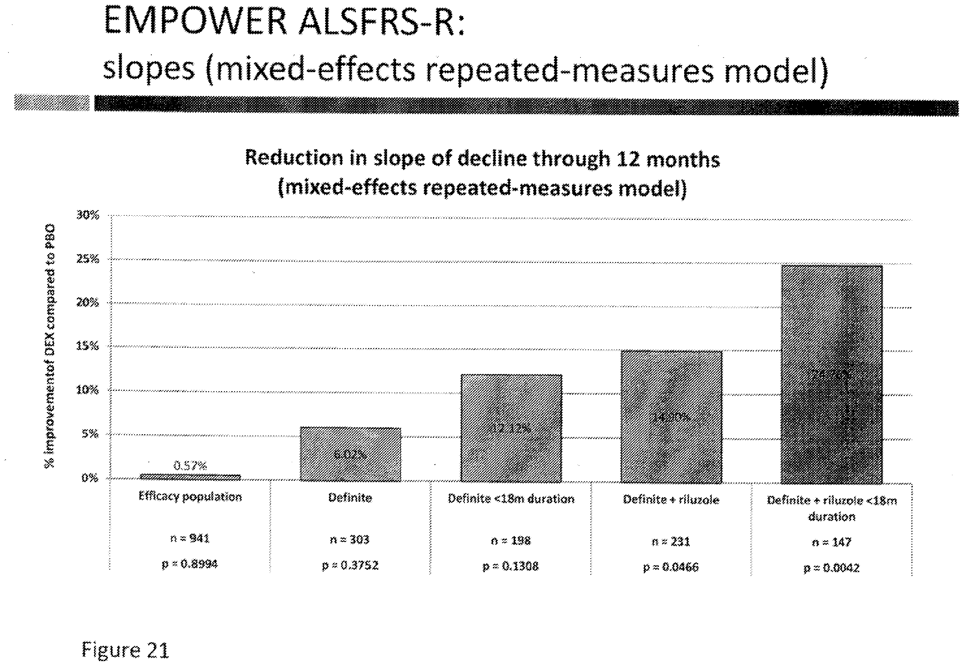

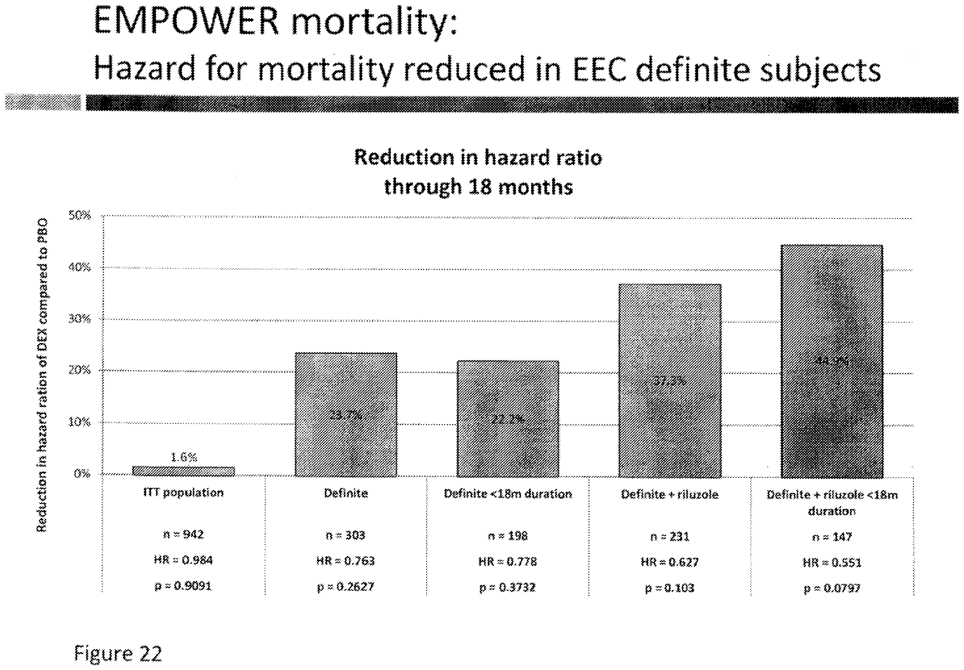

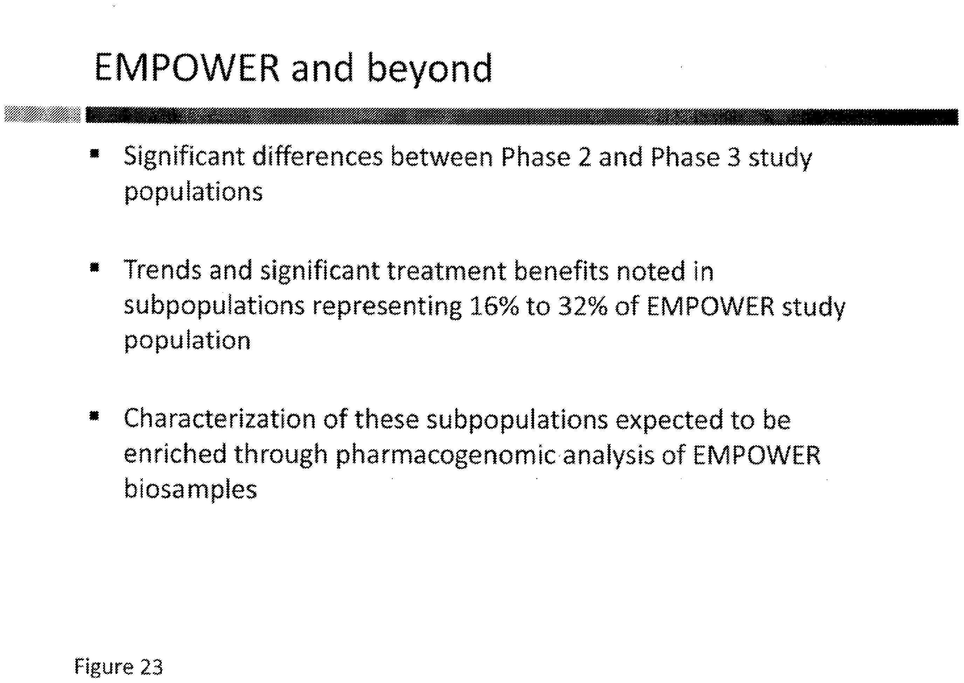

The initial analysis of the results of EMPOWER are set forth in Figure 6 and Figure 7. A comparison of the covariate distribution between the phase 2 CL201 and phase 3 EMPOWER studies are set forth in Figure 8 and 9. The effect of the EMPOWER enrollment patterns on El Escorial definite subjects is set forth in Figure 10 and the impact on CAFS rank outcome is set forth in Figure 11. A post-hoc analysis of the covariate impact on the results of CL201 is set forth in Figure 12 and in particular the impact of the CAFS score in ALS definite subjects in Figure 13. Figure 14 shows the difference in CAFS value, ALSFRS-R scores and mortality in the ALS definite and non-ALS definite subjects in EMPOWER. Figure 15 summarizes the significant differences in the patient populations and effects on treatment of ALS between the CL201 and the EMPOWER studies not previously identified. Figures 16- 23 depict the results of dexpramipexole treatment in certain subpopulations of patients studied in EMPOWER. In particular, Figure 16 depicts the improvement of CAFS rank in ALS definite subjects (as defined by El Escorial criteria). Figure 17 depicts the improvement in CAFS rank in ALS definite subjects (as defined by El Escorial criteria) with symptom duration of less than 18 months. Figure 18 depicts the improvement in CAFS rank in ALS definite subjects (as defined by El Escorial criteria) with concomitant riluzole administration. Figure 19 depicts the improvement in CAFS rank in ALS definite subjects (as defined by El Escorial criteria) with symptom duration of less than 18 months and concomitant riluzole administration. Figure 20 depicts the percent improvement of dexpramipexole compared to placebo in the reduction in slope of decline in the ALSFRS-R score in ALS definite patients, ALS definite patients with symptom duration of less than 18 months, ALS definite patients with concomitant riluzole administration and ALS definite patients with symptom duration of less than 18 months, as well as concomitant riluzole administration. Figure 21 depicts the percent improvement of dexpramipexole compared to placebo in the reduction in slope of decline in the ALSFRS-R score (using the mixed-effects repeated-measures model) in ALS definite patients, ALS definite patients with symptom duration of less than 18 months, ALS definite patients with concomitant riluzole administration and ALS definite patients with symptom duration of less than 18 months, as well as concomitant riluzole administration. Further, Figure 22 depicts the reduction in hazard ratio of dexpramipexole compared to placebo in ALS definite patients, ALS definite patients with symptom duration of less than 18 months, ALS definite patients with concomitant riluzole administration and ALS definite patients with symptom duration of less than 18 months, as well as concomitant riluzole administration.

A Monte Carlo analysis was done (Immunetrics, Inc., Pittsburgh, PA) to identify characteristics of subjects in the phase 3 EMPOWER studies that were predictive of improved outcomes. Among the baseline characteristics most predictive of outcomes was a serum creatinine level greater than about 72 µmol/L. The analysis identified 369 patients meeting this criterion, of which 174 had been randomized to the placebo group and 195 to the treatment group.

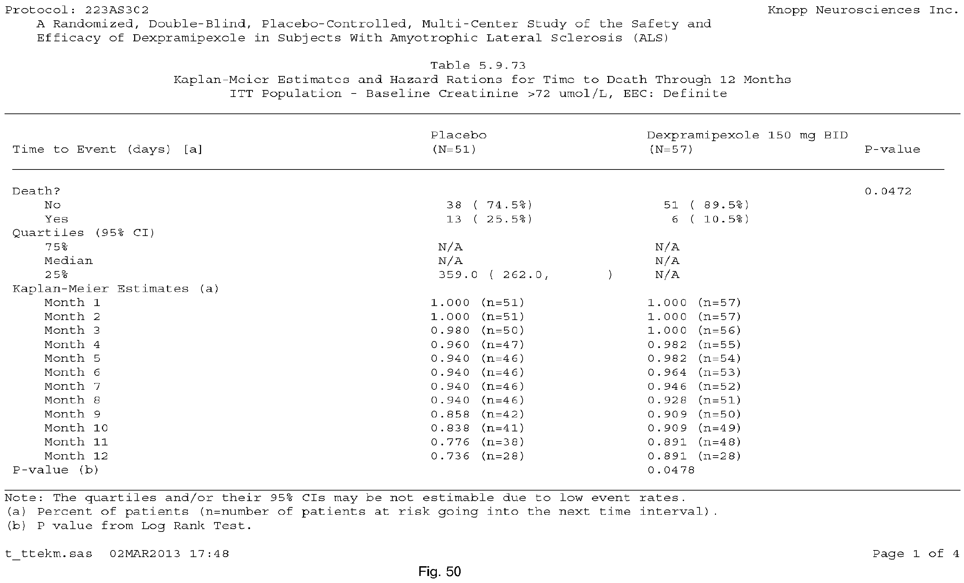

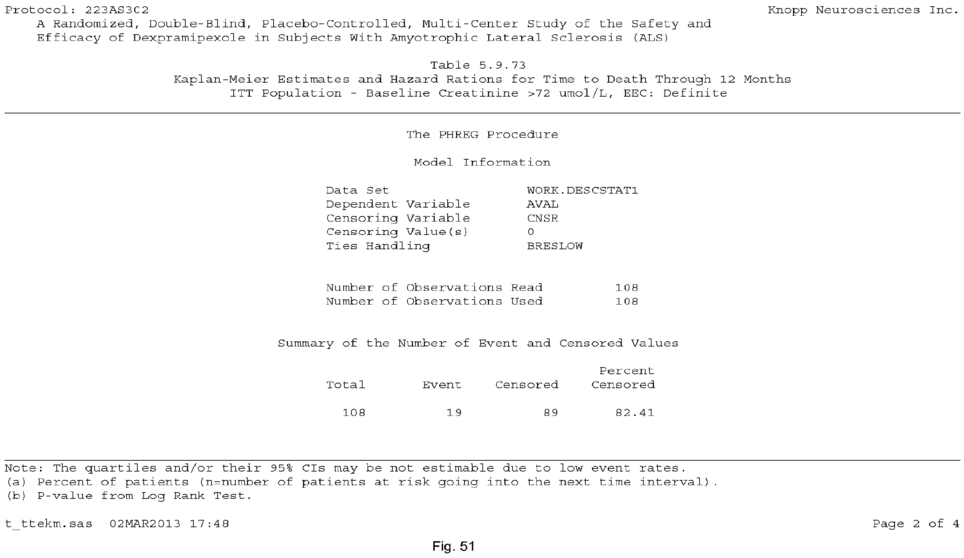

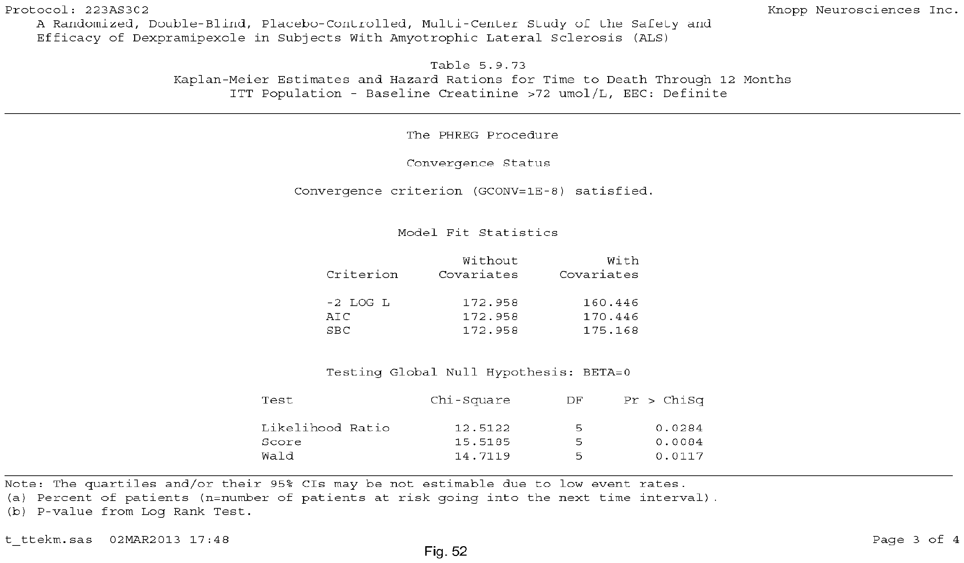

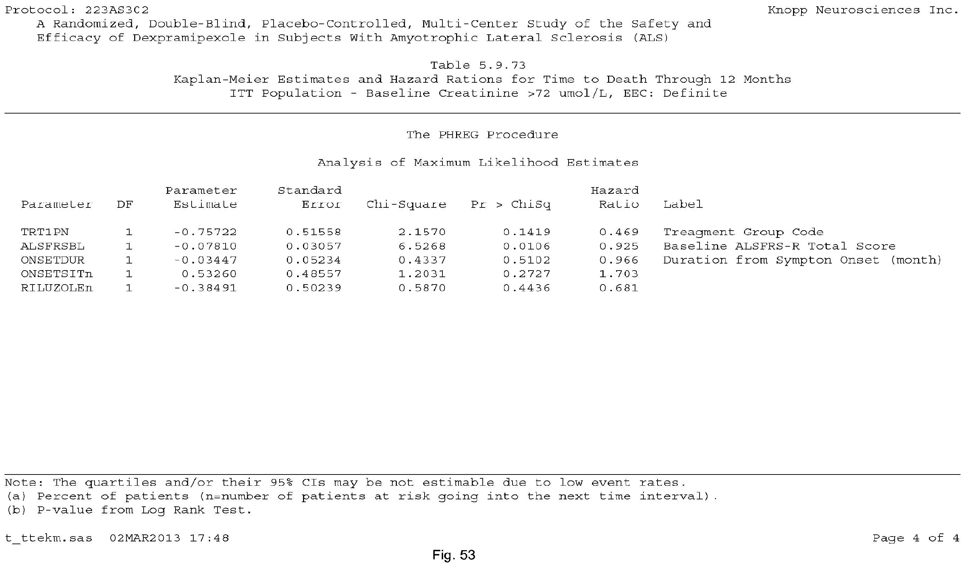

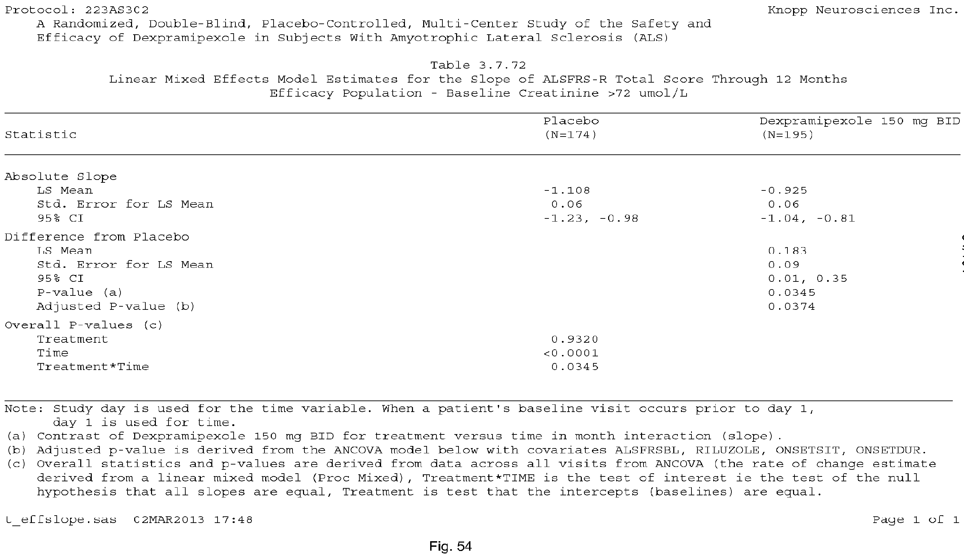

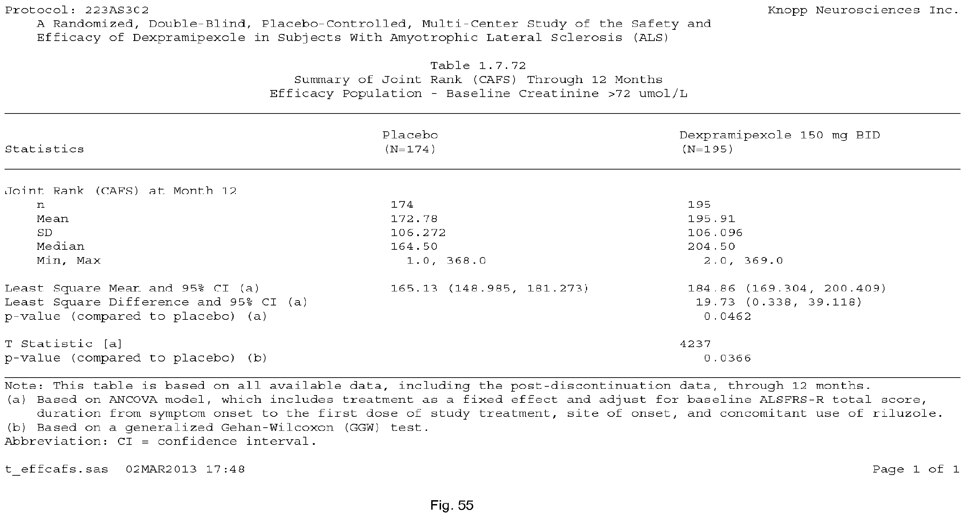

A subsequent statistical analysis (Macrostat Inc., Wilmington, DE) was directed to treatment effects of dexpramipexole in the above-identified high-creatinine patient group. In a combined assessment of function and survival (CAFS), the high-creatinine group demonstrated a statistically significant 19% improvement in least square means between treatment and placebo (184.86 vs. 165.13, respectively; p=0.0366). See Figure 55. In a separate analysis of the slope of decline on the ALS Functional Rating Scale (Revised), the high-creatinine group demonstrated a statistically significant 18% improvement in least square means between treatment and placebo (-0.925 vs. -1.108; p=0.0345). See Figure 54. In a Kaplan-Meier analysis of mortality, the high-creatinine group demonstrated a trend toward improved survival, as demonstrated by a 10.3% rate of mortality in the treatment group at 12 months vs. a 16.1% rate of mortality in the placebo group at 12 months (p=0.1207). See Figures 50-53.

The effects of dexpramipexole in simultaneously preserving serum creatinine levels and establishing improved outcomes for ALS subjects was further confirmed in an analysis of laboratory values and outcomes in a separate Phase 2 clinical study. This study has been reported to have shown dose-dependent benefits of twice-daily administration of dexpramipexole across both of the two parts of the study, in which 102 subjects were randomized in Part One to placebo or total daily doses of 50 mg, 150 mg, or 300 mg of dexpramipexole, after which, following a one-month placebo washout for all patients, subjects were randomized in Part Two to total daily doses of either 50 mg or 300 mg of dexpramipexole.

Treatment with dexpramipexole in both the Phase 2 and Phase 3 studies was associated with a reduction in the decline of serum creatinine levels as well as a reduction in the progression of disease.

Without wishing to be bound by theory, the use of serum creatinine as a biomarker to predict the treatment effects of dexpramipexole in patients with ALS may be related to either or both of at least two mechanisms. First, the biomarker may be based on creatinine as a marker of muscle loss, since levels of serum creatinine are known to be associated with the loss of muscle mass in human subjects. Second, the biomarker may also be a marker of the mechanistic effects of dexpramipexole at its molecular sites of action, including the mitochondria of either nerve cells or muscle cells or both.

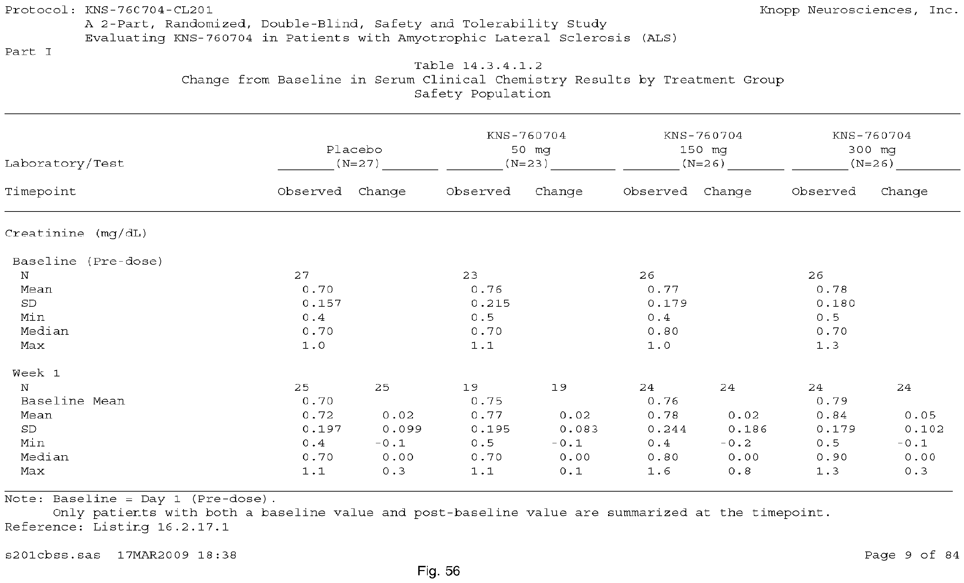

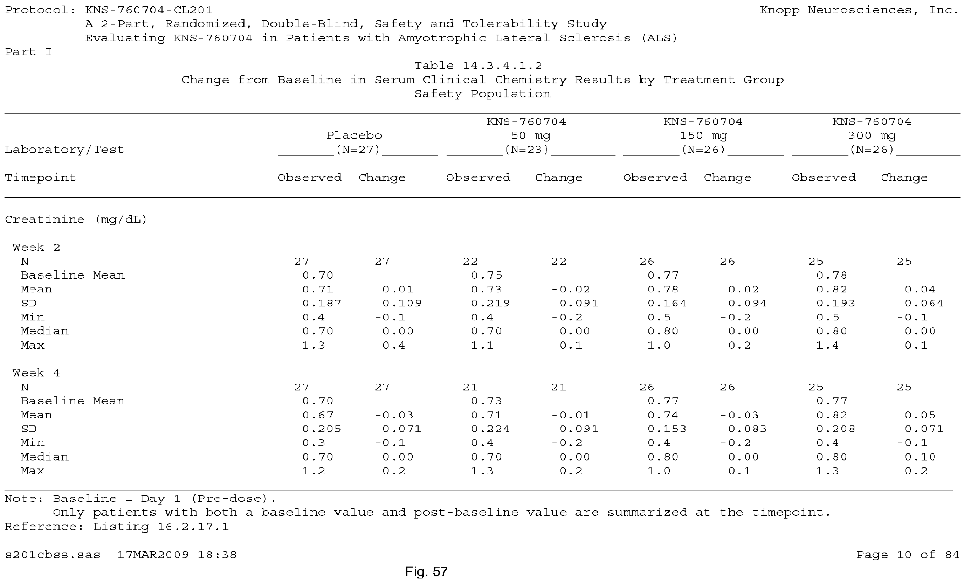

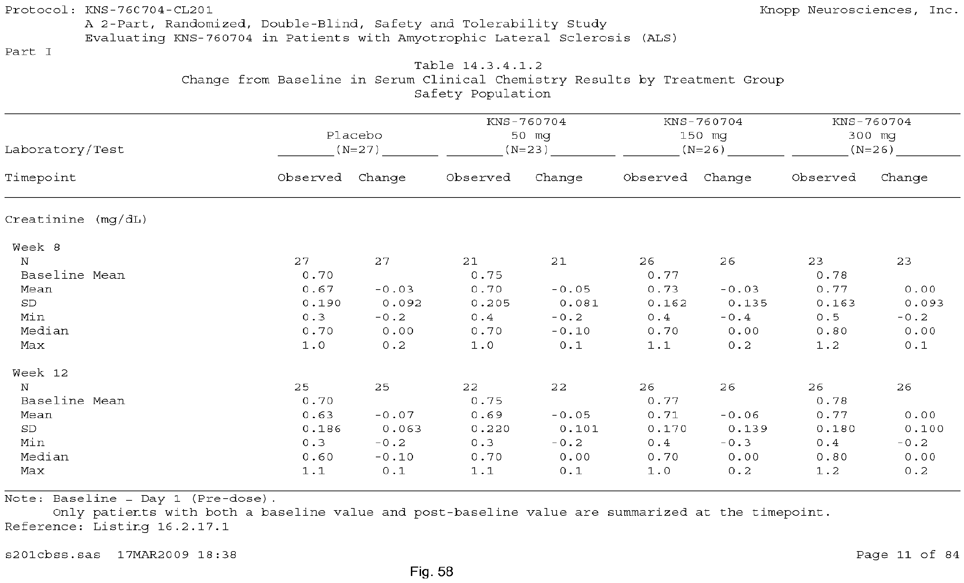

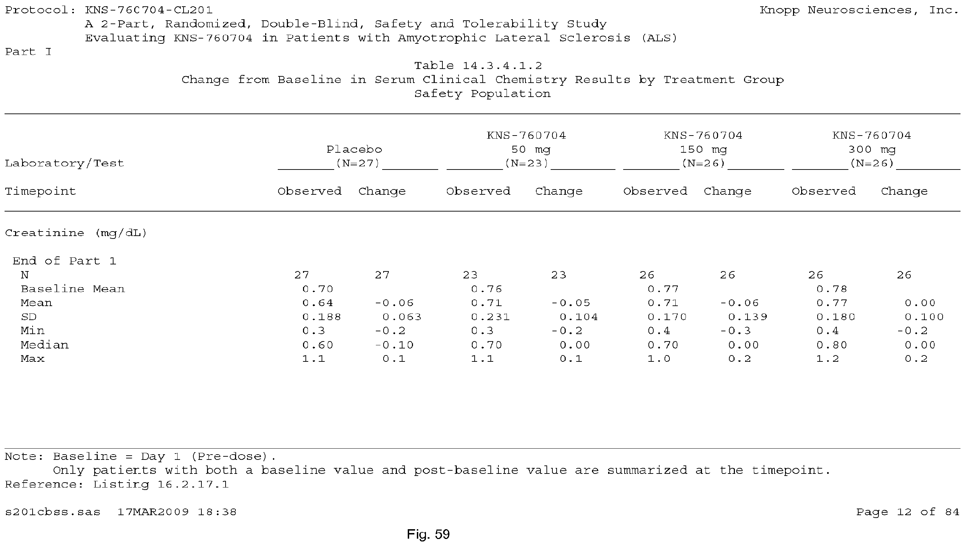

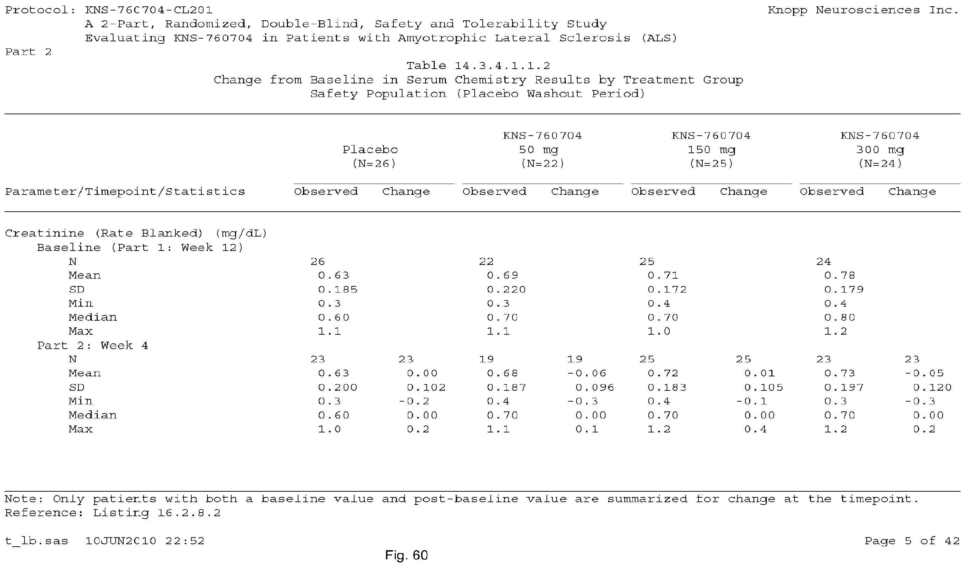

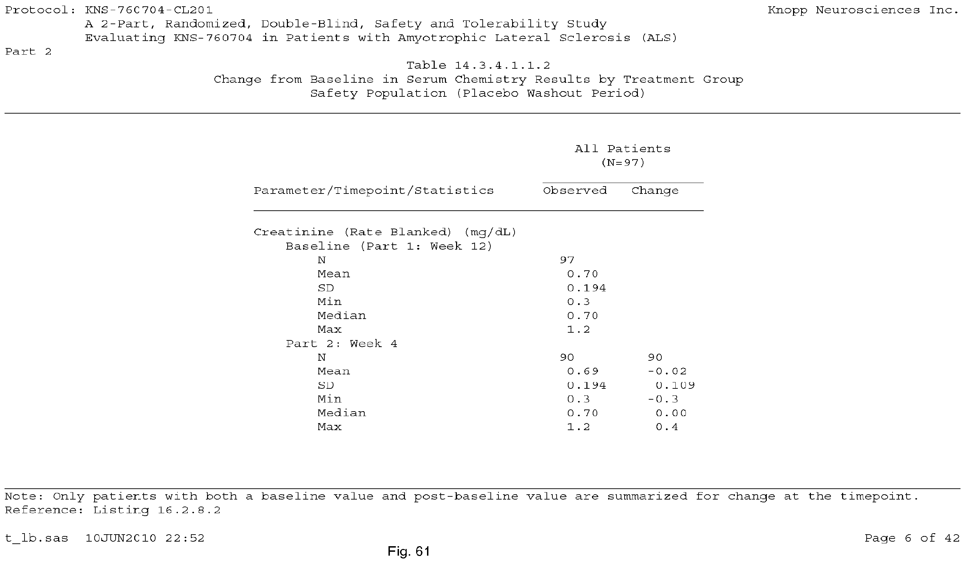

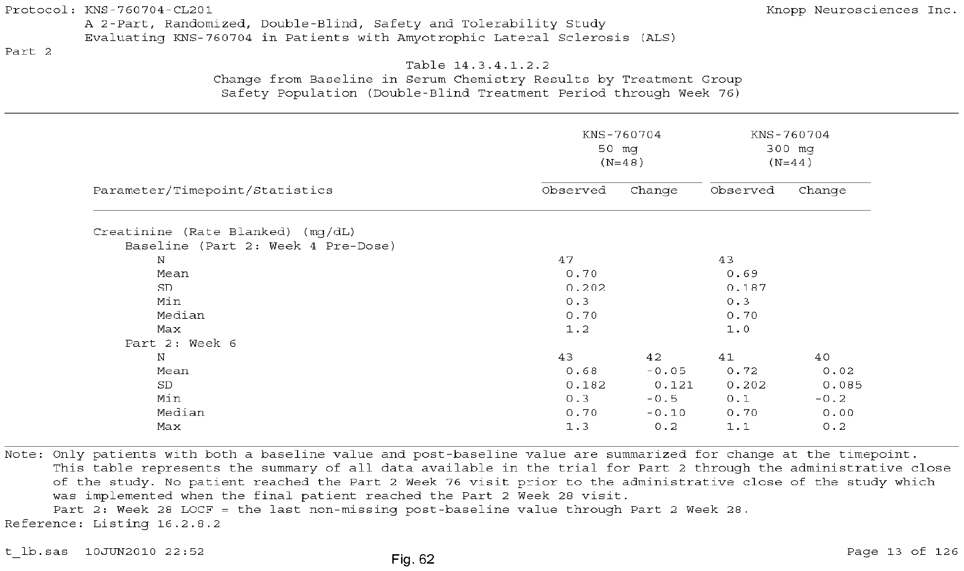

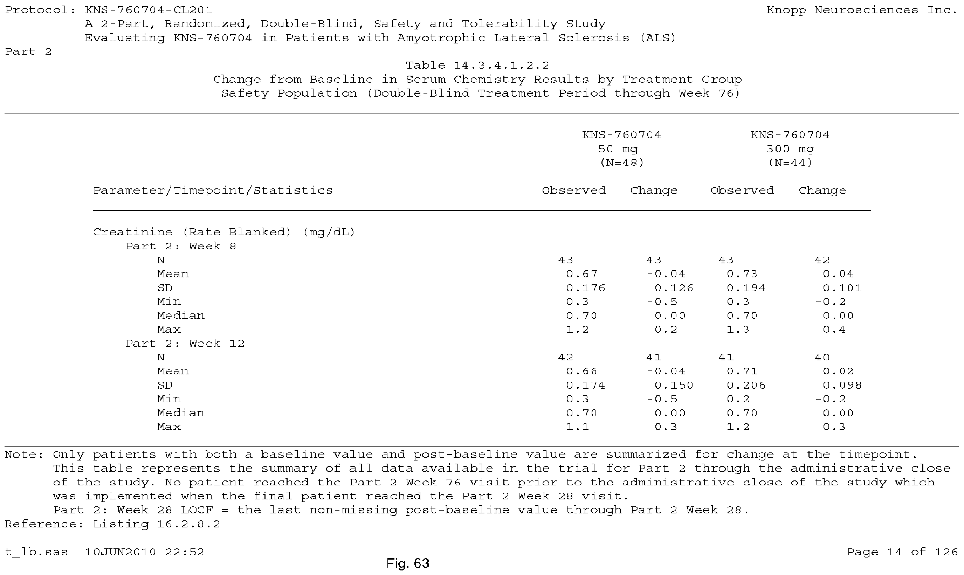

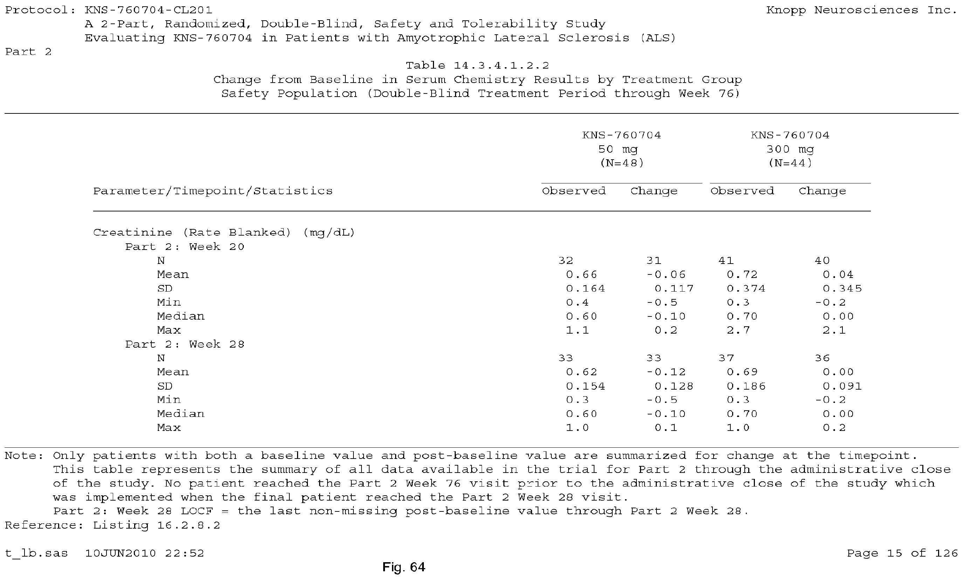

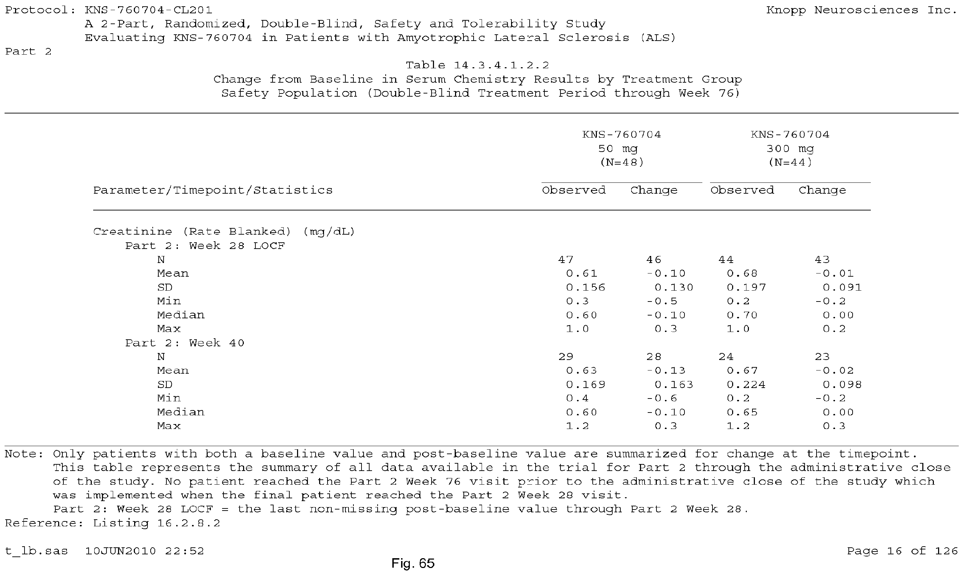

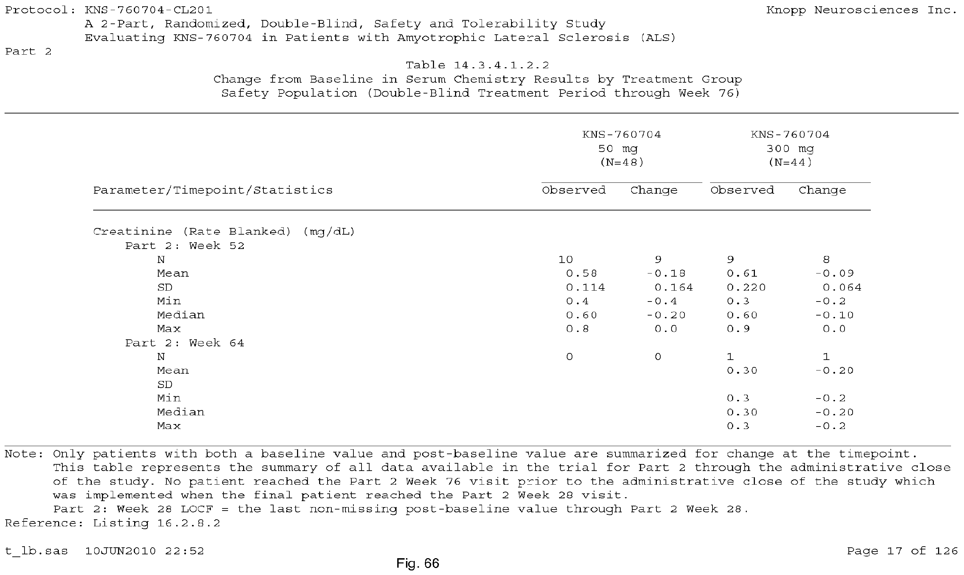

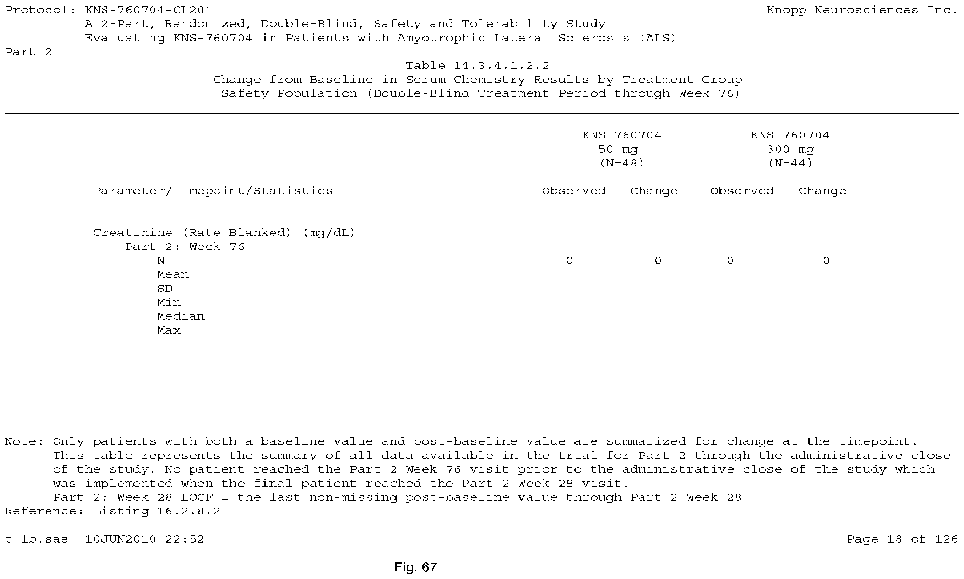

An analysis of the results of the EMPOWER study includes the Kaplan-Meier estimates and hazard ratios for time to death through twelve months for subjects with a baseline creatinine of greater than about 72 µmol/L is set forth in tables in Figures 50-53. Linear mixed effects model estimates for the slope of ALSFRS-R total score through twelve months for subjects with a baseline creatinine of greater than about 72 µmol/L is set forth in a table in Figure 54. A summary of joint rank (CAFS) through twelve months for subjects with a baseline creatinine of greater than about 72 µmol/L is set forth in a table in Figure 55. The change from baseline in serum creatinine results by treatment group is shown in tables in Figures 56-67.

The Monte Carlo analysis further seeks to identify subgroups of patients that exhibit strong "response" to treatment, as evidenced by comparing statistics of treated and untreated members of these subgroups. Response is defined according to two criteria:

1. reduction of disease symptom progression in treated patients as compared to placebo patients in the same subgroup;

2. reduction of end-of-study mortality in treated patients as compared to placebo patients in the same subgroup.

This analysis examined subgroup definitions based on both single and dual attribute criteria, and evaluated the performance of each subgroup with respect the two criteria above. To facilitate ranking of subgroups, a scoring metric aggregated these two-dimensional scores into a single scalar called "score." Larger scores are better: scores increase when mortality rates decrease, and when ALSFRS-R changes from baseline become more positive (i.e. score decreases are reduced). This scoring scheme serves to guide our maximization process and provide convenient rankings of subgroup quality.

4. Minimum subgroup size filters were imposed on results to exclude promising signals due to sampling artifacts, or which would be too narrow for consideration in future studies. Analyses were run for minimum group sizes of both 50 patients (data not shown) and 100 patients (i.e. for a cutoff of N, we require at least N patients in that subgroup for both the treated and untreated groups).

### Results

The Monte Carlo analysis identified numerous subgroups which show very large treatment effects with respect to both response criteria.

Subgroups identified with positive responses to treatment in the single-criterion Monte Carlo analysis included groups with any of the following baseline values: a pulse rate greater than 81 beats per minute, a creatinine value of greater than 72.0 µmol/L, a phosphorous value of less than or equal to 1.090 µmol/L, a cholesterol value of less than or equal to 5.3 mmol/L, a lactate dehydrogenase value of less than or equal to 161.0 U/L, a creatinine phosphokinase value of less than or equal to 184.0 U/L, a bicarbonate value of less than or equal to 21.6 mmol/L, a uric acid level of greater than 37.0 U/L.

### EXAMPLE 2 - IDENTIFICATION OF CLINICAL OUTCOMES AND CREATININE-SPARING EFFECT OF DEXPRAMIPEXOLE BASED ON SIGNIFICANT INTER STUDY DIFFERENCES IN THE PHASE 2 AND PHASE 3 (EMPOWER) CLINICAL TRIALS IN ALS

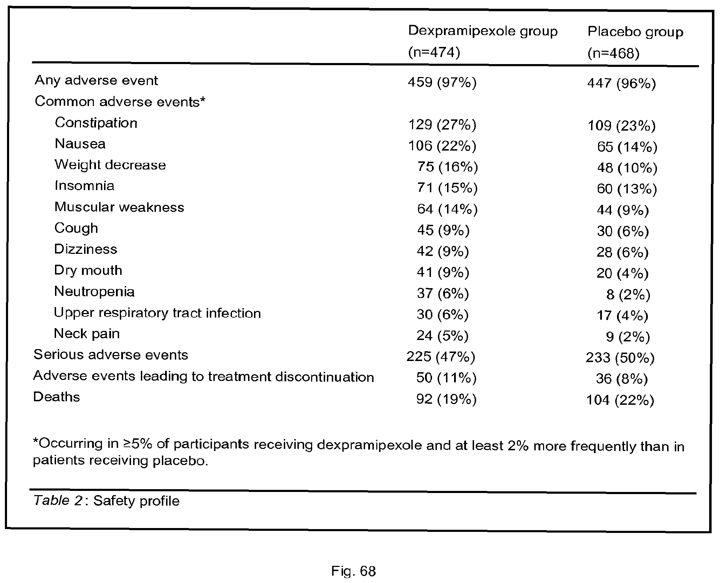

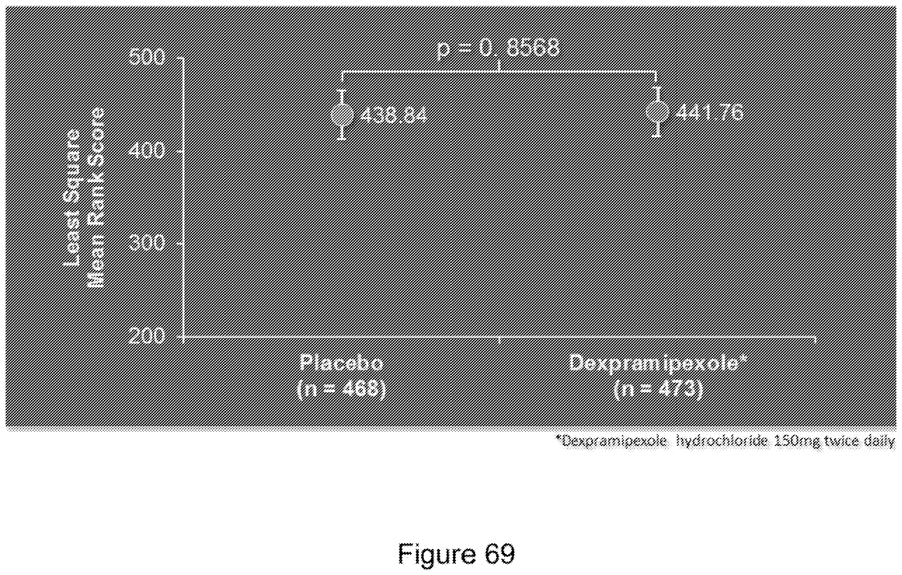

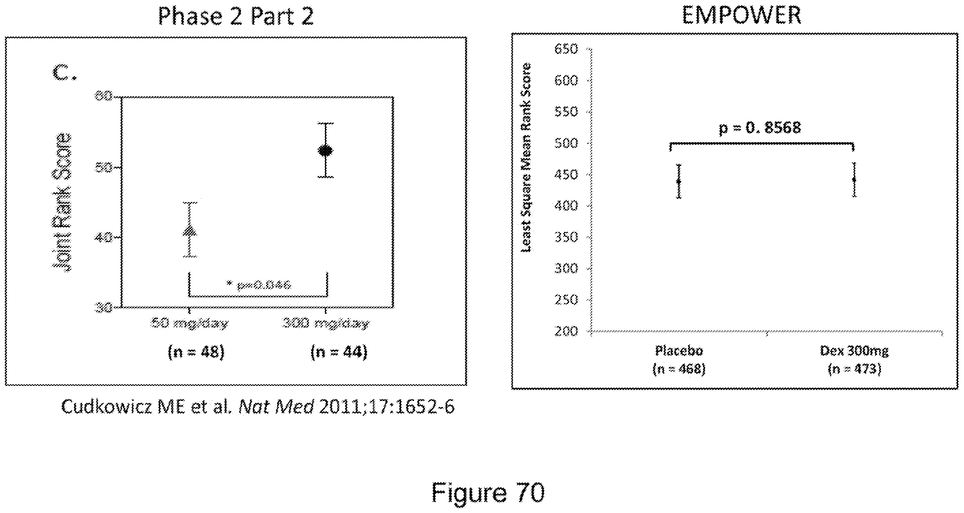

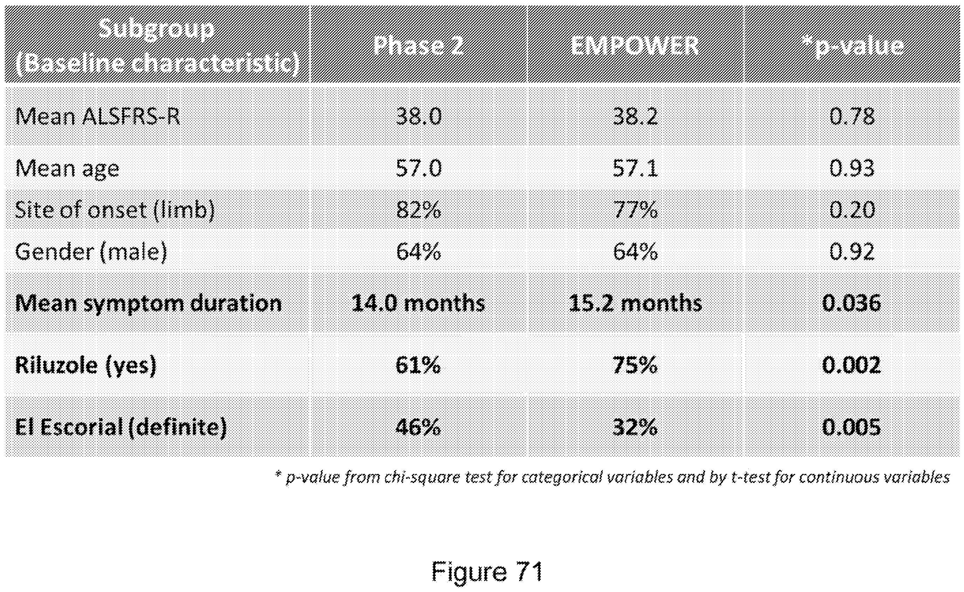

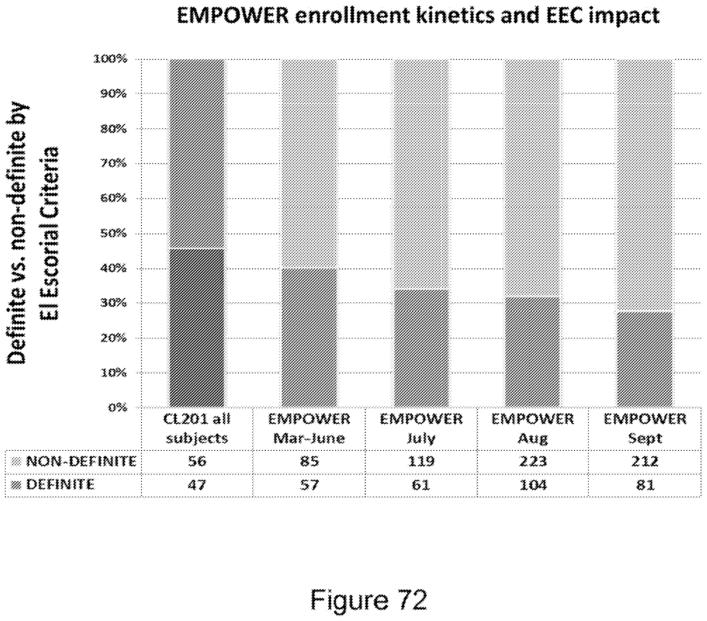

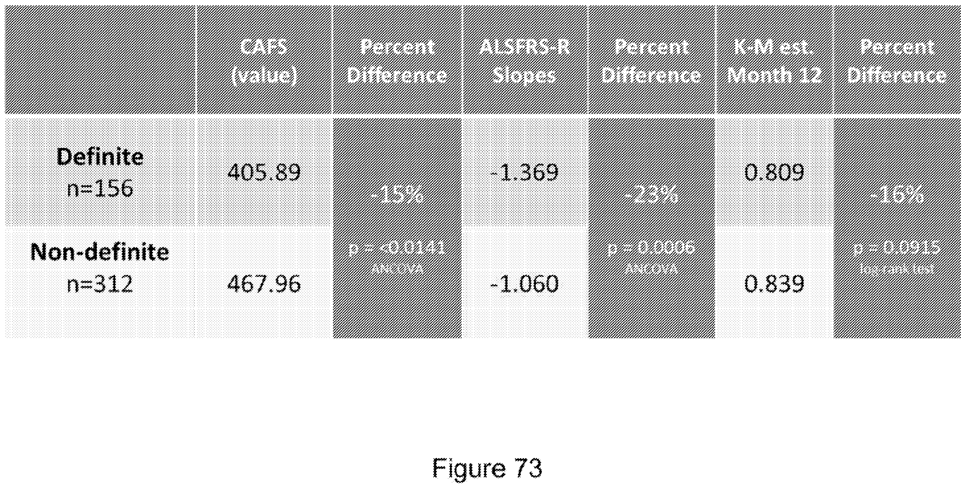

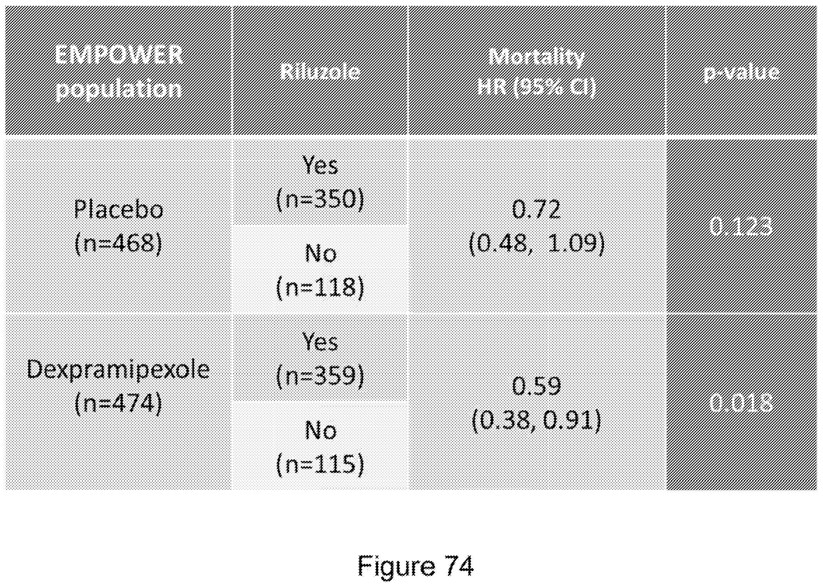

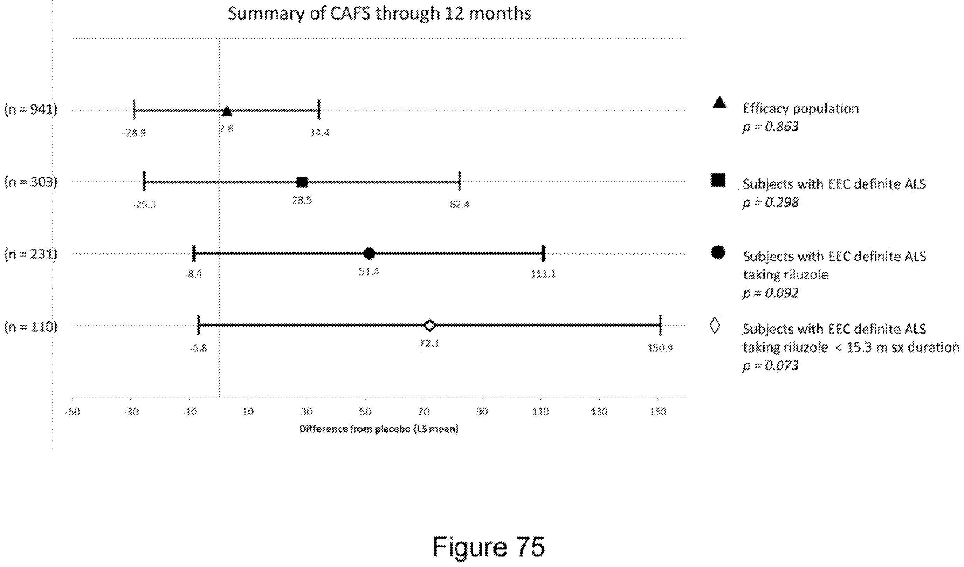

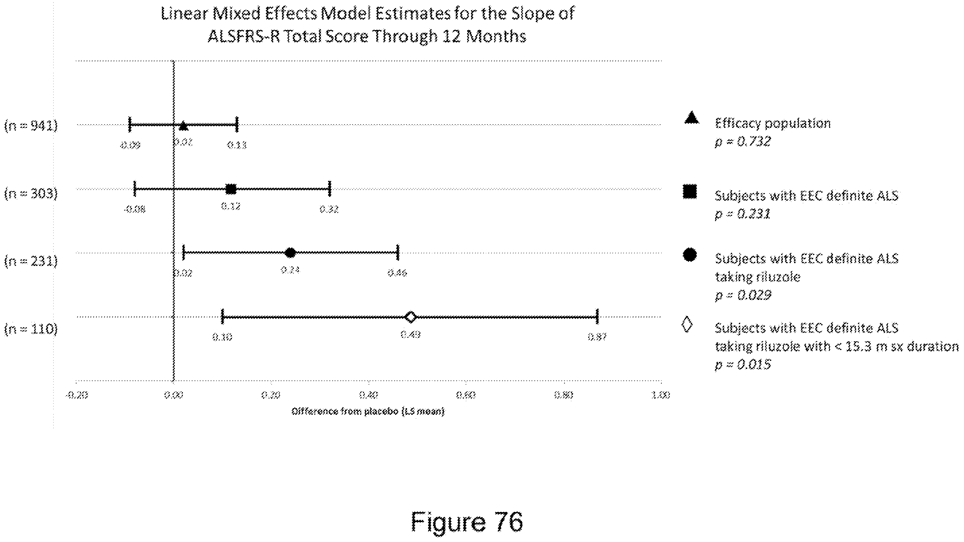

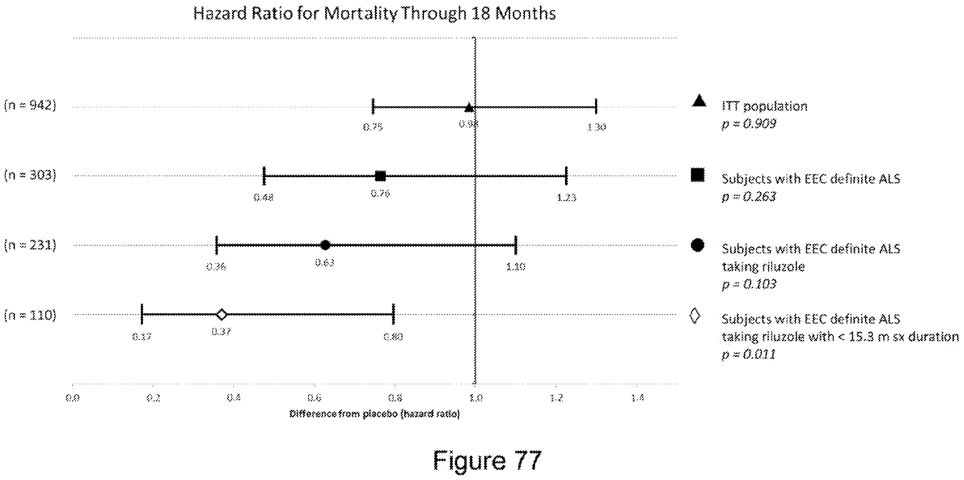

Dexpramipexole at 300 mg/day was well tolerated (Figure 68) but dexpramipexole 300 mg/day failed to show efficacy on any prespecified endpoint and had no significant effect over placebo in the EMPOWER study (Figure 69 and 70). A *post hoc* analysis of EMPOWER subgroups revealed that there are significant inter-study differences despite nearly identical enrollment criteria (Figure 71) and that enrollment kinetics led to milder EMPOWER disease severity (Figure 72). The study revealed that El Escorial definite EMPOWER placebo subjects were fast decliners (Figure 73) and that the apparent riluzole benefit increased in the dexpramipexole population versus the placebo population (Figure 74). The effect of dexpramipexole was increased on background of riluzole and with shorter symptom duration (Figure 75) and that the effect increased on background of riluzole and with shorter symptom duration (Figure 76). A similar finding was observed with the effect of dexpramipexole on hazard for mortality in EEC definite versus efficacy population where the Effect increased on background of riluzole and with shorter symptom duration (Figure 77).

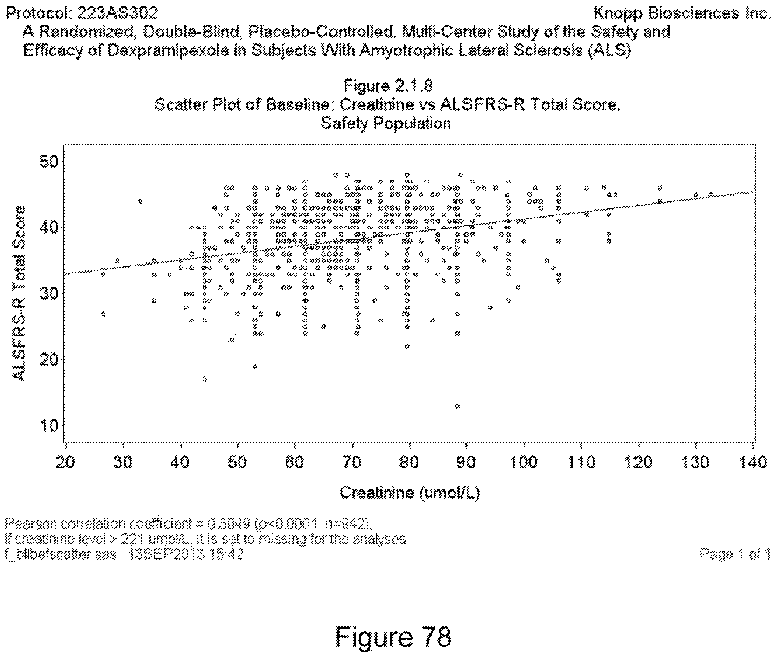

Data from the EMPOWER study demonstrates that reduced creatinine levels are seen in patients with more advanced ALS (with greater loss of function) as can be seen in the scatter plot (Figure 78 - baseline: creatinine vs. ALSFRS-R total score in the safety population). These data are consistent with previous findings and support the concept of creatinine as a candidate biomarker.

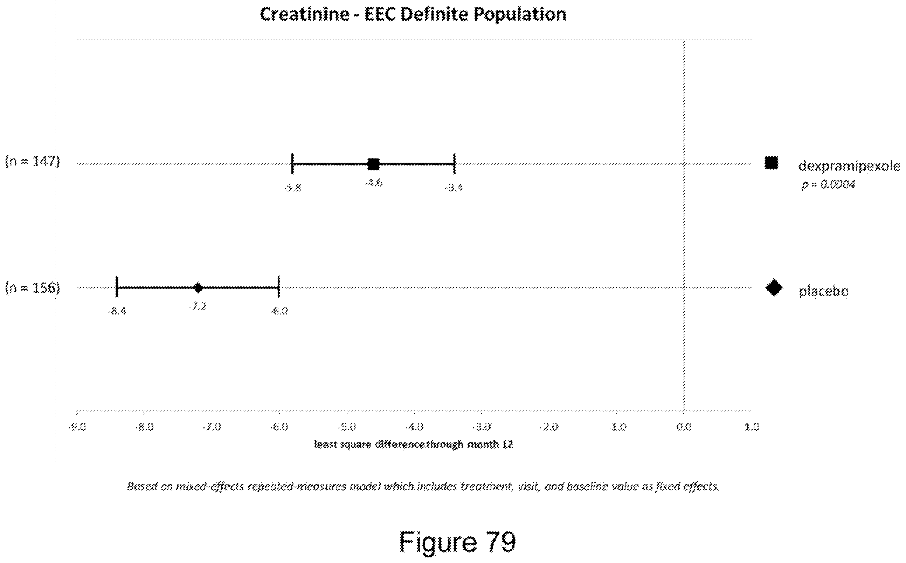

Among EEC definite ALS subjects, treatment with dexpramipexole significantly reduced the loss of creatinine over the course of the study. Figure 79 is a *post hoc* analysis of the dexpramipexole effects on time average difference change in creatinine performed using a time averaged difference method for the difference from start to finish in change from baseline in creatinine in the two groups. No therapeutic agent used for ALS has ever shown a creatinine sparing effect prior to this finding and in this subgroup it correlates with an improved clinical outcome. The observed creatinine effect may not simply be a reflection of muscle mass preservation because weight loss (fat or muscle, visceral or subcutaneous) is greater in dexpramipexole treated subjects compared to placebo. Additionally, dexpramipexole may improve clinical outcomes in ALS subjects by preserving creatinine relative to placebo through a mechanism that is yet to be determined but that is independent of weight loss per se.

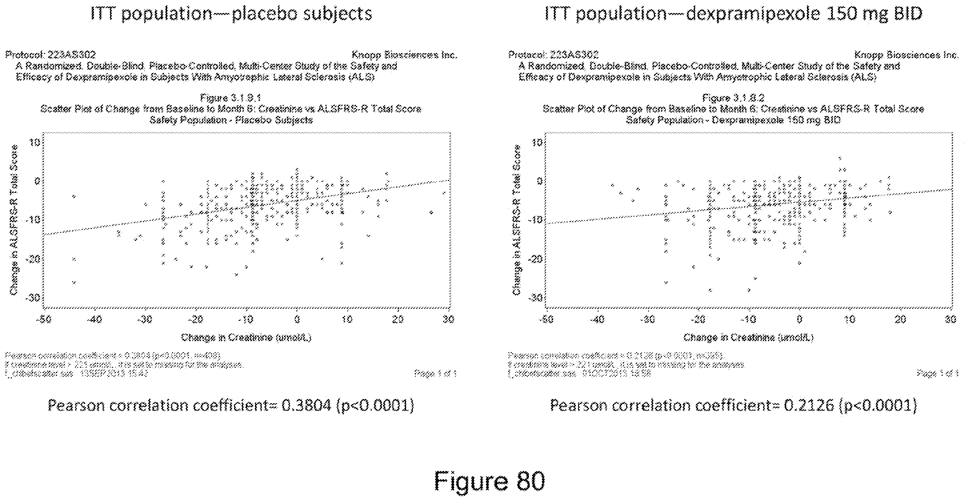

Loss of creatinine has a lower correlation to reduction in ALSFRS-R score (i.e., loss of function) in dexpramipexole treated patients compared to placebo. This is consistent with the findings in Figure 79 and dexpramipexole treatment appears to partially "de-link" creatinine loss to disease progression in ALS. However, the disease may progress despite relative creatinine sparing following dexpramipexole treatment suggesting that dex's effects on creatinine at least at a dose of 300 mg/day only partially modify disease progression. Figure 80 shows scatter plots of change from baseline to month 6 (creatinine vs. ALSFRS-R total score; safety population) and depicts the dexpramipexole effect on correlation of change in creatinine to change in ALSFRS-R score in placebo subjects and subjects receiving 150 mg BID from the ITT population.

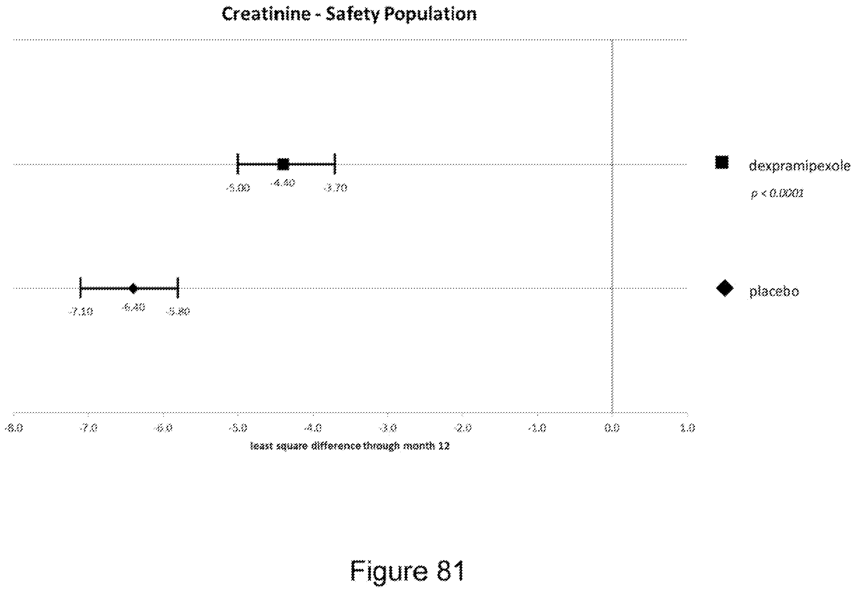

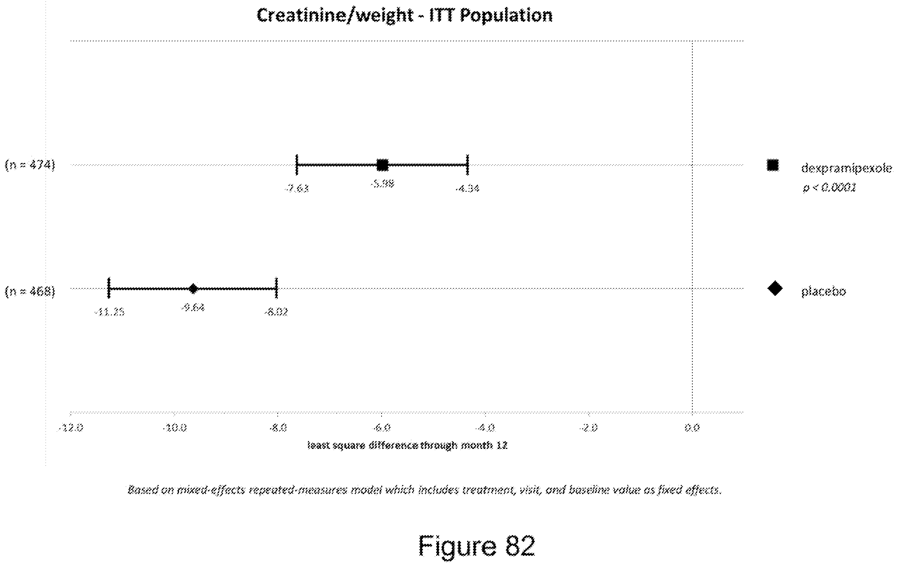

Using the time averaged difference (TAD) method (versus a slopes method to estimate end of study creatinine change from baseline where the effect was not significant), dexpramipexole has a significant effect on preserving creatinine in the total EMPOWER
(intent to treat) population. Since there was no significant clinical effect of dexpramipexole in the ITT group, this finding could suggest the implications from Figured 79 and 80 are spurious as the TAD method is overly sensitive and therefore a false positive predictor of clinical benefit of dex, or that TAD creatinine sparing by dexpramipexole is a leading indicator of potential clinical benefit since when TAD increases in magnitude (as in Figure 79) there is a significant benefit by the slopes method for end of study creatinine change that correlate with a clinically significant benefit. In addition, there was a directional (but not significant) clinical benefit of dexpramipexole in the ITT population. Figure 81 shows the creatinine-sparing effects of dexpramipexole observed in full ITT population and the magnitude of effect smaller than in EEC definite subgroup but still significant. In addition, Figure 82 demonstrates that the creatinine-sparing effects of dexpramipexole are not a passive marker of weight preservation.

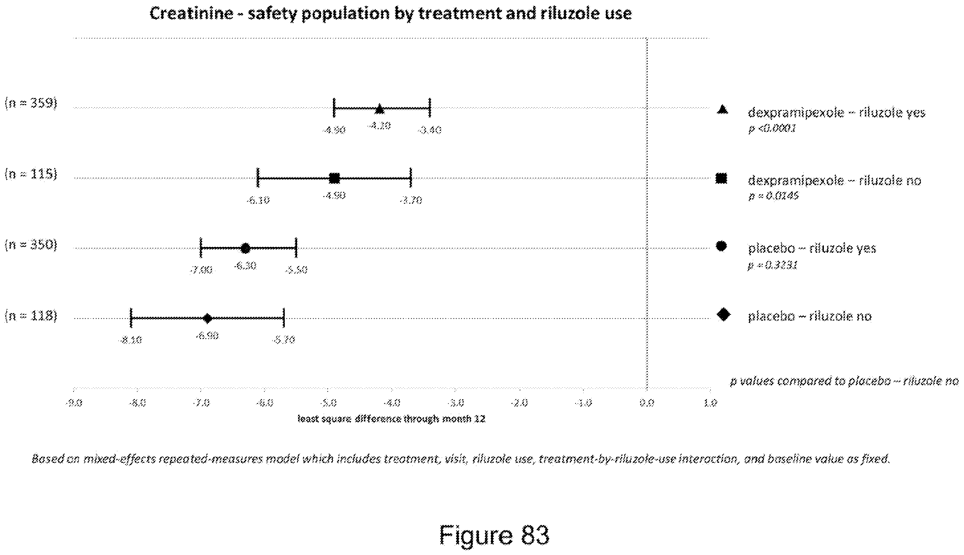

Riluzole produces directional but not significant creatinine sparing (Figure 83). In the presence of dex, this benefit is additive. Since riluzole produces clinical benefit in the absence of a creatinine effect, and since the creatinine sparing effects of dexpramipexole appear to be additive and highly significant with concomitant riluzole treatment, and since this is the subgroup (dexpramipexole + riluzole) with the greatest clinical benefit in some patients, it suggests the potential for dexpramipexole plus riluzole combination use through an additive (or potentially but less likely, synergistic) biochemical mechanism. It also supports the continued study of the drug combination in ALS subjects to improve clinical outcomes. Figure 82 shows the creatinine-sparing effects of dexpramipexole not a passive marker of weight preservation and the effect adjusted for weight by taking ratio of creatinine to weight per visit.

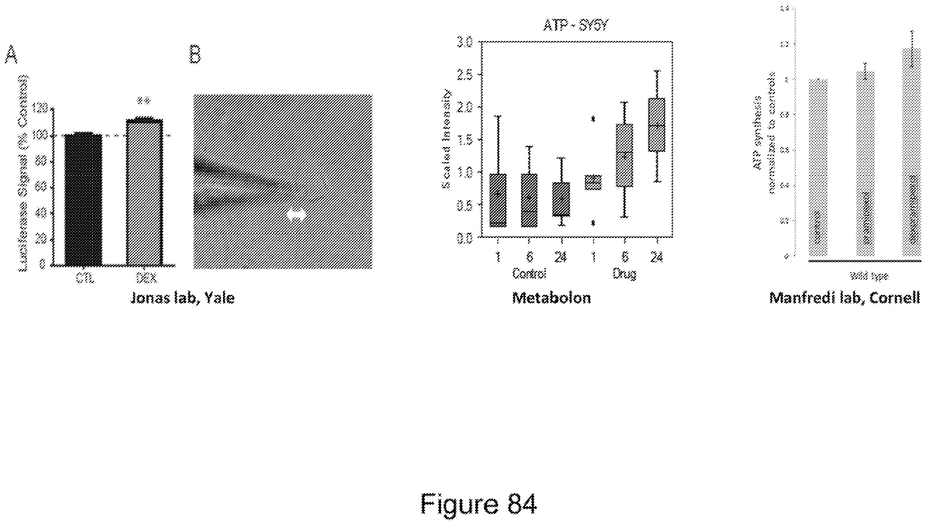

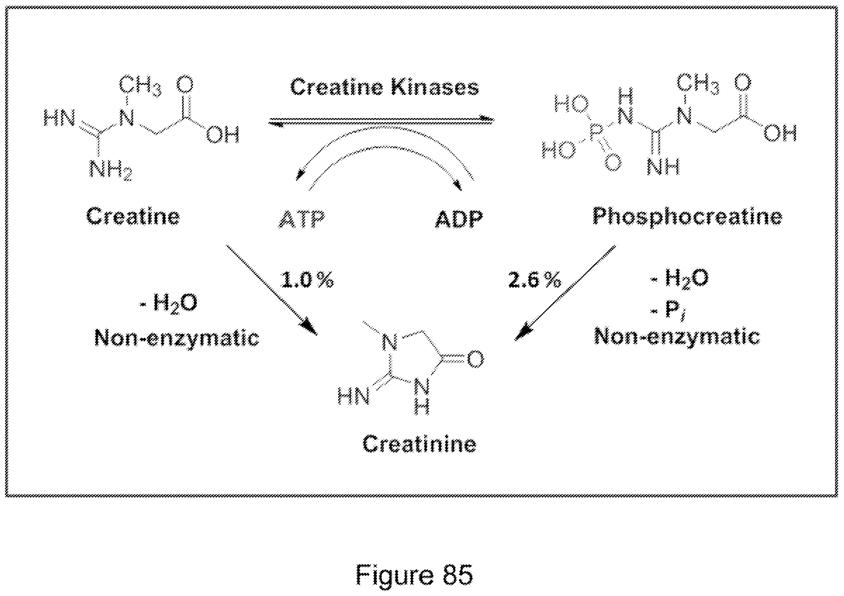

Dexpramipexole may increase ATP synthesis (Figure 84). dexpramipexole is known to increase ATP synthesis, and one potential consequence could be to drive the equilibrium reaction of creatine (Cr) to phosphocreatine (PCr) synthesis to the right as can be seen in Figure 85. If the ratio of PCr/Cr in the pool of total body creatine is increased, then creatinine levels should also increase since PCr is spontaneously and non-enzymatically degraded to creatinine at a rate 2.6 times faster than the Cr to creatinine degradation. This could account for the creatinine sparing effect of dexpramipexole in ALS subjects. Also, there are known energy deficiencies in ALS subjects that include a deficiency in Cr/PCr metabolism that may contribute to disease phenotype and progression. This mechanism then could also potentially account for the clinical benefit of dexpramipexole.

### EXAMPLE 3 - EMPOWER SUBJECTS WITH EL ESCORIAL DEFINITE ALS EXHIBITED SIGNIFICANT BASELINE DIFFERENCES INDEPENDENT OF DISEASE DURATION AND EXPERIENCED SIGNIFICANTLY WORSE OUTCOMES

### Introduction

The heterogeneity of amyotrophic lateral sclerosis (ALS) presents challenges for both clinical management and clinical research. We investigated whether the El Escorial Criteria (EEC) classification system presents an opportunity to discriminate ALS subpopulation phenotypes. A baseline diagnosis of EEC-definite ALS, involving clinical disease detection in 3 or 4 out of 4 possible regions, has repeatedly been associated with worse outcomes. However, it remains uncertain in what proportion EEC-definite ALS represents the effect of time on disease progression or a surrogate for a disease subtype associated with diffuse presentation.

We sought to characterize EEC-definite ALS patients at presentation and overtime by retrospectively analyzing data sets from the largest clinical trial ever conducted in ALS, the Phase 3 trial of dexpramipexole in ALS (EMPOWER, n=943). We tested the hypothesis that EEC-definite subjects exhibited distinct disease characteristics, including metabolic parameters, independent of disease duration. Methods

EMPOWER subjects with a baseline classification of EEC-definite ALS (n=303) were compared to a group compromising subjects with possible, probable, and probable-laboratory supported ALS (EEC non-definite ALS, n=639). Baseline demographics, ALS history, clinical laboratory values, and vital signs were compared using a two-sample t-test for continuous variables and chi square test for categorical variables.

To assess group differences in disease progression while eliminating any potential role of dexpramipexole investigational treatment, placebo-treated subjects (n=468) were grouped by baseline classification of EEC-definite (n=156) or EEC non-definite (n=312) ALS. The mortality difference through 18 months was tested by hazard ratio from the Cox proportional hazards model, with Kaplan-Meier estimates of death rate presented at 12 months. For other clinical outcomes, the slope of decline through 12 months was estimated using a linear mixed-effects slopes model. For lab tests and vital signs, changes from baseline to month 12 were estimated by a mixed effects repeated measures model.

### Results

Significant differences in numerous parameters characterized EEC-definite subjects entering the EMPOWER study (Table 5). EEC-definite subjects were significantly younger and more female than subjects classified as EEC-not definite. The mean pre-study progression rate, as measured by ALSFRS-R, was significantly greater for EEC-definite subjects, as was disease severity as measured by baseline ALFRS-R, ALSAQ-5, and pulmonary function. All four ALSFRS-R domains were lower in the EEC-definite group, with fine motor most reduced. Yet the duration of symptoms was nearly identical between groups. No significant differences existed in riluzole use or frequency of bulbar-onset disease (data not shown).

**Table 5 - Notable baseline differences, EEC-definite vs. EEC-not definite groups**

| **Baseline means** | **EEC non-definite n=639** | **EEC definite n=303** | **Δ not - definite to definite** | **p=** |
|---|---|---|---|---|
| **Demographics** | | | | |
| Age (years) | 57.66 | 55.79 | -3.2% | 0.018 |
| Female study subjects | 33.3% | 40.9% | 22.8% | 0.024 |

| **ALS history** | | | | |
|---|---|---|---|---|
| Symptom duration (months) | 15.16 | 15.35 | 1.2% | 0.625 |
| Pre-study progression rate | 0.705 | 0.801 | 123.6% | 0.016 |
| ALSAQ-5 (higher is worse) | 29.0 | 35.6 | 22.8% | <0.001 |
| ALSFRS-R total score | 38.9 | 36.7 | -5.47% | <0.001 |
| Fine motor domain | 8.8 | 8.0 | -9.1% | <0.001 |
| Bulbar domain | 10.4 | 9.8 | -5.8% | <0.001 |
| Gross motor domain | 8.4 | 7.8 | -7.1% | 0.003 |
| Respiratory domain | 11.3 | 11.2 | -0.9% | 0.157 |
| % predicted slow vital capacity | 90.4 | 85.6 | -5.3% | <0.001 |

| **Select Tabs** | | | | |
|---|---|---|---|---|
| Creatinine (µmol/L) | 71.321 | 68.249 | -4.1% | 0.010 |
| Cholesterol (mmol/L) | 5.624 | 5.492 | -2.3% | 0.080 |
| Bicarbonate (mmol/L | 23.579 | 23.299 | -1.2% | 0.115 |

| **Select vital signs** | | | | |
|---|---|---|---|---|
| Diastolic BP (mmHg) | 79.419 | 82.238 | 3.5% | <0.001 |
| Systolic BP (mmHg) | 127.670 | 129.399 | 1.4% | 0.110 |
| Pulse rate I (bpm) | 74.014 | 75.865 | 2.5% | 0.029 |

Three clinical laboratory parameters previously associated with disease outcomes (creatinine, cholesterol, and bicarbonate) also differed between the groups, although only creatinine, a measure of change in muscle mass and of metabolic activity, was significantly different. An intriguing difference was noted in certain baseline vital signs, i.e., significantly elevated pulse rate and diastolic BP and a trend toward higher systolic BP in the EEC-definite group. Modest hypertension has been previously reported in ALS, including in early disease, with speculation of associated autonomic imbalance and hypoxia. To our knowledge these cardiac parameters, which may also be associated with hypermetabolism noted in ALS, have not been previously associated with disease severity as measured by EEC.

Within the EMPOWER placebo group, EEC-definite ALS patients had significantly worse 12-month outcomes (Table 6). The difference in a Combined Assessment of Function and Survival was highly numerically and statistically significant, with the difference driven more by functional decline than mortality. Disease progression was steeper when measured by ALSAQ-5 than by ALSFRS-R.

Creatinine and bicarbonate showed significantly greater declines from baseline in EEC-definite subjects. Cardiac vitals with significant differences at baseline showed only trending differences over 12 months, suggesting any effect in discriminating EEC-definite ALS occurs in early disease.

**Table 6 - Differences in select study outcomes, EEC-definite vs. EEC-not definite groups**

| **Mean changes** | **EEC non-definite N+312** | **EEC definite N=156** | **101Δ not- definite to definite** | **P =** |
|---|---|---|---|---|
| **Outcome measures** | | | | |
| Combined assessment function+survival | 502.1 | 398.4 | -20.7% | <0.001 |
| ALSFRS-R slope | -1.060 | -1.369 | 29.2% | <0.001 |
| Kaplan-Myer mortality analysis | 0.161 | 0.191 | 18.6% | 0.093 |
| ALSAQ-5 slope (higher is worse) | 1.488 | 2.153 | 44.7% | <0.001 |
| % predicted slow vital capacity | -2.456 | -3.148 | 28.2% | 0.004 |

| **Select labs** | | | | |
|---|---|---|---|---|
| Creatinine (µmol/L) | -12.5 | -16.5 | -32.0% | 0.007 |
| Cholesterol (mmol/L) | -0.148 | -0.213 | 43.9% | 0.448 |
| Bicarbonate (mmol/L) | 0.50 | 1.44 | 188.0% | 0.008 |
| **Select vital signs** | | | | |

| Diastolic BP (mmHg) | -1.4 | 0.6 | -142.9% | 0.062 |
|---|---|---|---|---|
| Systolic BP (mmHg) | -2.4 | -3.5 | 45.8% | 0.442 |
| Pulse rate (bpm) | 4.7 | 7.5 | 59.6% | 0.038 |

### Conclusions

In the largest ALS clinical trial ever conducted, EEC-definite ALS was discriminated from non-definite ALS by age, gender, measures of disease severity, and certain laboratory parameters linked to metabolic function, but not discriminated by disease duration. This suggests that a diagnosis of EEC-definite ALS is not solely or even primarily a function of progression over time. Moreover, baseline EEC-definite ALS was highly predictive of faster progression on multiple outcome measures and of increased creatinine loss. With its diffuse presentation, EEC-definite ALS may represent an especially aggressive ALS phenotype. This analysis strengthens the relevance of the El Escorial Criteria in both clinical management and clinical research, including the potential for population enrichment in a disease whose heterogeneity continues to confound clinical trials.

### EXAMPLE 4 - SAFETY OF DEXPRAMIPEXOLE FOR THE TREATMENT OF ALS: RESULTS FROM THE RANDOMIZED, DOUBLE-BLIND, PLACEBO-CONTROLLED STUDY EMPOWER

### Introduction

In a two-part phase 2 study, dexpramipexole (25-150 mg twice daily) was well tolerated for ≤ 9 months, with dose-dependent trends in slowing the rate of functional decline and in reducing mortality in amyotrophic lateral sclerosis (ALS). EMPOWER was an international, prospective, randomized, double-blind, placebo-controlled phase 3 trial of dexpramipexole in familial or sporadic ALS.
To evaluate the safety of dexpramipexole in the EMPOWER phase 3 study of patients with ALS and determine if dose-limiting toxicities were present.

### Methods

EMPOWER randomized 942 subjects with familial or sporadic ALS to treatment with dexpramipexole or placebo. Patients (18-80 years) with symptom onset ≤ 24 months before randomization, slow vital capacity ≥65% of predicted, and no history of or current neutropenia were included. Patients were randomized 1:1 to dexpramipexole 150 mg or placebo twice daily for 12 to 18 months. Patients on a stable dose of riluzole for ≥ 60 days
before study start and those not taking riluzole were eligible. Safety was evaluated by monitoring adverse events and laboratory evaluations throughout the study.

### Results

The safety of dexpramipexole observed in EMPOWER was similar to previous studies. As shown in Table 7, most subjects in the dexpramipexole and placebo treated groups reported an AE (97% dexpramipexole, 96% placebo). Similar incidences of AEs were observed for both treatment groups with respect to AEs considered moderate or severe (81% dexpramipexole, 80% placebo), AEs considered severe (39% dexpramipexole, 40% placebo), serious adverse events (SAEs) (47% dexpramipexole, 50% placebo), subjects who discontinued study treatment due to an AE (11% dexpramipexole, 8% placebo), and subjects withdrawing from the study due to an AE (13% dexpramipexole, 13% placebo). More subjects treated with dexpramipexole experienced AEs related to study treatment (46%) than those who were treated with placebo (32%).

**Table 7 - Analysis of Treatment-Emergent Adverse Events**

| Adverse Events | Placebo | Dexpramipexole 150 mg twice daily |
|---|---|---|
| Subjects in safety population (%) | 468 (100) | 474 (100) |
| Subjects with an event (%) | 447 (96) | 459 (97) |
| Subjects with a moderate or severe event (%) | 373 (80) | 386 (81) |
| Subjects with a severe event (%) | 189 (40) | 187 (39) |
| Subjects with a related event (%) | 150 (32) | 220 (46) |
| Subjects with a serious event (%) | 233 (50) | 225 (47) |
| Subjects who died (%) | 104 (22) | 92 (19) |
| Subjects discontinuing study treatment due to an event (%) | 36 (8) | 50 (11) |
| Subjects withdrawing from study due to an event (%) | 59 (13) | 63 (13) |

As shown in Table 8, the most common adverse events (at least 5% incidence and 2% more than placebo) were constipation, nausea, weight decreased; insomnia, muscular weakness, cough, dizziness, dry mouth, neutropenia, upper respiratory tract infection, and neck pain.

**Table 8 - Incidence of Most Common Adverse Events**

| Adverse Events | Placebo | Dexpramipexole 150 mg twice daily |
|---|---|---|
| Subjects in safety population (%) | 468 (100) | 474 (100) |
| Subjects with an event (%) | 253 (54) | 328 (69) |
| CONSTIPATION | 109 (23) | 129 (27) |
| NAUSEA | 65 (14) | 106 (22) |
| WEIGHT DECREASED | 48 (10) | 75 (16) |
| INSOMNIA | 60 (13) | 71 (15) |
| MUSCULAR WEAKNESS | 44 (9) | 64 (14) |
| COUGH | 30 (6) | 45 (9) |
| DIZZINESS | 28 (6) | 42 (9) |
| DRY MOUTH | 20 (4) | 41 (9) |
| NEUTROPENIA | 8 (2) | 37 (8) |
| UPPER RESPIRATORY TRACT INFECTION | 17 (4) | 30 (6) |
| NECK PAIN | 9 (2) | 24 (5) |

Consistent with previous reports, the incidence of neutropenia was higher in participants treated with dexpramipexole (8%) compared with those who received placebo (2%), as shown in Table 9.

**Table 9 - Incidence of Neutropenia Defined by Laboratory Value**

| Adverse Events | Placebo | Dexpramipexole 150 mg twice daily |
|---|---|---|
| Subjects in safety population | 468 | 474 |
| Subjects with at least one post-baseline value (%) | 468 (100) | 473 (100) |
| Subjects whose ANC < 2.0 x 10^{9/}L (%) | 35 (7) | 76 (16) |
| Grade 1: ANC >=1.5 - < 2.0 x 10^{9/}L (%) | 27 (6) | 47 (10) |
| Grade 2: ANC >=1.0 - < 1.5 x 10^{9/}L (%) | 7 (1) | 13 (3) |
| Grade 3: ANC >=0.5 - < 1.0 x 10^{9/}L (%) | 0 | 5 (1) |
| Grade 4: ANC < 0.5 x 10^{9/}L (%) | 1 (<1) | 11 (2) |
| Subjects whose ANC < 1.5 x 10^{9/}L (neutropenia) (%) | 8 (2) | 29 (6) |

The incidence of deaths in the treatment groups was similar: 19% of subjects in the dexpramipexole group and 22% of subjects in the placebo group.

### Conclusions

Dexpramipexole was well tolerated in EMPOWER with comparable overall incidences of SAEs, discontinuations due to AEs, and withdrawals due to AEs between the dexpramipexole and placebo groups. No previously unidentified safety issues emerged compared with the earlier phase 2 study of dexpramipexole. No dose-limiting safety signals were observed.

### EXAMPLE 5 - CREATININE AS A BIOMARKER OF DISEASE SUBTYPE, DISEASE PROGRESSION, AND DRUG RESPONSE IN PATIENTS WITH ALS IN THE PHASE 3 EMPOWER STUDY

### Introduction

ALS is heterogeneous disease with a highly variable clinical course, including survival ranging from a few months to 10 years or more. Validated ALS biomarkers are needed to aid diagnosis, measure and predict disease progression, and serve as surrogate markers of drug response.

Changes in creatinine levels have previously been correlated with disease progression, suggesting creatinine as a candidate biomarker for ALS progression. Creatinine is a measure of both muscle degradation and of metabolic function. Dexpramipexole has demonstrated neuroprotective properties through a mechanism believed to be associated with enhanced bioenergetic efficiency.

We retrospectively analyzed data sets from the EMPOWER Phase 3 trial of dexpramipexole, the largest clinical trial ever conducted in ALS (n=943), to assess potential correlations of creatinine levels to disease progression, treatment effects, and outcomes of treatment-responsive subgroups.

### Methods

Baseline clinical laboratory values for creatinine were correlated with baseline clinical characteristics, including ALSFRS-R score and classification of definite ALS by El Escorial Criteria (EEC). Changes from baseline in creatinine levels were correlated with clinical outcomes by treatment groups. Subjects with baseline creatinine > 221 µmol/L were censored in all analyses.

Dexpramipexole and riluzole effects on creatinine were estimated using a mixed effects repeated measures model to compare effects averaged over time through month 12. The model included terms for treatment, visit, and treatment by visit interaction, baseline laboratory value and baseline laboratory value by visit interaction. The coefficient for
treatment main effect represented the mean change in creatinine averaged over each visit (time-averaged creatinine loss). The difference in the coefficients for treatments (placebo minus dexpramipexole) represented the time-averaged-difference (TAD) in creatinine loss. Creatinine loss from baseline to month 12 for EEC-definite participants compared with EEC-not definite participants was analyzed using the same repeated measures model described above.

### Results

In EMPOWER, a strong correlation existed between levels of creatinine at baseline and baseline ALSFRS-R, with greater creatinine levels associated with higher ALSFRS-R at randomization (See Figure 78). (Pearson correlation coefficient = 0.3049, p < 0.0001). In addition, reduced baseline creatine correlated significantly with a baseline diagnosis of definite ALS by El Escorial Criteria (data not shown).

A significant correlation existed between reduction in creatinine and decline in ALSFRS-R at month 6 (Figure 80, left panel). This correlation was significantly reduced with dexpramipexole treatment (Figure 80, right panel, p = 0.0447). Creatinine loss from baseline to month 12 (data not shown) was also greater for placebo participants than dexpramipexole participants (-6.0 µm/l vs -4.8 µm/l, p=0.0359). This finding is consistent with the phase 2 study of dexpramipexole in ALS (data not shown), in which participants receiving dexpramipexole 300 mg daily had reduced creatinine loss at month 3 compared with placebo participants (0.0 µm/l vs -5.3 µm/l, p=0.011).

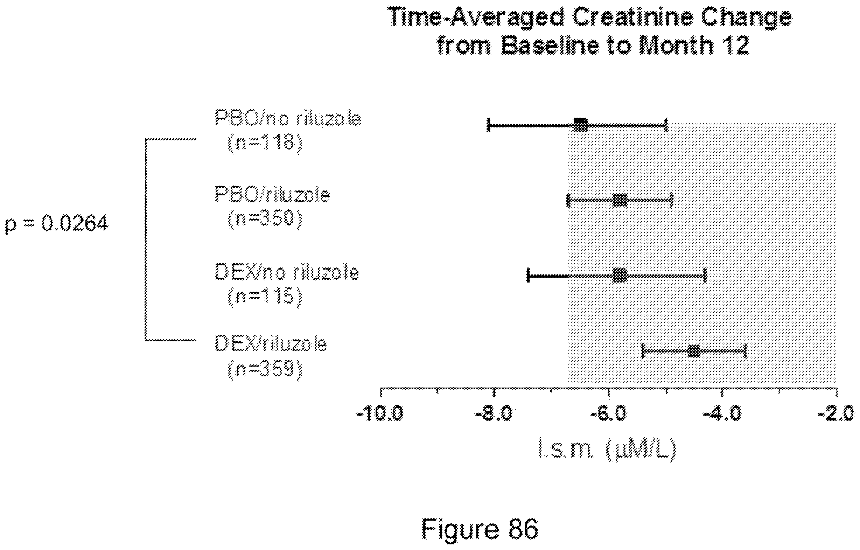

Creatinine loss was greatest for EMPOWER placebo subjects not receiving riluzole (-6.5 µm/l, 95% CI -8.1,-5.0), followed by dexpramipexole-treated subjects not on riluzole (-5.8 µm/l, 95% CI -7.4,-4.3) and placebo participants on riluzole (-5.8 µm/l, 95% CI - 6.7,-4.9) (Figure 86). Creatinine preservation was greatest for dexpramipexole-treated subjects on riluzole (-4.5 µm/l, 95% CI -5.4,-3.6). This suggests an additive benefit of riluzole and dexpramipexole on creatinine preservation, consistent with outcome benefits seen in subjects receiving dexpramipexole plus riluzole in EMPOWER *post hoc efficacy* analyses.

Creatinine-sparing effects of dexpramipexole by treatment-responder subgroups, measured by the least-square difference between dexpramipexole treatment and placebo: Creatinine preservation in the ITT population (1.2 µm/l; 95% CI 0.1,2.2) increased in the the EEC definite subgroup (2.1 µm/l; 95% CI 0.1,4.0), further increased in the EEC definite + riluzole subgroup (2.8 µm/l; 95% CI 0.7,5.0), and was greatest for EEC-definite subjects receiving riluzole who entered the study in the lower median (<15.3 months) of
symptom duration (4.8 µm/l; 95% CI 1.6,8.0) This creatinine-sparing effect by subpopulation is consistent with outcome benefits seen with EMPOWER *post hoc efficiacy* analyses.

### Conclusions

In EMPOWER, creatinine levels correlated significantly with disease severity at baseline and over 12 months of study. Creatinine-sparing effects were associated with both dexpramipexole and riluzole treatment, with the combination of drugs significantly attenuating creatinine loss. Preservation of creatinine was most significant in the subpopulation of dexpramipexole responders defined by EEC-definite classification, concomitant riluzole use, and lower-median symptom duration at study start.

These analyses strengthen the hypothesis that creatinine is a biomarker of ALS progression and potentially of positive treatment effect. Both hypotheses require prospective confirmation in well-controlled clinical studies. Further research is also needed to strengthen the mechanistic association between creatinine loss and disease progression.

### EXAMPLE 6 - LEARNING FROM EMPOWER: CREATININE AND OTHER METABOLIC MARKERS IN ALS

Creatinine background - Creatinine is the non-enzymatic, spontaneous, and irreversible breakdown product of creatine and phosphocreatine. The daily Crn production rate is -1.7% total body Cr (1.1% Cr + 2.6% PCr). Cr is either dietary or synthesized and transported into tissues. About 90% of Cr/PCr is in muscle. Resting type 1 skeletal muscle has -16 mM PCr and ∼7 mM Cr.

Is creatinine more than a passive marker of muscle mass in ALS? Creatinine levels were below the normal range for 5.9 % of the patients. For these patients, this low value was a strong predictor of death. This number is far less than the number of patients with atrophy of limbs, suggesting that there is no clear relation between these two variables, and that this decrease is probably independent of muscle atrophy. We have no clear explanation for the relationship between a decrease in the creatinine levels and poor survival. Creatinine is a non-enzymatic breakdown product of creatine phosphate and its levels therefore are a direct reflection of the creatine pool that plays an important role in muscle metabolism.

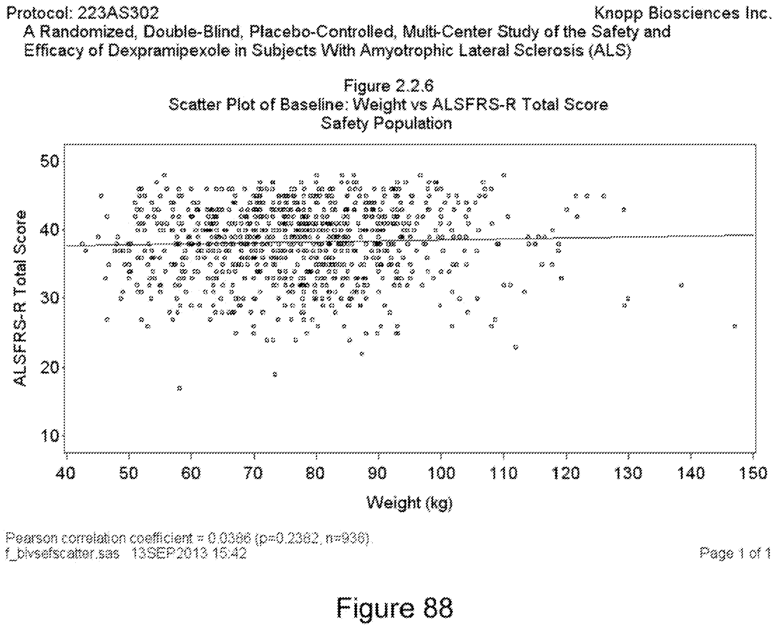

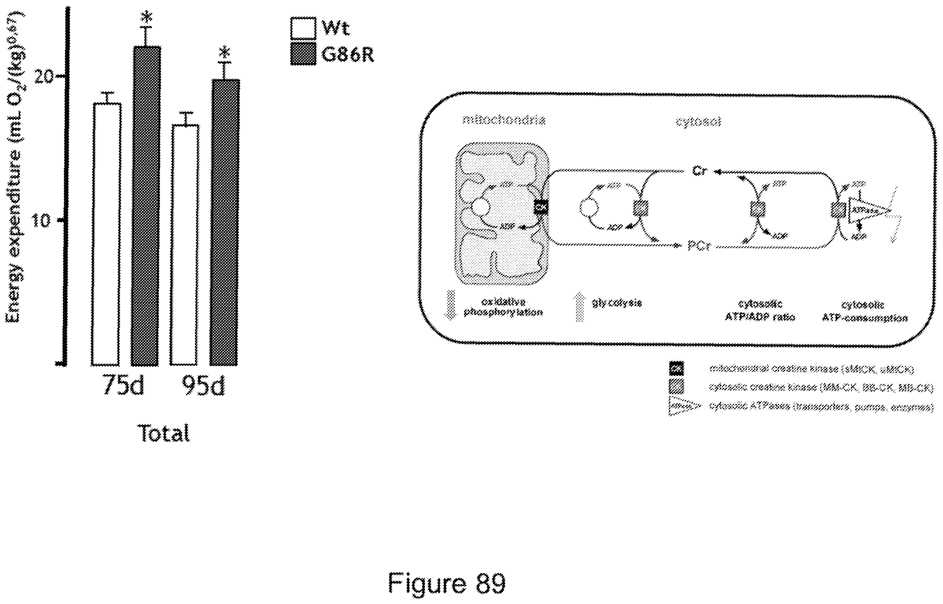

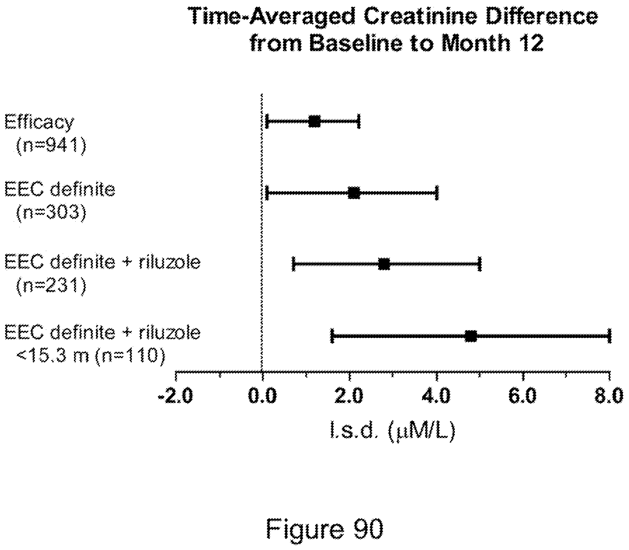

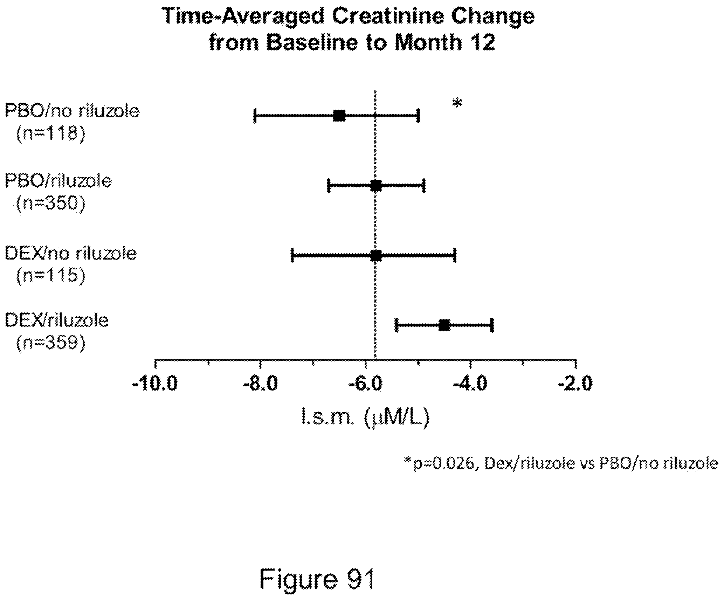

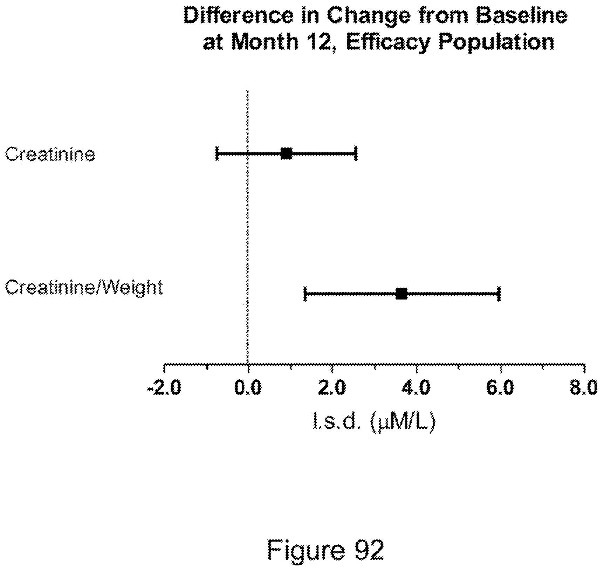

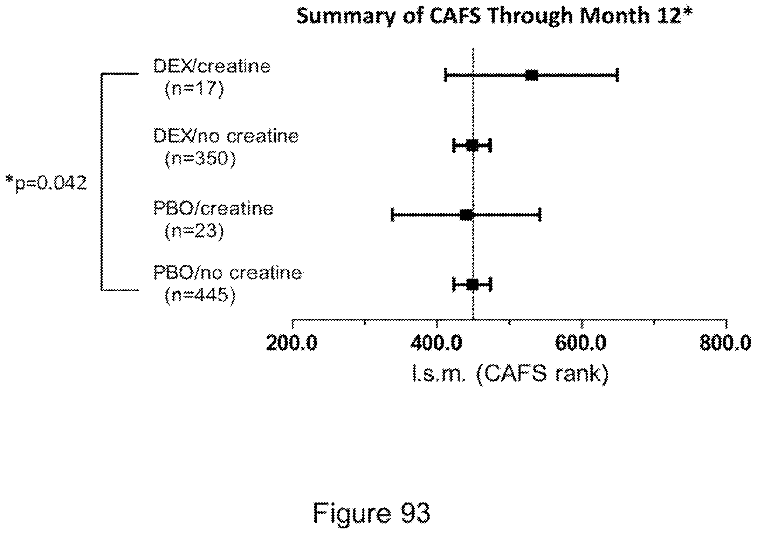

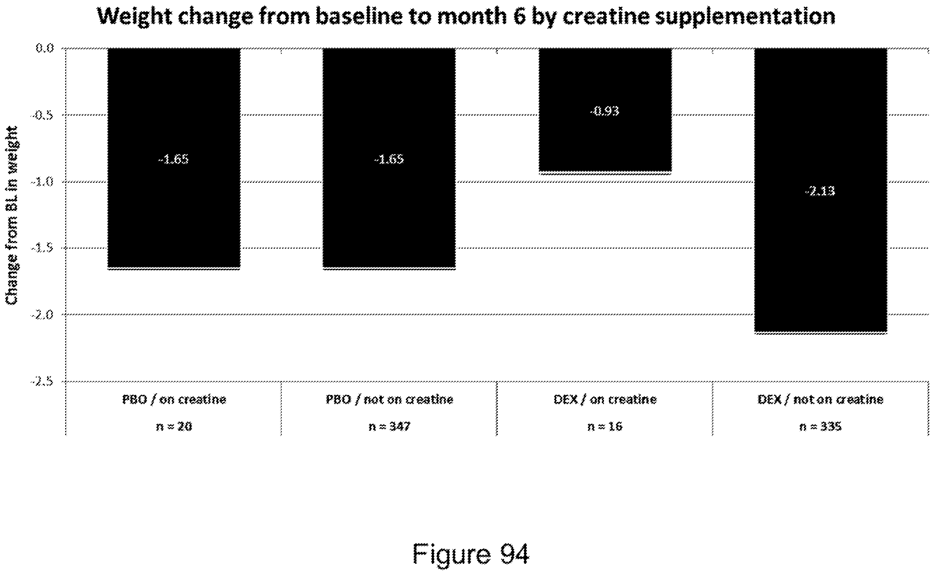

Baseline creatinine levels correlated significantly with ALSFRS-R in EMPOWER (See Figure 78). However, Baseline weight was not significantly correlated with baseline ALSFRS-R (See Figure 88). Figure 89 shows data supporting a correlating between creatinine loss and energy deficiency in ALS. As can be seen in Figure 80 (Left Panel), creatinine loss correlated significantly with ALSFRS-R decline in EMPOWER. As can be seen in Figure 76, the effect in the EEC definite subgroup increased on background of riluzole and with shorter symptom duration in a *post hoc* analysis of dexpramipexole effects on function in EMPOWER subgroups. Figure 90 shows that creatinine loss significantly reduced in dexpramipexole-treated EEC definite subgroups in a *post hoc* analysis of dexpramipexole effects on time average difference change in creatinine. Figure 91 shows the additive effects on creatinine-sparing seen with riluzole and dexpramipexole. The creatinine-sparing effects of dexpramipexole are not a passive marker of weight preservation as can be seen in Figure 92 which shows the creatinine-sparing effect of dexpramipexole adjusted for weight by taking ratio of creatinine to weight per visit based on mixed-effects repeated-measures model which includes treatment, visit, and baseline value as fixed effects. The dexpramipexole effects in increasing ATP synthesis confirmed in three independent labs (See Figure 84) and increased ATP synthesis with a corresponding increase in phosphocreatine could account for creatinine sparing (see Figure 85). Figure 93 shows a summary of the *post hoc* analysis of EMPOWER creatine use and raises the question as to whether dexpramipexole facilitates the effective conversion of creatine to phosphocreatine? Figure 94 shows that less weight loss observed in dexpramipexole subjects on creatine and raises the question as to whether dexpramipexole attenuate hypermetabolism in ALS?

EMPOWER data support exploration of creatinine as a biomarker of disease status and progression in ALS. Data consistent with the thesis that creatinine is more than a simple surrogate for muscle atrophy. Creatinine may be a marker of ALS energy deficit reflecting diminished phosphocreatine production. Creatinine may be useful in defining ALS phenotypes. *Post hoc* dexpramipexole clinical benefit in EEC definite subgroups is mirrored by creatinine sparring effect. Dexpramipexole may promote energy efficiency in ALS by enhancing phosphocreatine production and this hypothesis could be tested in experimental medicine paradigms.

### EXAMPLE 7 - A POST-HOC ANALYSIS OF THE PHASE 3 CLINICAL TRIAL (EMPOWER) OF DEXPRAMIPEXOLE IN ALS: POTENTIAL RESPONDER SUBGROUPS AND CREATININE AS A BIOMARKER OF DISEASE SEVERITY AND DRUG EFFECT

### Summary

Background: The failure of dexpramipexole to demonstrate efficacy in the phase 3 EMPOWER trial underscores the challenge of replicating positive phase 2 results in amyotrophic lateral sclerosis (ALS). We compared the phase 2 and phase 3 dexpramipexole study populations to determine whether inter-study population differences could account for conflicting results and to identify potential responder subgroups and biomarkers of drug effect.

Methods: We compared baseline characteristics of participants in the phase 2 and EMPOWER trials and used inter-study factors that differed significantly to identify subgroups for testing in a post-hoc analysis of EMPOWER efficacy outcomes. We also correlated changes from baseline in clinical laboratory tests with EMPOWER outcomes and analysed the effect of dexpramipexole on these parameters.

Findings: We found significant baseline differences in the proportion of phase 2 and phase 3 El Escorial Criteria (EEC) definite ALS participants (46% vs 32%, p=0.005), riluzole use (61% vs 75%, p=0.002), and mean symptom duration (14.0 months vs 15.2 months, p=0.037). Baseline creatinine level correlated highly with baseline ALSFRS-R score (p<0.001) in EMPOWER and creatinine loss correlated highly with ALSFRS-R decline (p<0.001). In the EMPOWER subgroup defined by riluzole use, EEC definite ALS, and <median symptom duration (15.3 months), participants receiving dexpramipexole (n=54) vs placebo (n=56) had less decline in ALSFRS-R slopes (0.49, 95% CI 0.10, 0.87), decreased mortality (H.R. 0.37, 95% CI 0.17,0.80), and reduced creatinine loss (4.8 µm/l; 95% CI 1.6,8.0).

Interpretation: Significant differences were present in the baseline characteristics of participants in the phase 2 and EMPOWER trials of dexpramipexole in ALS. In a post-hoc analysis of EMPOWER subgroups selected for these differences, potential clinical benefits of dexpramipexole effect were identified in the subgroup of riluzole-treated, short-symptom duration participants with definite ALS. Creatinine loss correlated with disease progression and was reduced in dexpramipexole-treated participants, suggesting it as a candidate biomarker.

### INTRODUCTION

Every investigational drug tested in a phase 3 clinical trial in amyotrophic lateral sclerosis (ALS) during the last 15 years has failed to demonstrate efficacy. The most recent of these phase 3 trials, known as EMPOWER, evaluated the investigational treatment dexpramipexole in the most comprehensive clinical trial ever conducted in ALS. Dexpramipexole failed to meet any prespecified endpoints in this study of 943 ALS
participants, despite an earlier 2-part phase 2 study in 102 participants demonstrating dose-dependent trends in reducing the rate of functional decline over 12 weeks (Part 1) and a significant difference (p=0.046) between the high- and low-dose treatment groups in a joint-rank test of mortality and function over 24 weeks (Part 2). Dexpramipexole at total daily doses up to 300 mg was well-tolerated in both the phase 2 and phase 3 studies with no dose-limiting toxicities.

Accordingly, we compared the phase 2 and phase 3 dexpramipexole trials to determine if inter-study population differences may have contributed to the divergent results. The goals of the analyses were to determine whether significant inter-study differences were present, whether such differences might suggest treatment-responsive EMPOWER subgroups and biomarkers of drug effect, and whether such findings could inform future ALS trial design.

The EMPOWER study was designed to replicate the phase 2 study in every feasible respect except size. The phase 2 study enrolled 102 participants at 20 U.S. centers in a primary safety and tolerability study. Pre-specified efficacy endpoints were the ALSFRS-R slope tested by the mixed-effects linear slopes model and mortality tested by the Cox proportional hazards regression model. The phase 3 study enrolled 943 participants in 11 countries and tested efficacy on the primary endpoint, the Combined Assessment of Function and Survival (CAFS), and on separate tests of the CAFS components: functional decline, measured by ALSFRS-R change, and mortality. Both studies included riluzole use, the only drug approved for ALS and which extends survival by approximately two to three months.

Baseline age, site of onset, ALSFRS-R score, and symptom duration are important covariates in predicting rates of disease progression and survival in ALS trials. Gender is also reported to affect disease course, though not consistently, and definite ALS classification by El Escorial Criteria (EEC) is emerging as a prognostic factor. We evaluated the impact of these factors and riluzole use on clinical outcomes in the EMPOWER placebo population for consistency with previous reports.

The need for biomarkers to monitor and predict disease progression and better inform patient selection in ALS clinical trials is widely recognized. While not diagnostic, changes in certain biochemical parameters, including creatinine, have been correlated with disease outcomes. We correlated changes from baseline in clinical laboratory tests with EMPOWER outcomes and analyzed the effect of dexpramipexole on select parameters.

### METHODS

Study Designs - The study designs, participants, endpoints and statistical analyses for the phase 2 and EMPOWER trials of dexpramipexole have been reported in detail previously.

Post-hoc Statistical Analyses - Differences in baseline participant characteristics between the phase 2 and EMPOWER studies, between EMPOWER treatment groups, and between the definite and non-definite El Escorial categories were tested by chi-square test for categorical variables and by t-test for continuous variables. Characteristics that differed significantly between phase 2 and phase 3 were used to select subgroups for efficacy testing. The order of subgroup testing-EEC definite, riluzole use, median symptom duration-was based on 1) the unexpected, significantly decreased representation in EMPOWER of an aggressive disease phenotype (definite ALS), and 2) the magnitude of the relative differences between phase 2 and EMPOWER baseline characteristics from greatest to least. The predictive association between baseline variables and the CAFS was determined by multiple regression. Baseline clinical laboratory tests were correlated with baseline clinical characteristics and changes from baseline in clinical laboratory tests were correlated with clinical outcomes by treatment groups. Dexpramipexole and riluzole effects on creatinine were estimated using a mixed-effects repeated-measures model to compare effects averaged from baseline through month 12. The model included terms for treatment, visit, and treatment by visit interaction, baseline laboratory value, and baseline laboratory value by visit interaction. The coefficient for treatment main effect represents the mean change in creatinine averaged over each visit (time-averaged creatinine loss). The difference in the coefficients for treatments (placebo minus dexpramipexole) represents the time-averaged-difference in creatinine loss. Creatinine loss from baseline to month 12 for EEC definite participants compared with not definite participants was analyzed using the repeated measures model described above.

Testing for treatment effects on the primary efficacy endpoint in subgroups selected by significant inter-study differences used the methods pre-specified in the EMPOWER statistical analysis plan. The CAFS is a joint-rank test of functional outcomes adjusted for mortality by ranking participants' time to death or change from baseline in ALSFRS-R scores, using follow-up data through the end of the 12-month primary evaluation period. CAFS ranks were analyzed using an ANCOVA model with treatment as a fixed effect, adjusted for ALSFRS-R, duration from symptom onset to first dose of study treatment, onset site, and concomitant riluzole use as baseline covariates. Functional change was measured by the ALSFRS-R slopes through month 12 analyzed by a linear mixed-effects model and time to death through month 18 was determined using the Cox proportional hazards model; both models adjusted for the same covariates used in the CAFS ANCOVA. The intent-to-treat (ITT) population (i.e., randomized participants who received at least one dose of study drug, n=942) was used for the survival analysis. The efficacy population (i.e., randomized participants receiving at least one dose of study drug and one on-study efficacy assessment, n=941) was used for all other endpoint analyses. Adjustments for multiple testing were not made.

### RESULTS

The dexpramipexole phase 2 and EMPOWER study populations were similar in baseline age (57.0 years vs 57.1 years), gender (64% male vs 64% male), ALSFRS-R (38.0 vs 38.2), and bulbar-onset ALS (18% vs 23%). (Figure 71) In contrast, there were significant baseline differences between phase 2 and EMPOWER EEC definite ALS participants (46% vs 32%, p=0.005), riluzole use (61% vs 75%, p=0.002), and symptom duration (14.0 months vs 15.2 months, p=0.037).

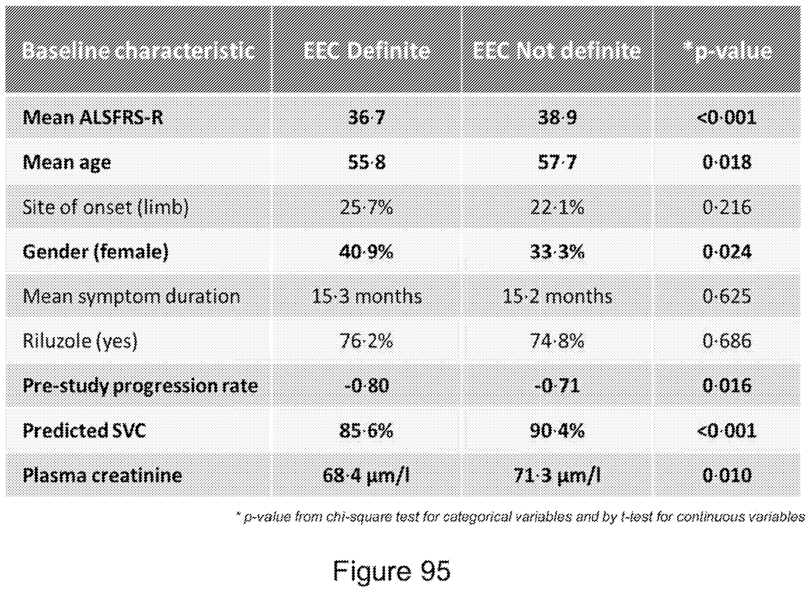

Consistent with prior reports, 6 EMPOWER EEC definite participants in the placebo group (n=156) had worse outcomes on CAFS (398.4 vs 502.1; p<0.001), ALSFRS-R slopes (-1.37 vs -1.06; p<0.001), and mortality (H.R. 1.39; p=0.093) compared with not definite participants (n=312). Creatinine loss from baseline to month 12 was also significantly greater for EEC definite participants compared with not definite participants (-16.5 µm/l vs - 12.5 µm/l, p=0.007). EMPOWER EEC definite participants were significantly younger (55.8 years vs 57.7 years, p=0.018) and more female (40.9% vs 33.3%, p=0.024) than not definite participants. (Figure 95) They also had significantly faster pre-study ALSFRS-R progression rates (-0.80 vs -0.71, p=0.016), and lower baseline ALSFRS-R scores (36.7 vs 38.9, p<0.001), predicted slow vital capacity (85.6% vs 90.4%, p<0.001), and plasma creatinine (68.4 µm/l vs 71.3 µm/l, p=0.010). There were no significant differences in riluzole use (76.2% vs 74.8%, p=0.686), bulbar onset (25.7% vs 22.1%, p=0.216), and symptom duration at baseline (15.3 months vs 15.2 months, p=0.625).

The effects of riluzole on mortality in EMPOWER were also consistent with previous reports. Among EMPOWER participants in the placebo group (n=468), the hazard for mortality was 0.72 (95% CI 0.48,1.09; p=0.123) for participants on riluzole (n=350) vs no riluzole (n=118). In participants receiving dexpramipexole (n=474), the hazard for mortality was 0.59 (95% CI 0.38,0.91; p=0.018) for participants on riluzole (n=359) vs no riluzole (n=115).

As shown in Figure 75, participants receiving dexpramipexole in the EMPOWER subgroups selected by significant inter-study differences (EEC definite; EEC definite on riluzole; EEC definite on riluzole with symptom duration <15.3 months) had improved CAFS outcomes compared with placebo-treated participants. In contrast to the overall efficacy population, in which no CAFS benefit was observed in dexpramipexole-treated participants (n=473) compared with placebo (n=468) (least square difference (l.s.d.) 2.8; 95% CI -28.9,34.4), the CAFS outcome was improved for dexpramipexole-treated participants (n=147) in the EEC definite subgroup (n=303) compared with placebo (n=156) (l.s.d. 28.5; 95% CI -25.3,82.4). The CAFS was further improved for dexpramipexole-treated participants (n=113) in the EEC definite subgroup on concomitant riluzole (n=231) compared with placebo (n=118) (l.s.d. 51.4; 95% CI -8.4,111.1), and for dexpramipexole-treated participants (n=54) in the EEC definite ALS, riluzole use, and short symptom duration (<15.3 months) subgroup (n=110) compared with placebo (n=56) (l.s.d. 72.1; 95% CI -6.8, 150.9).

Corresponding dexpramipexole benefits were observed in these subgroups on function, defined by reduced ALSFRS-R slopes decline, and in a decreased hazard for mortality, as shown in Figure 76 and Figure 77. Compared with the efficacy population, in which there was no difference in the ALSFRS-R monthly slope between dexpramipexole- and placebo-treated subjects (0.02; 95% CI -0.09,0.13), the monthly ALSFRS-R slopes decline was improved for participants receiving dexpramipexole vs placebo in the EEC definite subgroup (0.12; 95% CI -0.08,0.32), the EEC definite with concomitant riluzole subgroup (0.24; 95% CI 0.02,0.46), and the EEC definite with concomitant riluzole and short symptom duration subgroup (0.49, 95% CI 0.10,0.87). Similarly, compared to the hazard for mortality in the ITT population (H.R. 0.98; 95% CI 0.75,1.30), the hazard for mortality for dexpramipexole treated participants was reduced in the EEC definite subgroup (H.R. 0.76; 95% CI 0.48,1.23), the EEC definite with concomitant riluzole subgroup (H.R. 0.63; 95% CI 0.36,1.10), and the EEC definite with concomitant riluzole and short symptom duration subgroup (H.R. 0.37, 95% CI 0.17,0.80).

Creatinine levels are significantly lower in ALS subjects compared with healthy controls and low plasma creatinine has been correlated with worse ALS outcomes. In EMPOWER, there was a significant correlation between baseline creatinine and baseline ALSFRS-R (Pearson correlation coefficient=0.305, p<0.001). There was also a significant correlation between creatinine reduction and ALSFRS-R decline at month 6 that was greater for placebo (Pearson correlation coefficient=0.380, p<0.001) than for dexpramipexole treatment (Pearson correlation coefficient=0.217, p<0.001).

Creatinine loss from baseline to month 12 was greater for placebo participants vs dexpramipexole participants in the EMPOWER ITT population (-6.0 µm/l vs - 4.8 µm/l, p=0.0359). This finding is consistent with the phase 2 study, in which participants receiving dexpramipexole 300 mg daily had reduced creatinine loss at month 3 compared with placebo participants (0.0 µm/l vs -5.3 µm/l, p=0.011). As shown in Figure 91, creatinine loss was greatest for placebo participants not on riluzole (-6.5 µm/l, 95% Cl -8.1,-5.0), followed by dexpramipexole participants not on riluzole (-5.8 µm/l, 95% Cl -7.4,-4.3), placebo participants on riluzole (-5.8 µm/l, 95% Cl -6.7,-4.9), and dexpramipexole participants on riluzole (-4.5 µm/l, 95% Cl -5.4,-3.6). Consistent with clinical outcomes in EMPOWER subgroups, creatinine sparing for dexpramipexole treated subjects was greatest among participants receiving dexpramipexole vs placebo in the EEC definite with concomitant riluzole and median symptom duration subgroup (4.8 µm/l; 95% Cl 1.6,8.0), followed by the EEC definite with concomitant riluzole subgroup (2.8 µm/l; 95% Cl 0.7,5.0), the EEC definite subgroup (2.1 µm/l; 95% Cl 0.1,4.0), and the ITT population (1.2 µm/l; 95% Cl 0.1,2.2) (Figure 90).

### DISCUSSION

Despite significant progress in understanding the genetic basis of ALS, in characterizing its molecular pathogenesis, and in developing improved clinical trial outcome measures and effective clinical trial consortia, no new drug has been approved for the treatment of ALS since riluzole in 1995. Particularly vexing has been the failure of drugs in phase 3 trials that showed promise in phase 2 or, in the case of IGF, failed to replicate a positive phase 3 trial. The reasons for these failures include the absence of biomarkers, disease heterogeneity, trial design limitation, participant selection inconsistency, and, of course, ineffective treatments.

The EMPOWER result has only increased the debate about strategies in ALS drug development. In some views, ineffective drugs are advancing to phase 3 trials because phase 2 trials are too small to rule out false-positive error. In other views, promising investigational drugs are failing in phase 3 because those trials are too large and non-selective to detect a treatment signal within a heterogeneous population.

These concerns in ALS clinical research are consistent with emerging regulatory policy regarding drug development in complex diseases. A current draft guidance from the U.S. Food and Drug Administration (FDA) encourages incorporating enrichment strategies into clinical trials, which the FDA defines as "the prospective use of any participant characteristic to select a study population in which detection of a drug effect (if one is in fact
present) is more likely than it would be in an unselected population." Of course, the key condition in this definition is that a drug effect is "in fact present."

In phase 2, dexpramipexole demonstrated several criteria important in advancing a drug candidate to late-stage testing: (1) evidence of a dose-dependent treatment effect, (2) replication of treatment effect across re-randomized cohorts, (3) drug exposures consistent with preclinical pharmacodynamic effects, and (4) consistency across endpoints. Despite these promising results, dexpramipexole failed to meet any pre-specified efficacy endpoints in EMPOWER.

Notable in the phase 2 study of dexpramipexole was a placebo population with more rapidly progressing disease (ALSFRS-R monthly slope estimate of -1.28) than reported for placebo groups in the recent phase 3 ALS trials of lithium, TCH346, and minocycline (ALSFRS-R slopes of -0.78, -0.94, and -1.04, respectively) or observed in EMPOWER. Like ceftriaxone, which showed promise in a phase 2 ALS trial against a rapidly progressing placebo group but failed to show benefit in phase 3 efficacy signals may be more easily detected in cohorts with aggressive disease simply because they exhibit greater dynamic range. More aggressive ALS may also represent a phenotype more responsive to specific interventions (e.g., modulation of EEAT2 gene expression or mitochondrial bioenergetics).

We conducted a post-hoc analysis of the phase 2 and EMPOWER dexpramipexole trials to determine if significant inter-study population differences were present and whether such differences might account for the inconsistent trial results. Compared to the phase 2 study, riluzole use was significantly increased, symptom duration was significantly longer, and the proportion of EEC definite ALS participants was significantly smaller in EMPOWER. Symptom duration and EEC diagnosis were significant predictors of CAFS outcomes, and the inter-study covariate differences may have contributed to the reduced rate of disease progression in EMPOWER vs phase 2. In particular, the marked decline in the proportion of EEC definite participants had the effect of diluting the number of participants with a more aggressive disease phenotype.

In addition to being clinically distinct at baseline and on study, EEC definite participants had significantly lower baseline creatinine levels and greater on-study creatinine loss than not definite participants. More importantly, creatinine emerged as a potential biomarker of dexpramipexole clinical effects in this population. Increased creatinine sparing was observed in EMPOWER subgroups with improved clinical outcomes, an effect also seen in the phase 2 trial.

A potential limitation of using the CAFS as a primary endpoint in ALS clinical trials emerged from the post-hoc analysis of EMPOWER. Under most circumstances, the sensitivity of the CAFS in detecting a significant difference between treatment groups is concordant with the sensitivity of its component tests. However, in the subgroup analysis of EEC participants definite on riluzole with symptom duration <15.3 months, the CAFS proved to be less powerful than either component test, the mixed effects model for ALSFRS-R slopes or the Cox proportional hazards model. The CAFS is a non-parametric rank test and, in this case, ranking the joint outcome difference of function adjusted for mortality significantly compressed estimate of treatment effect compared with either of the separate parametric tests of function or mortality.

The effect of riluzole use in the EMPOWER placebo group might be considered an internal validation of the robustness of its design and execution. Consistent with the literature, riluzole produced a reduction in the hazard for mortality among placebo participants that trended toward statistical significance. Interestingly, the magnitude of the clinical effect increased and reached statistical significance in the dexpramipexole-treated group, suggesting the potential of an additive benefit of the drugs, in contrast to previous clinical trials that showed no benefit of riluzole in combination with the investigational drug or suggested a negative interaction. This clinical observation was consistent with the apparent additive effects of the drugs in reducing creatinine loss. Riluzole has no effect on the pharmacokinetics of and no known pharmacodynamic interactions with dexpramipexole. Both, however, are benzothiazoles, a class of molecules possessing broad biological activity, including neuroprotective properties in models of acute and chronic neurodegeneration, and they have similar chemical structures.

Subgroup analyses, both prespecified and post-hoc, are an important tool in the analysis of clinical trials of investigational drugs and established treatments. In trials of disease-modifying agents in neurodegenerative disorders, for example, post-hoc analyses have been reported extensively in assessing the heterogeneity of treatment effects and comparing the efficacy of multiple sclerosis therapeutics. Subgroup analyses can inform treatment guidelines, enhance trial designs, improve participant selection, and generate clinical hypotheses for future testing. Such analyses must be considered with caution, however, as they can be over-interpreted and should be viewed against both the methodology used in the analyses and the total body of evidence for the investigational new drug or established treatment being evaluated.

Subgroup analyses, especially post-hoc, are by definition subject to bias. However, some steps can be taken to reduce bias. These include conducting exploratory analyses on the basis of prior hypotheses, accounting for imbalances in the distribution of prognostic baseline characteristics within subgroups, reporting absolute and relative risk reductions with 95% confidence intervals, and adjusting for multiple comparisons. We hypothesized that significant differences in inter-study populations might have accounted for the divergent phase 2 and phase 3 dexpramipexole results and tested for potential responder subgroups in EMPOWER based on the identification of those differences. We also used the primary endpoint and the analysis techniques prespecified in the EMPOWER protocol.

Nonetheless, there are important limitations to the EMPOWER subgroup analyses we conducted. EMPOWER was neither designed nor powered to test for treatment differences within subgroups or heterogeneity across subgroups. Furthermore, we made no adjustments for multiple comparisons. The potential beneficial effects of dexpramipexole observed in the EMPOWER subgroup defined by riluzole use, EEC definite ALS, and short symptom duration should therefore be viewed as hypothesis generating and requiring further investigation. Significance testing does not establish the validity of subgroup analyses; only replication does.

ALS remains an intractable disease. Indeed, there is considerable discussion of whether ALS is a single disease or a spectrum of disorders manifesting a common phenotype. Consensus is emerging, however, following failures such as EMPOWER, that smaller, targeted phase 3 trials are necessary to reduce participant heterogeneity and enrich study populations to more effectively detect drug responses. EMPOWER attempted to replicate the phase 2 dexpramipexole study, but in scaling by nearly a factor of 10, significant population differences emerged that may have accounted for the divergent results. Future studies of dexpramipexole should be conducted on a background of riluzole use, be enriched for participants with EEC definite, rapidly-progressing ALS, and incorporate creatinine as a biomarker.

### RESEARCH IN CONTEXT

Systematic review - We searched PubMed for randomized placebo-controlled studies of dexpramipexole in amyotrophic lateral sclerosis published in English before Nov 10, 2013, with the search terms "dexpramipexole" and "amyotrophic lateral sclerosis." The two dexpramipexole publications retrieved were the phase 2 and EMPOWER studies. We also identified other recently published post-hoc analyses of randomized studies of drugs that
have been developed in multiple sclerosis as well as publications reviewing the role and method for conducting post-hoc analyses of clinical trials.

Interpretation - In this post-hoc analysis, we compared the baseline characteristics of participants randomized to the phase 2 and phase 3 dexpramipexole clinical studies to determine whether inter-study population differences could provide an explanation for the conflicting study outcomes. We found symptom duration was significantly longer and the proportion of EEC definite ALS participants was significantly smaller in EMPOWER relative to phase 2, factors that contributed to reduced disease severity among phase 3 participants, and riluzole use was significantly increased. Increased disease severity of EEC definite ALS participants relative to not definite participants, an increasingly cited risk factor for disease progression and survival, was confirmed in the EMPOWER placebo group by both clinical and laboratory baseline and on-study parameters. The significantly different outcomes between these groups underscore the heterogeneity of ALS, the challenge of replicating study phase 2 populations in phase 3, and the imperative for developing clinical and laboratory criteria that increase the uniformity of trial participants and improving the signal detection of therapies under development.

This post-hoc analysis also showed the effect of riluzole on mortality among EMPOWER placebo participants to be consistent with its label and with previous literature reports. Of note, the clinical effect of riluzole appeared to be augmented by the addition of dexpramipexole, and this effect was also consistent with the apparent additive benefit of the two drugs in combination in reducing creatinine loss. These findings raise the question of whether benzothiazoles may be a useful chemotype for developing ALS treatments, and, as both riluzole and dexpramipexole are relatively low potency compounds with evidence of dose dependent effects, whether dosage of the two agents could be further optimized in future ALS trials.

Importantly, creatinine levels at baseline and creatinine loss over time were strongly correlated with disease severity in the post-hoc analysis of EMPOWER, substantially strengthening prior observations of creatinine loss as a potential biomarker of disease progression. Creatinine also emerged as a potential biomarker of drug effect among participants receiving dexpramipexole and may prove to be useful in any future clinical studies of the drug.

### EXAMPLE 8 - EFFECTS OF DEXPRAMIPEXOLE ON WHITE BLOOD CELLS IN A MINIPIG TOXICOLOGY STUDY AND FROM TWO CLINICAL TRIALS IN PATIENTS WITH ALS

### Introduction

Background - Dexpramipexole is a small, orally bioavailable molecule with demonstrated cytoprotective properties in *in vitro* and *in vivo* preclinical studies. The drug is in development for the treatment of amyotrophic lateral sclerosis (ALS).

Objectives - In preclinical, phase 2, and phase 3 studies of dexpramipexole as a potential treatment for ALS, white blood cell status was monitored as a component of routine clinical laboratory assessments and to assess neutropenia.

### Methods

A 39-week toxicology study was conducted in minipigs. The phase 2 study randomized 102 subjects and the phase 3 study randomized 943 subjects in double-blind, placebo-controlled trials to assess the safety and efficacy of dexpramipexole in ALS, respectively. Subjects were randomized to 25 mg, 75 mg, or 150 mg dexpramipexole twice daily or placebo for up to 9 months (phase 2) or 150 mg dexpramipexole twice daily or placebo for up to 18 months (phase 3). CBCs were obtained monthly in both clinical studies and at 13, 26, and 39 weeks in the preclinical toxicology study.

### Results

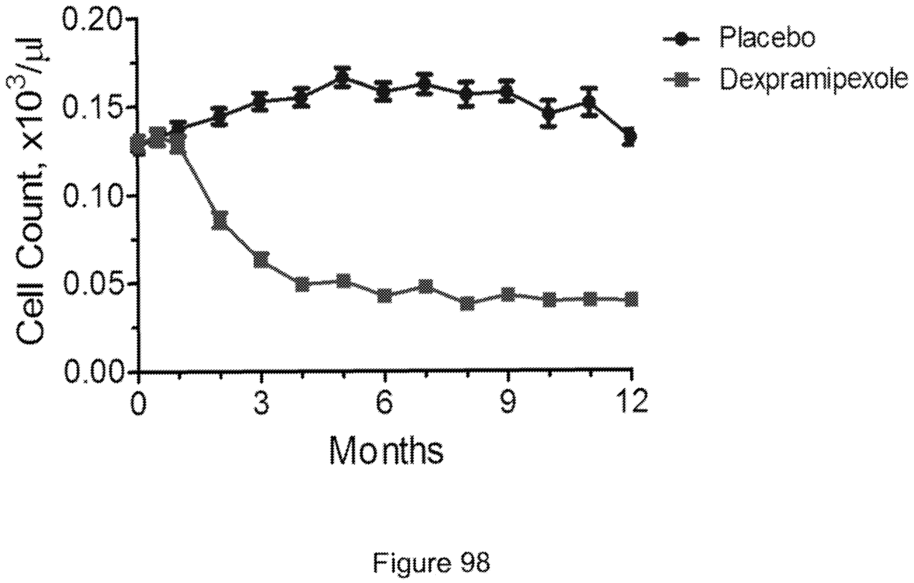

In the chronic toxicology study in minipigs, a time- and dose-dependent decrease in eosinophils was observed. (Fig. 98). The reduction of eosinophils was observed in minipigs in chronic toxicity studies (n=3-5 per group).

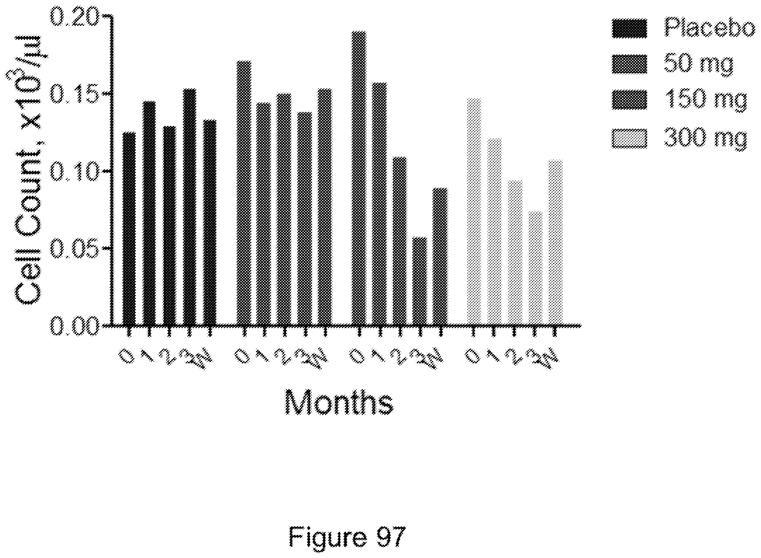

In part 1 of the two-part phase 2 study, a dose-dependent decrease in eosinophil count was observed following 12 weeks of treatment with dexpramipexole at doses of 25 mg, 50 mg, and 150 mg twice daily versus placebo (Fig. 97). Following a 4-week, single-blind drug washout, subjects in part 2 re-randomized to 150mg twice daily had a greater decline in eosinophils than subjects re-randomized to 25mg twice daily (data not shown). The part 1 decrease in eosinophil count was partially reversed by the end of the 4-week washout period (Fig. 97). Eosinophil reduction was observed over 3 months in Phase 2 (n=22-25 per group). W represents the washout at week 4 following Part 1 and the start of Part 2 baseline.

In phase 3, a profound decrease in blood eosinophil count was observed after 8-12 weeks of treatment with dexpramipexole that persisted for the duration of the trial (Fig.
98). In the dexpramipexole group, n=474 at baseline and n=328 at 12 months. In the placebo group, n=467 at baseline and n=340 in the placebo group. Counts were reduced by approximately 70% in the treated group, while there was a trend towards increased eosinophil count in patients receiving placebo. The treatment effect on eosinophils was observed in most patients, with three-quarters of dexpramipexole-treated subjects experiencing a 50% or greater decline in eosinophil count after 6 months of treatment (data not shown).

ALS is not typically associated with a systemic inflammatory response, and baseline eosinophil counts in the treated and placebo groups were comparable at 0.129 and 0.127 x 10⁹/L, respectively. However, the eosinophil-lowering effect of dexpramipexole was not diminished in patients (n=42) with higher eosinophil counts (i.e. >0.25 x 109/L), in whom a 75% decrease was observed after 6 months of treatment (data not shown).

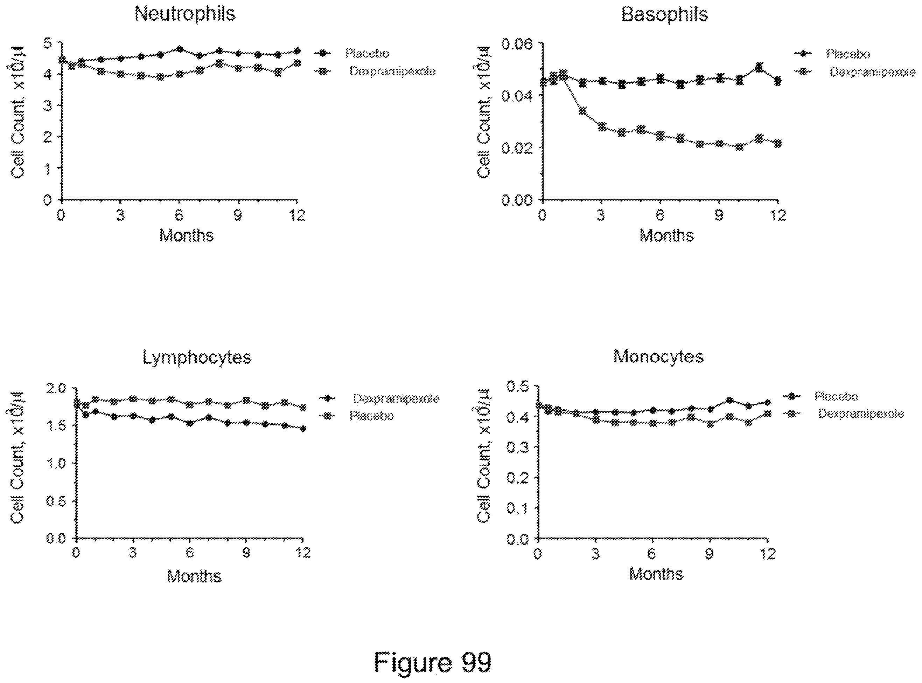

Basophils were also significantly reduced (Fig. 99). In the dexpramipexole group, n=474 at baseline and n=328 at 12 months. In the placebo group, n=467 at baseline and n=340 in the placebo group. There were no clinically significant changes in monocytes and lymphocytes, although these reductions were statistically significant. Neutropenia (i.e., ANC < 1.5 x 109/L) was observed in 29 dexpramipexole-treated patients (6.1%) and 8 (1.7%) of patients receiving placebo, and was reversible upon withdrawal of treatment. Dexpramipexole had no effect on red blood cell counts or platelet levels in EMPOWER (data not shown).

### Conclusions

Dexpramipexole treatment at 300 mg/day produces a variable degree of leukopenia depending on cell type, with the greatest magnitude observed on eosinophils. In ALS patients, statistically significant effects on eosinophils are observed at month 1, while the effect is maximal by 4 months. Eosinophil reduction occurs broadly: most patients show a 50% decrease or more by month 6. Eosinophil reduction are observed when baseline counts are elevated. Similar effects are seen on basophils. Sporadic, reversible neutropenia occurs in ∼ 6% of patients. Lymphocyte and monocyte counts show statistically but not clinically significant reductions.

### EXAMPLE 9 - THE EOSINOPHIL MODULATING EFFECTS OF DEXPRAMIPEXOLE IN CLINICAL STUDIES

### Abstract

The reliance on and insufficiency of off-label chronic corticosteroid therapy for eosinophil-associated diseases (EAD) underscores the need for targeted, non-steroidal treatments. Dexpramipexole, an orally available small molecule under development in amyotrophic lateral sclerosis, has been observed during routine hematology monitoring to demonstrate significant, dose- and time-dependent eosinophil-lowering activity, with less pronounced reductions on other leukocyte counts. Analysis of hematology values across phase 2 and 3 clinical trials encompassing more than 1,000 subjects demonstrated that dexpramipexole consistently and markedly lowered peripheral blood eosinophils. This effect developed after 1 month on treatment, required 3-4 months to reach its maximum, remained constant throughout treatment, and partial reversal upon drug withdrawal. All doses tested were well tolerated, with no increase in infection rate associated with eosinophil-lowering effects. Further studies will determine whether the eosinophil-lowering effect of dexpramipexole in peripheral blood may translate into benefits for patients with pathological systemic or organ-specific eosinophil activity.

### Introduction

Eosinophils are white blood cells of myeloid lineage with multiple roles, including the innate response to parasitic infection, modulation of adaptive immune responses, and maintenance of tissue homeostasis. Under a variety of conditions, eosinophils differentiate and proliferate in the bone marrow, enter the blood stream, migrate across endothelial tissues, and infiltrate target organs, secreting inflammatory proteins that may cause serious, potentially irreversible, tissue damage. Hypereosinophilic syndrome (HES) is defined as a persistent elevation of blood eosinophils above 1.5 X 109/L and may be reactive (including in allergic asthma and eosinophilic esophagitis), primary (including in hematopoietic neoplasms), or idiopathic (including in hypereosinophilic syndrome) in nature.

While the introduction of imantinib has improved treatment for a subset of HES patients with the FIP1L1/PDGFRA fusion gene, most forms of HES remain treated with chronic corticosteroid administration, or, in refractory patients, with a cytotoxic agent such as hydroxyurea. These chronic treatments have well-established side-effect profiles. Monoclonal antibodies targeting eosinophil-activating cytokines show promise in EAD, but none is yet approved. Overall, treatment options remain limited and the need remains for more effective, less toxic eosinopenia inducing drugs.

Dexpramipexole is a renally excreted, synthetic benzothiazole with high oral bioavailability, linear pharmacokinetics, and an established safety profile. Its eosinophil-lowering effect observed initially in phase 2 studies was recently confirmed in a large phase 3
trial (n=942) in amyotrophic lateral sclerosis (ALS) patients, a population with a generally normal hematologic status. An agent that safely, significantly, and persistently lowers circulating eosinophils merits investigation for its potential to normalize the hypereosinophilia characteristic of many EADs.

### Methods

Data sets were retrospectively reviewed from a chronic toxicology study of dexpramipexole in Göttingen minipigs and from two randomized, double-blind, placebo-controlled, clinical trials in ALS. Baseline hematology values were compared to the reference ranges prespecified for each study. Serum eosinophil levels were summarized over all available time points (to a maximum of 12 months in the case of the phase 3 study) and analyzed by ANOVA testing the effect of treatment vs. placebo on mean changes in serum eosinophil counts from baseline. Human subjects with baseline eosinophils from 0 to 0.02x103/µL (constituting less than 2% of all subjects analyzed) were censored from the primary analysis because of the inherent limitation to observe a decline from baseline. In the phase 3 study, changes from baseline for all other peripheral blood cells were also assessed to evaluate the broader hematologic effects of the drug. Adverse events were reviewed by preferred term and organ class and their incidences compared between the treatment and placebo groups to assess the potential risk of dexpramipexole-associated immunological side effects.

### Results and Discussion

The phase 2 clinical trial was a two-part, double-blind study that evaluated the safety, tolerability, and clinical effects of dexpramipexole in ALS patients. In Part 1, subjects were randomized to placebo (n=27), 50 mg/day (n=23), 150 mg/day (n=26), or 300 mg/day dexpramipexole (n=26) for 12 weeks. From baseline to week 12, mean serum eosinophils increased by 29.2% in the placebo group and declined by 18.2% (p=0.0370), 69.9%, (p<0.0001), and 42.9% (p=0.0008) in the 50 mg, 150 mg, and 300 mg groups, respectively (Fig. 97). During a one-month washout following week 12, mean eosinophils at week 16 recovered to 47%, and 73% of baseline levels in the 150, and 300 mg/day groups, respectively.

Dexpramipexole was well-tolerated in the phase 2 study, with no dose-limiting toxicities and with adverse event rates similar across treatment groups. However, two subjects from the 300 mg dose group experienced Grade 2 neutropenia (neutrophil count between 1000/µL and 1500/µL), which was reversed during the placebo washout period; neutropenia was not observed at daily doses below 300 mg.

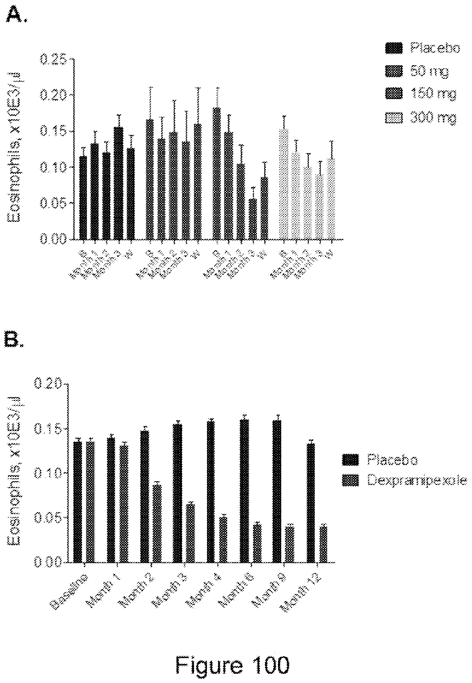

The phase 3 study was a double-blind trial of dexpramipexole in ALS patients randomized 1:1 to placebo or dexpramipexole 300 mg daily treatment. As in earlier studies, the eosinophil-lowering effect developed slowly, reached plateau at month 4, and persisted through month 12 (Figure 98). Figure 100 shows time- and dose-dependent eosinophil lowering effects of dexpramipexole in Phase 2 and Phase 3 clinical trials. (A) In the Phase 2 study (n=102), a 12 week dose ranging study in ALS subjects, mean serum eosinophils rose by 29.2% in the placebo group and declined by 18.2% (p=0.0370), 69.9%, (p<0.0001), and 42.9% (p=0.0008) in the 50 mg, 150 mg, and 300 mg groups from baseline to week 12, respectively. (B) In Phase 3 (n=928 after censoring subjects with a baseline eosinophil count of 0 to 0.02x10⁹/L), a profound decrease in peripheral blood eosinophil count was observed after 8-12 weeks of treatment with dexpramipexole that persisted for the duration of the trial. Eosinophil counts were reduced from baseline levels by approximately 70% in the dexpramipexole-treated group, while there was a slight increase in eosinophil count in patients receiving placebo.

At month 12, the change from baseline in serum eosinophil counts was -2% in the placebo group and -70% in the dexpramipexole-treated group (p<0.0001). The effect of dexpramipexole in reducing eosinophil counts was observed in most patients, with 76% of dexpramipexole-treated subjects experiencing a 50% or greater decline in eosinophil count after 6 months of treatment.

Dexpramipexole was well-tolerated in the phase 3 study, with no dose-limiting toxicities and adverse event rates similar between treatment groups. The number of treatment-emergent adverse events classified as infections was similar in the placebo group (57%) compared with the dexpramipexole-treated group (56%). Neutropenia (ANC < 1500/µL) occurred more frequently among dexpramipexole-treated subjects (8%) than placebo subjects (2%). Neutropenia resolved following discontinuation of study treatment in all but one case (for which follow-up was not available). Fifteen dexpramipexole-treated subjects with neutropenia resumed study treatment after resolution, among which eight neutropenia recurred.

The hematologic effect of dexpramipexole was not limited to eosinophils. At month 6 in the phase 3 study, all lymphoid and myeloid cell types measured, except neutrophils, showed statistically significant mean reductions from baseline, although the magnitude of the effect was greatest for eosinophils and basophils (Table 10). In the Phase 3 trial (n=942, no censoring rules applied), dexpramipexole treatment significantly lowered circulating cell counts for all leukocytes measured at month 6. These changes were sustained
through month 12 (data not shown). Notably among hematology parameters, neither red blood cells nor platelets showed clinically or statistically significant changes in the treatment group. In contrast, no changes in red blood cell or platelet counts were observed between the treatment groups.

**Table 10 - Effects of dexpramipexole treatment on circulating leukocytes in a Phase 3 trial.**

| Parameter | Cohort | Baseline | Month 6 | Change | p-value |
|---|---|---|---|---|---|
| White blood cells x10⁹/L | Placebo | 6.90 | 7.2 | 4.3% | < 0.0001 |
| | Treatment | 6.79 | 6.01 | -11.5% | |
| Eosinophils x10⁹/L | Placebo | 0.133 | 0.159 | 19.5% | < 0.0001 |
| | Treatment | 0.133 | 0.042 | -68.4% | |
| Basophils x10⁹/L | Placebo | 0.045 | 0.046 | 2.2% | < 0.0001 |
| | Treatment | 0.044 | 0.024 | -45.5% | |
| Neutrophils x10⁹/L | Placebo | 4.467 | 4.794 | 7.3% | < 0.0001 |
| | Treatment | 4.388 | 4.024 | -8.3% | |
| Lymphocytes x10⁹/L | Placebo | 1.818 | 1.782 | -2.0% | < 0.0001 |
| | Treatment | 1.784 | 1.54 | -13.7% | |
| Monocytes x10⁹/L | Placebo | 0.437 | 0.419 | -4.1% | < 0.0001 |
| | Treatment | 0.433 | 0.377 | -12.9% | |
| Red blood cells x10¹²/L | Placebo | 4.69 | 4.71 | 0.4% | = 0.1123 |
| | Treatment | 4.71 | 4.77 | 1.3% | |
| Platelets x10⁹/L | Placebo | 255.9 | 276.6 | 8.1% | = 0.4925 |
| | Treatment | 250.3 | 266.9 | 6.6% | |

In a 39-week toxicology study in Göttingen minipigs, a dose-and time-dependent eosinophil-lowering effect was also observed with no associated macroscopic or microscopic pathologic findings (data not shown).

Across all three studies, the systemic cell-depleting effect of dexpramipexole was slow to develop, generally requiring 3-4 months to reach maximum effect, and persisted for the duration of treatment. This suggests that dexpramipexole may modulate hematopoiesis of specific myeloid cell lines during differentiation or promote apoptosis, or perhaps both, although the mechanism of the effect remains under investigation.

These empirical observations across multiple studies demonstrate a consistent, robust effect of dexpramipexole on decreasing peripheral blood eosinophils from patients with normal eosinophil counts, and to a smaller extent other leukocyte counts, at doses well
tolerated clinically. Future research will address the effect of the drug on eosinophil levels in bone marrow and target tissues, its effect on activated cells, and whether the effects observed in normal hematologic states may be relevant in pathological conditions, especially in HES, where high levels of eosinophils are correlated with disease severity.

### EXAMPLE 10 - A RANDOMIZED, DOUBLE-BLIND, PLACEBO-CONTROLLED, MULTICENTER STUDY OF THE EFFICACY AND SAFETY OF DEXPRAMIPEXOLE (KNS-760704) IN SUBJECTS WITH AMYOTROPHIC LATERAL SCLEROSIS

### Rationale for the Study

Currently, only 1 medicine, Rilutek® (riluzole, Sanofi Aventis, approved by the United States [US] Food and Drug Administration in 1995 and the European Medicines Agency in 1996), is available for the treatment of ALS. Considering the seriousness of the disease, the lack of robust efficacy of riluzole, and limited options for further treatment, there remains a pressing unmet medical need for effective and safe treatments for ALS.

In a 2-part Phase 2 study conducted by Knopp Neurosciences Inc. (KNS-760704-CL201 [CL201]), dexpramipexole appeared to be well tolerated at doses ranging from 25 mg to 150 mg twice daily. Study results also showed a dose-dependent trend in slowing the rate of functional decline, as measured by change in the ALS Functional Rating Scale-Revised (ALSFRS-R), and a trend toward reduction in mortality of the 150 mg twice daily group compared to the 25 mg twice daily group.

In a Phase 3 study conducted by Biogen Idec Inc., dexpramipexole failed to meet the primary endpoint (CAFS) or key secondary endpoints. In that study, dexpramipexole showed no clinical benefit compared to placebo when dosed for 12 months.

Despite nearly identical entrance criteria between the Phase 2 and Phase 3 studies, an analysis of the inter-study differences identified significant differences between the Phase 2 and Phase 3 study populations. In particular, there were statistically significant differences in symptom duration (p=0.038), riluzole use (p=0.002), and El Escorial definite (p=0.005) populations, each favoring a generally slower progressing population in the Phase 3 study compared to the Phase 2 study. Each of these significant baseline characteristics have been show to impact outcome in ALS, a fact that was validated within the placebo population of the Phase 3 study.

### Study Design

Multi-center, multi-national, randomized, double-blind, placebo-controlled study stratified by investigational site, onset site (bulbar onset or others), and riluzole usage.

### Rationale for Dose and Schedule Selection

The dexpramipexole dose and regimen will be both 300 mg per day (150 mg administered twice daily) and 600 mg per day (300 mg administered daily). The selection of dose and regimen is based on the results of the Phase 2 and Phase 3 studies; in which oral study treatment was administered every 12 hours. In the only study conducted to date with a dose ranging element to the study design, subjects in Part 1 of Study CL201 were treated with either placebo, 50 mg (25 mg twice daily), 150 mg (75 mg twice daily), or 300 mg (150 mg twice daily) over 12 weeks. In Part 2 of Study CL201, subjects were treated with either 50 mg (25 mg twice daily), or 300 mg (150 mg twice daily) over 24 weeks. The greatest apparent benefit of dexpramipexole was observed at 150 mg twice daily, as measured by the ALSFRS-R in Part 1 and CAFs, the ALSFRS-R, and mortality in Part 2.

The dose selected for study in the first Phase 3 study of dexpramipexole in ALS (223AS302) conducted by Biogen Idec was 300 mg twice daily. The use of placebo, 150 mg twice daily, and 300 mg twice daily will enable a direct comparison of results of this study with the Biogen Idec study and help determine if the results seen in the post-hoc analysis of Study 233AS302 can be confirmed or enriched for in ALS subjects with diffuse disease, with a short time from symptom onset.

### Study Objectives and Endpoints

### Objectives

Primary:
The primary objective of the study is to evaluate the efficacy of oral administration of dexpramipexole 150 mg and 300 mg twice daily compared to placebo for 18 months in subjects with ALS.

### Secondary:

The secondary objectives of the study are to evaluate the safety and pharmacokinetic (PK) profiles of oral administration of 150 mg twice daily dexpramipexole.

### Endpoints

### Primary: Time to death using all available data up to 18 months

### Secondary:

Efficacy (ranked in order; will be evaluated, unless specified otherwise):
A joint rank of functional outcomes adjusted for mortality. The joint rank analysis is based on change from baseline in ALSFRS-R score and time to death using follow up data through 12 months.

Respiratory decline: time to reach ≤50% of predicted upright slow vital capacity (SVC) or death using all available data up to 18 months.

Time to death or respiratory insufficiency (DRI) Respiratory insufficiency is defined as receipt of a tracheostomy or the use of non-invasive ventilation (NIV) for ≥22 hours per day for ≥10 consecutive days. If NIV is used to meet the criteria for respiratory insufficiency, no measured slow vital capacity (SVC) at any subsequent assessment may be >50%.

### Pharmacodynamics:

### Final parameters will depend on the population PD model.

Relationship between changes from baseline in efficacy endpoints (e.g., ALSFRS-R) and population PD parameters

### Safety:

Incidence of adverse events (AEs) (including serious adverse events [SAEs]), vital signs, clinical laboratory assessments, physical examination, electrocardiogram (ECG) tests, and body weight.

### Study Location

### Multi-national; approximately 50 study centers

### Number of Planned Subjects

Approximately 450 subjects will be enrolled.

### Study Population

This study will be conducted in subjects with a clinical diagnosis of familial or sporadic ALS who meet the definite criteria for a diagnosis of ALS according to the World Federation of Neurology El Escorial criteria (revised according to the Airlie House Conference 1998.

At screening, eligible subjects must be 18 to 80 years (inclusive), must have an upright SVC ≥65% of predicted capacity for age, height, and gender, and onset of first ALS symptoms must be ≤18 months prior to Day 1.

Subjects must be on a stable dose (>60 days) of prescription strength riluzole at the time of randomization.

Men and women of child-bearing age at screening are eligible for inclusion as long as they meet specific protocol requirements.

### Treatment Groups

Subjects will be randomly assigned in a 1:1:1 ratio to 1 of 3 treatment groups: oral dexpramipexole 150 mg twice daily, oral dexpramipexole 300 mg twice daily, or matching placebo twice daily.

### Duration of Treatment and Follow-up

Subjects will remain on randomized, placebo-controlled, double-blind treatment until either the Month 18 visit. When a subject has study treatment discontinued prematurely, the subject will continue to be followed monthly for assessment of living status, collection of ALSFRS-R scores, and monitoring of adverse events (AEs) through 18 months or until study completion, whichever comes first.

### Criteria for evaluation

Effectiveness: Living status; ALSFRS-R; SVC, Documentation of the use of major medical interventions (recommendation for feeding tube placement, and use of NIV).

### Safety

Physical examination, vital signs (systolic and diastolic systemic blood pressure, respiratory rate, heart rate, and temperature), body weight, 12-lead ECG, clinical laboratory assessments (hematology, blood chemistry and urinalysis), pregnancy testing, AE/SAE, and concomitant medication monitoring throughout the study.

### Statistical Methods

### General Considerations

Data will be summarized using descriptive statistics (number of subjects [N], mean, standard deviation [SD], median, minimum, and maximum) for continuous variables, and frequency and percentage for discrete variables. Statistical tests will be 2-sided, with a significance level of α = 0.05.

### Primary Endpoint Analysis

Treatment differences will be evaluated using a Cox Proportional Hazard model and Kaplan-Meier estimates for time to death up to 18 months.

### Secondary Endpoint Analysis

The analyses of secondary endpoints will be based on a sequentially closed testing procedure, ranked in the order of the secondary endpoints.

An analysis of covariance (ANCOVA) model will be used as an analysis to analyze the ranked score using treatment as a fixed effect and adjusting for the following covariates: baseline ALSFRS-R, duration from onset of symptoms to randomization, site of
onset (bulbar or others), and concomitant use of riluzole. Differences between the treatment groups (dexpramipexole vs. placebo) in the joint rank will be compared using the generalized Gehan-Wilcoxon (GGW) test as a secondary analysis.

Treatment differences will be evaluated using a Cox Proportional Hazard model for respiratory decline.

### Safety Endpoint Analysis

Safety endpoints will be summarized by treatment group.

### Sample Size Determination

A sample size of 450 subjects per treatment group will have approximately 80% power to detect a true mean difference to detect a 37% reduction in the hazard ratio (dexpramipexole vs. placebo) and is based on a sample size of 225 subjects per treatment group.

The secondary endpoint (CAFS) is will have approximately 90% power to detect a true mean difference of 2.7 in the ALSFRS-R score at Month 18 between the 2 treatment groups. This power calculation is based on a 2-sided Wilcoxon test with a final significance level of 0.05 and SD of 7.4. The SD estimate is based on ALSFRS-R data from Study CL201 as well as data from 2 studies (Gordon et al, 6 Lancet Neurol. 1045-53 (2007) and Meininger, 10 AMYOTROPH LATERAL SCLER. 378-83 (2009)). The power for the ALSFRS-R score is calculated based on a sample size of 250 subjects per treatment group with a 20% drop-out rate.

## Claims

1. A therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject,
wherein the subject is a subject with definite ALS, amyotrophic lateral sclerosis symptom onset duration of less than about 18 months, a creatinine value of greater than 72.0 µmol/L, and concomitant riluzole administration; and
wherein the amyotrophic lateral sclerosis is treated.

2. A therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject according to claim 1, wherein definite ALS is defined by the El Escorial diagnosis criteria.

3. A therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject according to claim 1, wherein the subject is a subject with a pulse rate of greater than 81.0 beats per minute, a cholesterol value of less than or equal to 5.3 mmol/L, a creatine phosphokinase value of less than or equal to 184.0 U/L, a bicarbonate value of less than or equal to 21.6 mmol/L, or any combination thereof.

4. A therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject according to claim 1, wherein the therapeutically effective amount of (6R)-2-amino-4,5,6,7-tetrahydro-6-(propylamino)benzothiazole or pharmaceutically acceptable salt thereof is from 150 milligrams to 3,000 milligrams per day.

5. A therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject according to claim 1, wherein the therapeutically effective amount of (6R)-2-amino-4,5,6,7-tetrahydro-6-(propylamino)benzothiazole or pharmaceutically acceptable salt thereof is 150 milligrams twice daily.

6. A therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject according to claim 1, wherein said therapeutically effective amount of (6R)-2-amino-4,5,6,7-tetrahydro-6-(propylamino)benzothiazole or pharmaceutically acceptable salt thereof is a pharmaceutical composition which is chirally pure for (6R)-2-amino-4,5,6,7-tetrahydro-6-(propylamino)benzothiazole or a pharmaceutically acceptable salt thereof.

7. A therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject according to claim 1, wherein treatment success is further selected from improved ALSFRS-R score, improved CAFS rank, decreased mortality, increased life expectancy, and combinations thereof.

8. A therapeutically effective amount of (6R)-2-amino-4,5,6,7-tetrahydro-6-(propylamino)benzothiazole or a pharmaceutically acceptable salt thereof, for use in the treatment of amyotrophic lateral sclerosis in a subject identified as a responder;
wherein the responder is El Escorial definite ALS and has a serum creatinine level greater than 72 µmol/L.

9. A therapeutically effective amount of (6R)-2-amino-4,5,6,7-tetrahydro-6-(propylamino)benzothiazole or a pharmaceutically acceptable salt thereof, for use in the treatment of El Escorial definite ALS in a subject identified as a responder according to claim, 8 wherein the responder is a responder having the presence of amyotrophic lateral sclerosis symptom onset duration of less than about 18 months, concomitant riluzole administration, a pulse rate of greater than 81.0 beats per minute, a cholesterol value of less than or equal to 5.3 mmol/L, a creatine phosphokinase value of less than or equal to 184.0 U/L, a bicarbonate value of less than or equal to 21.6 mmol/L, or any combination thereof.

10. A therapeutically effective amount of (6R)-2-amino-4,5,6,7-tetrahydro-6-(propylamino)benzothiazole or a pharmaceutically acceptable salt thereof, for use in the treatment of El Escorial definite ALS in a subject identified as a responder according to claim 8, wherein the responder has the presence of amyotrophic lateral sclerosis symptom onset duration of less than about 18 months, and concomitant riluzole administration.

## Patentansprüche

1. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von amyotropher Lateralsklerose in einem Subjekt;
wobei das Subjekt ein Subjekt mit definitiver ALS ist, mit einem Symptombeginnzeitraum von amyotropher Lateralsklerose von weniger als etwa 18 Monaten, einem Kreatininwert von über 72,0 µmol/L und begleitender Riluzolverabreichung; und
wobei die amyotrophe Lateralsklerose behandelt wird.

2. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von amyotropher Lateralsklerose in einem Subjekt nach Anspruch 1, wobei die definitive ALS durch die El Escorial-Diagnosekriterien bestimmt ist.

3. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von amyotropher Lateralsklerose in einem Subjekt nach Anspruch 1, wobei das Subjekt ein Subjekt mit einer Pulsfrequenz von mehr als 81,0 Schlägen pro Minute, einem Cholesterinwert von kleiner oder gleich 5,3 mmol/L, einem Kreatinphosphokinasewert von kleiner oder gleich 184,0 U/L, einem Bicarbonatwert von kleiner oder gleich 21,6 mmol/L oder einer beliebigen Kombination dieser ist.

4. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von amyotropher Lateralsklerose in einem Subjekt nach Anspruch 1, wobei die therapeutisch wirksame Menge von (6R)-2-Amino-4,5,6,7-tetrahydro-6-(propylamino)benzothiazol oder eines pharmazeutisch akzeptablen Salzes davon zwischen 150 Milligramm und 3.000 Milligramm pro Tag beträgt.

5. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von amyotropher Lateralsklerose in einem Subjekt nach Anspruch 1, wobei die therapeutisch wirksame Menge von (6R)-2-Amino-4,5,6,7-tetrahydro-6-(propylamino)benzothiazol oder eines pharmazeutisch akzeptablen Salzes davon 150 Milligramm zweimal täglich beträgt.

6. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von amyotropher Lateralsklerose in einem Subjekt nach Anspruch 1, wobei die therapeutisch wirksame Menge von (6R)-2-Amino-4,5,6,7-tetrahydro-6-(propylamino)benzothiazol oder eines pharmazeutisch akzeptablen Salzes davon eine pharmazeutische Zusammensetzung ist, die für (6R)-2-Amino-4,5,6,7-tetrahydro-6-(propylamino)benzothiazol oder ein pharmazeutisch akzeptables Salz davon chiral rein ist.

7. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von amyotropher Lateralsklerose in einem Subjekt nach Anspruch 1, wobei der Behandlungserfolg ferner ausgewählt ist aus verbessertem ALSFRS-R-Score, verbessertem CAFS-Rang, reduzierter Mortalität, höherer Lebenserwartung und Kombinationen davon.

8. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von amyotropher Lateralsklerose in einem als Responder identifizierten Subjekt;
wobei der Responder mit nach El Escorial definitiver ALS diagnostiziert ist und einen Serumkreatininwert von über 72 µmol/L aufweist.

9. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von nach El Escorial definitiver ALS in einem als Responder identifizierten Subjekt nach Anspruch 8, wobei der Responder ein Responder ist mit einem Symptombeginnzeitraum von amyotropher Lateralsklerose von weniger als etwa 18 Monaten, begleitender Riluzolverabreichung, einer Pulsfrequenz von mehr als 81,0 Schlägen pro Minute, einem Cholesterinwert von kleiner oder gleich 5,3 mmol/L, einem Kreatinphosphokinasewert von kleiner oder gleich 184,0 U/L, einem Bicarbonatwert von kleiner oder gleich 21,6 mmol/L oder einer beliebigen Kombination dieser.

10. Therapeutisch wirksame Menge von (6R)-2-Amino-4,5,6,7-tetrahydro-6-(propylamino)benzothiazol oder eines pharmazeutisch akzeptablen Salzes davon zur Verwendung in der Behandlung von nach El Escorial definitiver ALS in einem als Responder identifizierten Subjekt nach Anspruch 8, wobei der Responder einen Symptombeginnzeitraum von amyotropher Lateralsklerose von weniger als etwa 18 Monaten und begleitende Riluzolverabreichung aufweist.

## Revendications

1. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la sclérose latérale amyotrophique chez un sujet,
le sujet étant un sujet avec une durée d'apparition des symptômes de la sclérose latérale amyotrophique, SLA, inférieure à environ 18 mois, une valeur de créatinine supérieure à 72,0 µmol/L et l'administration concomitante de riluzole ;
la sclérose latérale amyotrophique étant traitée.

2. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la sclérose latérale amyotrophique chez un sujet selon la revendication 1, la SLAdéfinie étant définie par les critères de diagnostic d'El Escorial.

3. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la sclérose latérale amyotrophique chez un sujet selon la revendication 1, le sujet étant un sujet ayant une fréquence cardiaque supérieure à 81,0 battements par minute, un taux de cholestérol inférieur ou égal à 5,3 mmol/L, une valeur de créatine phosphokinase inférieure ou égale à 184,0 U/L, une valeur de bicarbonate inférieure ou égale à 21,6 mmol/L, ou toute combinaison de ceux-ci.

4. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la sclérose latérale amyotrophique chez un sujet selon la revendication 1, la quantité thérapeutiquement efficace de (6R)-2-amino-4,5,6,7-tétrahydro-6-(propylamino)benzothiazole ou d'un sel pharmaceutiquement acceptable de celui-ci étant de 150 milligrammes à 3 000 milligrammes par jour.

5. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la sclérose latérale amyotrophique chez un sujet selon la revendication 1, la quantité thérapeutiquement efficace de (6R)-2-amino-4,5,6,7-tétrahydro-6-(propylamino)benzothiazole ou d'un sel pharmaceutiquement acceptable de celui-ci étant de 150 milligrammes deux fois par jour.

6. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la sclérose latérale amyotrophique chez un sujet selon la revendication 1, ladite quantité thérapeutiquement efficace de (6R)-2-amino-4,5,6,7-tétrahydro-6-(propylamino)benzothiazole ou d'un sel pharmaceutiquement acceptable de celui-ci étant une composition pharmaceutique qui est chiralement pure pour le (6R)-2-amino-4,5,6,7-tétrahydro-6-(propylamino)benzothiazole ou le sel pharmaceutiquement acceptable de celui-ci.

7. Quantité thérapeutiquement efficace de (6R)-2-amino-4,5,6,7-tétrahydro-6-(propylamino)benzothiazole ou un sel pharmaceutiquement acceptable de celui-ci, destinée à être utilisée dans le traitement de la sclérose latérale amyotrophique chez un sujet selon la revendication 1, le succès du traitement étant en outre choisi parmi un score SLAFRS-R amélioré, un rang CAFS amélioré, une mortalité réduite, une espérance de vie accrue, et des combinaisons de ceux-ci.

8. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la sclérose latérale amyotrophique chez un sujet identifié comme répondeur ;
le répondeur étant une SLA définie par El Escorial et ayant un niveau de créatinine sérique supérieur à 72 µmol/L.

9. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la SLA définie par El Escorial chez un sujet identifié comme répondeur selon la revendication 8, le répondeur étant un répondeur ayant la présence d'une durée d'apparition des symptômes de la sclérose latérale amyotrophique inférieure à environ 18 mois, une administration concomitante de riluzole, une fréquence cardiaque supérieure à 81,0 battements par minute, une valeur de cholestérol inférieure ou égale à 5,3 mmol/L, une valeur de créatine phosphokinase inférieure ou égale à 184,0 U/L, une valeur de bicarbonate inférieure ou égale à 21,6 mmol/L, ou toute combinaison de ces éléments.

10. Quantité thérapeutiquement efficace de (6R)-2-amino-4,5,6,7-tétrahydro-6-(propylamino)benzothiazole ou d'un sel pharmaceutiquement acceptable de celui-ci, destinée à être utilisée dans le traitement de la SLA définie par El Escorial chez un sujet identifié comme répondeur selon la revendication 8, le répondeur ayant la présence de symptômes de sclérose latérale amyotrophique d'une durée d'apparition inférieure à environ 18 mois, et l'administration concomitante de riluzole.
